# EUROPEAN PATENT APPLICATION

(11) **EP 4 847 499 A1**
(43) Date of publication of application: **02.12.2026**
(21) Application number: 25745185.6
(22) Date of filing: 23.01.2025
(51) Int. Cl.: C07D 401/14, A61K 31/517, A61K 31/5377, A61K 31/5386, A61P 35/00, C07D 471/10, C07D 487/08, C07D 498/10

(54) **HETEROCYCLIC COMPOUND FOR INDUCING DEGRADATION OF KRAS PROTEIN**

(30) Priority: 24.01.2024 JP 2024008762
(71) Applicant: Astellas Pharma Inc., Tokyo 103-8411 (JP)
(72) Inventor: MORIKAWA, Takahiro, Tokyo 103-8411 (JP); IMADA, Sunao, Tokyo 103-8411 (JP); UKEGAWA, Naoya, Tokyo 103-8411 (JP); SATO, Ryo, Tokyo 103-8411 (JP); KAWAMINAMI, Eiji, Tokyo 103-8411 (JP); OKUMURA, Mitsuaki, Tokyo 103-8411 (JP); FUJIWARA, Yuta, Tokyo 103-8411 (JP); YOSHINARI, Tomohiro, Tokyo 103-8411 (JP); NISHIZONO, Yoshihiro, Tokyo 103-8411 (JP)
(74) Representative: Potter Clarkson
(86) International application number: PCT/JP2025/001994
(87) International publication number: WO 2025/159142

(57) **Abstract**

Provided is a compound useful as an active ingredient in a pharmaceutical composition for treatment of cancer.

The present inventors have conducted research on compounds useful as an active ingredient in a pharmaceutical composition for treatment of cancer, and have completed the present invention by finding that the heterocyclic compounds represented by formulas (I) and (I-I) have degradation-inducing action of mutated KRAS proteins and wild-type KRAS proteins, particularly, degradation-inducing action of G12C mutated, G12D mutated, G12V mutated, and G13D mutated KRAS proteins, and inhibitory activity on mutated KRAS and inhibitory activity on KRAS in the presence of amplification of a wild-type KRAS gene, particularly inhibitory activity on G12C mutated, G12D mutated, G12V mutated, and G13D mutated KRASs. The heterocyclic compound or a salt thereof according to the present invention can be used as a therapeutic agent for cancer.

## Description

### TECHNICAL FIELD

The present invention relates to a heterocyclic compound which has excellent degradation-inducing action of mutated KRAS proteins, particularly, excellent inductive action on degradation of G12C mutated, G12D mutated, G12V mutated, and G13D mutated KRAS proteins and wild-type KRAS proteins and/or excellent inhibitory action on mutated KRAS, particularly, excellent inhibitory action on G12C mutated, G12D mutated, G12V mutated, and G13D mutated KRASs and excellent inhibitory action on KRAS in the presence of amplification of a wild-type KRAS gene, and which is expected to be useful as an active ingredient of a pharmaceutical composition such as a pharmaceutical composition for treatment of cancer.

### BACKGROUND ART

RAS proteins are low-molecular (about 21 kDa) guanosine triphosphate (GTP)-binding proteins each composed of 188 to 189 amino acids, and include four main proteins (KRAS (KRAS4A and KRAS4B), NRAS, HRAS) generated from three genes, i.e., KRAS, NRAS, and HRAS genes. The RAS proteins include those of an GTP-binding form as an active form and a GDP-binding form as an inert form. The RAS proteins are activated by exchanging guanosine diphosphate (GDP) for GTP as a result of ligand stimulation to a cell membrane receptor such as EGFR. The active-form RASs bind to as many as 20 effector proteins such as RAF, PI3K, and RALGDS, and activates the downstream signal cascade. On the other hand, the active-form RAS becomes the inert form as a result of conversion of GTP to GDP due to endogenous GTP hydrolysis (GTPase) activity. The GTPase activity is accelerated by a GTPase activating protein (GAP). Thus, RAS has an important function as a "molecular switch" in an intracellular signaling pathway such as EGFR, and plays an important role in the processes such as growth of cells, proliferation, and generation of blood vessels (Nature Rev. Cancer, 2011, 11, p. 761-774, Nature Rev. Drug Discov., 2014, 13, p. 828-851, Nature Rev. Drug Discov., 2016, 15, p.771-785).

When spontaneous mutation of the RAS gene causes amino acid substitution, RAS is constantly activated due to its reduced function as the GTPase or a reduced reaction with GAP, and continuously transmits the signal to the downstream side. Such an excess signal causes the onset of cancers or an enhancement of proliferation of cancers. For example, mutation of the KRAS genes is observed in 90% or more of patients having pancreatic ductal adenocarcinoma, and the mutation is present from the initial state called pancreatic intraepithelial neoplasia (PanIN). In lung cancer and colorectal cancer, the mutation of the KRAS genes is also highly frequently observed. As the mutation of the KRAS genes, many examples of point mutation of codon 12 located in KRAS exon 2 (G12C mutation, G12D mutation, G12V mutation, etc.) are known (Nat. Rev. Cancer, 2018, 18, p. 767-777).

Recently, a plurality of inhibitors selective for G12C mutated KRAS has been developed, among of which Sotorasib and adagrasib are approved as curative medicines for non-small cell lung cancer by FDA (J. Exp. Clin. Cancer. Res., 41 27, 2022). About 14% of patients having lung cancer have G12C mutated KRAS, and it is reported that about 5% and about 7% of the patients have G12V mutation and G12D mutation, respectively. On the other hand, in pancreatic cancer, G12D mutation and G12V mutation are observed frequently, i.e., about 40% and about 28% of the patients, respectively, and in colon cancer, G12D mutation, G12V mutation, G12C mutation, and G13D mutation are observed in about 15%, about 10%, about 3%, and about 8% of the patients, respectively. A high proportion of mutation other than G12C mutation in a variety of cancers leads to a high expectation of a curative medicine for a wide range of mutated KRAS (npj Precis. Onco., 6 91, 2022).

WO 2016/049565, WO 2016/049568, and WO 2017/172979 disclose RAS inhibitors, and WO 2016/049568 and WO 2017/172979 discloses the compound represented by formula (A) and that represented by formula (B) below, respectively (for the meanings of the symbols in the formulas, see these publications). Patent Documents 1, 2, and 3 state that the compounds are useful for cancers having mutation of codon 12 in KRAS and the mutation includes G 12C mutation and G 12V mutation, while no specific action of the compounds against G12C mutated KRAS and G12V mutated KRAS is described.

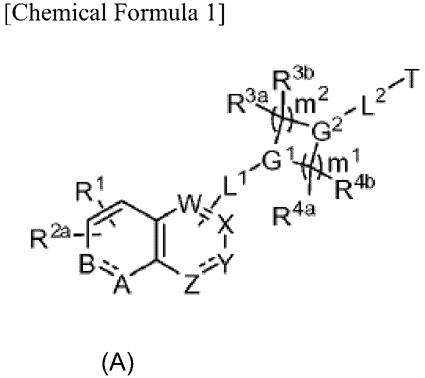

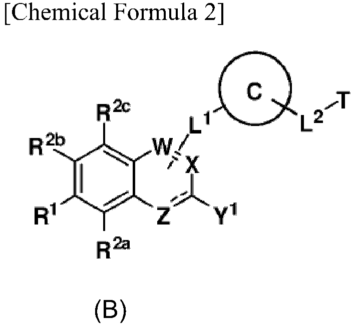

WO 2022/132200 discloses a pan-KRAS inhibitor.

Recently, bifunctional compounds generally called PROTAC (PROteolysis-TArgeting Chimera), SNIPER (Specific and Nongenetic IAP-dependent Protein Eraser) and the like are found as a technique of inducing degradation of the target protein, and are expected to be one of novel drug development modalities (Drug. Discov. Today Technol., 2019, 31, p15-27). Such a bifunctional compound promotes formation of a composite form of the target protein and E3 ligase within the cell, and use of the ubiquitin-proteasome system induces degradation of the target protein. The ubiquitin-proteasome system is one of intracellular protein degradation mechanisms. The protein called E3 ligase identifies and ubiquitinizes the protein to be degraded, causing its degradation to proceed in the proteasome.

E3 ligase includes 600 or more of E3 ligases present in the living body, and is mainly classified into four subclasses, HECT-domain E3s, U-box E3s, monomeric RING E3s, and multi-subunit E3s. Currently, the E3 ligases used in the bifunctional degradation inducing agents called PROTAC, SNIPER and the like are limited, and typical examples thereof include Von Hippel-Lindau (VHL), celebron (CRBN), inhibitor of apoptosis protein (IAP), and mouse double minute 2 homolog (MDM2). In particular, VHL is reported in WO 2013/106643, and CRBN is reported in WO 2015/160845.

The bifunctional compound is a compound in which the ligand of the target protein is linked to the ligand of E3 ligase via a linker, and bifunctional compounds which degrade KRAS proteins are reported so far (Cell. Chem. Biol., 2020, 27, p. 19-31, ACS Cent. Sci., 2020, 6, p. 1367-1375, U.S. Patent Application No. 2018/0015087, WO 2019/195609, WO 2020/018788, WO 2021/051034, WO 2021/207172, WO 2022/111521, WO 2022/061348, WO 2022/148422, WO 2022/228576, WO 2023/059609, WO 2023/077441, WO 2023/280026, China Patent Application Nos. 113956233 and 115785199, and WO 2023/138524).

Among PROTACs which degrade KRAS having G12D mutation, compounds are reported, in which the substituent at the 8-position of quinoline and quinazoline is linked to the ligand of E3 ligase (Patent Documents 1 and 2).

In addition, pan-KRAS PROTAC is also reported (Patent Documents 3 to 9).

### CITATION LIST

### PATENT DOCUMENT

PATENT DOCUMENT 1: WO 2022/173032
PATENT DOCUMENT 2: WO 2023/171781
PATENT DOCUMENT 3: WO 2022/087335
PATENT DOCUMENT 4: WO 2022/212611
PATENT DOCUMENT 5: WO 2022/266249
PATENT DOCUMENT 6: WO 2022/271823
PATENT DOCUMENT 7: WO 2023/099620
PATENT DOCUMENT 8: WO 2023/130012
PATENT DOCUMENT 9: WO 2023/141570

### SUMMARY OF INVENTION

### TECHNICAL PROBLEM

Provided is a heterocyclic compound which has excellent degradation-inducing action of mutated KRAS proteins, particularly, excellent degradation-inducing action of G12C mutated, G12D mutated, G12V mutated, and G13D mutated KRAS proteins and wild-type KRAS proteins, and/or excellent inhibitory action on mutated KRAS, particularly, excellent inhibitory action on G12C mutated, G12D mutated, G12V mutated, and G13D mutated KRASs and excellent inhibitory action on KRAS in the presence of amplification of a wild-type KRAS gene, and which is expected to be useful as an active ingredient of a pharmaceutical composition such as a pharmaceutical composition for treatment of cancer, particularly, mutated KRAS-positive cancer, particularly, G12C mutated, G12D mutated, G12V mutated, and G13D mutated KRAS-positive cancers and wild-type KRAS gene amplification-positive cancer.

### SOLUTION TO PROBLEM

The present inventors have conducted extensive research about a compound useful as an active ingredient of a pharmaceutical composition for treatment of cancer, and as a result have completed the present invention by finding that a heterocyclic compound represented by formula (I), particularly, a bifunctional compound represented by formula (I) wherein a substituent at a carbon atom adjacent to a carbon atom bonded to R¹ of a heterocyclic compound represented by formula (I) (the adjacent carbon atom corresponds to the 8-position of quinazoline that is the compound wherein A is N and E is CH) is linked to the ligand of E3 ligase or a substituent at a carbon atom adjacent to a carbon atom bonded to R¹ of a heterocyclic compound (the adjacent carbon atom corresponds to the 8-position of quinazoline that is the compound wherein A is N and E is CH) is linked to the ligand of E3 ligase via a linker has excellent degradation-inducing action of mutated KRAS proteins, excellent inhibitory activity on mutated KRAS, excellent degradation-inducing action of wild-type KRAS proteins, and excellent inhibitory activity on KRAS in the presence of amplification of a wild-type KRAS gene.

In other words, the present invention relates to a compound represented by formula (I) or a salt thereof, and a pharmaceutical composition including a compound represented by formula (I) or a salt thereof, and one or more pharmaceutically acceptable excipients. 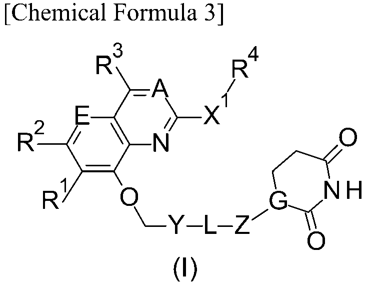 (wherein
A is CR^{A} or N,
R^{A} is H, C₁₋₃ alkyl, -CN, or -O-(C₁₋₃ alkyl),
E is CH or N,
X¹ is -CH₂-, -O-, or -NR^{X1}-,
R^{X1} is H or optionally substituted C₁₋₃ alkyl,
provided that when X¹ is -NR^{X1}-, R^{X1} and R⁴ present on the same nitrogen atom, together with the nitrogen atom adjacent thereto, may form optionally substituted 4- to 11-membered saturated hetero ring group,
R¹ is represented by the following formula (II), (III), (IV), (V), (VI), or (VII): 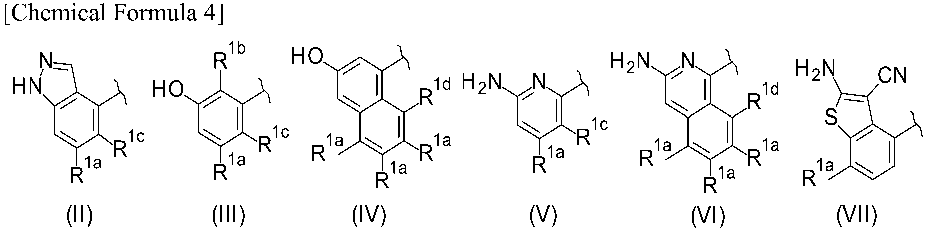
R^{1a} and R^{1b} are the same or different, and are each H, methyl, F, or Cl,
R^{1c} is F, Cl, methyl, ethyl, trifluoromethyl, or cyclopropyl,
R^{1d} is H, methyl, ethyl, F, Cl, or -C≡C-H,
R² is H, halogen, optionally substituted C₁₋₃ alkyl, cyclopropyl, or vinyl,
R³ is a group selected from the group consisting of the following formulas (VIII), (XI), (X), (XI), (XII), (XIII), (XIV), (XV), (XVI), and (XVII): 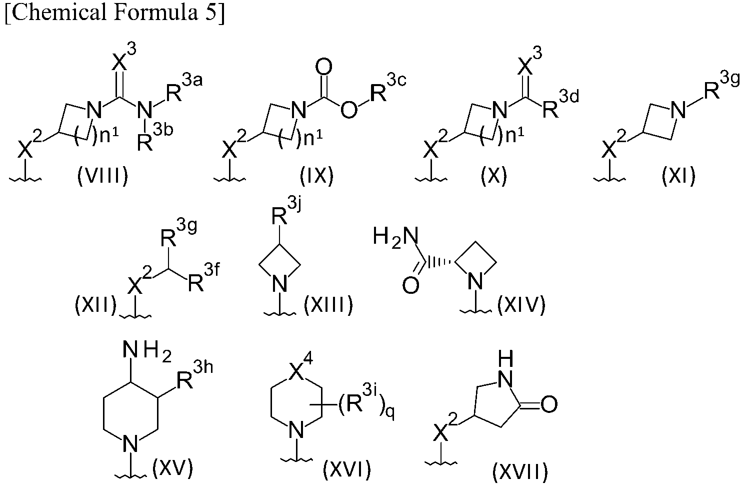
R^{3a} is -(CH₂)ₚCHR^{3e}-NR^{N1}R^{N2}, -(CH₂)ₚCHR^{3e}-OR^{3f}; 4- to 6-membered saturated hetero ring group optionally substituted with a group selected from the group consisting of C₁₋₃ alkyl, -C₁₋₃ alkylene-OR^{3f}, -C₁₋₃ alkylene-NR^{N1}R^{N2}, and -NR^{N1}R^{N2}; or C₃₋₆ cycloalkyl optionally substituted with a group selected from the group consisting of C₁₋₃ alkyl, -C₁₋₃ alkylene-OR^{3f}, -C₁₋₃ alkylene-NR^{N1}R^{N2}, -OR^{3f}, and -NR^{N1}R^{N2},
R^{3b} is H or C₁₋₃ alkyl,
R^{3c} and R^{3d} are -(CH₂)ₚCHR^{3e}-NR^{N1}R^{N2}; -(CH₂)ₚCHR^{3e}-OR^{3f}; 4- to 6-membered saturated hetero ring group optionally substituted with a group selected from the group consisting of C₁₋₃ alkyl, -C₁₋₃ alkylene-OR^{3f}, -C₁₋₃ alkylene-NR^{N1}R^{N2}, and -NR^{N1}R^{N2}; or C₃₋₆ cycloalkyl optionally substituted with a group selected from the group consisting of C₁₋₃ alkyl, -C₁₋₃ alkylene-OR^{3f}, -C₁₋₃ alkylene-NR^{N1}R^{N2}, -OR^{3f}, and -NR^{N1}R^{N2},
R^{3e} is H, F, or C₁₋₃ alkyl,
R^{3f} is the same or different, and is H or C₁₋₃ alkyl,
R^{3g} is optionally substituted C₃₋₆ cycloalkyl, optionally substituted 5-membered heteroaryl, optionally substituted 6-membered heteroaryl, or optionally substituted 4- to 6-membered saturated hetero ring group,
R^{3h} is H, F, or C₁₋₃ alkyl,
R³ⁱ is the same or different, and is a group selected from the group consisting of H, OH, optionally substituted C₁₋₃ alkyl, -O-optionally substituted C₁₋₃ alkyl, -NH-optionally substituted C₁₋₃ alkyl, -N-(optionally substituted C₁₋₃ alkyl)₂, halogen, -CN, and oxo, or
two R³ⁱs present on the same carbon atom together with the carbon atom adjacent thereto may form a spiro ring having a ring selected from the group consisting of C₃₋₆ cycloalkane and 4- to 6-membered saturated hetero ring, wherein the spiro ring is optionally substituted with 1 to 2 groups selected from the group of C₁₋₃ alkyl, -O-(C₁₋₃ alkyl), OH, halogen, and oxo, or
R³ⁱ present on two adjacent carbon atoms together with the two carbon atoms may form a fused ring having a ring selected from the group consisting of C₃₋₆ cycloalkane and 4-to 6-membered saturated hetero ring, wherein the fused ring is optionally substituted with 1 to 2 groups selected from the group consisting of C₁₋₃ alkyl, -O-(C₁₋₃ alkyl), OH, halogen, and oxo, or
R³ⁱ present on two non-adjacent carbon atoms together with the two carbon atoms may form bridged structure composed of 1 to 2 carbon atoms, and ring having the bridged structure is optionally substituted with 1 to 2 groups selected from the group consisting of C₁₋₃ alkyl, -O-(C₁₋₃ alkyl), OH, halogen, and oxo,
R^{N1} and R^{N2} are the same or different, and are each H or C₁₋₃ alkyl, or
R^{N1} and R^{N2} together with the nitrogen atom bonded thereto may form optionally substituted 4- to 6-membered saturated hetero ring group, or
R^{3e} and R^{N1} together with the carbon atom and the nitrogen atom bonded thereto may form optionally substituted 4- to 6-membered saturated hetero ring group,
R^{3j} is a group selected from the group consisting of H, OH, halogen, optionally substituted C₁₋₃ alkyl, -O-optionally substituted C₁₋₃ alkyl, optionally substituted C₃₋₆ cycloalkyl, optionally substituted 5-membered heteroaryl, optionally substituted 6-membered heteroaryl, and -CN,
X² is -O-, -NH-, or -N(C₁₋₃ alkyl)-,
X³ is O or S,
X⁴ is -CH₂-, -CH₂-CH₂-, -O-CH₂-, -O-, or -O-NH-,
n¹ is 1 or 2,
p is 1 or 2,
q is 1 to 8,
R⁴ is optionally substituted C₁₋₆ alkyl, optionally substituted piperidinyl, or optionally substituted tetrahydropyranyl,
Y is phenylene or pyridinediyl, wherein the phenylene is optionally substituted with F,
L is -(L^{A}-L^{B}-L^{C}-L^{D}-L^{E}-L^{F})-,
L^{A}, L^{B}, L^{C}, L^{D}, L^{E}, and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O,
R^{L1A} is H or C₁₋₃ alkyl,
Z is a group selected from the group consisting of the following formulas (XVIII), (XIX), (XX), (XXI), (XXII), (XXIII), (XXIV), (XXV), (XXVI), (XXVII), (XXVIII), and (XXIX): 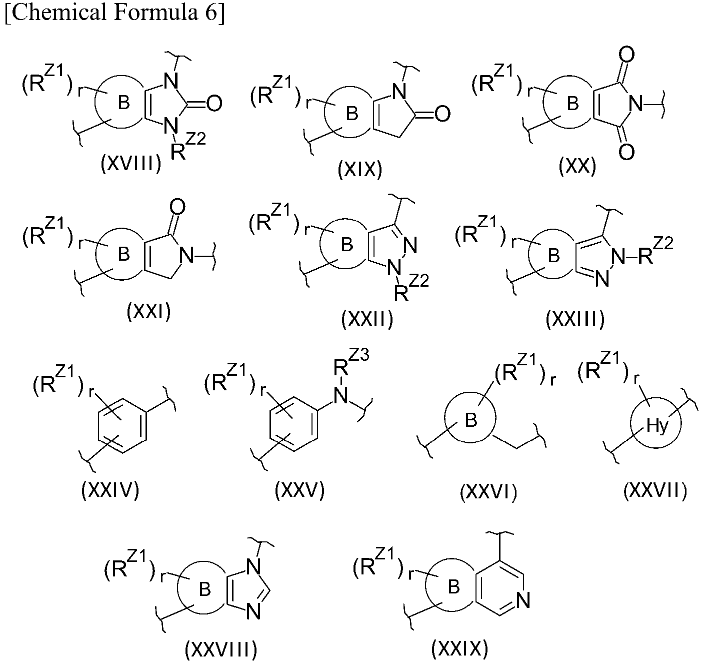
ring B is benzene ring or 6-membered hetero ring,
ring Hy is 5- or 6-membered hetero ring,
R^{Z1} is H, C₁₋₃ alkyl, halogen, -O-(C₁₋₃ alkyl), -NR^{Z4}₂, -CN, -CONR^{Z4}₂, or - NR^{Z4}COR^{Z5},
R^{Z2} is H or C₁₋₃ alkyl,
R^{Z3} is H or C₁₋₃ alkyl,
R^{Z4} is the same or different, and is H or C₁₋₃ alkyl,
R^{Z5} is C₁₋₃ alkyl,
L is bonded to ring B in the above formulas (XVIII), (XIX), (XX), (XXI), (XXII), (XXIII), (XXVI), (XXVIII), and (XXIX), to the benzene ring in formulas (XXIV) and (XXV), and to ring Hy in formula (XXVII),
r is 1 or 2, and
G is N or CH,
provided that when G is N, Z is the above formula (XVIII), (XXII), (XXIII), (XXIV), (XXVI), (XXVII), or (XXIX))

Further, the compound having degradation-inducing action of target proteins, particularly, mutated KRAS proteins, inhibitory activity on mutated KRAS, and/or degradation-inducing action of wild-type KRAS proteins, and inhibitory activity on KRAS in the presence of amplification of a wild-type KRAS gene, may be, for example, formula (I-I) below. The compound having degradation-inducing action of target proteins, particularly, mutated RAS proteins, inhibitory activity on mutated RAS, and/or degradation-inducing action of wild-type RAS proteins, and inhibitory activity on RAS in the presence of amplification of a wild-type RAS gene, may also be, for example, formula (I-I) below. In other words, the present invention relates to a compound represented by formula (I-I) or a salt thereof, and a pharmaceutical composition comprising the compound represented by formula (I-I) or a salt thereof, and one or more pharmaceutically acceptable excipients. 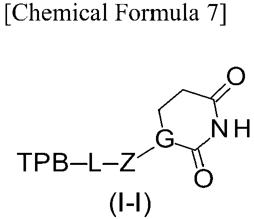 (wherein
TPB is a group having an ability to bind to a target protein,
L is -(L^{A}-L^{B}-L^{C}-L^{D}-L^{E}-L^{F})-,
L^{A}, L^{B}, L^{C}, L^{D}, L^{E}, and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O,
R^{L1A} is H or C₁₋₃ alkyl,
Z is a group selected from the group consisting of the following formulas (XVIII), (XIX), (XX), (XXI), (XXII), (XXIII), (XXIV), (XXV), (XXVI), (XXVII), (XXVIII), and (XXIX): 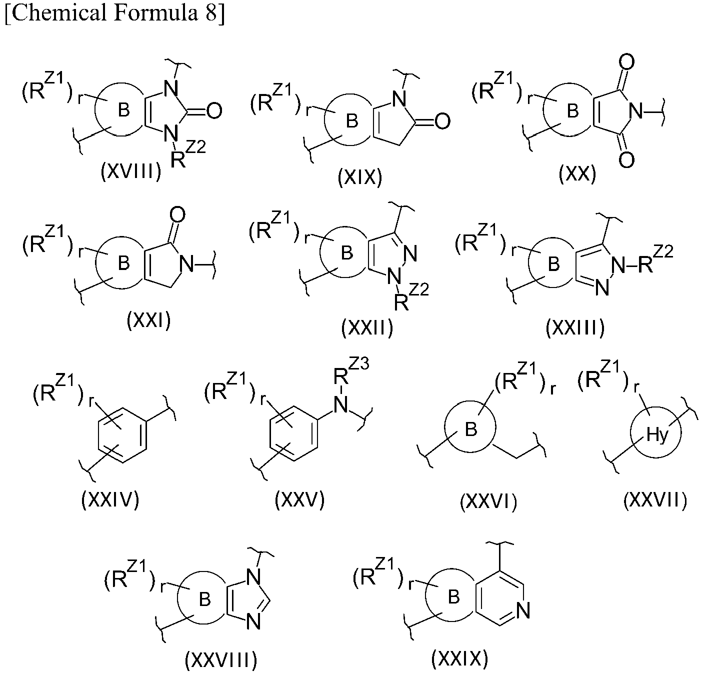
ring B is benzene ring or 6-membered hetero ring,
ring Hy is 5- or 6-membered hetero ring,
R^{Z1} is H, C₁₋₃ alkyl, halogen, -O-(C₁₋₃ alkyl), -NR^{Z4}₂, -CN, -CONR^{Z4}₂, or - NR^{Z4}COR^{Z5},
R^{Z2} is H or C₁₋₃ alkyl,
R^{Z3} is H or C₁₋₃ alkyl,
R^{Z4} is the same or different, and is H or C₁₋₃ alkyl,
R^{Z5} is C₁₋₃ alkyl,
L is bonded to ring B in the above formulas (XVIII), (XIX), (XX), (XXI), (XXII), (XXIII), (XXVI), (XXVIII), and (XXIX), to the benzene ring in formulas (XXIV) and (XXV), and to ring Hy in formula (XXVII),
r is 1 or 2, and
G is N or CH,
provided that when G is N, Z is the above formula (XVIII), (XXII), (XXIII), (XXIV), (XXVI), (XXVII), or (XXIX))

When symbols in chemical formulas in this specification are also used in other chemical formulas, the same symbols indicate the same meanings unless otherwise specified.

The present invention also relates to a pharmaceutical composition including a compound represented by formula (I) or a salt thereof, and one or more pharmaceutically acceptable excipients. In one embodiment, it relates to a pharmaceutical composition for treatment of cancer. In one embodiment, it relates to a pharmaceutical composition for treatment of mutated KRAS-positive cancer, particularly a pharmaceutical composition for treatment of mutated KRAS-positive cancer having one or more mutations selected from the group consisting of G12C mutation, G12D mutation, G12V mutation, and G13D mutation. In one embodiment, it relates to a pharmaceutical composition for treatment of metastatic cancer. In one embodiment, it relates to a pharmaceutical composition for treatment of locally advanced cancer. In one embodiment, it relates to a pharmaceutical composition for treatment of recurrent or refractory cancer. In one embodiment, it relates to a pharmaceutical composition for treatment of cancer in a patient untreated and/or having a treatment history. In one embodiment, it relates to a pharmaceutical composition for treatment of metastatic mutated KRAS-positive cancer, particularly a pharmaceutical composition for treatment of metastatic mutated KRAS-positive cancer having one or more mutations selected from the group consisting of G12C mutation, G12D mutation, G12V mutation, and G13D mutation. In one embodiment, it relates to a pharmaceutical composition for treatment of locally advanced mutated KRAS-positive cancer, particularly a pharmaceutical composition for treatment of locally advanced mutated KRAS-positive cancer having one or more mutations selected from the group consisting of G12C mutation, G12D mutation, G12V mutation, and G13D mutation. In one embodiment, it relates to a pharmaceutical composition for treatment of recurrent or refractory mutated KRAS-positive cancer, particularly a pharmaceutical composition for treatment of recurrent or refractory mutated KRAS-positive cancer having one or more mutations selected from the group consisting of G12C mutation, G12D mutation, G12V mutation, and G13D mutation. In one embodiment, it relates to a pharmaceutical composition for treatment of mutated KRAS-positive cancer in a patient untreated and/or having a treatment history, particularly a pharmaceutical composition for treatment of mutated KRAS-positive cancer having one or more mutations selected from the group consisting of G12C mutation, G12D mutation, G12V mutation, and G13D mutation in a patient untreated and/or having a treatment history. The pharmaceutical composition for treatment of cancer including a compound represented by formula (I) or a salt thereof and one or more pharmaceutically acceptable excipients encompasses a treatment agent for cancer, and in one embodiment, the treatment agent for mutated KRAS-positive cancer having one or more mutations selected from the group consisting of G12C mutation, G12D mutation, G12V mutation, and G13D mutation.

The present invention also relates to a pharmaceutical composition including a compound represented by formula (I) or a salt thereof and one or more pharmaceutically acceptable excipients. In one embodiment, it relates to a pharmaceutical composition for treating cancer. In one embodiment, it relates to a pharmaceutical composition for treating metastatic wild-type KRAS gene amplification-positive cancer. In one embodiment, it relates to a pharmaceutical composition for treating locally advanced wild-type KRAS gene amplification-positive cancer. In one embodiment, it relates to a pharmaceutical composition for treating recurrent or refractory wild-type KRAS gene amplification-positive cancer. In one embodiment, it relates to a pharmaceutical composition for treating wild-type KRAS gene amplification-positive cancer in a patient untreated and/or having a treatment history. The pharmaceutical composition for treating cancer including a compound represented by formula (I) or a salt thereof, and one or more pharmaceutically acceptable excipients encompasses a treatment agent for cancer, and in one embodiment, wild-type KRAS gene amplification-positive cancer, the treatment agent containing a compound represented by formula (I) or a salt thereof.

The present invention also relates to use of a compound represented by formula (I) or a salt thereof for the manufacture of a pharmaceutical composition for treating cancer, in one embodiment, mutated KRAS-positive cancer, particularly mutated KRAS-positive cancer having one or more mutations selected from the group consisting of G12C mutation, G12D mutation, G12V mutation, and G13D mutation, in one embodiment, metastatic cancer, in one embodiment, locally advanced cancer, in one embodiment, recurrent or refractory cancer, in one embodiment, cancer in a patient untreated and/or having a treatment history, in one embodiment, metastatic mutated KRAS-positive cancer, particularly mutated KRAS-positive cancer having one or more mutations selected from the group consisting of G12C mutation, G12D mutation, G12V mutation, and G13D mutation, in one embodiment, locally advanced mutated KRAS-positive cancer, particularly mutated KRAS-positive cancer having one or more mutations selected from the group consisting of G12C mutation, G12D mutation, G12V mutation, and G13D mutation, in one embodiment, recurrent or refractory mutated KRAS-positive cancer, particularly mutated KRAS-positive cancer having one or more mutations selected from the group consisting of G12C mutation, G12D mutation, G12V mutation, and G13D mutation, in one embodiment, mutated KRAS-positive cancer in a patient untreated and/or having a treatment history, particularly mutated KRAS-positive cancer having one or more mutations selected from the group consisting of G12C mutation, G12D mutation, G12V mutation, and G13D mutation. The present invention relates to use of a compound represented by formula (I) or a salt thereof for treating cancer, in one embodiment, mutated KRAS-positive cancer, particularly mutated KRAS-positive cancer having one or more mutations selected from the group consisting of G12C mutation, G12D mutation, G12V mutation, and G13D mutation. The present invention relates to a compound represented by formula (I) or a salt thereof for use in treatment of cancer, in one embodiment, mutated KRAS-positive cancer, particularly mutated KRAS-positive cancer having one or more mutations selected from the group consisting of G12C mutation, G12D mutation, G12V mutation, and G13D mutation; and a method for treating cancer, in one embodiment, mutated KRAS-positive cancer, particularly mutated KRAS-positive cancer having one or more mutations selected from the group consisting of G12C mutation, G12D mutation, G12V mutation, and G13D mutation, the method including administrating an effective amount of a compound represented by formula (I) or a salt thereof to a subject.

The present invention relates to use of a compound represented by formula (I) or a salt thereof for the manufacture of a pharmaceutical composition for treating cancer, in one embodiment, wild-type KRAS gene amplification-positive cancer, in one embodiment, metastatic wild-type KRAS gene amplification-positive cancer, in one embodiment, locally advanced wild-type KRAS gene amplification-positive cancer, in one embodiment, recurrent or refractory wild-type KRAS gene amplification-positive cancer, in one embodiment, wild-type KRAS gene amplification-positive cancer in a patient untreated and/or having a treatment history. The present invention also relates to use of a compound represented by formula (I) or a salt thereof for treating cancer, in one embodiment, wild-type KRAS gene amplification-positive cancer. The present invention also relates to a compound represented by formula (I) or a salt thereof for use in treatment of cancer, in one embodiment, wild-type KRAS gene amplification-positive cancer, and a method for treating cancer, in one embodiment, wild-type KRAS gene amplification-positive cancer, the method including administrating an effective of a compound represented by formula (I) or a salt thereof.

The present invention also relates to a compound represented by formula (I) or a salt thereof which is a mutated KRAS protein degradation inducing agent and/or a mutated KRAS inhibitor, particularly, a compound represented by formula (I) or a salt thereof which is a mutated KRAS protein degradation inducing agent having one or more mutations selected from the group consisting of G12C mutation, G12D mutation, G12V mutation, and G13D mutation and/or a mutated KRAS inhibitor having one or more mutations selected from the group consisting of G12C mutation, G12D mutation, G12V mutation, and G13D mutation.

The present invention also relates to a compound represented by formula (I) or a salt thereof which is a wild-type KRAS protein degradation inducing agent and/or an inhibitor of KRAS in the presence of amplification of a wild-type KRAS gene.

The "subject" indicates a person or other animals in need of the treatment, and in one embodiment, indicates a person in need of prevention or treatment.

### ADVANTAGEOUS EFFECTS OF INVENTION

The compound represented by formula (I) or a salt thereof has degradation-inducing action of mutated KRAS protein, inhibitory activity on mutated KRAS, particularly, inhibitory activity on mutated KRAS having one or more mutations selected from the group consisting of G12C mutation, G12D mutation, G12V mutation, and G13D mutation, and inhibitory activity on mutated KRAS having one or more mutations selected from the group consisting of G12C mutation, G12D mutation, G12V mutation, and G13D mutation, and can be used as a treatment agent for cancer, particularly, mutated KRAS-positive cancer having one or more mutations selected from the group consisting of G12C mutation, G12D mutation, G12V mutation, and G13D mutation.

The compound represented by formula (I) or a salt thereof has degradation-inducing action of wild-type KRAS proteins and inhibitory activity on KRAS in the presence of amplification of a wild-type KRAS gene, and can also be used as a treatment agent for cancer, particularly, wild-type KRAS gene amplification-positive cancer.

### DESCRIPTION OF EMBODIMENTS

Hereinafter, the present invention will be described in detail.

In this specification, "optionally substituted" indicates that the group is not substituted, or has 1 to 5 substituents. In one embodiment, it indicates that the group is not substituted, or has 1 to 3 substituents. When a plurality of substituents is present, these substituents may be the same or different.

"C₁₋₁₂ alkyl" is a linear or branched alkyl having 1 to 12 carbon atoms, such as methyl, ethyl, n-propyl, isopropyl, n-butyl, isobutyl, sec-butyl, tert-butyl, n-pentyl, n-hexyl, or dodecyl (hereinafter, the number of carbon atoms is expressed as above). In one embodiment, it is ethyl or dodecyl.

Likewise, "C₁₋₆ alkyl" is a linear or branched alkyl having 1 to 6 carbon atoms, such as methyl, ethyl, n-propyl, isopropyl, dimethylpropyl, n-butyl, isobutyl, sec-butyl, tert-butyl, n-pentyl, or n-hexyl. In one embodiment, it is methyl, ethyl, n-propyl, isopropyl, or sec-butyl. In one embodiment, it is methyl, ethyl, isopropyl, or tert-butyl. In one embodiment, it is methyl, ethyl, n-propyl, isopropyl, n-butyl, or dimethylpropyl. In one embodiment, it is methyl. In one embodiment, it is ethyl. In one embodiment, it is dimethylpropyl.

Likewise, "C₁₋₃ alkyl" is a linear or branched alkyl having 1 to 3 carbon atoms, such as methyl, ethyl, n-propyl, or isopropyl. In one embodiment, it is methyl or ethyl. In one embodiment, it is n-propyl or isopropyl. In one embodiment, it is methyl or isopropyl. In one embodiment, it is ethyl or isopropyl. In one embodiment, it is methyl. In one embodiment, it is ethyl. In one embodiment, it is isopropyl. In one embodiment, it is n-propyl.

Likewise, "C₂₋₃ alkyl" is a linear alkyl having 2 to 3 carbon atoms, such as ethyl or n-propyl. In one embodiment, it is ethyl. In one embodiment, it is n-propyl.

"C₃₋₆ cycloalkane" is a cycloalkane having 3 to 6 carbon atoms, such as cyclopropane, cyclobutane, cyclopentane, or cyclohexane.

"C₃₋₆ cycloalkyl" is a cycloalkyl having 3 to 6 carbon atoms, such as cyclopropyl, cyclobutyl, cyclopentyl, or cyclohexyl. In one embodiment, it is a cyclobutyl, cyclopentyl, or cyclohexyl. In one embodiment, it is cyclobutyl or cyclopentyl. In one embodiment, it is cyclopentyl or cyclohexyl. In one embodiment, it is cyclopropyl or cyclobutyl. In one embodiment, it is cyclopropyl. In one embodiment, it is cyclobutyl. In one embodiment, it is cyclopentyl. In one embodiment, it is cyclohexyl.

"C₁₋₃ alkylene" is a divalent group formed by removing a hydrogen atom from a C₁₋₃ alkyl, and is a linear or branched C₁₋₃ alkylene, such as methylene, ethylene, trimethylene, methylmethylene, or 1,1-dimethylmethylene. In one embodiment, it is a linear or branched C₁₋₃ alkylene. In one embodiment, it is methylene, ethylene, or trimethylene. In one embodiment, it is methylene or ethylene. In one embodiment, it is methylene. In one embodiment, it is ethylene. In one embodiment, it is trimethylene.

Likewise, "C₂₋₃ alkylene" is a divalent group formed by removing a hydrogen atom from C₂₋₃ alkyl, and in one embodiment, is ethylene or trimethylene. In one embodiment, it is ethylene. In one embodiment, it is trimethylene.

"C₃₋₆ cycloalkylene" is a divalent group formed by removing a hydrogen atom from C₃₋₆ cycloalkyl, such as cyclopropanediyl, cyclobutanediyl, cyclopentanediyl, or cyclohexanediyl. In one embodiment, it is cyclopropanediyl. In one embodiment, it is cyclobutanediyl. In one embodiment, it is cyclopentanediyl. In one embodiment, it is cyclohexanediyl.

"C₂₋₃ alkynediyl" is a divalent group having a triple bond in which four hydrogen atoms are removed from C₂₋₃ alkylene. In one embodiment, it is -C≡C-. In one embodiment, it is -CH₂-C≡C-.

"Saturated hetero ring" is a saturated hydrocarbon ring containing a heteroatom selected from the group consisting of oxygen, sulfur, and nitrogen as a ring forming atom. Here, part of the saturated hetero ring may have an unsaturated bond. The sulfur atom as a ring forming atom of the saturated hetero ring may be oxidized. Furthermore, the saturated hetero ring may have a bridged structure, and further, the saturated hetero ring may have a spiro structure. Accordingly, the "4- to 6-membered saturated hetero ring" is a 4- to 6-membered saturated hydrocarbon ring containing a heteroatom selected from the group consisting of oxygen, sulfur, and nitrogen as the ring forming atom, and may have a bridged structure, and may have a spiro structure. Likewise, the "4- to 11-membered saturated hetero ring" is a 4- to 11-membered saturated hydrocarbon ring containing a heteroatom selected from the group consisting of oxygen, sulfur, and nitrogen as the ring forming atom, and may have a bridged structure, and may have a spiro structure. One aspect of the "4- to 6-membered saturated hetero ring" is a 4- to 6-membered saturated hetero ring containing 1 to 2 heteroatoms selected from the group consisting of oxygen, sulfur, and nitrogen as the ring forming atom. In one embodiment, it is oxetane, tetrahydrofuran, tetrahydropyran, azetidine, pyrrolidine, piperidine, oxazolidine, imidazolidine, piperazine, morpholine, thiomorpholine, dioxothiomorpholine, or tetrahydropyridine.

The "saturated hetero ring group" is a saturated hydrocarbon ring group containing a heteroatom selected from the group consisting of oxygen, sulfur, and nitrogen as the ring forming atom. Here, part of the ring of the saturated hetero ring group may have an unsaturated bond. The sulfur atom as the ring forming atom of the saturated hetero ring group may be oxidized. Furthermore, the saturated hetero ring group may have a bridged structure, and further, the saturated hetero ring group may have a spiro structure.

Accordingly, the "4- to 6-membered saturated hetero ring group" is a 4- to 6-membered saturated hetero ring group containing a heteroatom selected from the group consisting of oxygen, sulfur, and nitrogen as the ring forming atom, and may have a bridged structure, and may have a spiro structure. One embodiment of the "4- to 6-membered saturated hetero ring group" is a 4- to 6-membered saturated hetero ring group containing 1 to 2 heteroatoms selected from the group consisting of oxygen, sulfur, and nitrogen as the ring forming atom. One embodiment of the 4- to 6-membered saturated hetero ring group containing 1 to 2 heteroatoms selected from the group consisting of oxygen, sulfur, and nitrogen as the ring forming atom is a 4- to 6-membered saturated hetero ring group containing one heteroatom selected from the group consisting of oxygen, sulfur, and nitrogen as the ring forming atom. In one embodiment, it is a 5- to 6-membered saturated hetero ring group containing 1 to 2 heteroatoms selected from the group consisting of oxygen, sulfur, and nitrogen as the ring forming atom. In one embodiment, it is a 4-membered saturated hetero ring group containing one heteroatom selected from the group consisting of oxygen, sulfur, and nitrogen as the ring forming atom. In one embodiment, it is a 5-membered saturated hetero ring group containing 1 to 2 heteroatoms selected from the group consisting of oxygen, sulfur, and nitrogen as the ring forming atom. In one embodiment, it is a 6-membered saturated hetero ring group containing 1 to 2 heteroatoms selected from the group consisting of oxygen, sulfur, and nitrogen as the ring forming atom. In one embodiment, it is oxetanyl, tetrahydrofuranyl, tetrahydropyranyl, azetidinyl, pyrrolidinyl, piperidinyl, oxazolidinyl, imidazolidinyl, piperazinyl, morpholinyl, thiomorpholinyl, dioxothiomorpholinyl, or tetrahydropyridyl. In one embodiment, it is oxetanyl, tetrahydrofuranyl, tetrahydropyranyl, azetidinyl, pyrrolidinyl, piperidinyl, piperazinyl, morpholinyl, thiomorpholinyl, or dioxothiomorpholinyl. In one embodiment, it is oxetanyl, tetrahydrofuranyl, tetrahydropyranyl, pyrrolidinyl, piperidinyl, piperazinyl, or morpholinyl. In one embodiment, it is oxetanyl, tetrahydrofuranyl, tetrahydropyranyl, pyrrolidinyl, piperazinyl, or piperidinyl. In one embodiment, it is oxetanyl, tetrahydrofuranyl, or tetrahydropyranyl. In one embodiment, it is piperazinyl.

Likewise, the "4- to 11-membered saturated hetero ring group" is a 4- to 11-membered saturated hetero ring group containing a heteroatom selected from the group consisting of oxygen, sulfur, and nitrogen as the ring forming atom, and may have a bridged structure, and may have a spiro structure. One embodiment of the "4- to 11-membered saturated hetero ring group" is a 4- to 11-membered saturated hetero ring group containing 1 to 2 heteroatoms selected from the group consisting of oxygen, sulfur, and nitrogen as the ring forming atom. In one embodiment, it is a 4- to 11-membered saturated hetero ring group containing one heteroatom selected from the group consisting of oxygen, sulfur, and nitrogen as the ring forming atom. In one embodiment, it is a 4- to 11-membered saturated hetero ring group containing 2 heteroatoms selected from the group consisting of oxygen, sulfur, and nitrogen as the ring forming atom. In one embodiment, it is 4- to 11-membered saturated hetero ring group containing 1 to 2 nitrogen atoms as the ring forming atom. In one embodiment, it is 4- to 11-membered saturated hetero ring group containing one nitrogen atom as the ring forming atom. In one embodiment, it is 4- to 11-membered saturated hetero ring group containing 2 nitrogen atoms as the ring forming atom. In one embodiment, it is oxetanyl, tetrahydrofuranyl, tetrahydropyranyl, azetidinyl, pyrrolidinyl, piperidinyl, oxazolidinyl, imidazolidinyl, piperazinyl, morpholinyl, thiomorpholinyl, dioxothiomorpholinyl, tetrahydropyridyl, 2,5-diazabicyclo[2.2.2]octan-2-yl, 2,5-diazabicyclo[2.2.1]heptan-2-yl, 3,6-diazabicyclo[3.1.1]heptan-3-yl, 3,6-diazabicyclo[3.1.1]heptan-6-yl, 2,7-diazaspiro[3.5]nonan-2-yl, 2,7-diazaspiro[3.5]nonan-7-yl, 2,8-diazaspiro[4.5]decan-2-yl, 2,8-diazaspiro[4.5]decan-8-yl, 3,9-diazaspiro[5.5]undecan-3-yl, 3,9-diazaspiro[5.5]undecan-9-yl, 4,7-diazaspiro[2.5]octan-4-yl, or 4,7-diazaspiro[2.5]octan-7-yl. In one embodiment, it is azetidinyl, pyrrolidinyl, piperidinyl, piperazinyl, morpholinyl, 2,5-diazabicyclo[2.2.1]heptan-2-yl, 2,8-diazaspiro[4.5]decan-8-yl, or 4,7-diazaspiro[2.5]octan-7-yl.

The "divalent group of saturated hetero ring" is a divalent group of a 4- to 11-membered saturated hetero ring group containing 1 to 2 nitrogen atoms as the ring forming atom, and may further contain 1 to 2 oxygen atoms, may be a divalent group of a saturated hetero ring having spiro ring and fused ring, or may have an unsaturated bond in part of the saturated hetero ring. One embodiment of the "divalent group of a saturated hetero ring" is a divalent group of a 4- to 11-membered saturated hetero ring containing 1 to 2 heteroatoms selected from the group consisting of oxygen, sulfur, and nitrogen as the ring forming atom. In one embodiment, it is a divalent group of a 4- to 11-membered saturated hetero ring containing 1 to 2 heteroatoms selected from the group consisting of oxygen and nitrogen as the ring forming atom. In one embodiment, it is a divalent group of a 4- to 11-membered saturated hetero ring containing 1 to 2 nitrogen atoms as the ring forming atom. One embodiment of the "divalent group of saturated hetero ring containing 1 to 2 nitrogen atoms" is azetidinediyl, pyrrolidinediyl, imidazolidinediyl, piperidinediyl, piperazinediyl, azepanediyl, diazepanediyl, azocanediyl, diazocanediyl, azonanediyl, diazonanediyl, tetrahydropyridinediyl, or divalent group represented by formula (XLIV), (XLV), (XLVI), (XLVII), or (XLVIII) below:

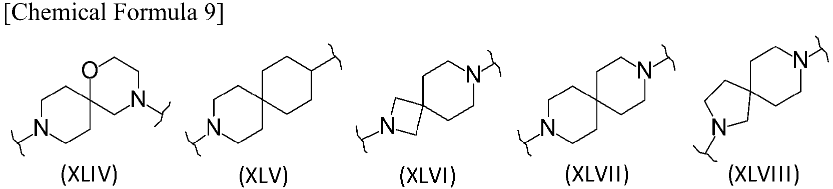

One embodiment of the "divalent group of saturated hetero ring" is pyrrolidinediyl, piperazinediyl, tetrahydropyridinediyl, or divalent group represented by formula (XLIV), (XLV), (XLVI), (XLVII), or (XLVIII) above. In one embodiment, it is tetrahydropyridinediyl or a divalent group represented by formula (XLIV), (XLV), (XLVI), (XLVII), or (XLVIII) above. In one embodiment, it is a divalent group represented by formula (XLIV), (XLV), (XLVI), (XLVII), or (XLVIII) above. In one embodiment, it is divalent group represented by formula (XLV) above. In one embodiment, it is divalent group represented by the following formula (XLIV), (XLV-1), (XLV-2), (XLVI), or (XLVII):

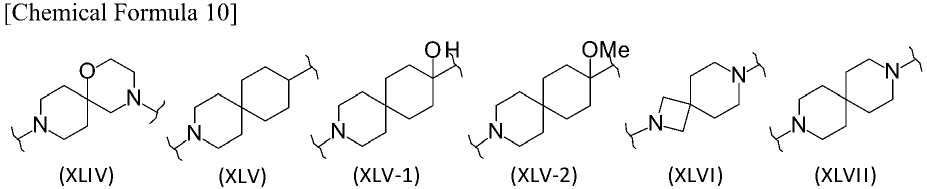

The "hetero ring" is an aromatic hydrocarbon ring containing heteroatom selected from the group consisting of oxygen, sulfur, and nitrogen as the ring forming atom.

Accordingly, the "6-membered hetero ring" is 6-membered aromatic hydrocarbon ring containing nitrogen atom as the ring forming atom. In one embodiment, it is pyridine ring, a pyridazine ring, a pyrimidine ring, pyrazine ring, or triazine ring. In one embodiment, it is pyridine ring.

The "5- or 6-membered hetero ring" is a 5- or 6-membered aromatic hydrocarbon ring containing heteroatom selected from the group consisting of oxygen, sulfur, and nitrogen as the ring forming atom. In one embodiment, it is pyrazole ring, triazole ring, imidazole ring, thiazole ring, isothiazole ring, oxazole ring, isoxazole ring, oxadiazole ring, thiadiazole ring, tetrazole ring, pyridine ring, pyridazin ring, pyrimidine ring, pyrazine ring, or triazine ring. In one embodiment, it is pyrazole ring, triazole ring, pyridine ring, or pyridazine ring. In one embodiment, it is pyridine ring. In one embodiment, it is pyridazine ring.

The "heteroaryl" is a hetero ring group containing heteroatom selected from the group consisting of oxygen, sulfur, and nitrogen as the ring forming atom.

Accordingly, the "5-membered heteroaryl" is 5-membered hetero ring group containing 1 to 4 heteroatoms selected from the group consisting of oxygen, sulfur, and nitrogen as the ring forming atom.

One aspect of the "5-membered heteroaryl" is a 5-membered hetero ring group containing 1 to 3 heteroatoms selected from the group consisting of oxygen, sulfur, and nitrogen as the ring forming atom. In one embodiment, it is pyrazolyl, imidazolyl, triazolyl, tetrazolyl, oxazolyl, isoxazolyl, thiazolyl, isothiazolyl, oxadiazolyl, or thiadiazolyl. In one embodiment, it is pyrazolyl, triazolyl, or oxazolyl. In one embodiment, it is pyrazolyl or triazolyl.

The "6-membered heteroaryl" is a 6-membered hetero ring group containing 1 to 3 nitrogen atoms as the ring forming atom. One aspect of the "6-membered heteroaryl" is pyridyl, pyrimidinyl, pyrazinyl, pyridazinyl, or triazinyl. In one embodiment, it is pyridyl or pyridazinyl. In one embodiment, it is pyridyl or pyrimidinyl. In one embodiment, it is pyridyl. In one embodiment, it is pyrimidinyl.

The "halogen" indicate F, Cl, Br, and I. In one embodiment, it is F, Cl, or Br. In one embodiment, it is F or Cl. In one embodiment, it is F or Br. In one embodiment, it is F. In one embodiment, it is Cl. In one embodiment, it is Br.

The "spiro ring" indicates polycyclic ring structure in which two ring structures are bonded with one shared spiro atom as a quaternary carbon, and the "fused ring" indicates a polycyclic ring structure in which two or more ring structures are bonded with two or more adjacent atoms comprising one of the rings being shared. The "bridged structure" is divalent chain structure linked to two non-adjacent atoms among the ring forming atoms in one ring.

One embodiment of acceptable substituents in "optionally substituted C₁₋₆ alkyl ", "optionally substituted C₁₋₃ alkyl ", and "optionally substituted C₁₋₃ alkylene " is F, OH, OCH₃, N(C₁₋₃ alkyl optionally substituted with F)₂, optionally substituted C₃₋₆ cycloalkyl, azabicyclo[3.3.0]octanyl, or optionally substituted 4- to 6-membered saturated hetero ring group. In one embodiment, it is F, OH, OCH₃, cyclopropyl, N(C₁₋₃ alkyl optionally substituted with F)₂, pyrrolidinyl optionally substituted with (C₁₋₃ alkyl optionally substituted with F), and tetrahydrofuranyl. In one embodiment, it is F, OH, OCH₃, N(CH₃)₂, hydroxymethyl, methoxymethyl, difluoroethyl, optionally substituted cyclopropyl, optionally substituted cyclobutyl, tetrahydrofuranyl, optionally substituted tetrahydropyranyl, optionally substituted morpholinyl, optionally substituted pyrrolidinyl, or optionally substituted piperidinyl. In one embodiment, it is F, OH, OCH₃, N(CH₃)₂, hydroxymethyl, methoxymethyl, optionally substituted cyclopropyl, optionally substituted cyclobutyl, optionally substituted morpholinyl, tetrahydrofuranyl, optionally substituted tetrahydropyranyl, or optionally substituted pyrrolidinyl. In one embodiment, it is F, OH, OCH₃, N(CH₃)₂, hydroxymethyl, methoxymethyl, cyclopropyl, (hydroxymethyl)cyclopropyl, (methoxymethyl)cyclopropyl, (dimethylaminomethyl)cyclobutyl, tetrahydrofuranyl, tetrahydropyranyl, (hydroxymethyl)tetrahydropyranyl, (methoxymethyl)tetrahydropyranyl, pyrrolidinyl, morpholinyl, fluoropyrrolidinyl, or methylpyrrolidinyl. In one embodiment, it is F, OH, OCH₃, (methoxymethyl)cyclopropyl, (dimethylaminomethyl)cyclobutyl, tetrahydrofuranyl, morpholinyl, fluoropyrrolidinyl, or methylpyrrolidinyl. In one embodiment, it is F, OH, or cyclopropyl. In one embodiment, it is OH, OCH₃, N(CH₃)₂, methylpyrrolidine, pyrrolidine optionally substituted with F, or morpholine. In one embodiment, it is N(CH₃)₂. In one embodiment, it is F, OH, or OCH₃. In one embodiment, it is OH or OCH₃. In one embodiment, it is F or OCH₃. In one embodiment, it is OH. In one embodiment, it is F. In one embodiment, it is OCH₃. In one embodiment, it is OH, OCH₃, N(CH₃)₂, methylpyrrolidinyl, morpholinyl, fluoropyrrolidinyl, (hydroxymethyl)cyclopropyl, or (dimethylaminomethyl)cyclobutyl.

One embodiment of acceptable substituents in the "optionally substituted 5-membered heteroaryl", "optionally substituted 6-membered heteroaryl", "optionally substituted 5- or 6-membered hetero ring", "optionally substituted benzene", and "optionally substituted oxazolyl" is C₁₋₃ alkyl optionally substituted with a group selected from the group consisting of OH and OCH₃, -SO₂CH₃, halogen, OH, OCH₃, or C₃₋₆ cycloalkyl. In one embodiment, it is C₁₋₃ alkyl optionally substituted with a group selected from the group consisting of OH and OCH₃. In one embodiment, it is C₁₋₃ alkyl optionally substituted with OH. In one embodiment, it is C₁₋₃ alkyl optionally substituted with OCH₃. In one embodiment, it is C₁₋₃ alkyl or halogen. In one embodiment, it is methyl, ethyl, methoxymethyl, or F. In one embodiment, it is methyl, ethyl, F, or Cl. In one embodiment, it is methyl, ethyl, or F.

One embodiment of acceptable substituents in the "optionally substituted 4- to 6-membered saturated hetero ring group", "optionally substituted 4- to 11-membered saturated hetero ring group", "optionally substituted pyrrolidinyl", "optionally substituted piperidinyl", "optionally substituted tetrahydropyranyl", "optionally substituted C₃₋₆ cycloalkyl", "optionally substituted C₃₋₆ cycloalkylene", and "divalent group of optionally substituted saturated hetero ring" is C₁₋₃ alkyl optionally substituted with a group selected from the group consisting of F, OH, OCH₃, and N(CH₃)₂, F, OH, OCH₃, oxo, or oxetanyl. In one embodiment, it is F, OH, or OCH₃. In one embodiment, it is OH or OCH₃. In one embodiment, it is OH or methyl. In one embodiment, it is F. In one embodiment, it is OH. In one embodiment, it is OCH₃. In one embodiment, it is C₁₋₃ alkyl optionally substituted with a group selected from the group consisting of F, OH, and OCH₃, F, oxo, or oxetanyl. In one embodiment, it is C₁₋₃ alkyl optionally substituted with a group selected from the group consisting of F, OH, OCH₃, and N(CH₃)₂, or oxo. In one embodiment, it is C₁₋₃ alkyl optionally substituted with a group selected from the group consisting of F, OH, OCH₃, and N(CH₃)₂. In one embodiment, it is C₁₋₃ alkyl optionally substituted with F. In one embodiment, it is C₁₋₃ alkyl optionally substituted with OH. In one embodiment, it is C₁₋₃ alkyl optionally substituted with OCH₃. In one embodiment, it is C₁₋₃ alkyl optionally substituted with N(CH₃)₂. In one embodiment, it is C₁₋₃ alkyl. In one embodiment, it is C₁₋₃ alkyl optionally substituted with N(CH₃)₂, or oxo. In one embodiment, it is C₁₋₃ alkyl optionally substituted with N(CH₃)₂, or F. In one embodiment, it is C₁₋₃ alkyl optionally substituted with OCH₃, dimethylaminomethyl, or F.

One embodiment of "C₁₋₃ alkyl optionally substituted with OH" is methyl optionally substituted with one OH group, or ethyl optionally substituted with 1 to 2 OH groups. Examples thereof include methyl, ethyl, hydroxymethyl, 1-hydroxyethyl, 2-hydroxyethyl, and 1,2-dihydroxyethyl. In one embodiment, it is methyl, ethyl, or hydroxymethyl. In one embodiment, it is methyl or hydroxymethyl. In one embodiment, it is hydroxymethyl or hydroxyethyl. In one embodiment, it is hydroxymethyl. In one embodiment, it is hydroxyethyl.

One aspect of the "C₁₋₆ alkyl optionally substituted with OCH₃" and "C₁₋₃ alkyl optionally substituted with OCH₃" is methyl optionally substituted with one OCH₃ group or ethyl optionally substituted with 1 to 2 OCH₃ groups. Examples thereof include methyl, ethyl, methoxymethyl, 1-methoxyethyl, 2-methoxyethyl, and 1,2-dimethoxyethyl. In one embodiment, it is methoxymethyl or methoxyethyl. In one embodiment, it is methoxymethyl. In one embodiment, it is methoxyethyl.

One aspect of "C₁₋₆ alkyl optionally substituted with N(C₁₋₃ alkyl)₂" is "C₁₋₃ alkyl optionally substituted with N(CH₃)₂". In one embodiment, it is methyl optionally substituted with one N(C₁₋₃ alkyl)₂, ethyl optionally substituted with one N(C₁₋₃ alkyl)₂, or n-propyl optionally substituted with one N(C₁₋₃ alkyl)₂. In one embodiment, it is methyl optionally substituted with one N(C₁₋₃ alkyl)₂, or ethyl optionally substituted with one N(C₁₋₃ alkyl)₂.

One embodiment of "C₁₋₃ alkyl optionally substituted with N(CH₃)₂" is methyl optionally substituted with one N(CH₃)₂, or ethyl optionally substituted with one N(CH₃)₂. In one embodiment, it is methyl, ethyl, dimethylaminomethyl, or dimethylaminoethyl. In one embodiment, it is methyl or dimethylaminomethyl. In one embodiment, it is dimethylaminomethyl. In one embodiment, it is dimethylaminoethyl.

One embodiment of "phenylene optionally substituted with F" is phenylene optionally substituted with 1 to 2 F atoms. In one embodiment, it is phenylene optionally substituted with one F atom. In one embodiment, it is phenylene or fluorophenylene. In one embodiment, it is phenylene. In one embodiment, it is 2-fluoro-1,4-phenylene. In one embodiment, it is 3-fluoro-1,4-phenylene.

The "attachment point" indicates a site which forms covalent bond with a specific atom or a substituent. Accordingly, "attachment point to L" indicates that the site having the "attachment point to L" and L form covalent bond.

The "mutated KRAS" is KRAS having mutation, and indicates G12C mutated KRAS, G12D mutated KRAS, G12V mutated KRAS, and/or G13D mutated KRAS, for example.

"G12C mutation" indicates a mutation in a wild-type protein in which the amino acid residue corresponding to codon 12 is converted from glycine to cysteine.

"G12C mutated KRAS" indicates KRAS having the "G12C mutation" above.

"G12D mutation" indicates a mutation in a wild-type protein in which the amino acid residue corresponding to codon 12 is converted from glycine to aspartic acid.

"G12D mutated KRAS" indicates KRAS having the "G12D mutation" above.

"G12V mutation" indicates a mutation in a wild-type protein in which the amino acid residue corresponding to codon 12 is converted from glycine to valine.

"G12V mutated KRAS" indicates KRAS having the "G12V mutation" above.

"G13D mutation" indicates a mutation in a wild-type protein in which the amino acid residue corresponding to codon 13 is converted from glycine to aspartic acid.

"G13D mutated KRAS" indicates KRAS having the "G13D mutation" above.

The "cancer" indicates a malignant tumor. In one embodiment, it is pancreatic cancer, lung cancer, colon cancer, skin cancer, uterus cancer, thyroid cancer, bladder cancer, gastric cancer, epithelia cancer, esophagus cancer, liver cancer, breast cancer, ovarian cancer, prostate cancer, kidney cancer, myeloma, lymphoma, or leukemia. In one embodiment, it is pancreatic cancer, lung cancer, colon cancer, or gastric cancer. In one embodiment, it is pancreatic cancer, lung cancer, or colon cancer. In one embodiment, it is also pancreatic cancer, lung cancer, colon cancer, skin cancer such as melanoma, cervical cancer, uterus cancer such as endometrial cancer, thyroid cancer, bladder cancer, gastric cancer, epithelia cancer, esophagus cancer, liver cancer, breast cancer, ovarian cancer, prostate cancer, renal cell carcinoma or kidney cancer, myelodysplastic syndrome, myeloma such as myeloproliferative neoplasm, lymphoma such as mature B-cell lymphoma, leukemia, esophagogastric junction cancer, head and neck cancer, glioma, hepatobiliary cancer, soft tissue sarcoma, appendix cancer, small intestinal cancer, germ cell tumor, neuroendocrine carcinoma, gallbladder cancer, peritoneal cancer, or fallopian tube cancer. In one embodiment, it is also germ cell tumor, esophagogastric junction cancer, ovarian cancer, and pancreatic cancer. In one embodiment, it is also solid cancer and hematological cancer. In one embodiment, it is solid cancer. In one embodiment, it is hematological cancer.

The "mutated KRAS-positive cancer" is mutated KRAS-positive cancer, particularly G12C mutated, G12D mutated, G12V mutated, and/or G13D mutated KRAS-positive cancer. For example, it is a cancer wherein KRAS G12C mutation, G12D mutation, G12V, mutation, and/or G13D mutation occur, and is a cancer showing high positive rates of G12C mutation, G12D mutation, G12V mutation, and/or G13DKRAS.

One embodiment of the "mutated KRAS-positive cancer" is:
G12C mutated, G12D mutated, and G12V mutated KRAS-positive cancer,
G12C mutated, G12D mutated, and G13D mutated KRAS-positive cancer,
G12C mutated, G12V mutated, and G13D mutated KRAS-positive cancer, and
G12D mutated, G12V mutated, and G13D mutated KRAS-positive cancer.

One embodiment of the "mutated KRAS-positive cancer" is:
G12C mutated and G12D mutated KRAS-positive cancer,
G12C mutated and G12V mutated KRAS-positive cancer,
G12D mutated and G12V mutated KRAS-positive cancer,
G12C mutated and G13D mutated KRAS-positive cancer,
G12D mutated and G13D mutated KRAS-positive cancer, and
G12V mutated and G13D mutated KRAS-positive cancer.

One embodiment of the "mutated KRAS-positive cancer" is:
G12C mutated KRAS-positive cancer,
G12D mutated KRAS-positive cancer,
G12V mutated KRAS-positive cancer, and
G13D mutated KRAS-positive cancer.

The "G12C mutated KRAS-positive cancer" is G12C mutated KRAS-positive cancer. For example, it is a cancer wherein KRAS G12C mutation occurs, and is a cancer showing a high positive rate of G12C mutated KRAS. In one embodiment, it is pancreatic cancer, lung cancer, or colon cancer showing a high positive rate of G12C mutated KRAS.

The "G12D mutated KRAS-positive cancer" is G12D mutated KRAS-positive cancer. For example, it is a cancer wherein KRAS G12D mutation occurs, and is a cancer showing a high positive rate of G12D mutated KRAS. In one embodiment, it is pancreatic cancer, lung cancer, or colon cancer showing a high positive rate of G12D mutated KRAS.

The "G12V mutated KRAS-positive cancer" is G12V mutated KRAS-positive cancer. For example, it is a cancer wherein KRAS G12V mutation occurs, and is a cancer showing a high positive rate of G12V mutated KRAS. In one embodiment, it is pancreatic cancer, lung cancer, or colon cancer showing a high positive rate of G12V mutated KRAS.

The "G13D mutated KRAS-positive cancer" is G13D mutated KRAS-positive cancer. For example, it is a cancer wherein KRAS G13D mutation occurs, and is a cancer showing a high positive rate of G13D mutated KRAS. In one embodiment, it is pancreatic cancer, lung cancer, or colon cancer showing a high positive rate of G13D mutated KRAS.

The "wild-type KRAS gene amplification" indicates that the KRAS gene is amplified to a high level due to copy number alterations of the wild-type KRAS gene.

The "wild-type KRAS gene amplification-positive cancer" is a cancer having the "wild-type KRAS gene amplification" above. In one embodiment, it is germ cell tumor, esophagogastric junction cancer, ovarian cancer, and pancreatic cancer having the wild-type KRAS gene amplification.

The "pancreatic cancer" is a malignant tumor formed in a pancreas. For example, it is pancreatic ductal cancer and pancreatic ductal adenocarcinoma. In one embodiment, it is pancreatic ductal cancer. In one embodiment, it is pancreatic ductal adenocarcinoma. Furthermore, in one embodiment, it is metastatic pancreatic cancer. In one embodiment, it is locally advanced pancreatic cancer. In one embodiment, it is recurrent or refractory pancreatic cancer. In one embodiment, it is pancreatic cancer in a patient untread and/or having a treatment history.

The "lung cancer" is a malignant tumor formed in a lung. For example, it is small cell lung cancer and non-small cell lung cancer. In one embodiment, it is small cell lung cancer. In one embodiment, it is non-small cell lung cancer. Furthermore, in one embodiment, it is metastatic lung cancer. In one embodiment, it is locally advanced lung cancer. In one embodiment, it is recurrent or refractory lung cancer. In one embodiment, it is lung cancer in a patient untread and/or having a treatment history. In one embodiment, it is non-squamous non-small cell lung cancer. As another aspect, it is squamous non-small cell lung cancer.

The "colon cancer" is a malignant tumor formed in a colon. For example, it is colonic cancer and rectal cancer. In one embodiment, it is colonic cancer. In one embodiment, it is rectal cancer. Furthermore, in one embodiment, it is metastatic colon cancer. In one embodiment, it is locally advanced colon cancer. In one embodiment, it is recurrent or refractory colon cancer. In one embodiment, it is colon cancer in a patient untread and/or having a treatment history.

The "gastric cancer" is a malignant tumor formed in a stomach. In one embodiment, it is metastatic gastric cancer. In one embodiment, it is locally advanced gastric cancer. In one embodiment, it is recurrent or refractory gastric cancer. In one embodiment, it is gastric cancer in a patient untread and/or having a treatment history.

The "esophagogastric junction cancer" is a malignant tumor formed in a junction of an esophagus and a stomach. In one embodiment, it is metastatic esophagogastric junction cancer. In one embodiment, it is locally advanced esophagogastric junction cancer. In one embodiment, it is recurrent or refractory esophagogastric junction cancer. In one embodiment, it is esophagogastric junction cancer in a patient untread and/or having a treatment history.

The "ovarian cancer" is a malignant tumor formed in an ovary. In one embodiment, it is metastatic ovarian cancer. In one embodiment, it is locally advanced ovarian cancer. In one embodiment, it is recurrent or refractory ovarian cancer. In one embodiment, it is ovarian cancer in a patient untread and/or having a treatment history.

The "germ cell tumor" is a tumor into which germ cells have been transformed for some reason. In one embodiment, it is a metastatic germ cell tumor. In one embodiment, it is a locally advanced germ cell tumor. In one embodiment, it is a recurrent or refractory germ cell tumor. In one embodiment, it is a germ cell tumor in a patient untread and/or having a treatment history.

The "solid cancer" is a cancer other than hematological cancer. In one embodiment, it is, pancreatic cancer, lung cancer, colon cancer, skin cancer such as melanoma, uterus cancer such as endometrial cancer, thyroid cancer, bladder cancer, gastric cancer, epithelia cancer, esophagus cancer, liver cancer, breast cancer, ovarian cancer, prostate cancer, renal cell carcinoma or kidney cancer, esophagogastric junction cancer, head and neck cancer, glioma, hepatobiliary cancer, soft tissue sarcoma, appendix cancer, small intestinal cancer, germ cell tumor, neuroendocrine carcinoma, gallbladder cancer, peritoneal cancer, or fallopian tube cancer.

The "hematological cancer" is a disease in which blood cells become cancerous and abnormally proliferate. In one embodiment, it includes leukemia such as acute myeloid leukemia, chronic myeloid leukemia, acute lymphocytic leukemia, and chronic lymphocytic leukemia, malignant lymphoma such as Hodgkin's lymphoma, non-Hodgkin's lymphoma, and mature B-cell lymphoma, multiple myeloma, myelodysplastic syndrome, polycythemia vera, essential thrombocythemia, primary myelofibrosis and the like.

The "ability to bind to a RAS protein" indicates that the compound can bond to the RAS protein. In one embodiment, it indicates that the compound can bond to the KRAS protein, HRAS protein, and/or NRAS protein contained in the RAS family. In one embodiment, it indicates that the compound shows inhibitory activity on the KRAS protein, HRAS protein, and/or NRAS protein contained in the RAS family. The RAS protein shown here includes mutated RAS proteins, such as G12C, G12D, G12V, and G13D mutated KRAS proteins. The ability to bind to a RAS protein can be evaluated by the methods described in Patent Document 1 and Patent Document 2 above or by a similar method thereof.

One embodiment of the "group having an ability to bind to a RAS protein" is a group having an ability to bind to the KRAS protein, HRAS protein, and/or NRAS protein contained in the RAS family. In one embodiment, it indicates that the group is a group formed by replacing any hydrogen atom on the compound having inhibitory activity on the KRAS protein, HRAS protein, and/or NRAS protein contained in the RAS family by an attachment point.

One embodiment of the "group having an ability to bind to a RAS protein" is a group formed by replacing any hydrogen atom on the compound having inhibitory activity on the KRAS protein by an attachment point, such as a group formed by replacing any hydrogen atom on the compound having inhibitory activity on the KRAS protein described in the following literatures by an attachment point:
WO 2022/132200, WO 2022/133038, WO 2022/047260, WO 2022/173870, WO 2022/061251, WO 2022/250170, WO 2023/001123, WO 2023/287896, WO 2023/287896, WO 2023/099623, WO 2023/099608, WO 2023/099592, WO 2023/099624, WO 2023/137223, WO 2023/138589, WO 2023/154766, WO 2023/114733, WO 2023/172737, WO 2023/183585, WO 2023/244599, WO 2023/244604, WO 2023/232776, WO 2023/244615, WO 2024/041621, WO 2024/041589, WO 2024/044667, WO 2024/086061, WO 2024/104425, WO 2023/230190, WO 2016/049568, WO 2017/172979, WO 2018143315, and WO 2022173033.

One embodiment of the "group having an ability to bind to a RAS protein" is a group serving as the ligand of the target protein in bifunctional compounds such as PROTACs which degrade KRAS proteins, such as a group serving as the ligand of the target protein in bifunctional compounds such as PROTACs which degrade KRAS proteins described in the following literatures:
Cell. Chem. Biol., 2020, 27, p. 19-31, ACS Cent. Sci., 2020, 6, p. 1367-1375, U.S. Patent Application No. 2018/0015087, WO 2019/195609, WO 2020/018788, WO 2021/051034, WO 2021/207172, WO 2022/111521, WO 2022/061348, WO 2022/148422, WO 2022/228576, WO 2023/059609, WO 2023/077441, WO 2023/280026, China Patent Application Nos. 113956233 and 115785199, WO 2023/138524, WO 2022/173032, WO 2023/171781, WO 2022/087335, WO 2022/212611, WO 2022/266249, WO 2022/271823, WO 2023/099620, WO 2023/130012, WO 2023/141570, WO 2024019103, WO 2024034591, WO 2024029613, and WO 2024034593.

One embodiment of the compound represented by formula (I) or a salt thereof according to the present invention is shown below.
(1-1) A compound represented by formula (I) or a salt thereof, wherein A is CR^{A} or N, and R^{A} is H, C₁₋₃ alkyl, -CN, or -O-(C₁₋₃ alkyl).
(1-2) A compound represented by formula (I) or a salt thereof, wherein A is CR^{A} or N, and R^{A} is H or C₁₋₃ alkyl.
(1-3) A compound represented by formula (I) or a salt thereof, wherein A is CR^{A} or N, and R^{A} is H.
(1-4) A compound represented by formula (I) or a salt thereof, wherein A is CR^{A} or N, and R^{A} is C₁₋₃ alkyl.
(1-5) A compound represented by formula (I) or a salt thereof, wherein A is N.
(1-6) A compound represented by formula (I) or a salt thereof, wherein A is CR^{A}, and R^{A} is H, C₁₋₃ alkyl, -CN, or -O-(C₁₋₃ alkyl).
(1-7) A compound represented by formula (I) or a salt thereof, wherein A is CR^{A}, and R^{A} is H.
(1A-1) A compound represented by formula (I) or a salt thereof, wherein E is CH or N.
(1A-2) A compound represented by formula (I) or a salt thereof, wherein E is CH.
(1A-3) A compound represented by formula (I) or a salt thereof, wherein E is N.
(2-1) A compound represented by formula (I) or a salt thereof, wherein X¹ is -CH₂-, -O-, or -NR^{X1}-,
   R^{X1} is H or optionally substituted C₁₋₃ alkyl, and
   provided that when X¹ is -NR^{X1}-, R^{X1} and R⁴ present on the same nitrogen atom, together with the nitrogen atom adjacent thereto, may form optionally substituted 4- to 11-membered saturated hetero ring group.
(2-1-1) A compound represented by formula (I) or a salt thereof, wherein X¹ is -CH₂-, -O-, or -NR^{X1}-,
   R^{X1} is H or optionally substituted C₁₋₃ alkyl, and
   provided that when X¹ is -NR^{X1}-, R^{X1} and R⁴ present on the same nitrogen atom, together with the nitrogen atom adjacent thereto, may form optionally substituted 4- to 6-membered saturated hetero ring group.
(2-2) A compound represented by formula (I) or a salt thereof, wherein X¹ is -O- or - NR^{X1}-,
   R^{X1} is H or C₁₋₃ alkyl, and
   provided that when X¹ is -NR^{X1}-, R^{X1} and R⁴ present on the same nitrogen atom, together with the nitrogen atom adjacent thereto, may form optionally substituted 4- to 11-membered saturated hetero ring group.
(2-2-1) A compound represented by formula (I) or a salt thereof, wherein X¹ is -O- or - NR^{X1}-,
   R^{X1} is H or C₁₋₃ alkyl, and
   provided that when X¹ is -NR^{X1}-, R^{X1} and R⁴ present on the same nitrogen atom, together with the nitrogen atom adjacent thereto, may form optionally substituted 4- to 6-membered saturated hetero ring group.
(2-3) A compound represented by formula (I) or a salt thereof, wherein X¹ is -O- or - NR^{X1}-, and
   provided that when X¹ is -NR^{X1}-, R^{X1} and R⁴ present on the same nitrogen atom, together with the nitrogen atom adjacent thereto, form optionally substituted 4- to 11-membered saturated hetero ring group.
(2-3-1) A compound represented by formula (I) or a salt thereof, wherein X¹ is -O- or - NR^{X1}-, and
   provided that when X¹ is -NR^{X1}-, R^{X1} and R⁴ present on the same nitrogen atom, together with the nitrogen atom adjacent thereto, form optionally substituted 4- to 6-membered saturated hetero ring group.
(2-4) A compound represented by formula (I) or a salt thereof, wherein X¹ is -O- or - NR^{X1}-, and
   R^{X1} is H or C₁₋₃ alkyl.
(2-5) A compound represented by formula (I) or a salt thereof, wherein X¹ is -O-.
(2-6) A compound represented by formula (I) or a salt thereof, wherein X¹ is -NR^{X1}-, and R^{X1} and R⁴ present on the same nitrogen atom, together with the nitrogen atom adjacent thereto, form optionally substituted 4- to 11-membered saturated hetero ring group.
(2-6-1) A compound represented by formula (I) or a salt thereof, wherein X¹ is -NR^{X1}-, and R^{X1} and R⁴ present on the same nitrogen atom, together with the nitrogen atom adjacent thereto, form optionally substituted 4- to 6-membered saturated hetero ring group.
(2-7) A compound represented by formula (I) or a salt thereof, wherein X¹ is -NR^{X1}-, and R^{X1} and R⁴ present on the same nitrogen atom, together with the nitrogen atom adjacent thereto, form 4- to 11-membered saturated hetero ring group optionally substituted with C₁₋₃ alkyl optionally substituted with N(CH₃)₂, or F.
(2-7-1) A compound represented by formula (I) or a salt thereof, wherein X¹ is -NR^{X1}-, and R^{X1} and R⁴ present on the same nitrogen atom, together with the nitrogen atom adjacent thereto, form 4- to 6-membered saturated hetero ring group optionally substituted with C₁₋₃ alkyl optionally substituted with N(CH₃)₂, or F.
(2-8) A compound represented by formula (I) or a salt thereof, wherein X¹ is -NR^{X1}-, and R^{X1} and R⁴ present on the same nitrogen atom, together with the nitrogen atom adjacent thereto, form a 4- to 11-membered saturated hetero ring group optionally substituted with C₁₋₃ alkyl.
(2-8-1) A compound represented by formula (I) or a salt thereof, wherein X¹ is -NR^{X1}-, and R^{X1} and R⁴ present on the same nitrogen atom, together with the nitrogen atom adjacent thereto, form a 4- to 6-membered saturated hetero ring group optionally substituted with C₁₋₃ alkyl.
(2-9) A compound represented by formula (I) or a salt thereof, wherein X¹ is -NR^{X1}-, and R^{X1} and R⁴ present on the same nitrogen atom, together with the nitrogen atom adjacent thereto, form piperazinyl group optionally substituted with C₁₋₃ alkyl.
(3-1) A compound represented by formula (I) or a salt thereof, wherein R¹ is represented by the following formula (II), (III), (IV), (V), (VI), or (VII): 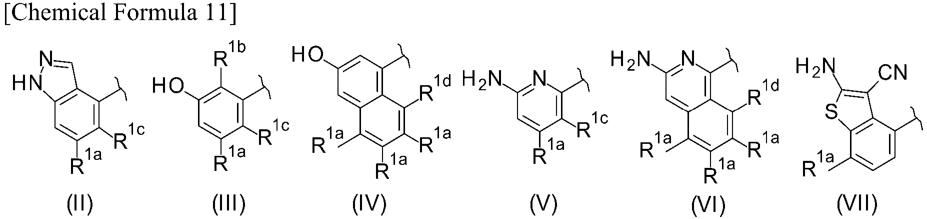
   R^{1a} and R^{1b} are the same or different, and are each H, methyl, F, or Cl,
   R^{1c} is F, Cl, methyl, ethyl, trifluoromethyl, or cyclopropyl, and
   R^{1d} is H, methyl, ethyl, F, Cl, or -C≡C-H.
(3-1-1) A compound represented by formula (I) or a salt thereof, wherein R¹ is represented by the following formula (II), (III), (IV), (V), (VI), or (VII): 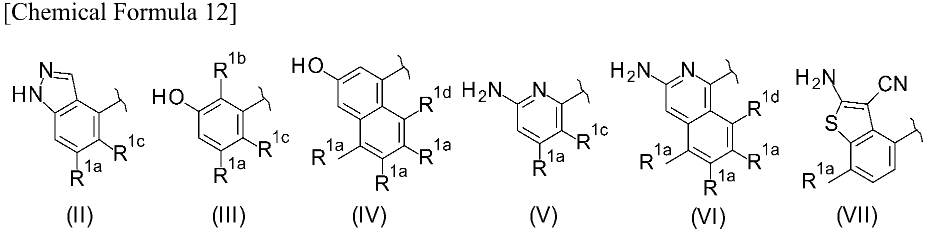
   R^{1a} is the same or different, and is H, methyl, F, or Cl,
   R^{1b} is H, methyl, F, or Cl,
   R^{1c} is F, Cl, methyl, ethyl, trifluoromethyl, or cyclopropyl, and
   R^{1d} is H, methyl, ethyl, F, Cl, or -C≡C-H.
(3-2) A compound represented by formula (I) or a salt thereof, wherein R¹ is represented by the following formula (II), (III), or (IV): 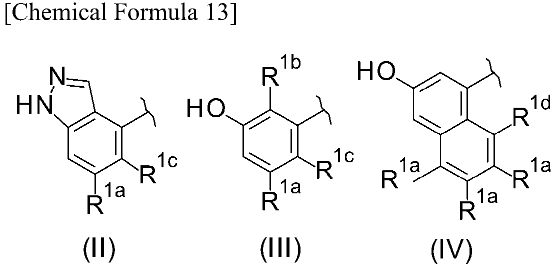
   R^{1a} is the same or different, and is H or F,
   R^{1b} is H,
   R^{1c} is methyl or cyclopropyl, and
   R^{1b} is H.
(3-3) A compound represented by formula (I) or a salt thereof, wherein R¹ is represented by the following formula (II): 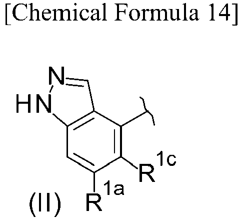
   R^{1a} is H, methyl, F, or Cl, and
   R^{1c} is F, Cl, methyl, or ethyl.
(3-4) A compound represented by formula (I) or a salt thereof, wherein R¹ is represented by the following formula (II): 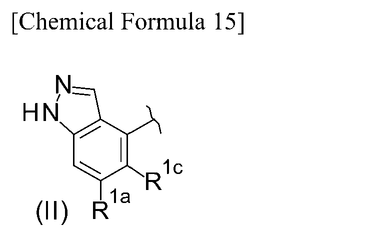
   R^{1a} is F, and
   R^{1c} is methyl.
(3-5) A compound represented by formula (I) or a salt thereof, wherein R¹ is represented by the following formula (III): 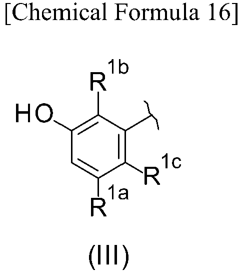
   R^{1a} is H or F,
   R^{1b} is H, and
   R^{1c} is methyl or cyclopropyl.
(3-6) A compound represented by formula (I) or a salt thereof, wherein R¹ is represented by the following formula (IV): 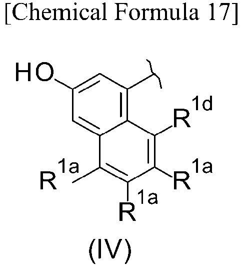
   R^{1a} is the same or different, and is H or F, and
   R^{1d} is H.
(4-1) A compound represented by formula (I) or a salt thereof, wherein R² is H, halogen, optionally substituted C₁₋₃ alkyl, cyclopropyl, or vinyl.
(4-2) A compound represented by formula (I) or a salt thereof, wherein R² is H, halogen, C₁₋₃ alkyl, cyclopropyl, or vinyl, and the C₁₋₃ alkyl is optionally substituted with a group selected from the group consisting of OH and OCH₃.
(4-3) A compound represented by formula (I) or a salt thereof, wherein R² is cyclopropyl or vinyl.
(4-4) A compound represented by formula (I) or a salt thereof, wherein R² is cyclopropyl.
(5-1) A compound represented by formula (I) or a salt thereof, wherein R³ is a group selected from the group consisting of the following formulas (VIII), (XI), (X), (XI), (XII), (XIII), (XIV), (XV), (XVI), and (XVII): 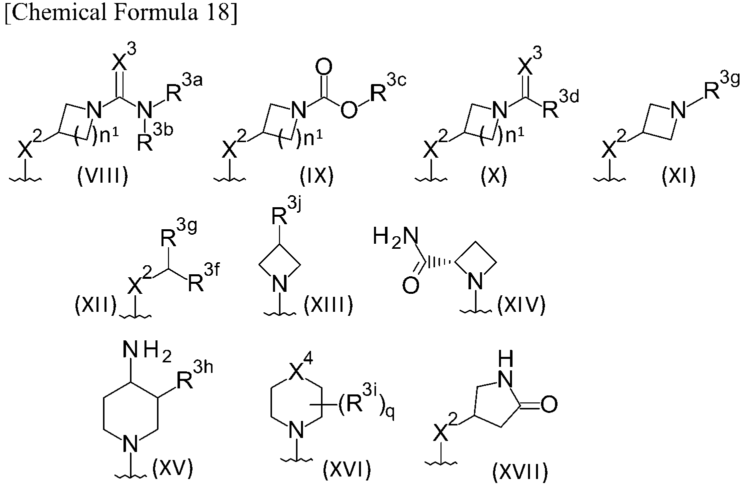
   R^{3a} is -(CH₂)ₚCHR^{3e}-NR^{N1}R^{N2}; -(CH₂)ₚCHR^{3e}-OR^{3f}; 4- to 6-membered saturated hetero ring group optionally substituted with a group selected from the group consisting of C₁₋₃ alkyl, -C₁₋₃ alkylene-OR^{3f}, -C₁₋₃ alkylene-NR^{N1}R^{N2}, and -NR^{N1}R^{N2}. or C₃₋₆ cycloalkyl optionally substituted with a group selected from the group consisting of C₁₋₃ alkyl, -C₁₋₃ alkylene-OR^{3f}, -C₁₋₃ alkylene-NR^{N1}R^{N2}, -OR^{3f}, and -NR^{N1}R^{N2},
   R^{3b} is H or C₁₋₃ alkyl,
   R^{3c} and R^{3d} are -(CH₂)ₚCHR^{3e}-NR^{N1}R^{N2}; -(CH₂)ₚCHR^{3e}-OR^{3f}; 4- to 6-membered saturated hetero ring group optionally substituted with a group selected from the group consisting of C₁₋₃ alkyl, -C₁₋₃ alkylene-OR^{3f}, -C₁₋₃ alkylene-NR^{N1}R^{N2}, and -NR^{N1}R^{N2}; or C₃₋₆ cycloalkyl optionally substituted with a group selected from the group consisting of C₁₋₃ alkyl, -C₁₋₃ alkylene-OR^{3f}, -C₁₋₃ alkylene-NR^{N1}R^{N2}, -OR^{3f}, and -NR^{N1}R^{N2},
   R^{3e} is H, F, or C₁₋₃ alkyl,
   R^{3f} is the same or different, and is H or C₁₋₃ alkyl,
   R^{3g} is optionally substituted C₃₋₆ cycloalkyl, optionally substituted 5-membered heteroaryl, optionally substituted 6-membered heteroaryl, or optionally substituted 4- to 6-membered saturated hetero ring group,
   R^{3h} is H, F, or C₁₋₃ alkyl,
   R³ⁱ is the same or different, and is a group selected from the group consisting of H, OH, optionally substituted C₁₋₃ alkyl, -O-optionally substituted C₁₋₃ alkyl, -NH-optionally substituted C₁₋₃ alkyl, -N-(optionally substituted C₁₋₃ alkyl)₂, halogen, -CN, and oxo, or
   two R³ⁱs present on the same carbon atom, together with the carbon atom adjacent thereto, may form a spiro ring having a ring selected from the group consisting of C₃₋₆ cycloalkane and 4- to 6-membered saturated hetero ring, and the spiro ring is optionally substituted with 1 to 2 groups selected from the group consisting of C₁₋₃ alkyl, -O-(C₁₋₃ alkyl), OH, halogen, and oxo, or
   R³ⁱ present on two carbon atoms adjacent thereto, together with the two carbon atoms, may form a fused ring having a ring selected from the group consisting of C₃₋₆ cycloalkane and 4- to 6-membered saturated hetero rings, and the fused ring is optionally substituted with 1 to 2 groups selected from the group consisting of C₁₋₃ alkyl, -O-(C₁₋₃ alkyl), OH, halogen, and oxo,
   R³ⁱ present on two carbon atoms not adjacent thereto, together with the two carbon atoms, may form a bridged structure composed of 1 to 2 carbon atoms, or the ring having the bridged structure is optionally substituted with 1 to 2 groups selected from the group consisting of C₁₋₃ alkyl, -O-(C₁₋₃ alkyl), OH, halogen, and oxo,
   R^{N1} and R^{N2} are the same or different, and are each H or C₁₋₃ alkyl, or
   R^{N1} and R^{N2} together with a nitrogen atom bonded thereto may form optionally substituted 4- to 6-membered saturated hetero ring group, or
   R^{3e} and R^{N1} together with a carbon atom and a nitrogen atom bonded thereto may form optionally substituted 4- to 6-membered saturated hetero ring group,
   R^{3j} is a group selected from the group consisting of H, OH, halogen, optionally substituted C₁₋₃ alkyl, -O-optionally substituted C₁₋₃ alkyl, optionally substituted C₃₋₆ cycloalkyl, optionally substituted 5-membered heteroaryl, optionally substituted 6-membered heteroaryl, and -CN,
   X² is -O-, -NH-, or -N(C₁₋₃ alkyl)-,
   X³ is O or S,
   X⁴ is -CH₂-, -CH₂-CH₂-, -O-CH₂-, -O-, or -O-NH-,
   n¹ is 1 or 2,
   p is 1 or 2, and
   q is 1 to 8.
(5-2) A compound represented by formula (I) or a salt thereof, wherein R³ is a group represented by the following formula (XII), (XIII), (XIV), or (XVII): 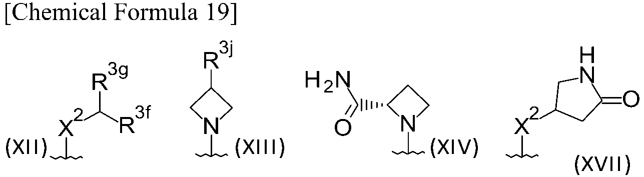
   R^{3f} is the same or different, and is H or C₁₋₃ alkyl,
   R^{3g} is optionally substituted C₃₋₆ cycloalkyl, optionally substituted 5-membered heteroaryl, optionally substituted 6-membered heteroaryl, or optionally substituted 4- to 6-membered saturated hetero ring group,
   R^{3j} is a group selected from the group consisting of H, OH, halogen, optionally substituted C₁₋₃ alkyl, -O-optionally substituted C₁₋₃ alkyl, optionally substituted C₃₋₆ cycloalkyl, optionally substituted 5-membered heteroaryl, optionally substituted 6-membered heteroaryl, and -CN, and
   X² is -O-, -NH-, or -N(C₁₋₃ alkyl)-.
(5-3) A compound represented by formula (I) or a salt thereof, wherein R³ is a group represented by (XIV): 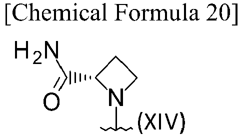
(6-1) A compound represented by formula (I) or a salt thereof, wherein R⁴ is optionally substituted C₁₋₆ alkyl, optionally substituted piperidinyl, or optionally substituted tetrahydropyranyl.
(6-2) A compound represented by formula (I) or a salt thereof, wherein R⁴ is C₁₋₆ alkyl or piperidinyl, and the C₁₋₆ alkyl is optionally substituted with a group selected from the group consisting of F; OH; OCH₃; N(R^{4a})₂; C₃₋₆ cycloalkyl optionally substituted with R^{4b}; pyrrolidinyl optionally substituted with R^{4b}, and morpholinyl optionally substituted with R^{4b}, and the piperidinyl is optionally substituted with R^{4b},
   R^{4a} is optionally substituted C₁₋₃ alkyl, and
   R^{4b} is optionally substituted C₁₋₃ alkyl or halogen.
(6-3) A compound represented by formula (I) or a salt thereof, wherein R⁴ is C₁₋₆ alkyl or piperidinyl, the C₁₋₆ alkyl is optionally substituted with a group selected from the group consisting of OH; OCH₃; N(R^{4a})₂; C₃₋₆ cycloalkyl optionally substituted with R^{4b}; pyrrolidinyl optionally substituted with R^{4b}, and morpholinyl optionally substituted with R^{4b}, and the piperidinyl is optionally substituted with R^{4b},
   R^{4a} is C₁₋₃ alkyl, and
   R^{4b} is C₁₋₃ alkyl optionally substituted with -O-(C₁₋₃ alkyl) or -N(C₁₋₃ alkyl)₂, or halogen.
(6-3-1) A compound represented by formula (I) or a salt thereof, wherein R⁴ is C₁₋₆ alkyl or piperidinyl, and the C₁₋₆ alkyl is optionally substituted with a group selected from the group consisting of OH; OCH₃; N(R^{4a})₂; C₃₋₆ cycloalkyl optionally substituted with R^{4b}; pyrrolidinyl optionally substituted with R^{4b}; and morpholinyl optionally substituted with R^{4b}, and the piperidinyl is optionally substituted with R^{4b},
   R^{4a} is C₁₋₃ alkyl, and
   R^{4b} is C₁₋₃ alkyl optionally substituted with a group selected from the group consisting of OH, -O-(C₁₋₃ alkyl), and -N(C₁₋₃ alkyl)₂, or halogen.
(6-4) A compound represented by formula (I) or a salt thereof, wherein R⁴ is C₁₋₆ alkyl optionally substituted with N(C₁₋₃ alkyl)₂.
(6-5) A compound represented by formula (I) or a salt thereof, wherein R⁴ is C₁₋₆ alkyl optionally substituted with N(Me)₂.
(7-1) A compound represented by formula (I) or a salt thereof, wherein Y is phenylene or pyridinediyl, and the phenylene is optionally substituted with F.
(7-2) A compound represented by formula (I) or a salt thereof, wherein Y is phenylene optionally substituted with F.
(7-3) A compound represented by formula (I) or a salt thereof, wherein Y is phenylene.
(7-4) A compound represented by formula (I) or a salt thereof, wherein Y is phenylene or pyridinediyl.
(8-1) A compound represented by formula (I) or a salt thereof, wherein L is -(L^{A}-L^{B}-L^{C}-L^{D}-L^{E}-L^{F})-,
   L^{A}, L^{B}, L^{C}, L^{D}, L^{E}, and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O, and
   R^{L1A} is H or C₁₋₃ alkyl.
(8-2) A compound represented by formula (I) or a salt thereof, wherein L is a group selected from the group consisting of the following formulas (XXX-L), (XXXI-L), (XXXII-L), (XXXIII-L), and (XXXIV-L): 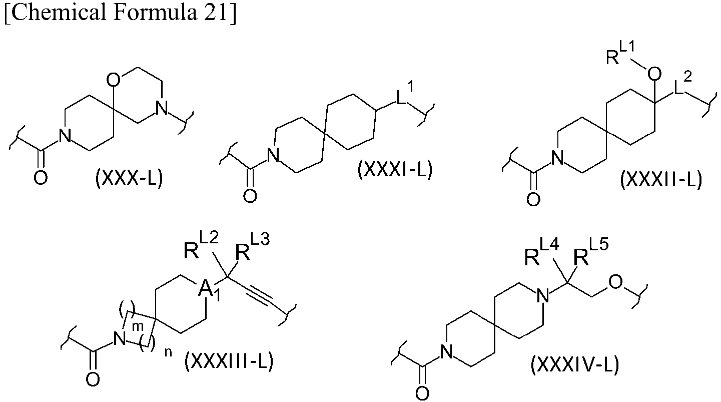
   L¹ is bond, C₁₋₃ alkylene, -C₁₋₃ alkylene-O-, -C₁₋₃ alkylene-C₂₋₃ alkynediyl, -C≡C-, or divalent group of optionally substituted saturated hetero ring,
   L² is -C₁₋₃ alkylene-C₂₋₃ alkynediyl or -C₁₋₃ alkylene-O-,
   R^{L1} is H or C₁₋₃ alkyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are the same or different, and are each 1 or 2,
   R^{L4} and R^{L5} are the same or different, and are each H or C₁₋₃ alkyl, or the R^{L4} and R^{L5} together with the carbon atom bonded thereto may form C₃₋₆ cycloalkane or the R^{L4} and R^{L5} together with the carbon atom bonded thereto may form carbonyl.
(8-2-1) A compound represented by formula (I) or a salt thereof, wherein L is a group selected from the group consisting of the following formulas (XXX-L), (XXXI-LX), (XXXII-L), (XXXIII-L), and (XXXIV-L): 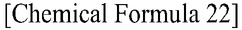 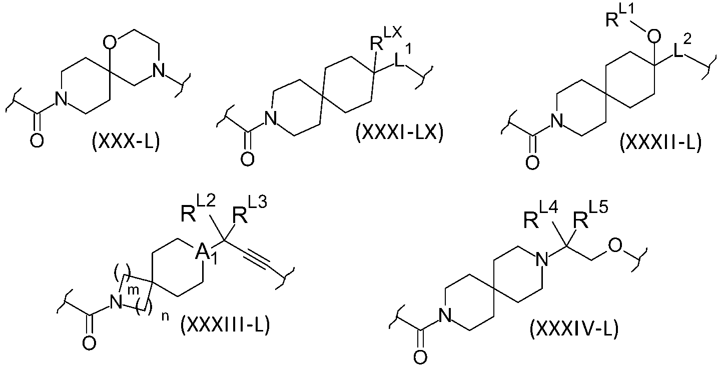
   L¹ is bond, C₁₋₃ alkylene, -C₁₋₃ alkylene-O-, -C₁₋₃ alkylene-C₂₋₃ alkynediyl, -C≡C-, or divalent group of optionally substituted saturated hetero ring,
   R^{LX} is H or OH,
   L² is -C₁₋₃ alkylene-C₂₋₃ alkynediyl or -C₁₋₃ alkylene-O-,
   R^{L1} is H or C₁₋₃ alkyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are the same or different, and are each 1 or 2, and
   R^{L4} and R^{L5} are the same or different, and are each H or C₁₋₃ alkyl, or the R^{L4} and R^{L5} together with the carbon atom bonded thereto may form C₃₋₆ cycloalkane, or the R^{L4} and R^{L5} together with the carbon atom bonded thereto may form carbonyl.
(8-3) A compound represented by formula (I) or a salt thereof, wherein L is a group selected from the group consisting of the following formulas (XXX-L), (XXXI-L), (XXXII-L), (XXXIII-L), and (XXXIV-L): 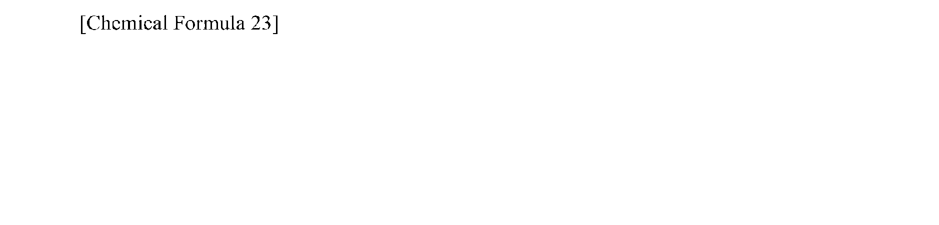 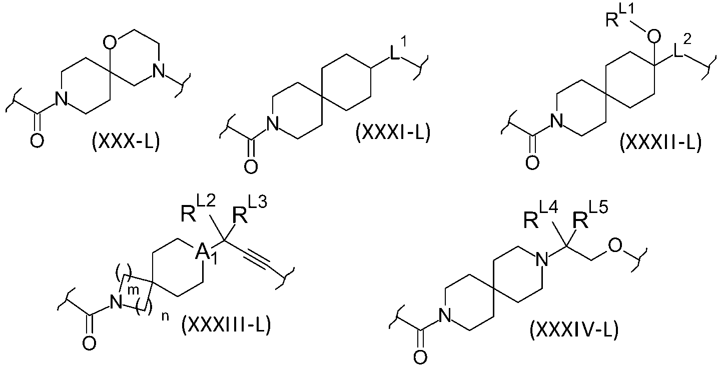
   L¹ is bond, C₁₋₃ alkylene, -CH₂-C≡C-, -C₁₋₃ alkylene-O-, or tetrahydropyridinediyl,
   L² is -CH₂-C≡C- or -CH₂CH₂-O-,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are both 1 or both 2, and
   R^{L4} and R^{L5} are both H, or the R^{L4} and R^{L5} together with the carbon atom bonded thereto may form cyclopropane.
(8-3-1) A compound represented by formula (I) or a salt thereof, wherein L is a group selected from the group consisting of the following formulas (XXX-L), (XXXI-LX), (XXXII-L), (XXXIII-L), and (XXXIV-L): 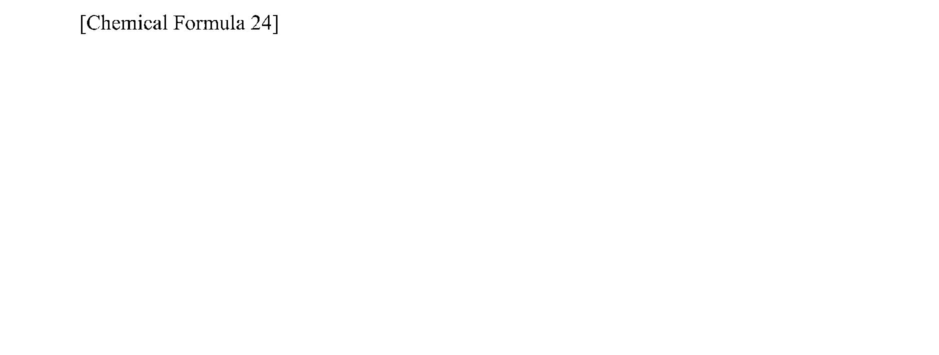 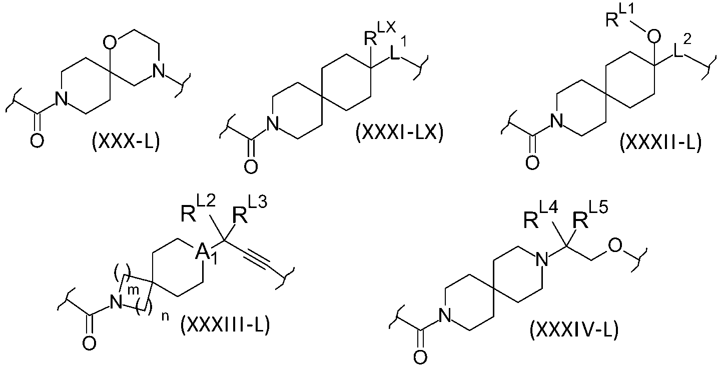
   L¹ is bond, C₁₋₃ alkylene, -CH₂-C≡C-, -C₁₋₃ alkylene-O-, or tetrahydropyridinediyl,
   R^{LX} is H or OH,
   L² is -CH₂-C≡C- or -CH₂CH₂-O-,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are both 1 or both 2, and
   R^{L4} and R^{L5} are both H, or the R^{L4} and R^{L5} together with the carbon atom bonded thereto may form cyclopropane.
(8-4) A compound represented by formula (I) or a salt thereof, wherein L is a group represented by the following formula (XXXI-L): 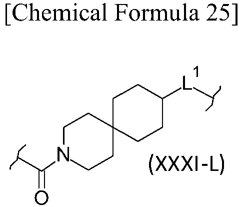 wherein L¹ is bond, C₁₋₃ alkylene, -CH₂-C≡C-, -C₁₋₃ alkylene-O-, or tetrahydropyridinediyl.
(8-4-1) A compound represented by formula (I) or a salt thereof, wherein L is a group represented by the following formula (XXXI-LX): 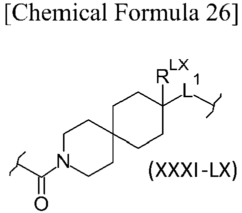
   wherein L¹ is bond, C₁₋₃ alkylene, -CH₂-C≡C-, -C₁₋₃ alkylene-O-, or tetrahydropyridinediyl, and
   R^{LX} is H or OH.
(8-5) A compound represented by formula (I) or a salt thereof, wherein L is a group represented by the following formula (XXXI-L): 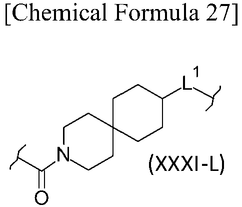 wherein L¹ is -CH₂CH₂-O- or -CH₂-C≡C-.
(8-6) A compound represented by formula (I) or a salt thereof, wherein L is a group represented by the following formula (XXXI-L): 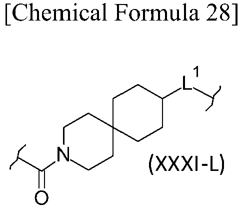 wherein L¹is -CH₂CH₂-O-.
(8-7) A compound represented by formula (I) or a salt thereof, wherein L is a group represented by the following formula (XXXI-L): 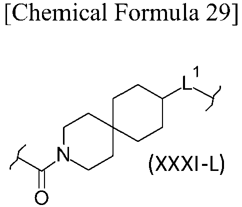 wherein L¹ is -CH₂-C≡C-.
(9-1) A compound represented by formula (I) or a salt thereof, wherein Z is a group selected from the group consisting of the following formulas (XVIII), (XIX), (XX), (XXI), (XXII), (XXIII), (XXIV), (XXV), (XXVI), (XXVII), (XXVIII), and (XXIX): 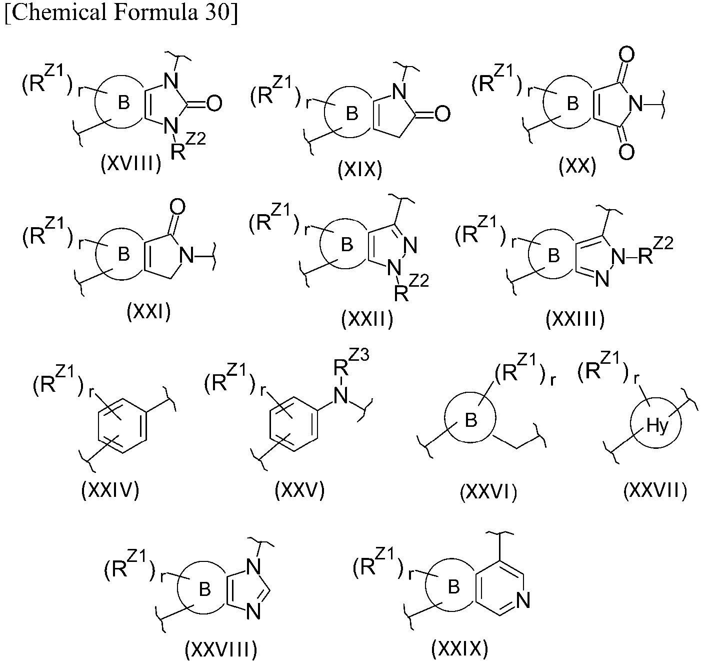
   wherein ring B is benzene ring or 6-membered hetero ring,
   ring Hy is 5- or 6-membered hetero ring,
   R^{Z1} is H, C₁₋₃ alkyl, halogen, -O-(C₁₋₃ alkyl), -NR^{Z4}₂, -CN, -CONR^{Z4}₂, or - NR^{Z4}COR^{Z5},
   R^{Z2} is H or C₁₋₃ alkyl,
   R^{Z3} is H or C₁₋₃ alkyl,
   R^{Z4} is the same or different, and is H or C₁₋₃ alkyl,
   R^{Z5} is C₁₋₃ alkyl,
   L is bonded to ring B in the above formulas (XVIII), (XIX), (XX), (XXI), (XXII), (XXIII), (XXVI), (XXVIII), and (XXIX), to the benzene ring in formulas (XXIV) and (XXV), and to ring Hy in formula (XXVII), and
   r is 1 or 2.
(9-2) A compound represented by formula (I) or a salt thereof, wherein Z is a group selected from the group consisting of formulas (XXII), (XXIV), (XXVI), and (XXVII): 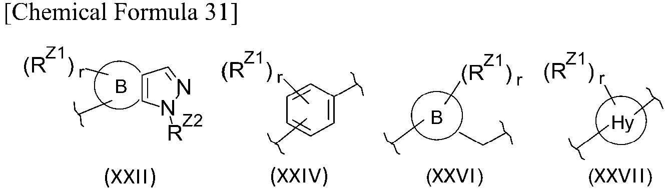
   ring B is benzene ring or a 6-membered hetero ring,
   ring Hy is 5- or 6-membered hetero ring,
   R^{Z1} is H, C₁₋₃ alkyl, halogen, -O-(C₁₋₃ alkyl), -NR^{Z4}₂, -CN, -CONR^{Z4}₂, or - NR^{Z4}COR^{Z5},
   R^{Z2} is H or C₁₋₃ alkyl,
   R^{Z4} is the same or different, and is H or C₁₋₃ alkyl,
   R^{Z5} is C₁₋₃ alkyl,
   r is 1 or 2, and
   L is bonded to ring B in the above formulas (XXII) and (XXVI), to the benzene ring in formula (XXIV), and to ring Hy in formula (XXVII).
(9-3) A compound represented by formula (I) or a salt thereof, wherein Z is a group selected from the group consisting of formulas (XXX-Z), (XXXI-Z), and (XXXII-Z): 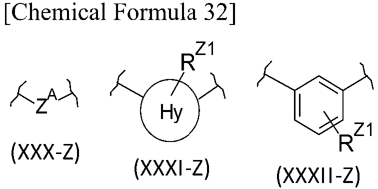
   Z^{A} is a group represented by the following formula (XXII-1) or (XXVI-1): 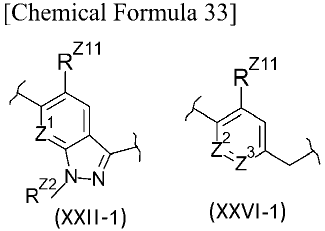
   R^{Z11} is H or halogen,
   Z¹, Z², and Z³ are the same or different, and are each CH or N,
   R^{Z2} is C₁₋₃ alkyl,
   ring Hy is 5- or 6-membered hetero ring, and
   R^{Z1} is H, halogen, C₁₋₃ alkyl, -O-(C₁₋₃ alkyl), or -CN.
(9-4) A compound represented by formula (I) or a salt thereof, wherein Z is a group selected from the group consisting of formulas (XXX-Z), (XXXI-Z), and (XXXII-Z): 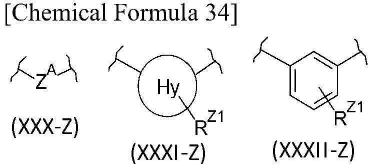
   Z^{A} is a group selected from the group consisting of the following formula (XXII-1) or (XXVI-2): 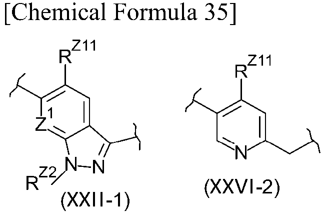
   R^{Z11} is halogen,
   Z¹ is CH or N,
   R^{Z2} is C₁₋₃ alkyl,
   ring Hy is 5- or 6-membered hetero ring, and
   R^{Z1} is H, C₁₋₃ alkyl, or halogen.
(9-5) A compound represented by formula (I) or a salt thereof, wherein Z is a group selected from the group consisting of formulas (XXX-Z), (XXXI-Z1), (XXXII-Z2), and (XXXIV-Z): 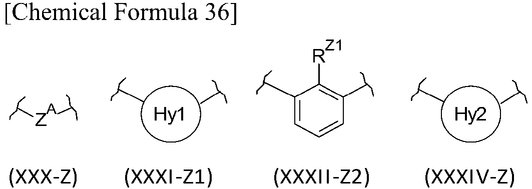
   Z^{A} is a group represented by formula (XXII-1) or (XXVI-2): 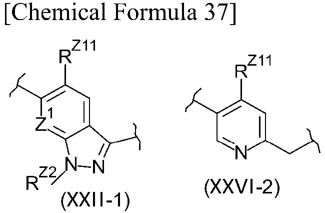
   R^{Z11} is halogen,
   Z¹ is CH or N,
   R^{Z2} is C₁₋₃ alkyl,
   ring Hy1 is a ring selected from the group consisting of the following formulas (XXXV), (XXXVI), (XXXVII), (XXXVIII), and (XXXIX): 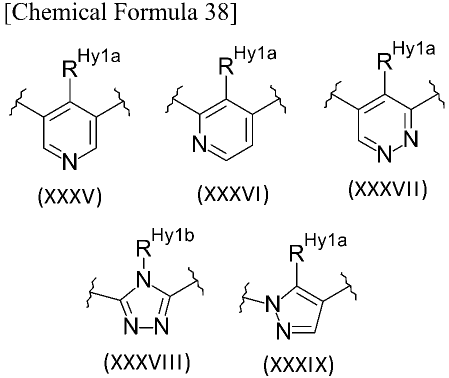
   ring Hy2 is of the following formula (XLIII): 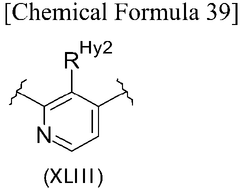
   R^{Z1} is H, C₁₋₃ alkyl, or halogen,
   R^{Hy1a} is H, C₁₋₃ alkyl, or halogen,
   R^{Hy1b} is H or C₁₋₃ alkyl, and
   R^{Hy2} is H, C₁₋₃ alkyl, or halogen.
(9-5-1) A compound represented by formula (I) or a salt thereof, wherein Z is a group selected from the group consisting of formulas (XXX-Z), (XXXI-Z1), (XXXII-Z2), and (XXXIV-Z): 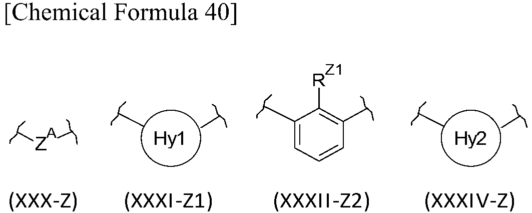
   Z^{A} is a group represented by the following formula (XXII-1) or (XXVI-2): 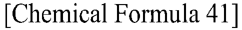 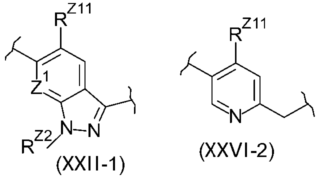
   R^{Z11} is halogen,
   Z¹ is CH or N,
   R^{Z2} is C₁₋₃ alkyl,
   ring Hy1 is a ring selected from the group consisting of the following formulas (XXXV), (XXXVI), (XXXVII), (XXXVII-2), (XXXVIII), and (XXXIX): 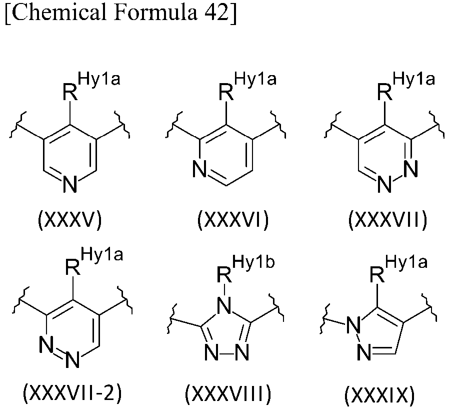
   ring Hy2 is of the following formula (XLIII): 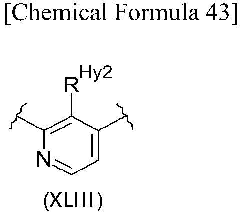
   R^{Z1} is H, C₁₋₃ alkyl, or halogen,
   R^{Hy1a} is H, C₁₋₃ alkyl, or halogen,
   R^{Hy1b} is H or C₁₋₃ alkyl, and
   R^{Hy2} is H, C₁₋₃ alkyl, or halogen.
(9-6) A compound represented by formula (I) or a salt thereof, wherein Z is a group selected from the group consisting of formulas (XXX-Z), (XXXI-Z1), (XXXII-Z2), and (XXXIV-Z): 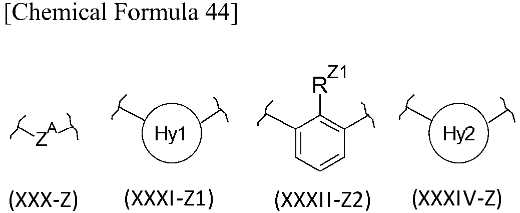
   Z^{A} is a group represented by the following formula (XXII-1) or (XXVI-2): 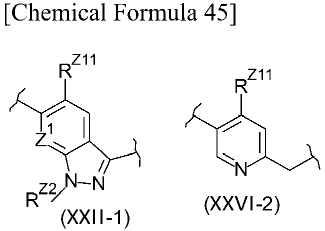
   R^{Z11} is halogen,
   Z¹ is CH or N,
   R^{Z2} is C₁₋₃ alkyl,
   ring Hy1 is a ring selected from the group consisting of the following formulas (XXXV), (XXXVI), (XXXVII), (XXXVIII), and (XXXIX): 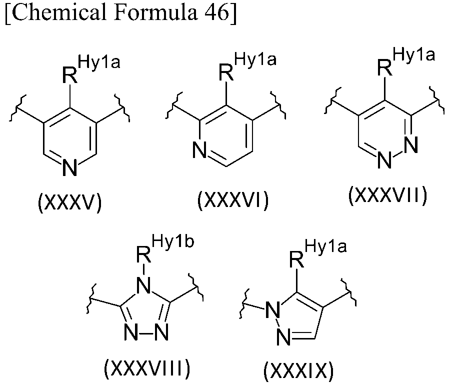
   ring Hy2 is of the following formula (XLIII): 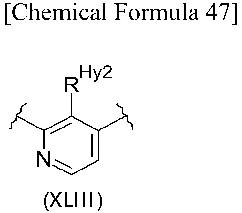
   R^{Z1} is H or halogen,
   R^{Hy1a} is H, C₁₋₃ alkyl, or halogen,
   R^{Hy1b} is H or C₁₋₃ alkyl, and
   R^{Hy2} is H or C₁₋₃ alkyl.
(9-6-1) A compound represented by formula (I) or a salt thereof, wherein Z is a group selected from the group consisting of formulas (XXX-Z), (XXXI-Z1), (XXXII-Z2), and (XXXIV-Z): 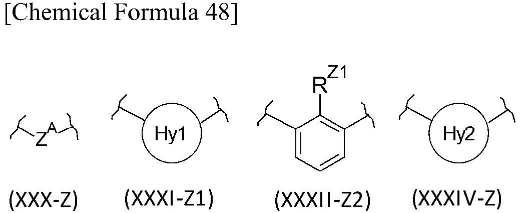
   Z^{A} is a group represented by the following formula (XXII-1) or (XXVI-2): 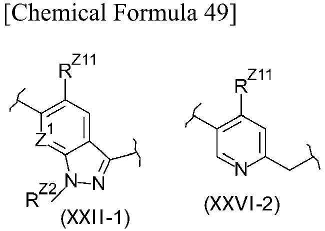
   R^{Z11} is halogen,
   Z¹ is CH or N,
   R^{Z2} is C₁₋₃ alkyl,
   ring Hy1 is a ring selected from the group consisting of the following formulas (XXXV), (XXXVI), (XXXVII), (XXXVII-2), (XXXVIII), and (XXXIX): 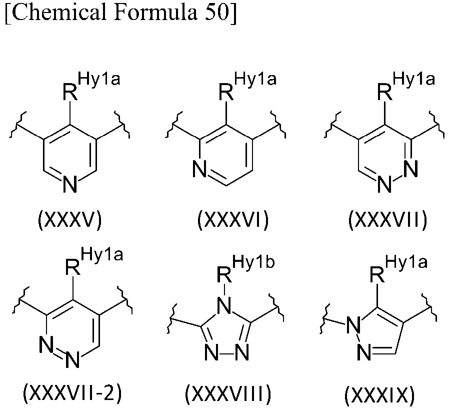
   ring Hy2 is of the following formula (XLIII): 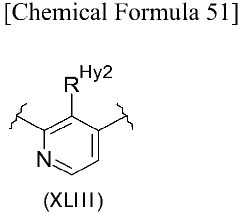
   R^{Z1} is H or halogen,
   R^{Hy1a} is H, C₁₋₃ alkyl, or halogen,
   R^{Hy1b} is H or C₁₋₃ alkyl, and
   R^{Hy2} is H or C₁₋₃ alkyl.
(9-7) A compound represented by formula (I) or a salt thereof, wherein Z is formula (XXXI-Z1): 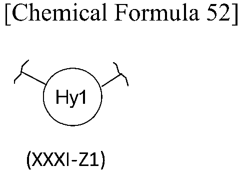
   ring Hy1 is a ring selected from the group consisting of the following formulas (XXXV) and (XXXVI): 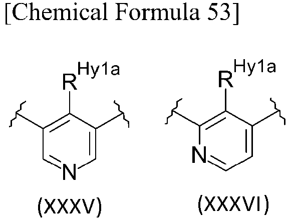
   R^{Hy1a} is H, C₁₋₃ alkyl, or halogen.
(9-8) A compound represented by formula (I) or a salt thereof, wherein Z is formula (XXXI-Z1): 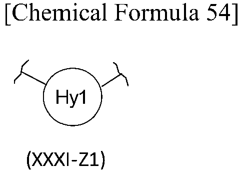
   ring Hy1 is a ring selected from the group consisting of the following formulas (XXXV) and (XXXVI): 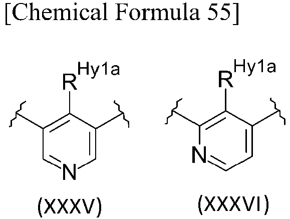
   R^{Hy1a} is H.
(10-1) A compound represented by formula (I) or a salt thereof, wherein L together with Z forms a group selected from the group consisting of the following formulas (XXX), (XXXI), (XXXII), (XXXIII-A), (XXXIII-B), (XXXIV), and (XXXI-A): 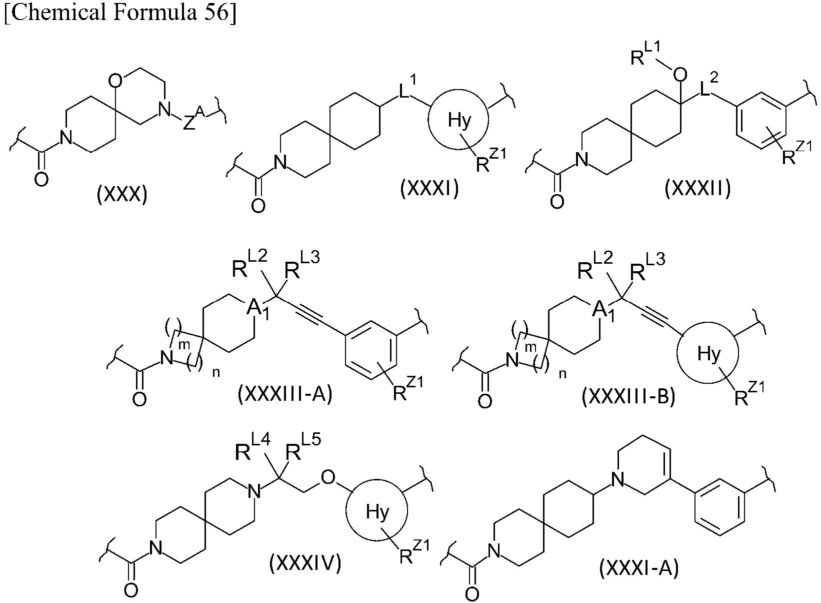
   Z^{A} is a group represented by the following formula (XXII-1) or (XXVI-1): 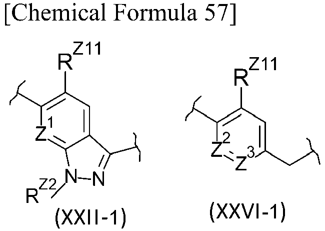
   R^{Z11} is H or halogen,
   Z¹, Z², and Z³ are the same or different, and are each CH or N,
   R^{Z2} is C₁₋₃ alkyl,
   L¹ is bond, C₁₋₃ alkylene, -C₁₋₃ alkylene-O-, -C₁₋₃ alkylene-C₂₋₃ alkynediyl, or -C=C-,
   ring Hy is 5- or 6-membered hetero ring,
   L² is -C₁₋₃ alkylene-C₂₋₃ alkynediyl or -C₁₋₃ alkylene-O-,
   R^{Z1} is H, halogen, C₁₋₃ alkyl, -O-(C₁₋₃ alkyl), or -CN,
   R^{L1} is H or C₁₋₃ alkyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are the same or different, and are each 1 or 2, and
   R^{L4} and R^{L5} are the same or different, and are each H or C₁₋₃ alkyl, and the R^{L4} and R^{L5} together with a carbon atom bonded thereto may form C₃₋₆ cycloalkane, or the R^{L4} and R^{L5} together with a carbon atom bonded thereto may form carbonyl.
(10-1-1) A compound represented by formula (I) or a salt thereof, wherein L together with Z forms a group selected from the group consisting of the following formulas (XXX), (XXXI-X), (XXXII), (XXXIII-A), (XXXIII-B), (XXXIV), and (XXXI-A): 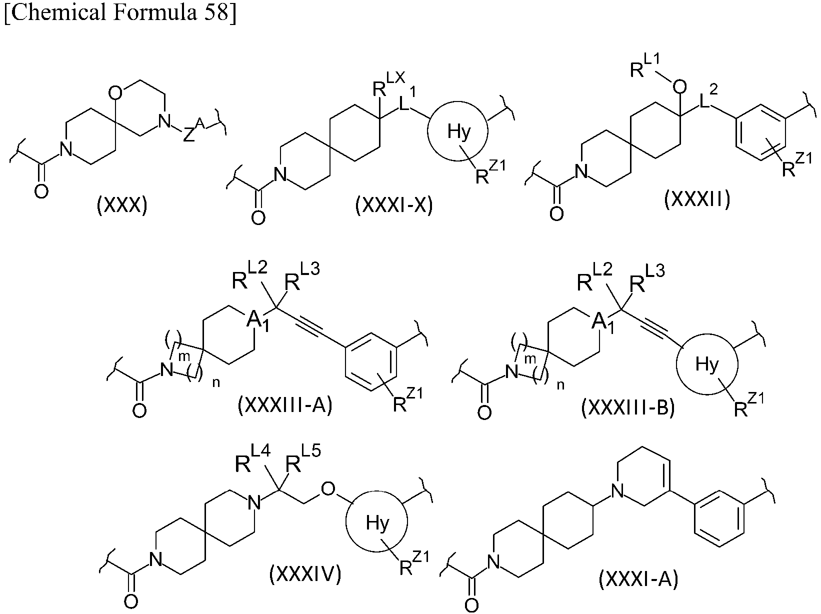
   Z^{A} is a group represented by the following formula (XXII-1) or (XXVI-1): 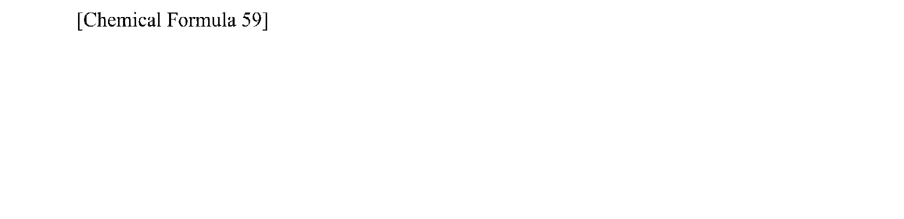 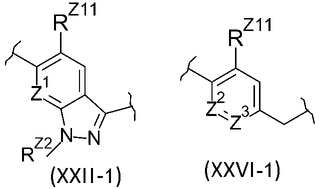
   R^{Z11} is H or halogen,
   Z¹, Z², and Z³ are the same or different, and are each CH or N,
   R^{Z2} is C₁₋₃ alkyl,
   L¹ is bond, C₁₋₃ alkylene, -C₁₋₃ alkylene-O-, -C₁₋₃ alkylene-C₂₋₃ alkynediyl, or -C≡C-,
   R^{LX} is H or OH,
   ring Hy is 5- or 6-membered hetero ring,
   L² is -C₁₋₃ alkylene-C₂₋₃ alkynediyl or -C₁₋₃ alkylene-O-,
   R^{Z1} is H, halogen, C₁₋₃ alkyl, -O-(C₁₋₃ alkyl), or -CN,
   R^{L1} is H or C₁₋₃ alkyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are the same or different, and are each 1 or 2, and
   R^{L4} and R^{L5} are the same or different, and are each H or C₁₋₃ alkyl, or the R^{L4} and R^{L5} together with the carbon atom bonded thereto may form C₃₋₆ cycloalkane or the R^{L4} and R^{L5} together with the carbon atom bonded thereto may form carbonyl.
(10-2) A compound represented by formula (I) or a salt thereof, wherein L and Z together are a group selected from the group consisting of the following formulas (XXX), (XXXI), (XXXII), (XXXIII-A), (XXXIII-B), (XXXIV), and (XXXI-A): 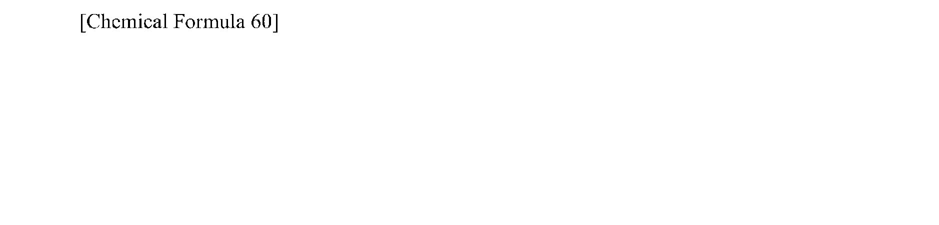 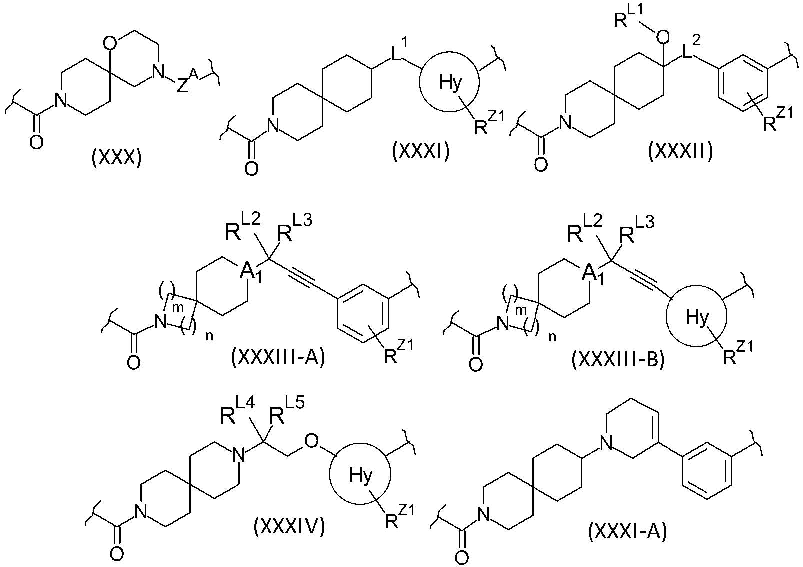
   Z^{A} is a group represented by the following formula (XXII-1) or (XXVI-2): 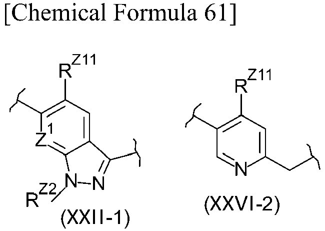
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C≡C-,
   ring Hy is 5- or 6-membered hetero ring,
   L² is -CH₂-C≡C- or -CH₂CH₂-O-,
   R^{Z1} is H, C₁₋₃ alkyl, or halogen,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are both 1 or both 2, and
   R^{L4} and R^{L5} are both H, or the R^{L4} and R^{L5} together with the carbon atom bonded thereto may form cyclopropane.
(10-2-1) A compound represented by formula (I) or a salt thereof, wherein L and Z together are a group selected from the group consisting of the following formulas (XXX), (XXXI-X), (XXXII), (XXXIII-A), (XXXIII-B), (XXXIV), and (XXXI-A): 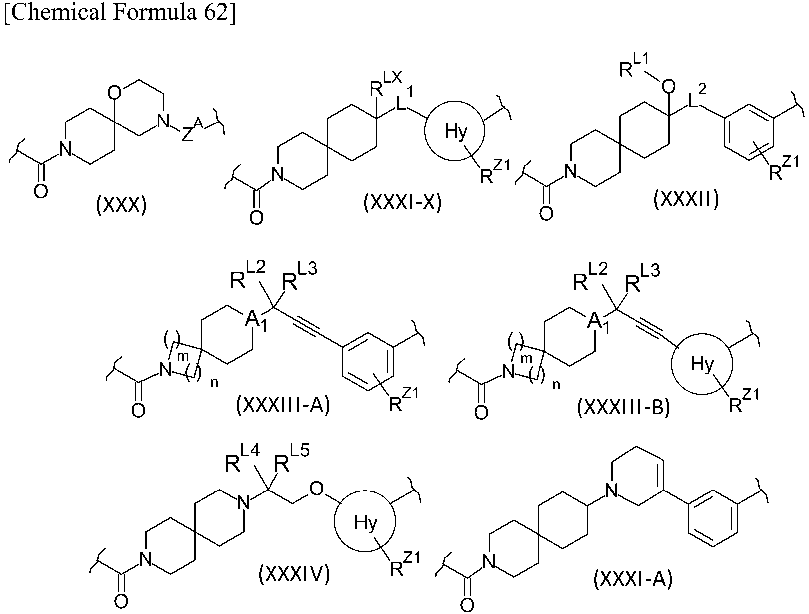
   Z^{A} is a group represented by the following formula (XXII-1) or (XXVI-2): 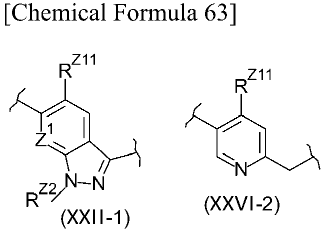
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C≡C-,
   R^{LX} is H or OH,
   ring Hy is 5- or 6-membered hetero ring,
   L² is -CH₂-C≡C- or -CH₂CH₂-O-,
   R^{Z1} is H, C₁₋₃ alkyl, or halogen,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are both 1 or both 2, and
   R^{L4} and R^{L5} are both H, or the R^{L4} and R^{L5} together with the carbon atom bonded thereto may form cyclopropane.
(10-3) A compound represented by formula (I) or a salt thereof, wherein L and Z together are a group selected from the group consisting of the following formulas (XXX), (XXXI-1), (XXXII-1), (XXXIII-A1), (XXXIII-B1), (XXXIV-1), and (XXXI-A): 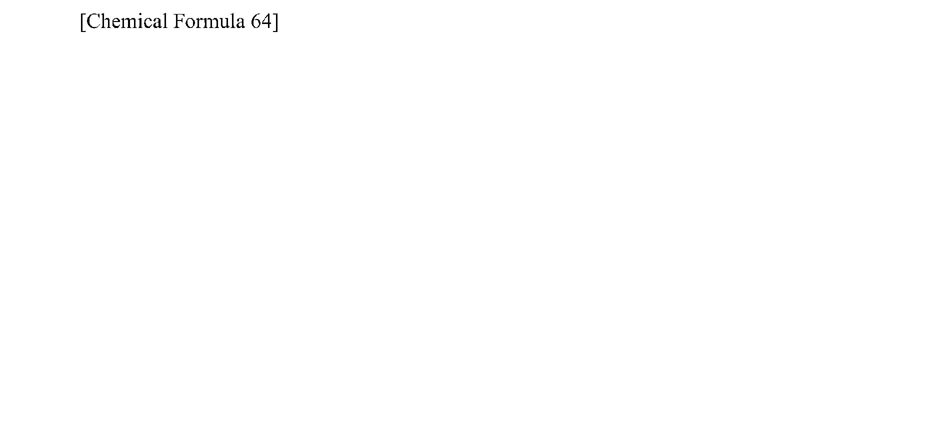 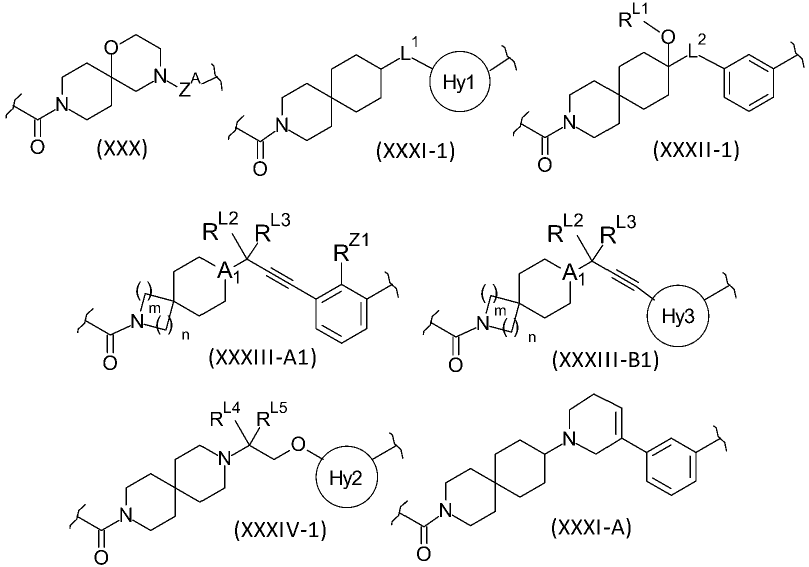
   Z^{A} is a group represented by the following formula (XXII-1) or (XXVI-2): 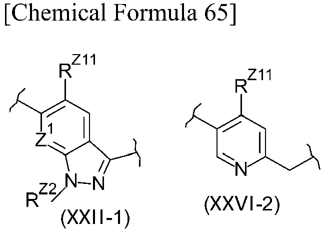
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C≡C-,
   ring Hy1 is a ring selected from the group consisting of the following formulas (XXXV), (XXXVI), (XXXVII), (XXXVIII), and (XXXIX): 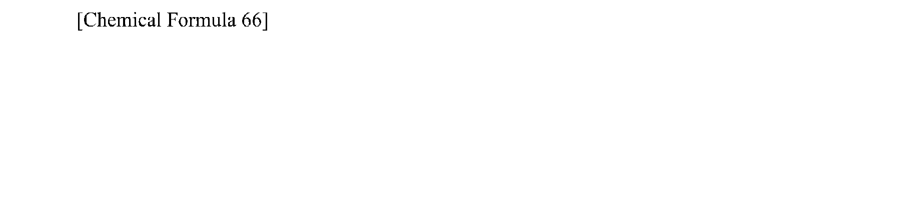 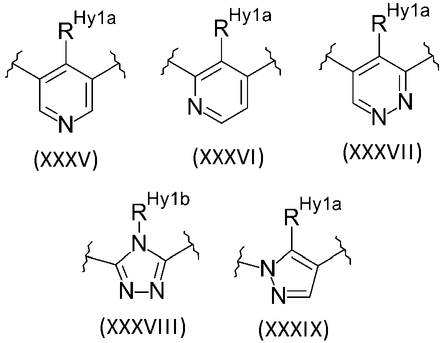
   R^{Hy1a} is H, C₁₋₃ alkyl, or halogen,
   R^{Hy1b} is H or C₁₋₃ alkyl,
   L² is -CH₂-C≡C- or -CH₂CH₂-O-,
   R^{Z1} is H, C₁₋₃ alkyl, or halogen,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 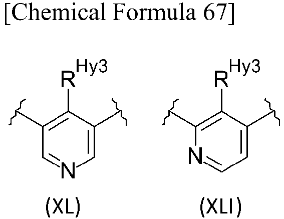
   R^{Hy3} is H, C₁₋₃ alkyl, or halogen,
   m and n are both 1 or both 2,
   ring Hy2 is of the following formula (XLIII): 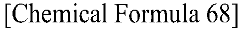 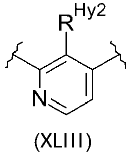
   R^{Hy2} is H, C₁₋₃ alkyl, or halogen, and
   R^{L4} and R^{L5} are both H, or the R^{L4} and R^{L5} together with the carbon atom bonded thereto may form cyclopropane.
(10-3-1) A compound represented by formula (I) or a salt thereof, wherein L and Z together are a group selected from the group consisting of the following formulas (XXX), (XXXI-1X), (XXXII-1), (XXXIII-A1), (XXXIII-B1), (XXXIV-1), and (XXXI-A): 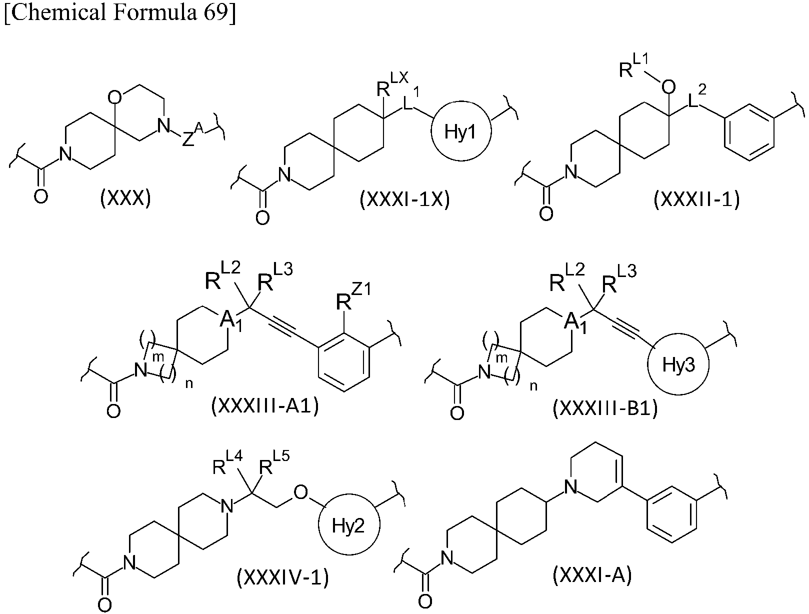
   Z^{A} is a group represented by the following formula (XXII-1) or (XXVI-2): 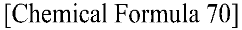 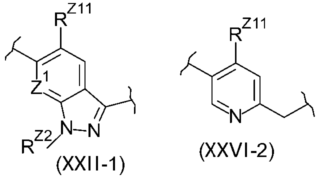
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C≡C-,
   R^{LX} is H or OH,
   ring Hy1 is a ring selected from the group consisting of the following formulas (XXXV), (XXXVI), (XXXVII), (XXXVII-2), (XXXVIII), and (XXXIX): 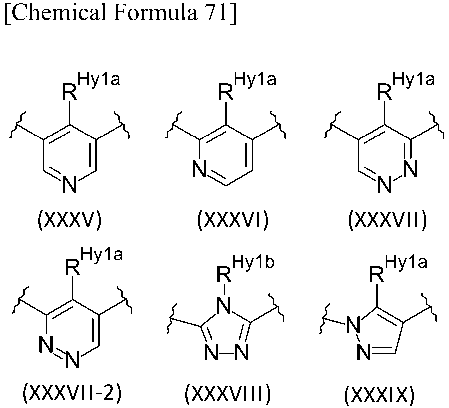
   R^{Hy1a} is H, C₁₋₃ alkyl, or halogen,
   R^{Hy1b} is H or C₁₋₃ alkyl,
   L² is -CH₂-C≡C- or -CH₂CH₂-O-,
   R^{Z1} is H, C₁₋₃ alkyl, or halogen,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 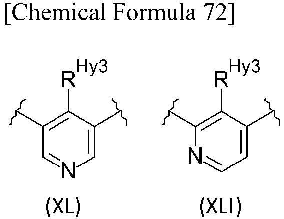
   R^{Hy3} is H, C₁₋₃ alkyl, or halogen,
   m and n are both 1 or both 2,
   ring Hy2 is of the following formula (XLIII): 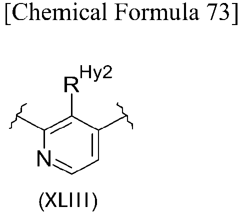
   R^{Hy2} is H, C₁₋₃ alkyl, or halogen, and
   R^{L4} and R^{L5} are both H, or the R^{L4} and R^{L5} together with the carbon atom bonded thereto may form cyclopropane.
(10-4) A compound represented by formula (I) or a salt thereof, wherein L and Z together are a group selected from the group consisting of the following formulas (XXX), (XXXI-1), (XXXII-1), (XXXIII-A1), (XXXIII-B1), (XXXIV-1), and (XXXI-A): 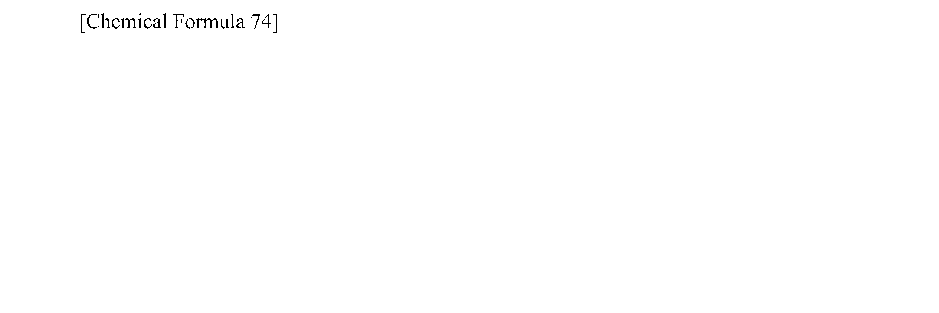 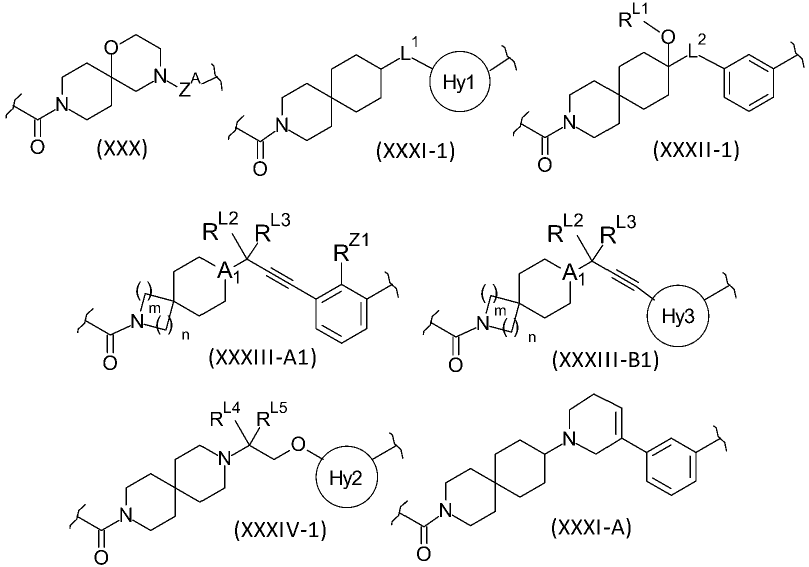
   Z^{A} is a group represented by the following formula (XXII-1) or (XXVI-2): 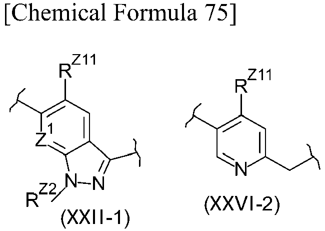
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C≡C-,
   ring Hy1 is a ring selected from the group consisting of the following formulas (XXXV), (XXXVI), (XXXVII), (XXXVIII), and (XXXIX): 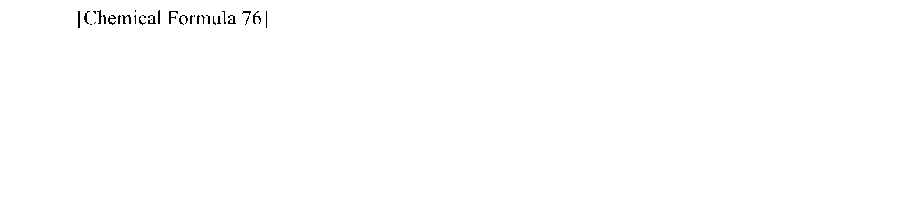 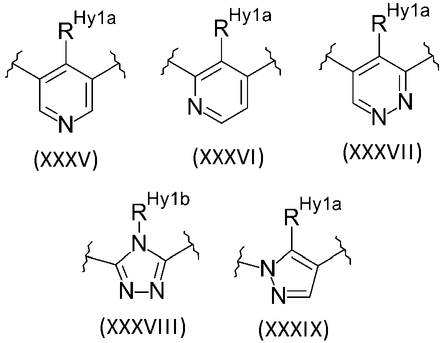
   R^{Hy1a} is H, C₁₋₃ alkyl, or halogen,
   R^{Hy1b} is H or C₁₋₃ alkyl,
   L² is -CH₂-C≡C- or -CH₂CH₂-O-,
   R^{Z1} is H or halogen,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 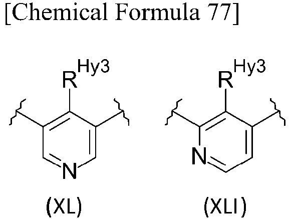
   R^{Hy3} is H or halogen,
   m and n are both 1 or both 2,
   ring Hy2 is of the following formula (XLIII): 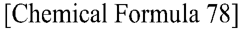 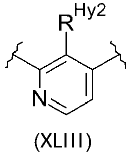
   R^{Hy2} is H or C₁₋₃ alkyl, and
   R^{L4} and R^{L5} are both H, or the R^{L4} and R^{L5} together with the carbon atom bonded thereto may form cyclopropane.
(10-4-1) A compound represented by formula (I) or a salt thereof, wherein L and Z together are a group selected from the group consisting of the following formulas (XXX), (XXXI-1X), (XXXII-1), (XXXIII-A1), (XXXIII-B1), (XXXIV-1), and (XXXI-A): 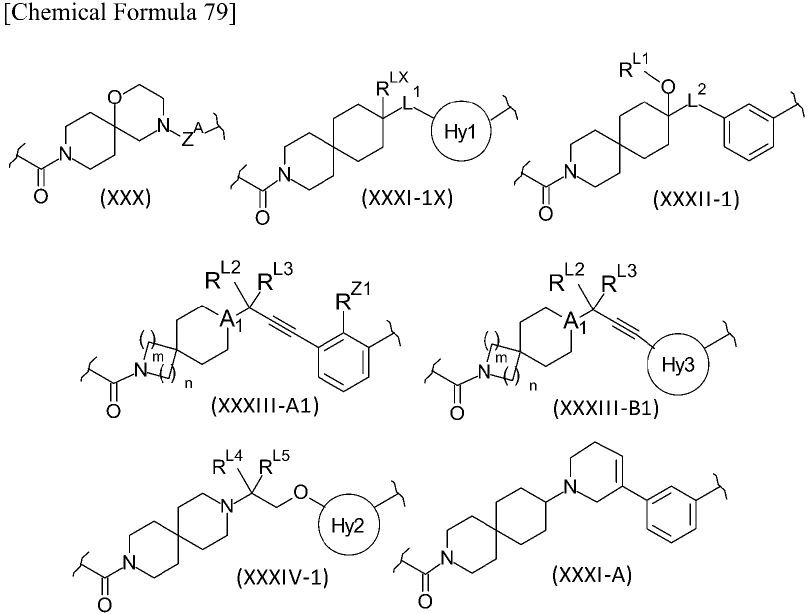
   Z^{A} is a group represented by the following formula (XXII-1) or (XXVI-2): 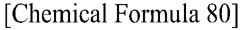 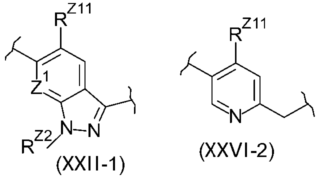
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C≡C-,
   R^{LX} is H or OH,
   ring Hy1 is a ring selected from the group consisting of the following formulas (XXXV), (XXXVI), (XXXVII), (XXXVIII), and (XXXIX): 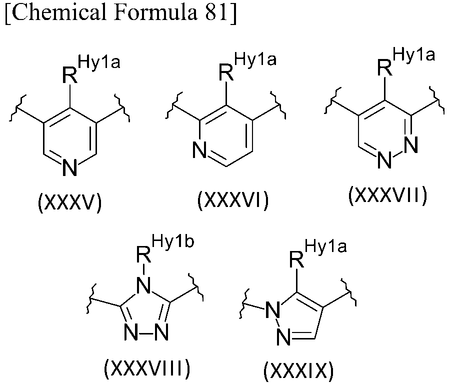
   R^{Hy1a} is H, C₁₋₃ alkyl, or halogen,
   R^{Hy1b} is H or C₁₋₃ alkyl,
   L² is -CH₂-C≡C- or -CH₂CH₂-O-,
   R^{Z1} is H or halogen,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 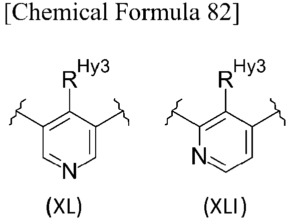
   R^{Hy3} is H or halogen,
   m and n are both 1 or both 2,
   ring Hy2 is of the following formula (XLIII): 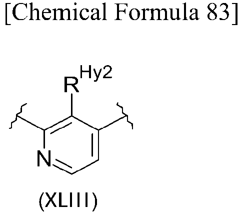
   R^{Hy2} is H or C₁₋₃ alkyl, and
   R^{L4} and R^{L5} are both H, or the R^{L4} and R^{L5} together with the carbon atom bonded thereto may form cyclopropane.
(10-5) A compound represented by formula (I) or a salt thereof, wherein L together with Z forms a group selected from the group consisting of the following formulas (XXX), (XXXI), (XXXIII-A), (XXXIII-B), (XXXIV), and (XXXI-A): 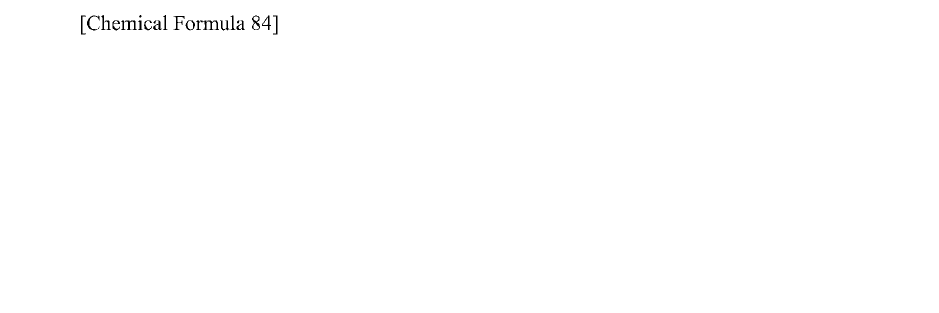 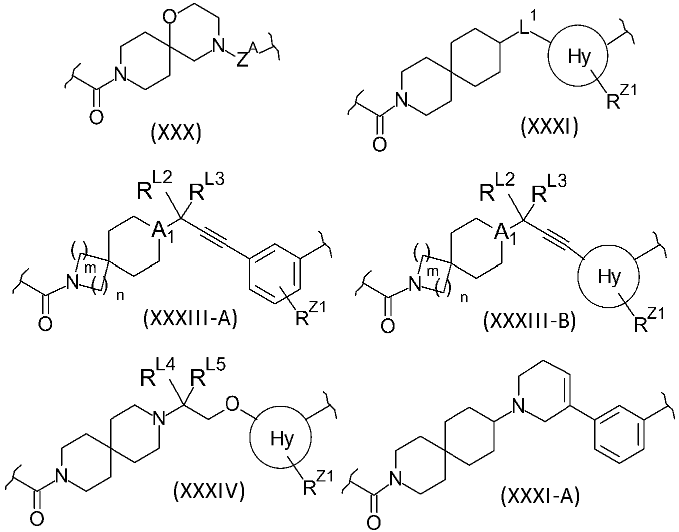
   Z^{A} is a group represented by the following formula (XXII-1) or (XXVI-1): 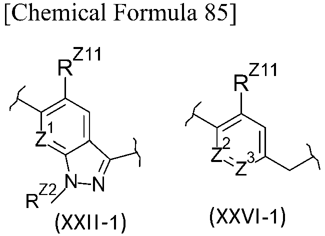
   R^{Z11} is H or halogen,
   Z¹, Z², and Z³ are the same or different, and are each CH or N,
   R^{Z2} is C₁₋₃ alkyl,
   L¹ is bond, C₁₋₃ alkylene, -C₁₋₃ alkylene-O-, -C₁₋₃ alkylene-C₂₋₃ alkynediyl, or -C=C-,
   ring Hy is 5- or 6-membered hetero ring,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   R^{Z1} is H, C₁₋₃ alkyl, or halogen,
   m and n are the same or different, and are each 1 or 2, and
   R^{L4} and R^{L5} are the same or different, and are each H or C₁₋₃ alkyl, or the R^{L4} and R^{L5} together with the carbon atom bonded thereto may form C₃₋₆ cycloalkane or the R^{L4} and R^{L5} together with the carbon atom bonded thereto may form carbonyl.
(10-5-1) A compound represented by formula (I) or a salt thereof, wherein L together with Z forms a group selected from the group consisting of the following formulas (XXX), (XXXI-X), (XXXIII-A), (XXXIII-B), (XXXIV), and (XXXI-A): 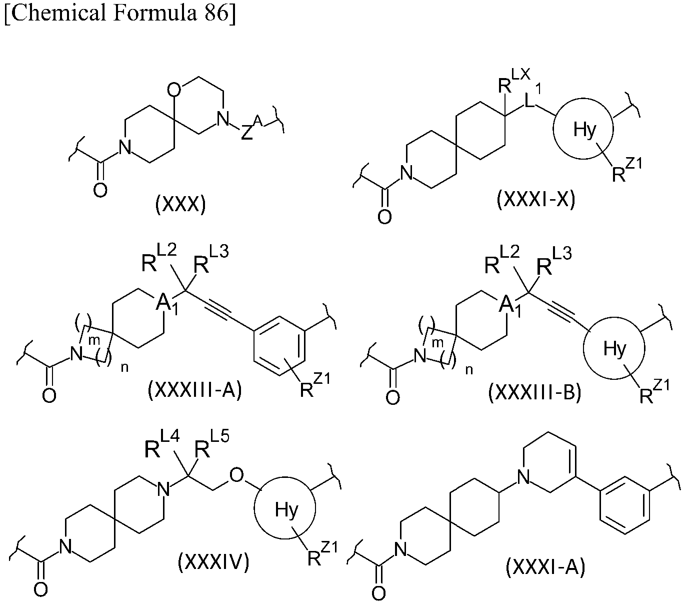
   Z^{A} is a group represented by the following formula (XXII-1) or (XXVI-1): 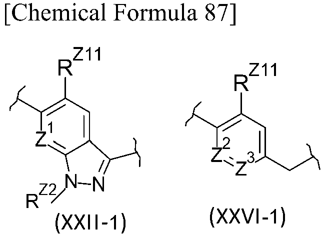
   R^{Z11} is H or halogen,
   Z¹, Z², and Z³ are the same or different, and are each CH or N,
   R^{Z2} is C₁₋₃ alkyl,
   L¹ is bond, C₁₋₃ alkylene, -C₁₋₃ alkylene-O-, -C₁₋₃ alkylene-C₂₋₃ alkynediyl, or -C≡C-,
   R^{LX} is H or OH,
   ring Hy is 5- or 6-membered hetero ring,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   R^{Z1} is H, C₁₋₃ alkyl, or halogen,
   m and n are the same or different, and are each 1 or 2, and
   R^{L4} and R^{L5} are the same or different, and are each H or C₁₋₃ alkyl, or the R^{L4} and R^{L5} together with the carbon atom bonded thereto may form C₃₋₆ cycloalkane or the R^{L4} and R^{L5} together with the carbon atom bonded thereto may form carbonyl.
(10-6) A compound represented by formula (I) or a salt thereof, wherein L and Z together are a group selected from the group consisting of the following formulas (XXX), (XXXI), (XXXIII-A), (XXXIII-B), (XXXIV), and (XXXI-A): 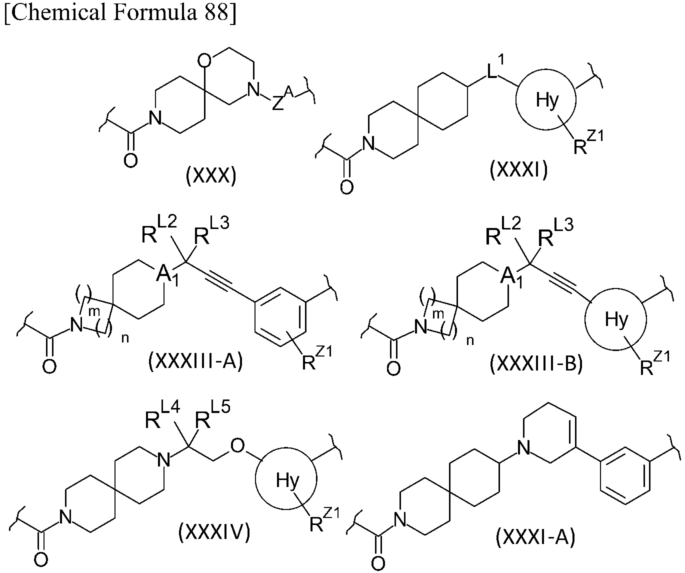
   Z^{A} is a group represented by the following formula (XXII-1) or (XXVI-2): 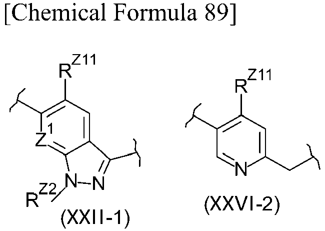
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C≡C-,
   ring Hy is 5- or 6-membered hetero ring,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   R^{Z1} is H, C₁₋₃ alkyl, or halogen,
   m and n are both 1 or both 2, and
   R^{L4} and R^{L5} are both H, or the R^{L4} and R^{L5} together with the carbon atom bonded thereto may form cyclopropane.
(10-6-1) A compound represented by formula (I) or a salt thereof, wherein L and Z together are a group selected from the group consisting of the following formulas (XXX), (XXXI-X), (XXXIII-A), (XXXIII-B), (XXXIV), and (XXXI-A): 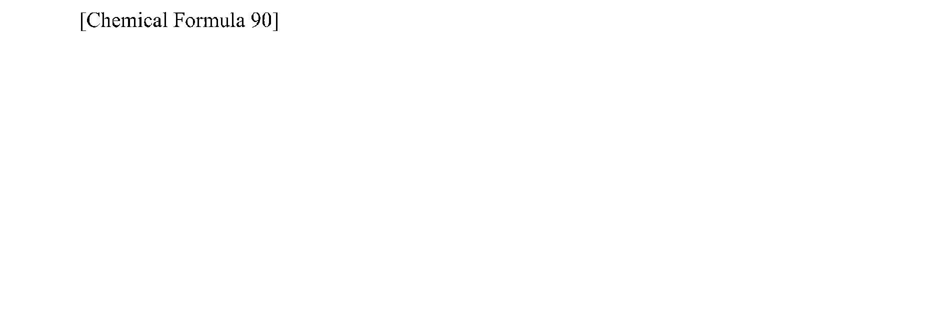 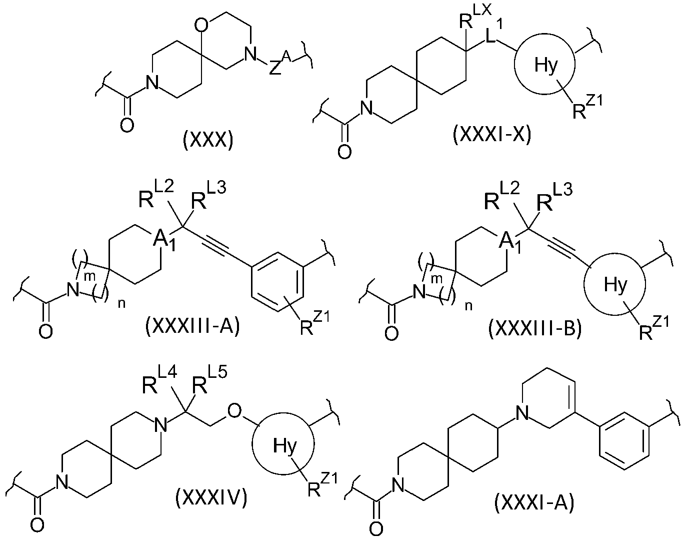
   Z^{A} is a group represented by the following formula (XXII-1) or (XXVI-2): 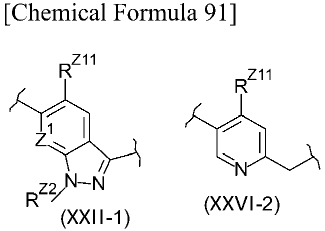
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C≡C-,
   R^{LX} is H or OH,
   ring Hy is 5- or 6-membered hetero ring,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   R^{Z1} is H, C₁₋₃ alkyl, or halogen,
   m and n are both 1 or both 2, and
   R^{L4} and R^{L5} are both H, or the R^{L4} and R^{L5} together with the carbon atom bonded thereto may form cyclopropane.
(10-7) A compound represented by formula (I) or a salt thereof, wherein L and Z together are a group selected from the group consisting of the following formulas (XXX), (XXXI-1), (XXXIII-A1), (XXXIII-B1), (XXXIV-1), and (XXXI-A): 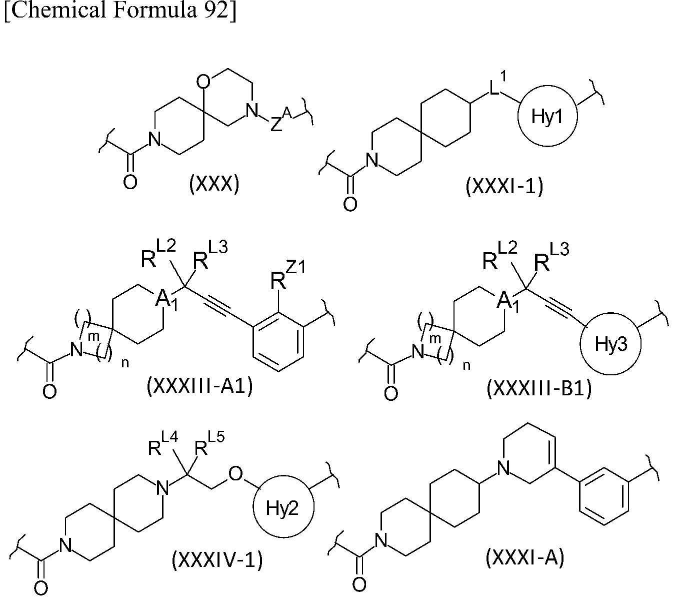
   Z^{A} is a group represented by the following formula (XXII-1) or (XXVI-2): 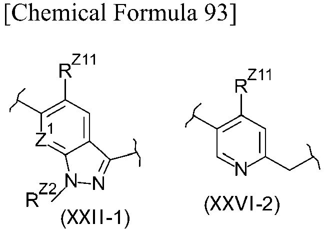
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C≡C-,
   ring Hy1 is a ring selected from the group consisting of the following formulas (XXXV), (XXXVI), (XXXVII), (XXXVIII), and (XXXIX): 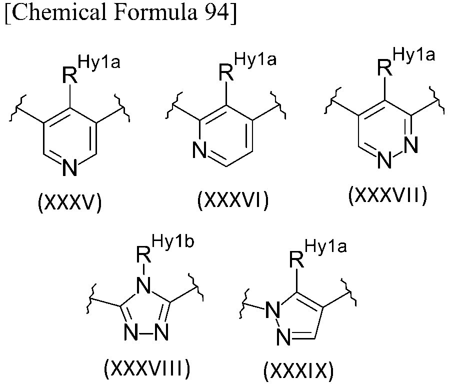
   R^{Hy1a} is H, C₁₋₃ alkyl, or halogen,
   R^{Hy1b} is H or C₁₋₃ alkyl,
   R^{Z1} is H, C₁₋₃ alkyl, or halogen,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 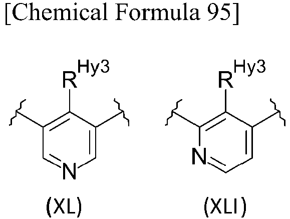
   R^{Hy3} is H, C₁₋₃ alkyl, or halogen,
   m and n are both 1 or both 2,
   ring Hy2 is of the following formula (XLIII): 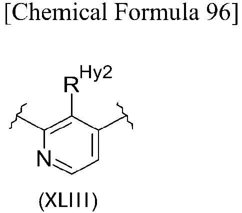
   R^{Hy2} is H, C₁₋₃ alkyl, or halogen, and
   R^{L4} and R^{L5} are both H, or the R^{L4} and R^{L5} together with the carbon atom bonded thereto may form cyclopropane.
(10-7-1) A compound represented by formula (I) or a salt thereof, wherein L and Z together are a group selected from the group consisting of the following formulas (XXX), (XXXI-1X), (XXXIII-A1), (XXXIII-B1), (XXXIV-1), and (XXXI-A): 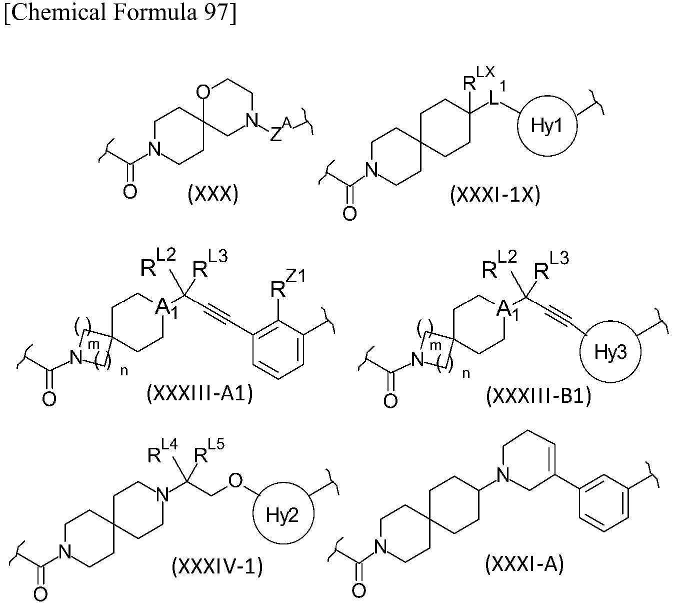
   Z^{A} is a group represented by the following formula (XXII-1) or (XXVI-2): 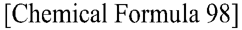 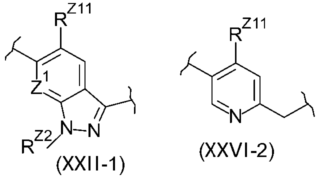
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C≡C-,
   R^{LX} is H or OH,
   ring Hy1 is a ring selected from the group consisting of the following formulas (XXXV), (XXXVI), (XXXVII), (XXXVIII), and (XXXIX): 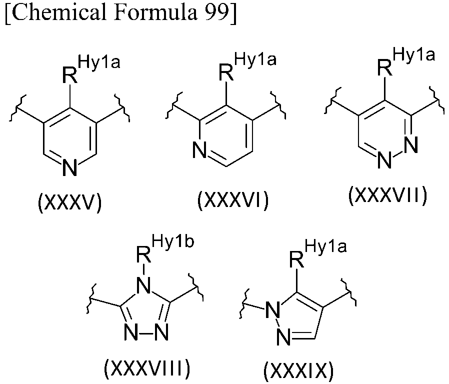
   R^{Hy1a} is H, C₁₋₃ alkyl, or halogen,
   R^{Hy1b} is H or C₁₋₃ alkyl,
   R^{Z1} is H, C₁₋₃ alkyl, or halogen,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 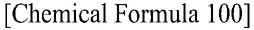 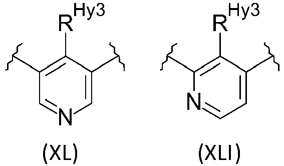
   R^{Hy3} is H, C₁₋₃ alkyl, or halogen,
   m and n are both 1 or both 2,
   ring Hy2 is of the following formula (XLIII): 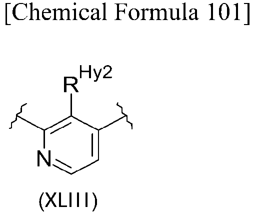
   R^{Hy2} is H, C₁₋₃ alkyl, or halogen, and
   R^{L4} and R^{L5} are both H, or the R^{L4} and R^{L5} together with the carbon atom bonded thereto may form cyclopropane.
(10-8) A compound represented by formula (I) or a salt thereof, wherein L and Z together are a group selected from the group consisting of the following formulas (XXX), (XXXI-1), (XXXIII-A1), (XXXIII-B1), (XXXIV-1), and (XXXI-A): 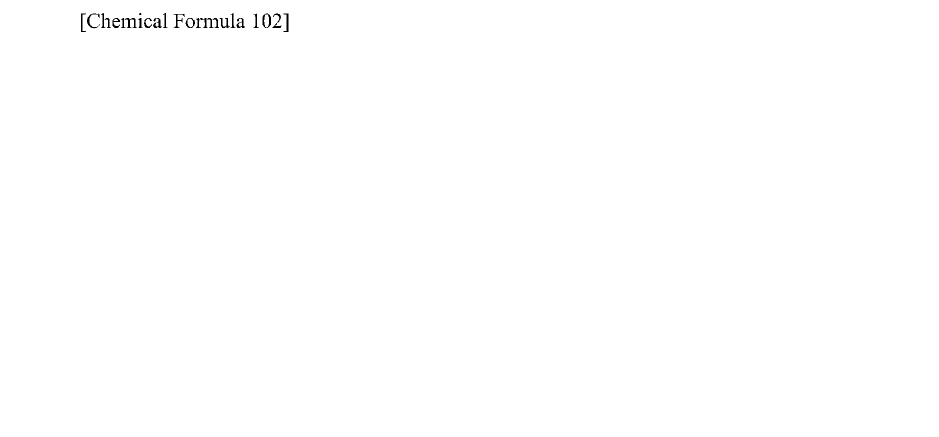 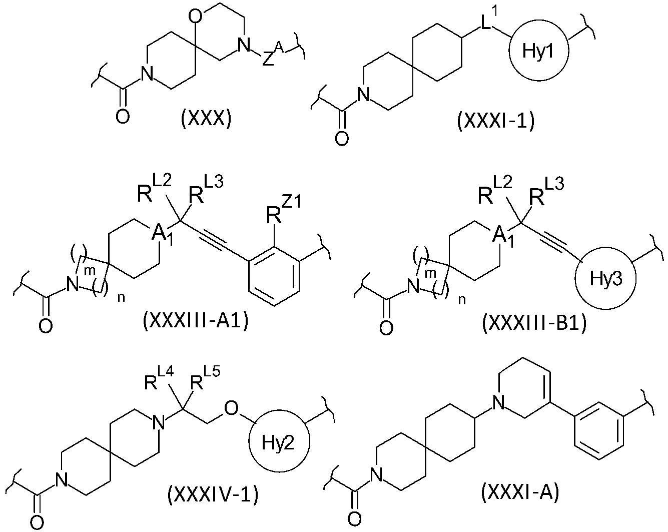
   Z^{A} is a group represented by the following formula (XXII-1) or (XXVI-2): 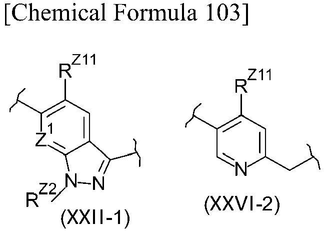
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C≡C-,
   ring Hy1 is a ring selected from the group consisting of the following formulas (XXXV), (XXXVI), (XXXVII), (XXXVIII), and (XXXIX): 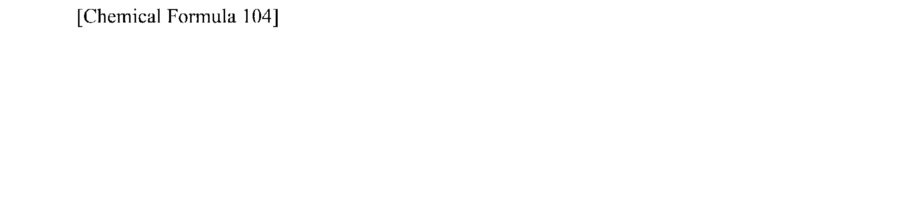 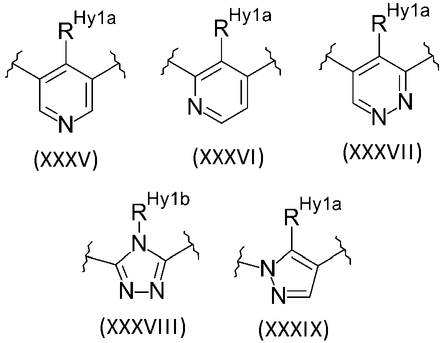
   R^{Hy1a} is H, C₁₋₃ alkyl, or halogen,
   R^{Hy1b} is H or C₁₋₃ alkyl,
   R^{Z1} is H or halogen,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 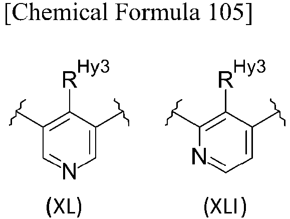
   R^{Hy3} is H or halogen,
   m and n are both 1 or both 2,
   ring Hy2 is of the following formula (XLIII): 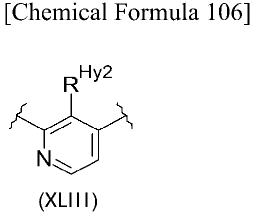
   R^{Hy2} is H or C₁₋₃ alkyl, and
   R^{L4} and R^{L5} are both H, or the R^{L4} and R^{L5} together with the carbon atom bonded thereto may form cyclopropane.
(10-8-1) A compound represented by formula (I) or a salt thereof, wherein L and Z together are a group selected from the group consisting of the following formulas (XXX), (XXXI-1X), (XXXIII-A1), (XXXIII-B1), (XXXIV-1), and (XXXI-A): 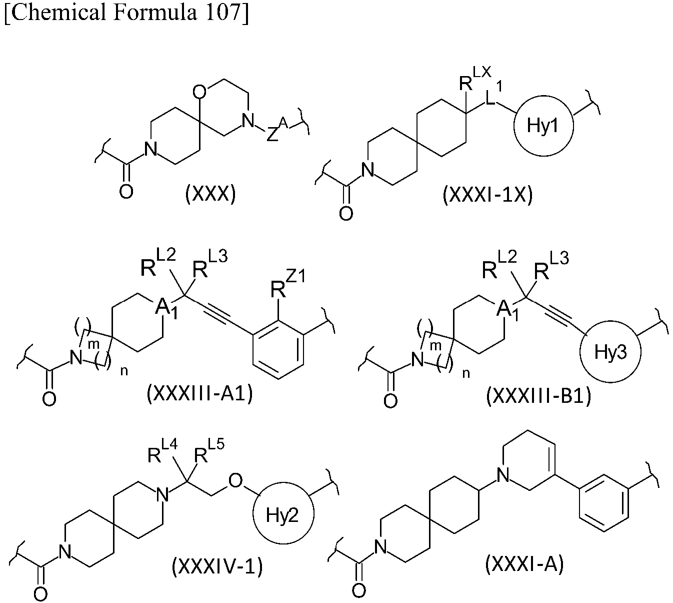
   Z^{A} is a group represented by the following formula (XXII-1) or (XXVI-2): 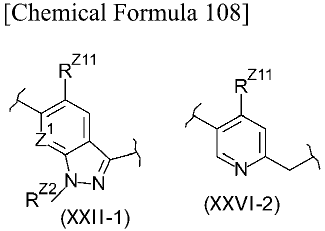
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C≡C-,
   R^{LX} is H or OH,
   ring Hy1 is a ring selected from the group consisting of the following formulas (XXXV), (XXXVI), (XXXVII), (XXXVIII), and (XXXIX): 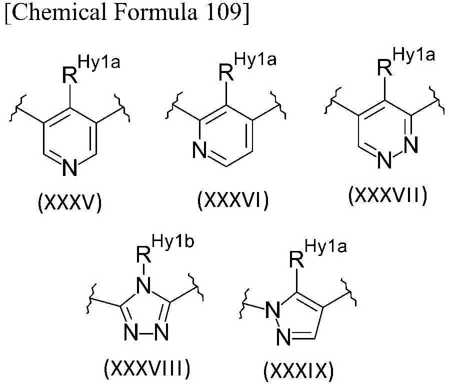
   R^{Hy1a} is H, C₁₋₃ alkyl, or halogen,
   R^{Hy1b} is H or C₁₋₃ alkyl,
   R^{Z1} is H or halogen,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 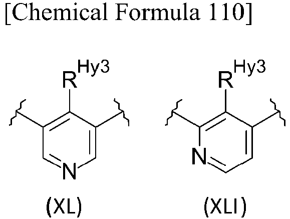
   R^{Hy3} is H or halogen,
   m and n are both 1 or both 2,
   ring Hy2 is of the following formula (XLIII): 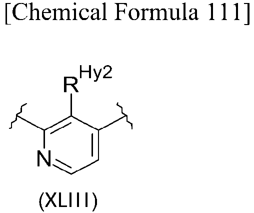
   R^{Hy2} is H or C₁₋₃ alkyl, and
   R^{L4} and R^{L5} are both H, or the R^{L4} and R^{L5} together with the carbon atom bonded thereto may form cyclopropane.
(10-9) A compound represented by formula (I) or a salt thereof, wherein L and Z together are a group represented by the formula (XXXI-1): 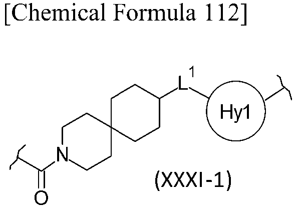
   L¹ is -CH₂CH₂-O- or -CH₂-C≡C-,
   ring Hy1 is a ring selected from the group consisting of the following formulas (XXXV) and (XXXVI): 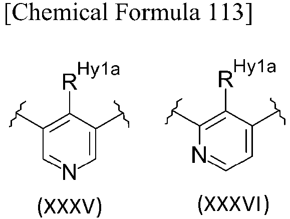
   R^{Hy1a} is H, C₁₋₃ alkyl, or halogen.
(10-9-1) A compound represented by formula (I) or a salt thereof, wherein L and Z together are a group represented by the formula (XXXI-1X): 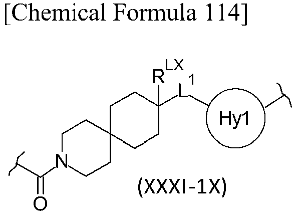
   L¹ is -CH₂CH₂-O- or -CH₂-C≡C-,
   R^{LX} is H,
   ring Hy1 is a ring selected from the group consisting of the following formulas (XXXV) and (XXXVI): 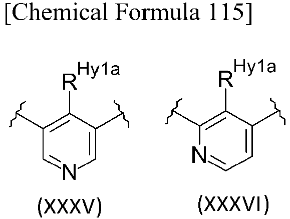
   R^{Hy1a} is H, C₁₋₃ alkyl, or halogen.
(10-10) A compound represented by formula (I) or a salt thereof, wherein L and Z together are a group represented by formula (XXXI-1A) or (XXXI-1B): 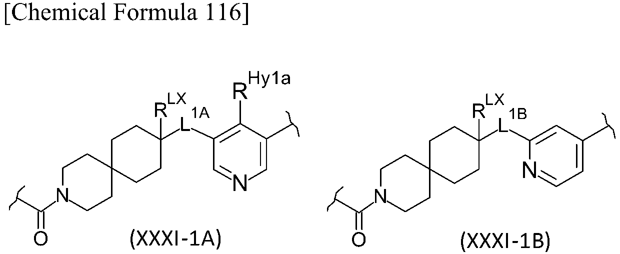
   R^{LX} is H,
   L^{1A} is -CH₂CH₂-O-,
   L^{1B} is -CH₂CH₂-O- or -CH₂-C≡C-, and
   R^{Hy1a} is H or C₁₋₃ alkyl.
(10-11) A compound represented by formula (I) or a salt thereof, wherein L and Z together are a group selected from the group consisting of the following formulas (XXX), (XXXI-1X), (XXXII-1), (XXXIII-A1), (XXXIII-B1), (XXXIV-1), and (XXXI-A): 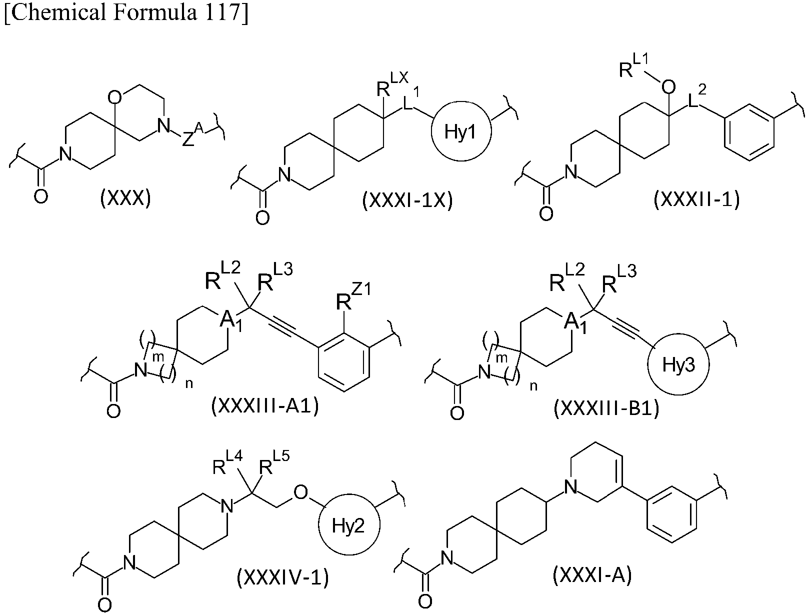
   Z^{A} is a group represented by the following formula (XXII-1) or (XXVI-2): 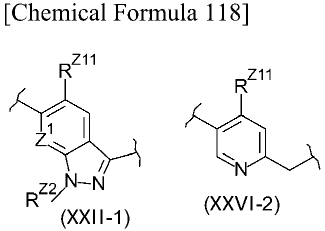
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C≡C-,
   R^{LX} is H or OH,
   ring Hy1 is a ring selected from the group consisting of the following formulas (XXXV), (XXXVI), (XXXVII), (XXXVII-2), (XXXVIII), and (XXXIX): 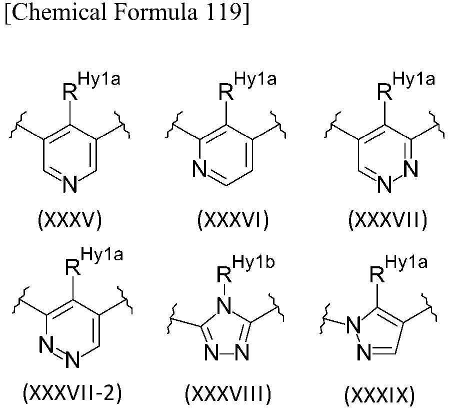
   R^{Hy1a} is H, C₁₋₃ alkyl, or halogen,
   R^{Hy1b} is H or C₁₋₃ alkyl,
   L² is -CH₂-C≡C- or -CH₂CH₂-O-,
   R^{Z1} is H or halogen,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 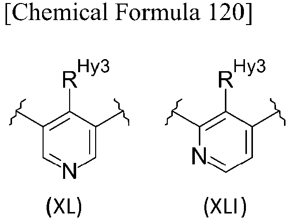
   R^{Hy3} is H or halogen,
   m and n are both 1 or both 2, and
   ring Hy2 is of the following formula (XLIII): 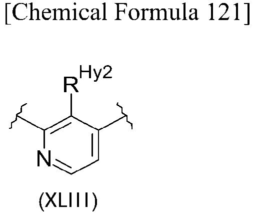
   R^{Hy2} is H or C₁₋₃ alkyl, and
   R^{L4} and R^{L5} are both H, or the R^{L4} and R^{L5} together with the carbon atom bonded thereto may form cyclopropane.
(10-12) A compound represented by formula (I) or a salt thereof, wherein L together with Z forms a group selected from the group consisting of the following formulas (XXXI-X), (XXXIII-A), (XXXIII-B), (XXXIV), and (XXXI-A): 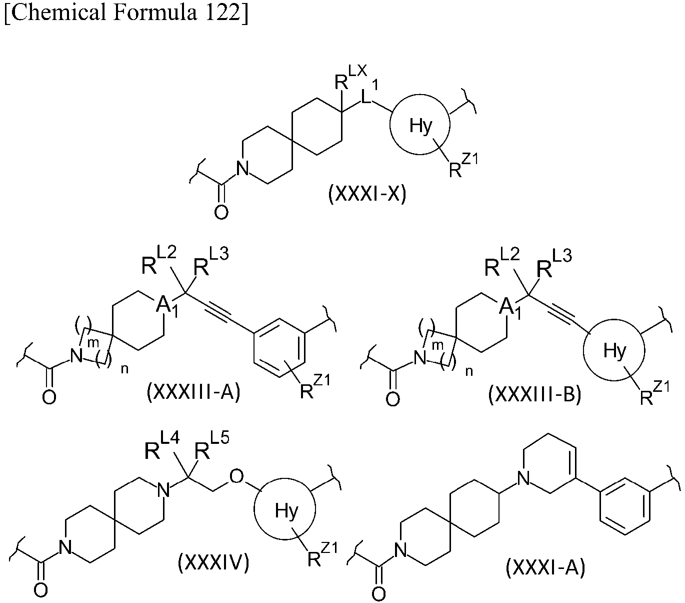
   L¹ is bond, C₁₋₃ alkylene, -C₁₋₃ alkylene-O-, -C₁₋₃ alkylene-C₂₋₃ alkynediyl, or -C≡C-,
   R^{LX} is H or OH,
   ring Hy is 5- or 6-membered hetero ring,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   R^{Z1} is H, C₁₋₃ alkyl, or halogen,
   m and n are the same or different, and are each 1 or 2, and
   R^{L4} and R^{L5} are the same or different, and are each H or C₁₋₃ alkyl, or the R^{L4} and R^{L5} together with the carbon atom bonded thereto may form C₃₋₆ cycloalkane or the R^{L4} and R^{L5} together with the carbon atom bonded thereto may form carbonyl.
(10-13) A compound represented by formula (I) or a salt thereof, wherein L and Z together are a group selected from the group consisting of the following formulas (XXXI-X), (XXXIII-A), (XXXIII-B), (XXXIV), and (XXXI-A): 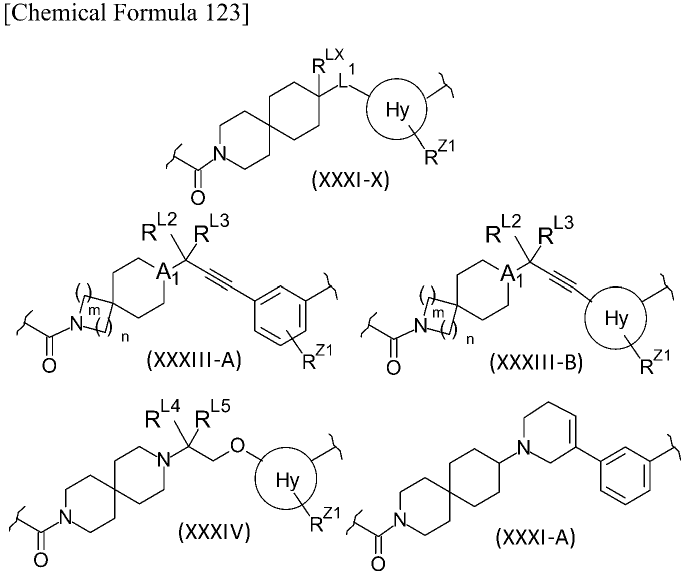
   L¹ is bond, C₁₋₃ alkylene, -C₁₋₃ alkylene-O-, or -CH₂-C≡C-,
   R^{LX} is H or OH,
   ring Hy is 5- or 6-membered hetero ring,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   R^{Z1} is H, C₁₋₃ alkyl, or halogen,
   m and n are both 1 or both 2, and
   R^{L4} and R^{L5} are both H, or the R^{L4} and R^{L5} together with the carbon atom bonded thereto may form cyclopropane.
(10-14) A compound represented by formula (I) or a salt thereof, wherein L and Z together are a group selected from the group consisting of the following formulas (XXXI-1X), (XXXIII-A1), (XXXIII-B1), (XXXIV-1), and (XXXI-A): 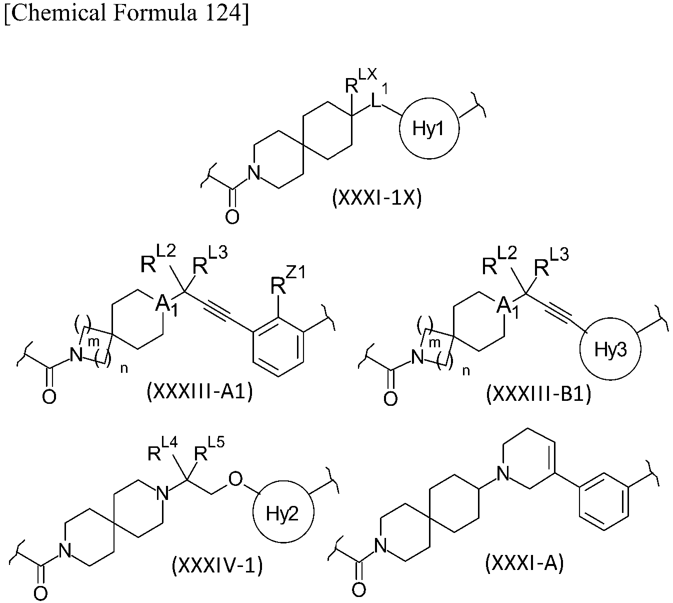
   L¹ is bond, C₁₋₃ alkylene, -C₁₋₃ alkylene-O-, or -CH₂-C≡C-,
   R^{LX} is H or OH,
   ring Hy1 is a ring selected from the group consisting of the following formulas (XXXV), (XXXVI), (XXXVII), (XXXVII-2), (XXXVIII), and (XXXIX): 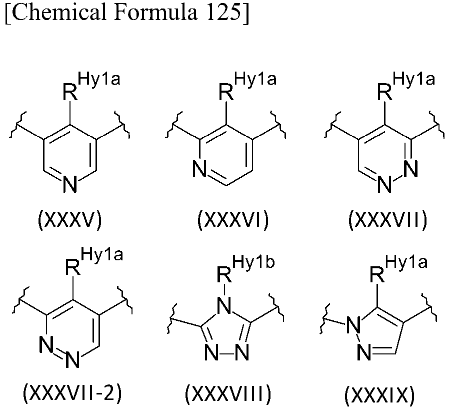
   R^{Hy1a} is H, C₁₋₃ alkyl, or halogen,
   R^{Hy1b} is H or C₁₋₃ alkyl,
   R^{Z1} is H, C₁₋₃ alkyl, or halogen,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 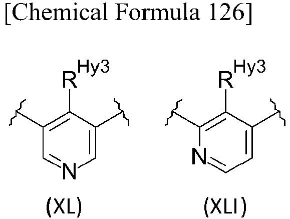
   R^{Hy3} is H, C₁₋₃ alkyl, or halogen,
   m and n are both 1 or both 2,
   ring Hy2 is of the following formula (XLIII): 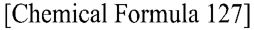 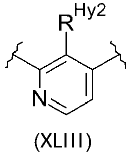
   R^{Hy2} is H, C₁₋₃ alkyl, or halogen, and
   R^{L4} and R^{L5} are both H, or the R^{L4} and R^{L5} together with the carbon atom bonded thereto may form cyclopropane.
(10-15) A compound represented by formula (I) or a salt thereof, wherein L and Z together are a group selected from the group consisting of the following formulas (XXXI-1X), (XXXIII-A1), (XXXIII-B1), (XXXIV-1), and (XXXI-A): 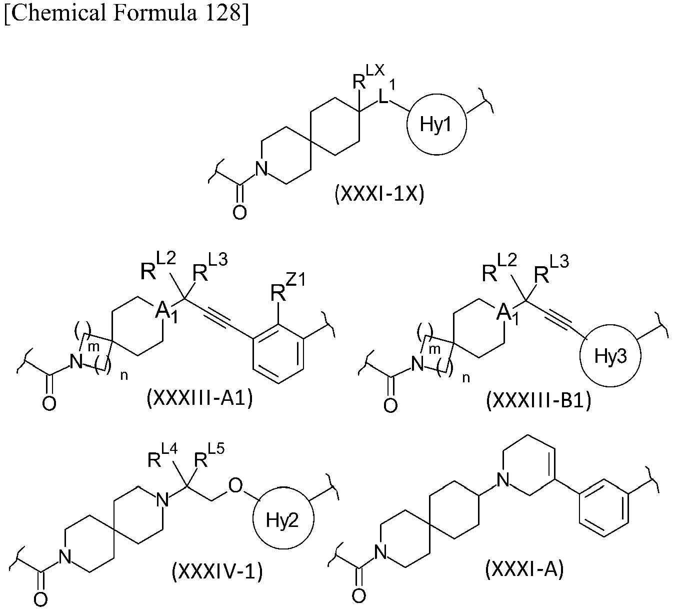
   L¹ is bond, C₁₋₃ alkylene, -C₁₋₃ alkylene-O-, or -CH₂-C≡C-,
   R^{LX} is H or OH,
   ring Hy1 is a ring selected from the group consisting of the following formulas (XXXV), (XXXVI), (XXXVII), (XXXVII-2), (XXXVIII), and (XXXIX): 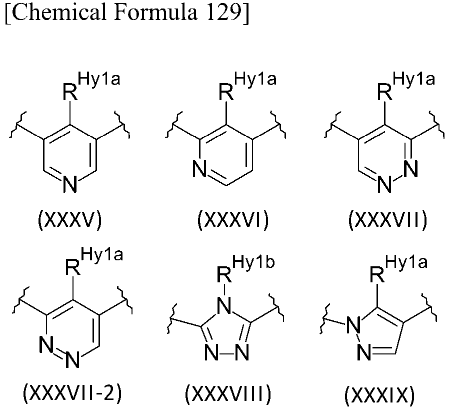
   R^{Hy1a} is H, C₁₋₃ alkyl, or halogen,
   R^{Hy1b} is H or C₁₋₃ alkyl,
   R^{Z1} is H or halogen,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 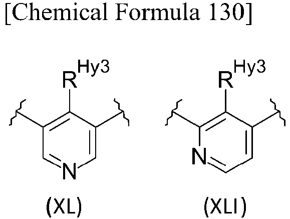
   R^{Hy3} is H or halogen,
   m and n are both 1 or both 2,
   ring Hy2 is of the following formula (XLIII): 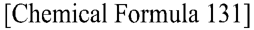 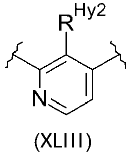
   R^{Hy2} is H or C₁₋₃ alkyl, and
   R^{L4} and R^{L5} are both H, or the R^{L4} and R^{L5} together with the carbon atom bonded thereto may form cyclopropane.
(10-16) A compound represented by formula (I) or a salt thereof, wherein L and Z together are a group selected from the group consisting of the following formulas (XXXI-1X), (XXXIII-A1), (XXXIII-B1), and (XXXIV-1): 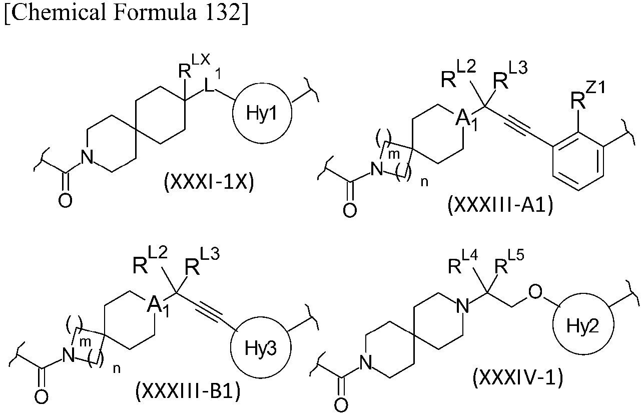
   L¹ is bond, C₁₋₃ alkylene, -C₁₋₃ alkylene-O-, or -CH₂-C≡C-,
   R^{LX} is H or OH,
   ring Hy1 is a ring selected from the group consisting of the following formulas (XXXV), (XXXVI), (XXXVII), (XXXVII-2), (XXXVIII), and (XXXIX): 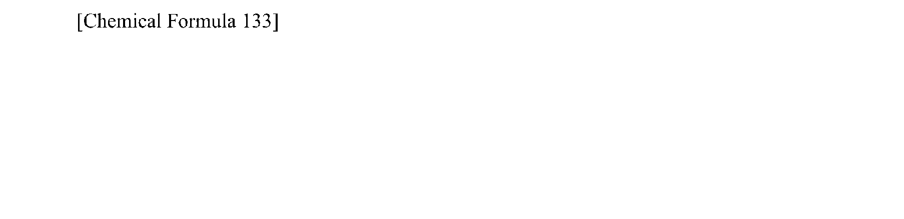 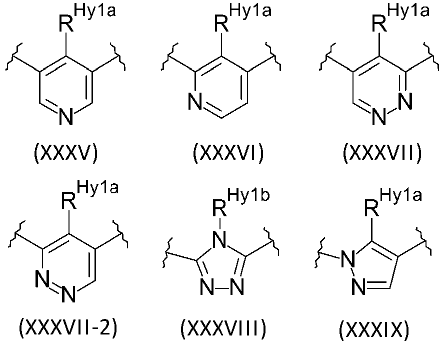
   R^{Hy1a} is H, C₁₋₃ alkyl, or halogen,
   R^{Hy1b} is H or C₁₋₃ alkyl,
   R^{Z1} is H or halogen,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 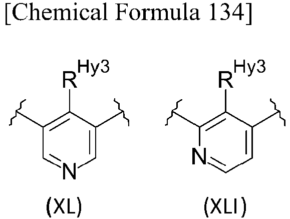
   R^{Hy3} is H or halogen,
   m and n are both 1 or both 2,
   ring Hy2 is of the following formula (XLIII): 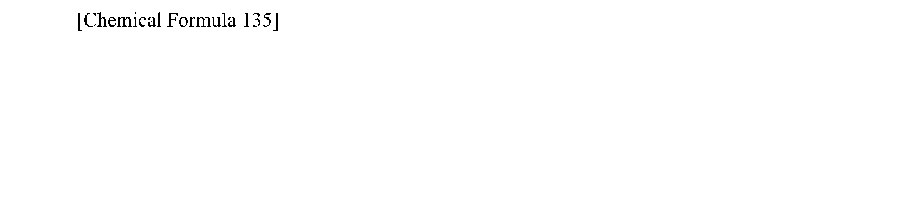 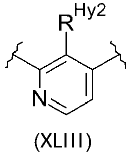
   R^{Hy2} is H or C₁₋₃ alkyl, and
   R^{L4} and R^{L5} are both H, or the R^{L4} and R^{L5} together with the carbon atom bonded thereto may form cyclopropane.
(11-1) A compound represented by formula (I) or a salt thereof, wherein G is N or CH,
   provided that when G is N, Z is formula (XVIII), (XXII), (XXIII), (XXIV), (XXVI), (XXVII), or (XXIX).
(11-2) A compound represented by formula (I) or a salt thereof, wherein G is N,
   Z is formula (XXII), (XXIV), (XXVI), or (XXVII).
(11-3) A compound represented by formula (I) or a salt thereof, wherein G is N.
(12) A compound or a salt thereof which is any noncontradictory combination of two or more of the embodiments according to (1-1) to (1-7), (1A-1) to (1A-3), (2-1) to (2-8-1), (3-1) to (3-4), (4-1) to (4-4), (5-1) to (5-3), (6-1) to (6-4), (7-1) to (7-4), (8-1) to (8-6), (9-1) to (9-7), (10-1) to (10-16), and (11-1) to (11-3).

Examples of the combination according to (12) above specifically include the following embodiments.
(12-1) A compound represented by formula (I) or a salt thereof: 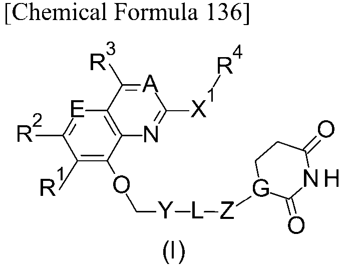 wherein
   A is CR^{A} or N,
   R^{A} is H, C₁₋₃ alkyl, -CN, or -O-(C₁₋₃ alkyl),
   E is CH or N,
   X¹ is -CH₂-, -O-, or -NR^{X1}-,
   R^{X1} is H or optionally substituted C₁₋₃ alkyl,
   provided that when X¹ is -NR^{X1}-, R^{X1} and R⁴ present on the same nitrogen atom, together with the nitrogen atom adjacent thereto, may form optionally substituted 4- to 11-membered saturated hetero ring group,
   R¹ is represented by the following formula (II), (III), (IV), (V), (VI), or (VII):

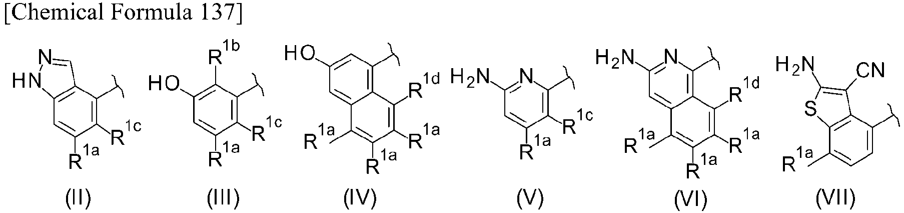

   R^{1a} and R^{1b} are the same or different, and are each H, methyl, F, or Cl,
   R^{1c} is F, Cl, methyl, ethyl, trifluoromethyl, or cyclopropyl,
   R^{1d} is H, methyl, ethyl, F, Cl, or -C=C-H,
   R² is H, halogen, optionally substituted C₁₋₃ alkyl, cyclopropyl, or vinyl,
   R³ is a group selected from the group consisting of the following formulas (VIII), (XI), (X), (XI), (XII), (XIII), (XIV), (XV), (XVI), and (XVII): 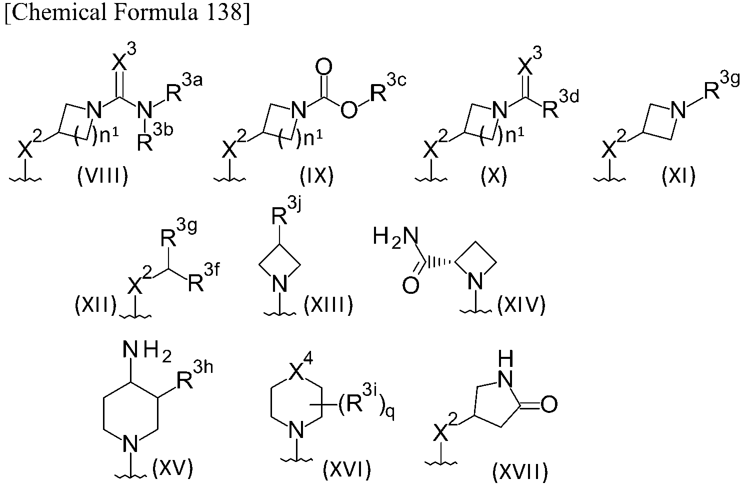
   R^{3a} is -(CH₂)ₚCHR^{3e}-NR^{N1}R^{N2}; -(CH₂)ₚCHR^{3e}-OR^{3f}; a 4- to 6-membered saturated hetero ring group optionally substituted with a group selected from the group consisting of C₁₋₃ alkyl, -C₁₋₃ alkylene-OR^{3f}, -C₁₋₃ alkylene-NR^{N1}R^{N2}, and -NR^{N1}R^{N2}. or C₃₋₆ cycloalkyl optionally substituted with a group selected from the group consisting of C₁₋₃ alkyl, -C₁₋₃ alkylene-OR^{3f}, -C₁₋₃ alkylene-NR^{N1}R^{N2}, -OR^{3f}, and -NR^{N1}R^{N2}
   R^{3b} is H or C₁₋₃ alkyl,
   R^{3c} and R^{3d} are -(CH₂)ₚCHR^{3e}-NR^{N1}R^{N2}; -(CH₂)ₚCHR^{3e}-OR^{3f}; 4- to 6-membered saturated hetero ring group optionally substituted with a group selected from the group consisting of C₁₋₃ alkyl, -C₁₋₃ alkylene-OR^{3f}, -C₁₋₃ alkylene-NR^{N1}R^{N2}, and -NR^{N1}R^{N2}; or C₃₋₆ cycloalkyl optionally substituted with a group selected from the group consisting of C₁₋₃ alkyl, -C₁₋₃ alkylene-OR^{3f}, -C₁₋₃ alkylene-NR^{N1}R^{N2}, -OR^{3f}, and -NR^{N1}R^{N2},
   R^{3e} is H, F, or C₁₋₃ alkyl,
   R^{3f} is the same or different, and is H or C₁₋₃ alkyl,
   R^{3g} is optionally substituted C₃₋₆ cycloalkyl, optionally substituted 5-membered heteroaryl, optionally substituted 6-membered heteroaryl, or optionally substituted 4- to 6-membered saturated hetero ring group,
   R^{3h} is H, F, or C₁₋₃ alkyl,
   R³ⁱ is the same or different, and is a group selected from the group consisting of H, OH, optionally substituted C₁₋₃ alkyl, -O-optionally substituted C₁₋₃ alkyl, -NH-optionally substituted C₁₋₃ alkyl, -N-(optionally substituted C₁₋₃ alkyl)₂, halogen, -CN, and oxo, or
   two R³ⁱs present on the same carbon atom together with the carbon atom adjacent thereto may form a spiro ring having a ring selected from the group consisting of C₃₋₆ cycloalkane and 4- to 6-membered saturated hetero ring, wherein the spiro ring is optionally substituted with 1 to 2 groups selected from the group of C₁₋₃ alkyl, -O-(C₁₋₃ alkyl), OH, halogen, and oxo, or
   R³ⁱ present on two adjacent carbon atoms together with the two carbon atoms may form a fused ring having a ring selected from the group consisting of C₃₋₆ cycloalkane and 4-to 6-membered saturated hetero ring, wherein the fused ring is optionally substituted with 1 to 2 groups selected from the group consisting of C₁₋₃ alkyl, -O-(C₁₋₃ alkyl), OH, halogen, and oxo, or
   R³ⁱ present on two non-adjacent carbon atoms together with the two carbon atoms may form a bridged structure composed of 1 to 2 carbon atoms, and a ring having the bridged structure is optionally substituted with 1 to 2 groups selected from the group consisting of C₁₋₃ alkyl, -O-(C₁₋₃ alkyl), OH, halogen, and oxo,
   R^{N1} and R^{N2} are the same or different, and are each H or C₁₋₃ alkyl, or
   R^{N1} and R^{N2} together with the nitrogen atom bonded thereto may form optionally substituted 4- to 6-membered saturated hetero ring group, or
   R^{3e} and R^{N1} together with the carbon atom and the nitrogen atom bonded thereto may form optionally substituted 4- to 6-membered saturated hetero ring group,
   R^{3j} is a group selected from the group consisting of H, OH, halogen, optionally substituted C₁₋₃ alkyl, -O-optionally substituted C₁₋₃ alkyl, optionally substituted C₃₋₆ cycloalkyl, optionally substituted 5-membered heteroaryl, optionally substituted 6-membered heteroaryl, and -CN,
   X² is -O-, -NH-, or -N(C₁₋₃ alkyl)-,
   X³ is O or S,
   X⁴ is -CH₂-, -CH₂-CH₂-, -O-CH₂-, -O-, or -O-NH-,
   n¹ is 1 or 2,
   p is 1 or 2,
   q is 1 to 8,
   R⁴ is optionally substituted C₁₋₆ alkyl, optionally substituted piperidinyl, or optionally substituted tetrahydropyranyl,
   Y is phenylene or pyridinediyl, wherein the phenylene is optionally substituted with F,
   L is -(L^{A}-L^{B}-L^{C}-L^{D}-L^{E}-L^{F})-,
   L^{A}, L^{B}, L^{C}, L^{D}, L^{E}, and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O,
   R^{L1A} is H or C₁₋₃ alkyl,
   Z is a group selected from the group consisting of the following formulas (XVIII), (XIX), (XX), (XXI), (XXII), (XXIII), (XXIV), (XXV), (XXVI), (XXVII), (XXVIII), and (XXIX): 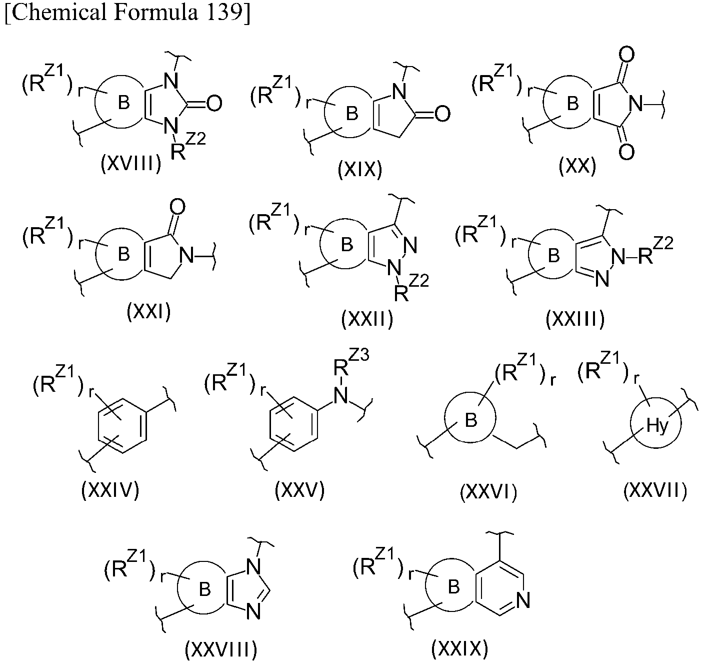
   ring B is benzene ring or 6-membered hetero ring,
   ring Hy is 5- or 6-membered hetero ring,
   R^{Z1} is H, C₁₋₃ alkyl, halogen, -O-(C₁₋₃ alkyl), -NR^{Z4}₂, -CN, -CONR^{Z4}₂, or - NR^{Z4}COR^{Z5},
   R^{Z2} is H or C₁₋₃ alkyl,
   R^{Z3} is H or C₁₋₃ alkyl,
   R^{Z4} is the same or different, and is H or C₁₋₃ alkyl,
   R^{Z5} is C₁₋₃ alkyl,
   L is bonded to ring B in the above formulas (XVIII), (XIX), (XX), (XXI), (XXII), (XXIII), (XXVI), (XXVIII), and (XXIX), to the benzene ring in formulas (XXIV) and (XXV), and to ring Hy in formula (XXVII),
   r is 1 or 2, and
   G is N or CH,
   provided that when G is N, Z is the above formula (XVIII), (XXII), (XXIII), (XXIV), (XXVI), (XXVII), or (XXIX).
(12-2) The compound or a salt thereof according to (12-1) above,
   wherein E is CH,
   R³ is a group represented by the following formula (XII), (XIII), (XIV), or (XVII): 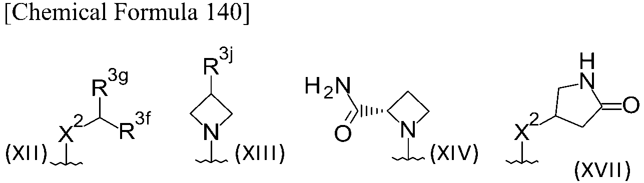
   R^{3f} is the same or different, and is H or C₁₋₃ alkyl,
   R^{3g} is optionally substituted C₃₋₆ cycloalkyl, optionally substituted 5-membered heteroaryl, optionally substituted 6-membered heteroaryl, or optionally substituted 4- to 6-membered saturated hetero ring group,
   R^{3j} is a group selected from the group consisting of H, OH, halogen, optionally substituted C₁₋₃ alkyl, -O-optionally substituted C₁₋₃ alkyl, optionally substituted C₃₋₆ cycloalkyl, optionally substituted 5-membered heteroaryl, optionally substituted 6-membered heteroaryl, and -CN,
   X² is -O-, -NH-, or -N(C₁₋₃ alkyl)-,
   L is -(L^{A}-L^{B}-L^{C}-L^{D}-L^{E}-L^{F})-,
   L^{A}, L^{B}, L^{C}, L^{D}, L^{E} and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O,
   R^{L1A} is H or C₁₋₃ alkyl,
   Z is a group selected from the group consisting of formulas (XXII), (XXIV), (XXVI), and (XXVII): 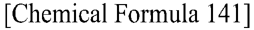 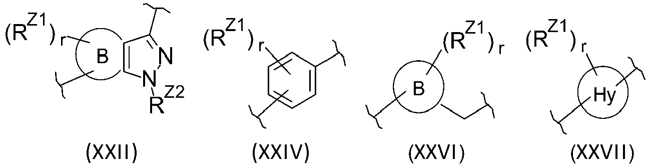
   ring B is benzene ring or 6-membered hetero ring,
   ring Hy is 5- or 6-membered hetero ring,
   R^{Z1} is H, C₁₋₃ alkyl, halogen, -O-(C₁₋₃ alkyl), -NR^{Z4}₂, -CN, -CONR^{Z4}₂, or - NR^{Z4}COR^{Z5},
   R^{Z2} is H or C₁₋₃ alkyl,
   R^{Z4} is the same or different, and is H or C₁₋₃ alkyl,
   R^{Z5} is C₁₋₃ alkyl,
   r is 1 or 2,
   L is bonded to ring B in the above formulas (XXII) and (XXVI), to the benzene ring in formulas (XXIV), and to ring Hy in formula (XXVII), and
   G is N.
(12-2-1) The compound or a salt thereof according to (12-2) above, wherein X¹ is -O- or -NR^{X1}-,
   provided that when X¹ is -NR^{X1}-, R^{X1} and R⁴ present on the same nitrogen atom, together with the nitrogen atom adjacent thereto, form optionally substituted 4- to 11-membered saturated hetero ring group,
   R¹ is represented by the following formula (II), (III), or (IV): 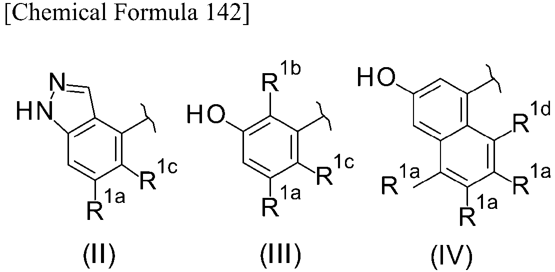
   R^{1a} is the same or different, and is H or F,
   R^{1b} is H,
   R^{1c} is methyl or cyclopropyl,
   R^{1d} is H,
   R² is cyclopropyl,
   R³ is a group represented by formula (XIV): 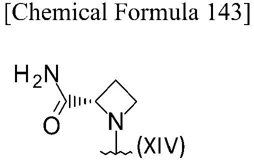
   R⁴ is C₁₋₆ alkyl or piperidinyl, and the C₁₋₆ alkyl is optionally substituted with a group selected from the group consisting of OH; OCH₃; N(R^{4a})₂; C₃₋₆ cycloalkyl optionally substituted with R^{4b}; pyrrolidinyl optionally substituted with R^{4b}; and morpholinyl optionally substituted with R^{4b}, and the piperidinyl is optionally substituted with R^{4b},
   R^{4a} is C₁₋₃ alkyl, and
   R^{4b} is C₁₋₃ alkyl optionally substituted with -O-(C₁₋₃ alkyl) or -N(C₁₋₃ alkyl)₂, or halogen,
   Y is phenylene,
   L is a group selected from the group consisting of the following formulas (XXX-L), (XXXI-L), (XXXII-L), (XXXIII-L), and (XXXIV-L): 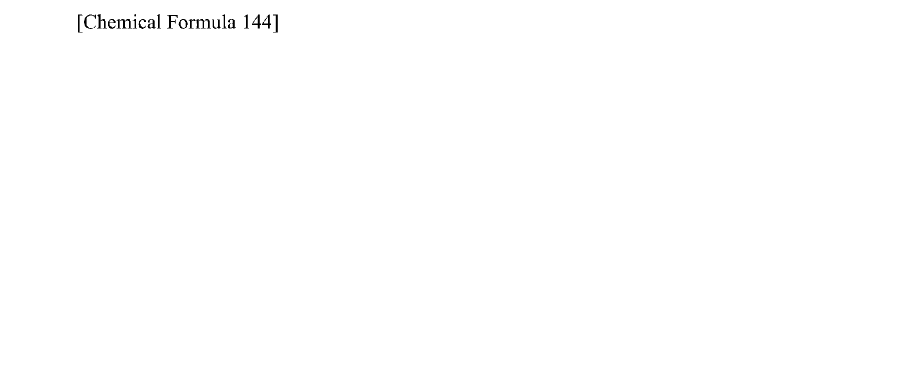 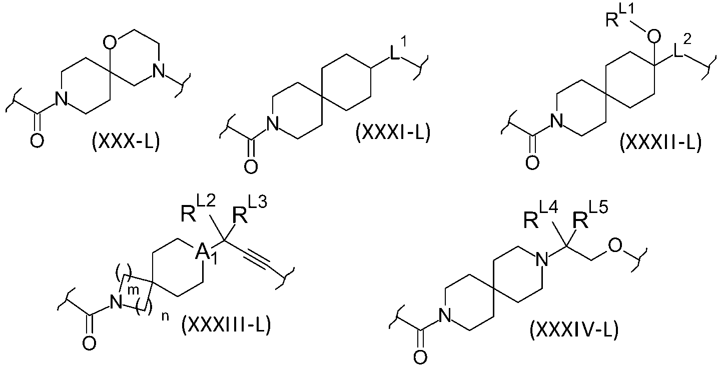
   L¹ is bond, C₁₋₃ alkylene, -CH₂-C=C-, -C₁₋₃ alkylene-O-, or tetrahydropyridinediyl,
   L² is -CH₂-C≡C- or -CH₂CH₂-O-,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are both 1 or both 2, and
   R^{L4} and R^{L5} are both H, or the R^{L4} and R^{L5} together with the carbon atom bonded thereto may form cyclopropane.
   Z is a group selected from the group consisting of formulas (XXX-Z), (XXXI-Z), and (XXXII-Z): 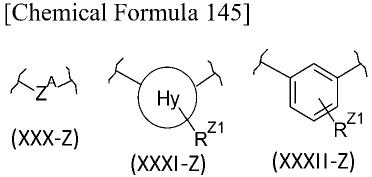
   Z^{A} is a group represented by the following formula (XXII-1) or (XXVI-2): 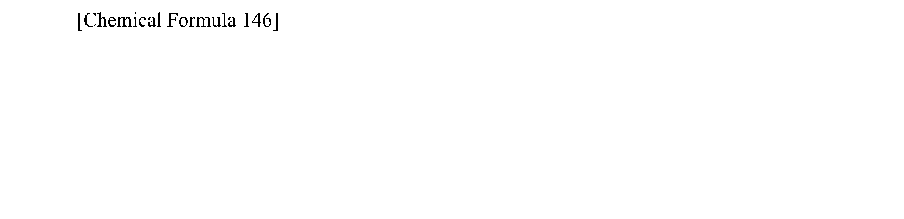 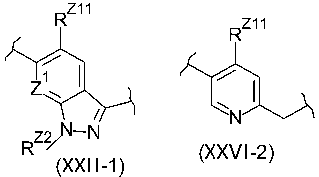
   R^{Z11} is halogen,
   Z¹ is CH or N,
   R^{Z2} is C₁₋₃ alkyl,
   ring Hy is 5- or 6-membered hetero ring, and
   R^{Z1} is H, C₁₋₃ alkyl, or halogen.
(12-3) The compound or a salt thereof according to (12-1) above,
   wherein E is CH,
   R³ is a group represented by formula (XIV): 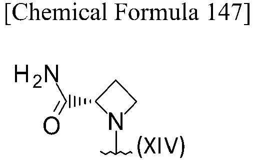
   Y is phenylene,
   L together with Z forms a group selected from the group consisting of the following formulas (XXX), (XXXI), (XXXII), (XXXIII-A), (XXXIII-B), (XXXIV), and (XXXI-A): 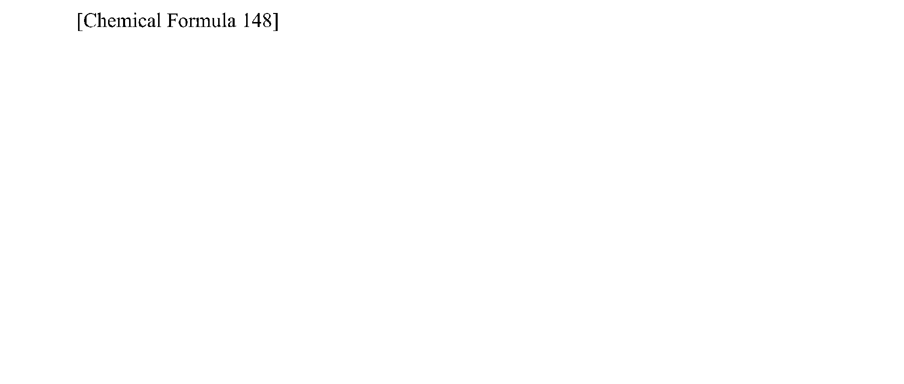 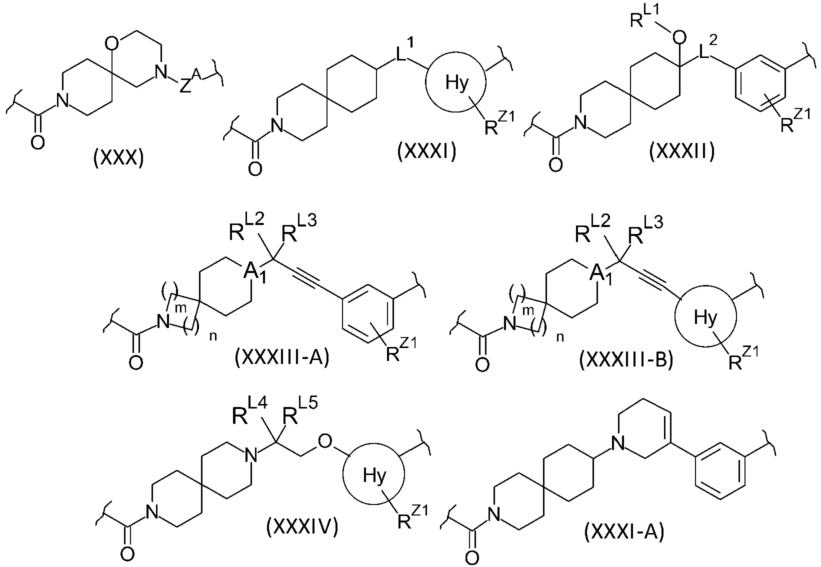
   Z^{A} is a group represented by the following formula (XXII-1) or (XXVI-1): 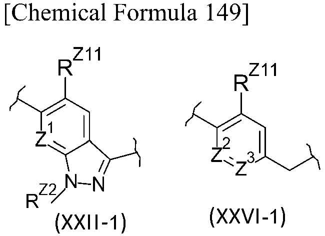
   R^{Z11} is H or halogen,
   Z¹, Z², and Z³ are the same or different, and are each CH or N,
   R^{Z2} is C₁₋₃ alkyl,
   L¹ is bond, C₁₋₃ alkylene, -C₁₋₃ alkylene-O-, -C₁₋₃ alkylene-C₂₋₃ alkynediyl, or -C=C-, ring Hy is 5- or 6-membered hetero ring,
   L² is -C₁₋₃ alkylene-C₂₋₃ alkynediyl or -C₁₋₃ alkylene-O-,
   R^{Z1} is H, halogen, C₁₋₃ alkyl, -O-(C₁₋₃ alkyl), or -CN,
   R^{L1} is H or C₁₋₃ alkyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are the same or different, and are each 1 or 2,
   R^{L4} and R^{L5} are the same or different, and are each H or C₁₋₃ alkyl, or the R^{L4} and R^{L5} together with the carbon atom bonded thereto may form C₃₋₆ cycloalkane, or the R^{L4} and R^{L5} together with the carbon atom bonded thereto may form carbonyl, and
   G is N.
(12-3-1) The compound or a salt thereof according to (12-1) above,
   wherein E is CH,
   X¹ is -O- or -NR^{X1}-,
   R^{X1} is H or C₁₋₃ alkyl,
   provided that when X¹ is -NR^{X1}-, R^{X1} and R⁴ present on the same nitrogen atom, together with the nitrogen atom adjacent thereto, may form optionally substituted 4- to 11-membered saturated hetero ring group,
   R¹ is represented by the following formula (II), (III), or (IV): 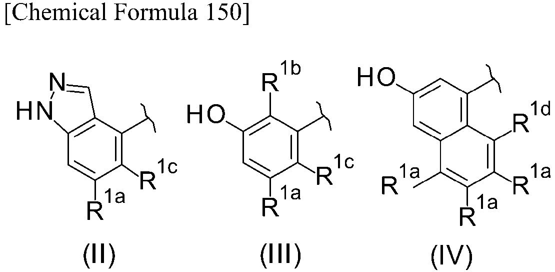
   R^{1a} is the same or different, and is H or F,
   R^{1b} is H,
   R^{1c} is methyl or cyclopropyl,
   R^{1d} is H,
   R³ is a group represented by the formula (XIV): 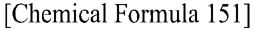 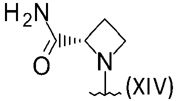
   R⁴ is C₁₋₆ alkyl or piperidinyl, and the C₁₋₆ alkyl is optionally substituted with a group selected from the group consisting of F; OH; OCH₃; N(R^{4a})₂; C₃₋₆ cycloalkyl optionally substituted with R^{4b}; pyrrolidinyl optionally substituted with R^{4b}, and morpholinyl optionally substituted with R^{4b}, and the piperidinyl is optionally substituted with R^{4b},
   R^{4a} is optionally substituted C₁₋₃ alkyl,
   R^{4b} is optionally substituted C₁₋₃ alkyl, or halogen,
   Y is phenylene,
   L together with Z forms a group selected from the group consisting of the following formulas (XXX), (XXXI-X), (XXXII), (XXXIII-A), (XXXIII-B), (XXXIV), and (XXXI-A): 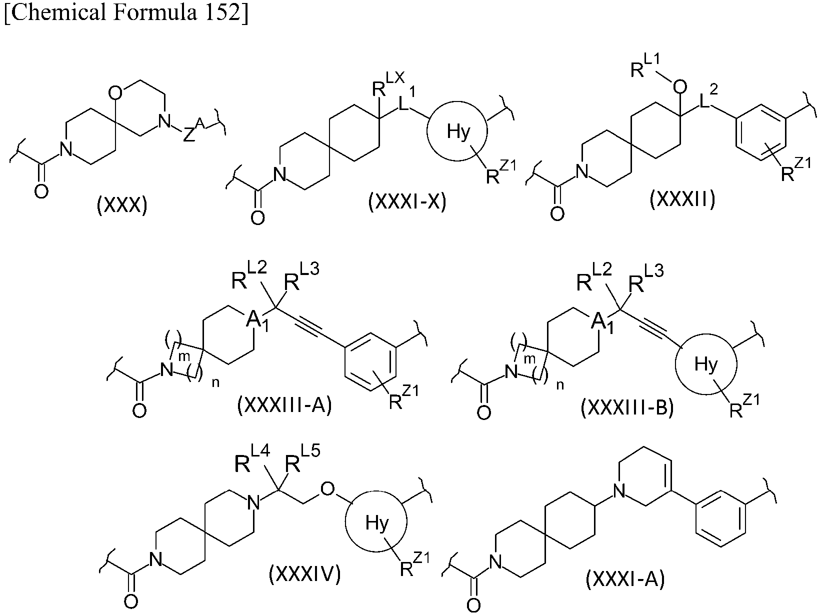
   Z^{A} is a group represented by the following formula (XXII-1) or (XXVI-1): 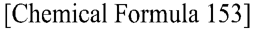 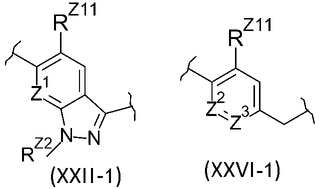
   R^{Z11} is H or halogen,
   Z¹, Z², and Z³ are the same or different, and are each CH or N,
   R^{Z2} is C₁₋₃ alkyl,
   L¹ is bond, C₁₋₃ alkylene, -C₁₋₃ alkylene-O-, -C₁₋₃ alkylene-C₂₋₃ alkynediyl, or -C≡C-,
   R^{LX} is H or OH,
   ring Hy is 5- or 6-membered hetero ring,
   L² is -C₁₋₃ alkylene-C₂₋₃ alkynediyl or -C₁₋₃ alkylene-O-,
   R^{Z1} is H, halogen, C₁₋₃ alkyl, -O-(C₁₋₃ alkyl), or -CN,
   R^{L1} is H or C₁₋₃ alkyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are the same or different, and are each 1 or 2,
   R^{L4} and R^{L5} are the same or different, and are each H or C₁₋₃ alkyl, or the R^{L4} and R^{L5} together with the carbon atom bonded thereto may form C₃₋₆ cycloalkane, or the R^{L4} and R^{L5} together with the carbon atom bonded thereto may form carbonyl, and
   G is N.
(12-4) The compound or a salt thereof according to (12-3) above,
   wherein A is N,
   X¹ is -O- or -NR^{X1}-,
   R^{X1} is H or C₁₋₃ alkyl,
   provided that when X¹ is -NR^{X1}-, R^{X1} and R⁴ present on the same nitrogen atom, together with the nitrogen atom adjacent thereto, may form optionally substituted 4- to 11-membered saturated hetero ring group,
   R⁴ is C₁₋₆ alkyl or piperidinyl, and the C₁₋₆ alkyl is optionally substituted with a group selected from the group consisting of F; OH; OCH₃; N(R^{4a})₂; C₃₋₆ cycloalkyl optionally substituted with R^{4b}; pyrrolidinyl optionally substituted with R^{4b}, and morpholinyl optionally substituted with R^{4b}, and the piperidinyl is optionally substituted with R^{4b},
   R^{4a} is optionally substituted C₁₋₃ alkyl,
   R^{4b} is optionally substituted C₁₋₃ alkyl, or halogen.
(12-4-1) The compound or a salt thereof according to (12-3-1) above, wherein A is N,
   X¹ is -O- or -NR^{X1}-,
   provided that when X¹ is -NR^{X1}-, R^{X1} and R⁴ present on the same nitrogen atom, together with the nitrogen atom adjacent thereto, form optionally substituted 4- to 11-membered saturated hetero ring group,
   R⁴ is C₁₋₆ alkyl or piperidinyl, and the C₁₋₆ alkyl is optionally substituted with a group selected from the group consisting of F; OH; OCH₃; N(R^{4a})₂; C₃₋₆ cycloalkyl optionally substituted with R^{4b}; pyrrolidinyl optionally substituted with R^{4b}, and morpholinyl optionally substituted with R^{4b}, and the piperidinyl is optionally substituted with R^{4b},
   R^{4a} is optionally substituted C₁₋₃ alkyl, and
   R^{4b} is optionally substituted C₁₋₃ alkyl, or halogen.
(12-5) The compound or a salt thereof according to (12-3),
   wherein A is N,
   X¹ is -O- or -NR^{X1}-,
   provided that when X¹ is -NR^{X1}-, R^{X1} and R⁴ present on the same nitrogen atom, together with the nitrogen atom adjacent thereto, form optionally substituted 4- to 11-membered saturated hetero ring group,
   R¹ is represented by the following formula (II), (III), or (IV): 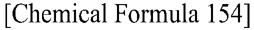 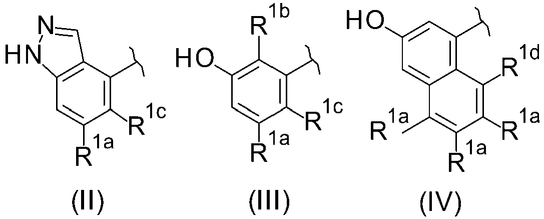
   R^{1a} is the same or different, H or F,
   R^{1b} is H,
   R^{1c} is methyl or cyclopropyl,
   R^{1d} is H,
   R² is cyclopropyl,
   R⁴ is C₁₋₆ alkyl or piperidinyl, and the C₁₋₆ alkyl is optionally substituted with a group selected from the group consisting of OH; OCH₃; N(R^{4a})₂; C₃₋₆ cycloalkyl optionally substituted with R^{4b}; pyrrolidinyl optionally substituted with R^{4b}; and morpholinyl optionally substituted with R^{4b}, and the piperidinyl is optionally substituted with R^{4b},
   R^{4a} is C₁₋₃ alkyl,
   R^{4b} is C₁₋₃ alkyl optionally substituted with -O-(C₁₋₃ alkyl) or -N(C₁₋₃ alkyl)₂, or halogen,
   L and Z together are a group selected from the group consisting of the following formulas (XXX), (XXXI), (XXXII), (XXXIII-A), (XXXIII-B), (XXXIV), and (XXXI-A): 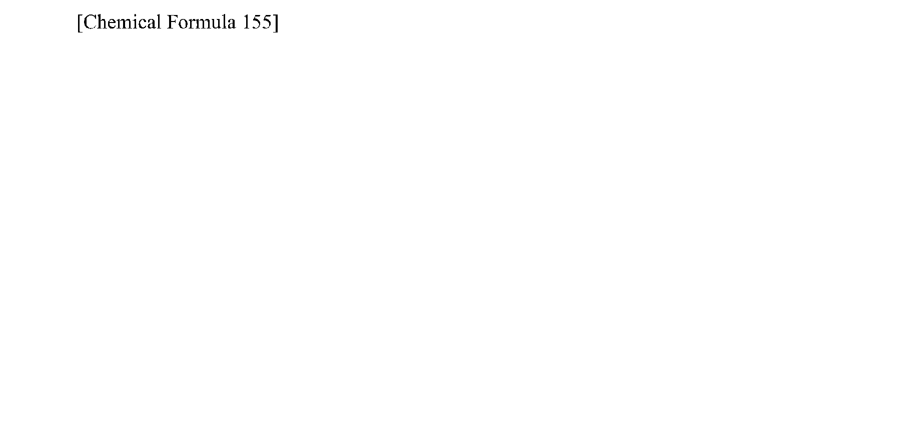 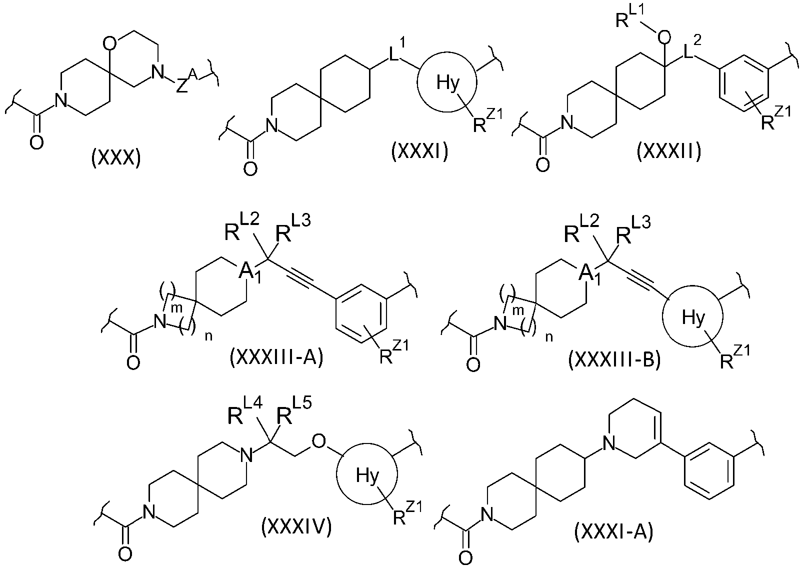
   Z^{A} is a group represented by the following formula (XXII-1) or (XXVI-2): 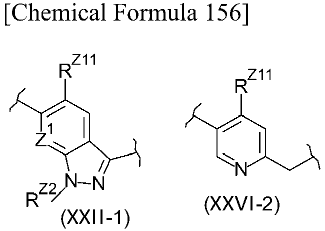
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C=C-,
   ring Hy is 5- or 6-membered hetero ring,
   L² is -CH₂-C≡C- or -CH₂CH₂-O-,
   R^{Z1} is H, C₁₋₃ alkyl, or halogen,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are both 1 or both 2, and
   R^{L4} and R^{L5} are both H, or the R^{L4} and R^{L5} together with the carbon atom bonded thereto may form cyclopropane.
(12-5-1) The compound or a salt thereof according to (12-3-1),
   wherein A is N,
   X¹ is -O- or -NR^{X1}-,
   provided that when X¹ is -NR^{X1}-, R^{X1} and R⁴ present on the same nitrogen atom, together with the nitrogen atom adjacent thereto, form optionally substituted 4- to 11-membered saturated hetero ring group,
   R¹ is represented by the following formula (II), (III), or (IV): 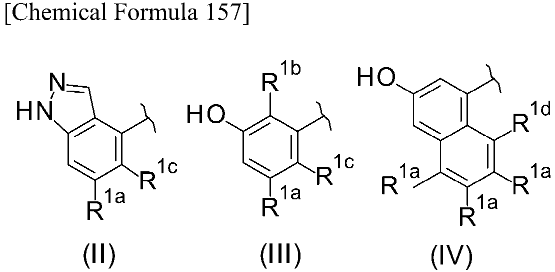
   R^{1a} is the same or different, and is H or F,
   R^{1b} is H,
   R^{1c} is methyl or cyclopropyl,
   R^{1d} is H,
   R² is cyclopropyl,
   R⁴ is C₁₋₆ alkyl or piperidinyl, and the C₁₋₆ alkyl is optionally substituted with a group selected from the group consisting of OH; OCH₃; N(R^{4a})₂; C₃₋₆ cycloalkyl optionally substituted with R^{4b}; pyrrolidinyl optionally substituted with R^{4b}; and morpholinyl optionally substituted with R^{4b}, and the piperidinyl is optionally substituted with R^{4b},
   R^{4a} is C₁₋₃ alkyl,
   R^{4b} is C₁₋₃ alkyl optionally substituted with a group selected from the group consisting of OH, -O-(C₁₋₃ alkyl) and -N(C₁₋₃ alkyl)₂, or halogen,
   L and Z together are a group selected from the group consisting of the following formulas (XXX), (XXXI-X), (XXXII), (XXXIII-A), (XXXIII-B), (XXXIV), and (XXXI-A): 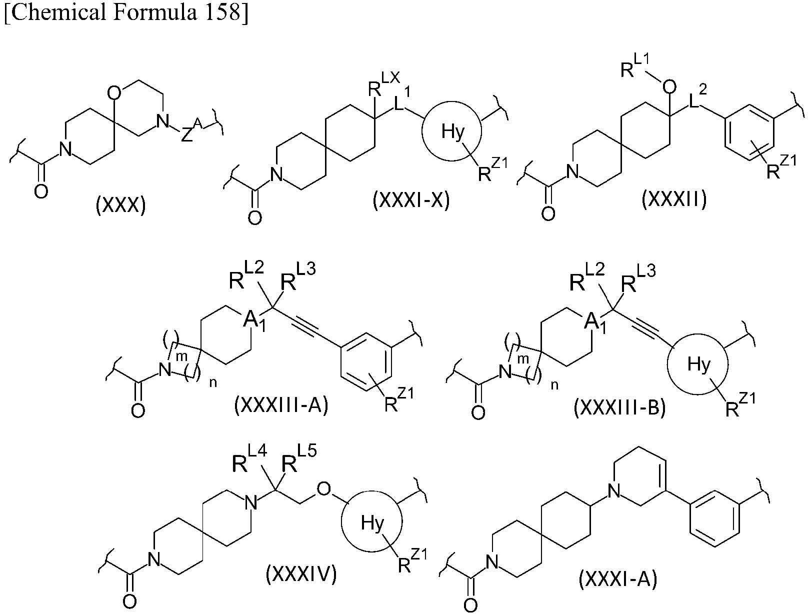
   Z^{A} is a group represented by the following formula (XXII-1) or (XXVI-2): 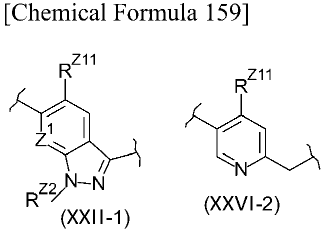
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C=C-,
   R^{LX} is H or OH,
   ring Hy is 5- or 6-membered hetero ring,
   L² is -CH₂-C≡C- or -CH₂CH₂-O-,
   R^{Z1} is H, C₁₋₃ alkyl, or halogen,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are both 1 or both 2, and
   R^{L4} and R^{L5} are both H, or the R^{L4} and R^{L5} together with the carbon atom bonded thereto may form cyclopropane.
(12-6) The compound or a salt thereof according to (12-5) above,
   wherein X¹ is -O- or -NR^{X1}-,
   provided that when X¹ is -NR^{X1}-, R^{X1} and R⁴ present on the same nitrogen atom, together with the nitrogen atom adjacent thereto, form optionally substituted 4- to 6-membered saturated hetero ring group,
   L and Z together are a group selected from the group consisting of the following formulas (XXX), (XXXI-1), (XXXII-1), (XXXIII-A1), (XXXIII-B1), (XXXIV-1), and (XXXI-A): 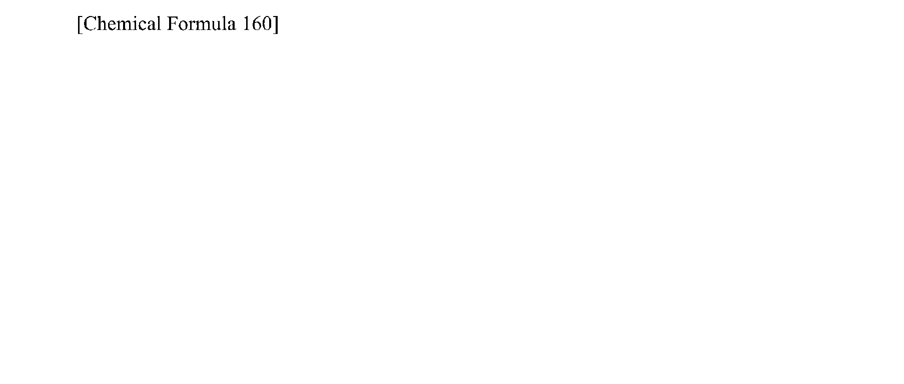 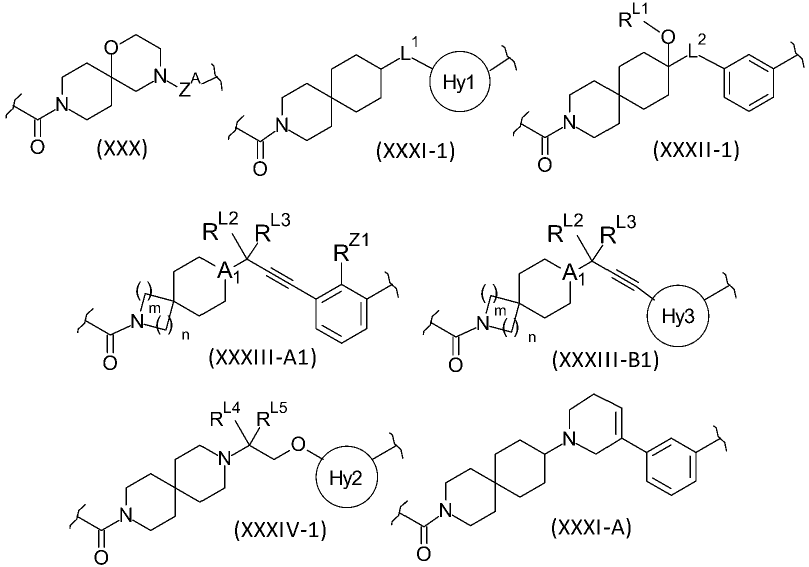
   Z^{A} is a group represented by the following formula (XXII-1) or (XXVI-2): 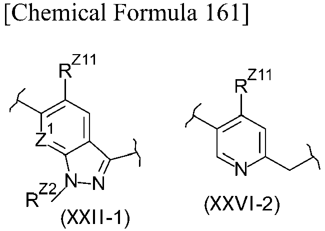
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C=C-,
   ring Hy1 is a ring selected from the group consisting of the following formulas (XXXV), (XXXVI), (XXXVII), (XXXVIII), and (XXXIX): 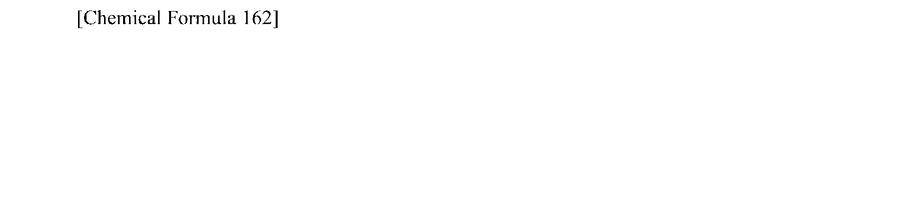 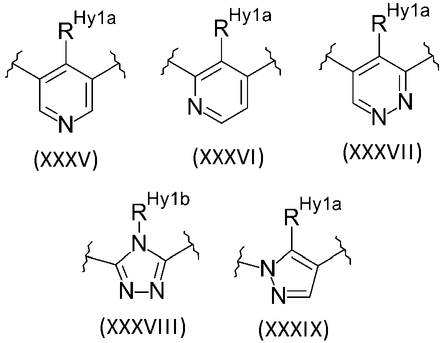
   R^{Hy1a} is H, C₁₋₃ alkyl, or halogen,
   R^{Hy1b} is H or C₁₋₃ alkyl,
   L² is -CH₂-C≡C- or -CH₂CH₂-O-,
   R^{Z1} is H, C₁₋₃ alkyl, or halogen,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 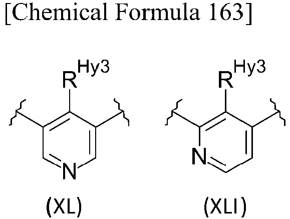
   R^{Hy3} is H, C₁₋₃ alkyl, or halogen,
   m and n are both 1 or both 2,
   ring Hy2 is of the following formula (XLIII): 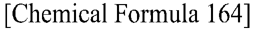 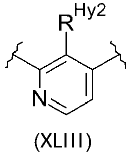
   R^{Hy2} is H, C₁₋₃ alkyl, or halogen, and
   R^{L4} and R^{L5} are both H, or the R^{L4} and R^{L5} together with the carbon atom bonded thereto may form cyclopropane.
(12-6-1) The compound or a salt thereof according to (12-5-1) above,
   wherein X¹ is -O- or -NR^{X1}-,
   provided that when X¹ is -NR^{X1}-, R^{X1} and R⁴ present on the same nitrogen atom, together with the nitrogen atom adjacent thereto, form optionally substituted 4- to 11-membered saturated hetero ring group,
   L and Z together are a group selected from the group consisting of the following formulas (XXX), (XXXI-1X), (XXXII-1), (XXXIII-A1), (XXXIII-B1), (XXXIV-1), and (XXXI-A): 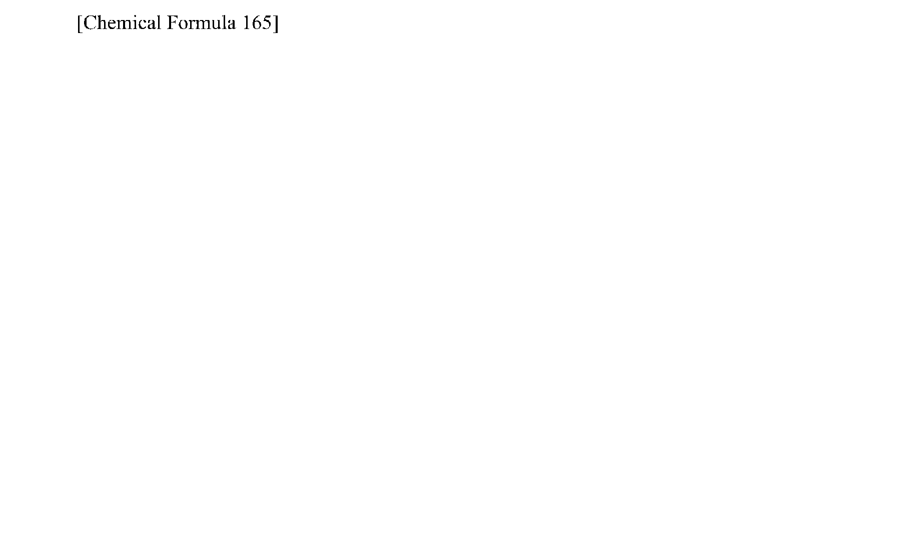 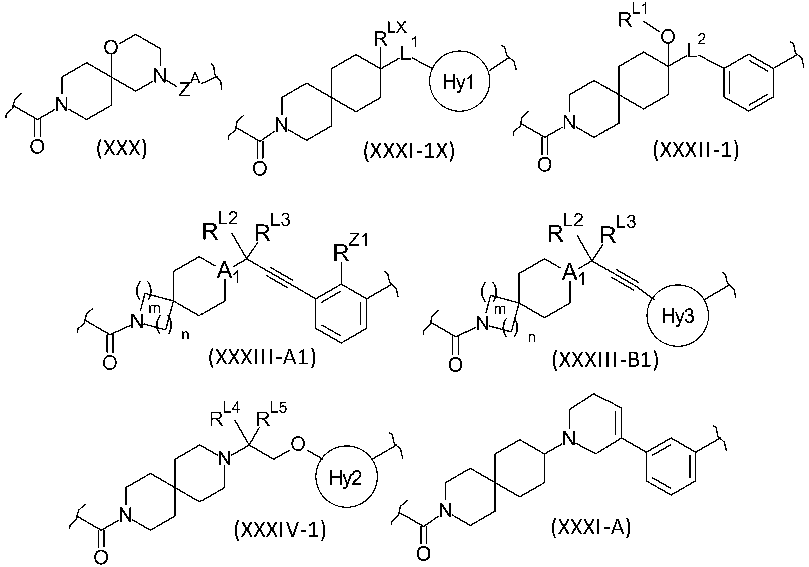
   Z^{A} is a group represented by the following formula (XXII-1) or (XXVI-2): 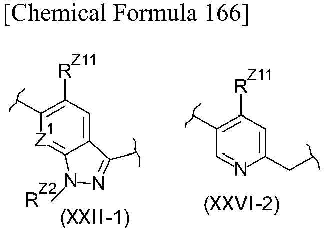
   R^{Z11} is halogen,
   Z¹ is CH or N,
   R^{Z2} is C₁₋₃ alkyl,
   L¹ is bond, C₁₋₃ alkylene, -C₁₋₃ alkylene-O-, or -CH₂-C=C-,
   R^{LX} is H or OH,
   ring Hy1 is a ring selected from the group consisting of the following formulas (XXXV), (XXXVI), (XXXVII), (XXXVII-2), (XXXVIII), and (XXXIX): 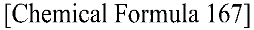 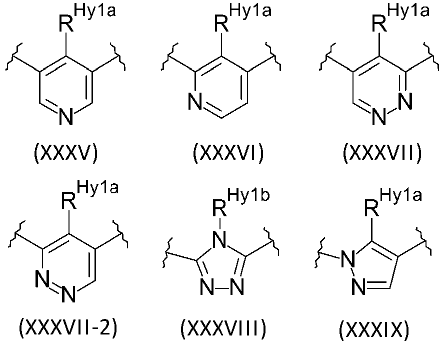
   R^{Hy1a} is H, C₁₋₃ alkyl, or halogen,
   R^{Hy1b} is H or C₁₋₃ alkyl,
   L² is -CH₂-C≡C- or -CH₂CH₂-O-,
   R^{Z1} is H, C₁₋₃ alkyl, or halogen,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 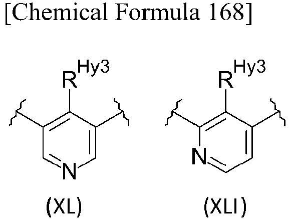
   R^{Hy3} is H, C₁₋₃ alkyl, or halogen,
   m and n are both 1 or both 2,
   ring Hy2 is of the following formula (XLIII): 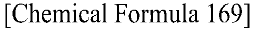 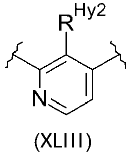
   R^{Hy2} is H, C₁₋₃ alkyl, or halogen, and
   R^{L4} and R^{L5} are both H, or the R^{L4} and R^{L5} together with the carbon atom bonded thereto may form cyclopropane.
(12-7) The compound or a salt thereof according to (12-6) above, wherein L and Z together are a group represented by the formula (XXXI-1): 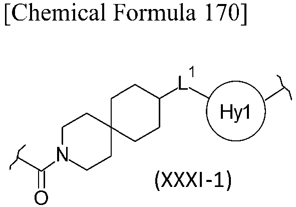
   L¹ is -CH₂CH₂-O- or -CH₂-C=C-,
   ring Hy1 is a ring selected from the group consisting of the following formulas (XXXV) and (XXXVI): 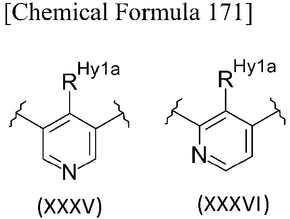
   R^{Hy1a} is H, C₁₋₃ alkyl, or halogen.
(12-7-1) The compound or a salt thereof according to (12-6-1) above, wherein L and Z together are a group represented by the formula (XXXI-1X): 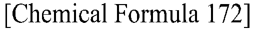 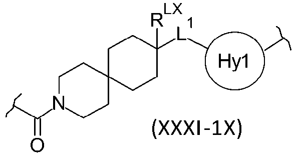
   L¹ is -CH₂CH₂-O- or -CH₂-C=C-,
   R^{LX} is H,
   ring Hy1 is a ring selected from the group consisting of the following formulas (XXXV) and (XXXVI): 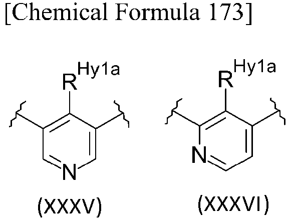
   R^{Hy1a} is H, C₁₋₃ alkyl, or halogen.
(12-8) The compound or a salt thereof according to (12-7-1) above, wherein R¹ is represented by the following formula (II): 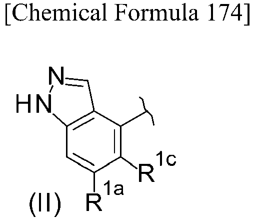
   R^{1a} is F,
   R^{1c} is methyl,
   R⁴ is C₁₋₆ alkyl optionally substituted with N(Me)₂,
   L and Z together are a group represented by formula (XXXI-1A) or (XXXI-1B): 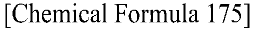 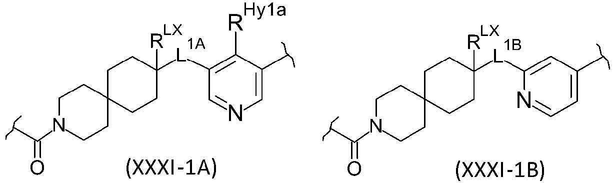
   R^{LX} is H,
   L^{1A} is -CH₂CH₂-O-,
   L^{1B} is -CH₂CH₂-O- or -CH₂-C=C-, and
   R^{Hy1a} is H or C₁₋₃ alkyl.
(12-9) The compound or a salt thereof according to (12-1) above,
   wherein E is CH,
   R³ is a group represented by formula (XIV): 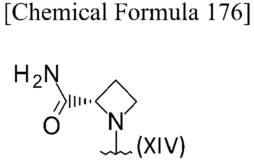
   Y is phenylene,
   L together with Z forms a group selected from the group consisting of the following formulas (XXXI-X), (XXXIII-A), (XXXIII-B), (XXXIV), and (XXXI-A): 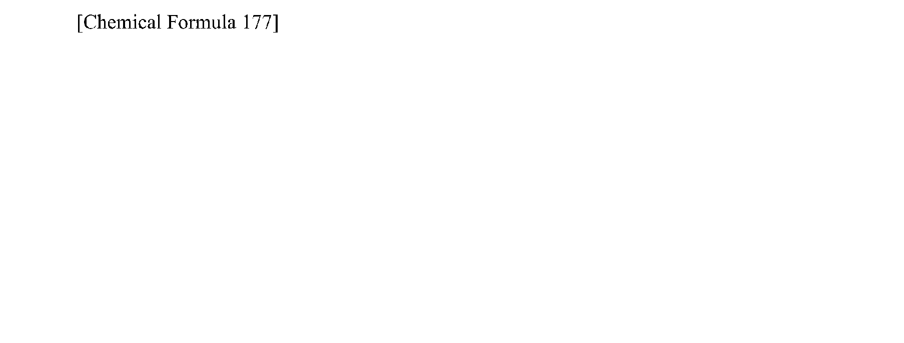 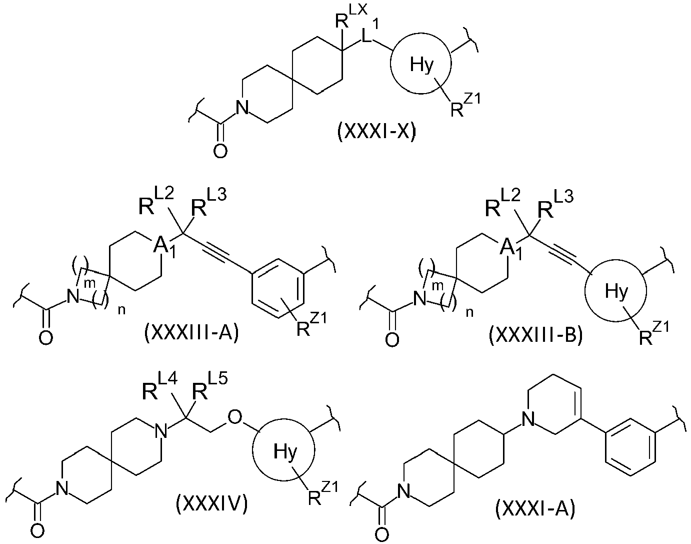
   L¹ is bond, C₁₋₃ alkylene, -C₁₋₃ alkylene-O-, -C₁₋₃ alkylene-C₂₋₃ alkynediyl, or -C≡C-,
   R^{LX} is H or OH,
   ring Hy is 5- or 6-membered hetero ring,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   R^{Z1} is H, C₁₋₃ alkyl, or halogen,
   m and n are the same or different, and are each 1 or 2,
   R^{L4} and R^{L5} are the same or different, and are each H or C₁₋₃ alkyl, or the R^{L4} and R^{L5} together with the carbon atom bonded thereto may form C₃₋₆ cycloalkane, or the R^{L4} and R^{L5} together with the carbon atom bonded thereto may form carbonyl, and
   G is N.,
(12-10) The compound or a salt thereof according to (12-9) above,
   wherein A is N,
   X¹ is -O- or -NR^{X1}-,
   R^{X1} is H or C₁₋₃ alkyl,
   provided that when X¹ is -NR^{X1}-, R^{X1} and R⁴ present on the same nitrogen atom, together with the nitrogen atom adjacent thereto, may form optionally substituted 4- to 11-membered saturated hetero ring group,
   R⁴ is C₁₋₆ alkyl or piperidinyl, and the C₁₋₆ alkyl is optionally substituted with a group selected from the group consisting of F; OH; OCH₃; N(R^{4a})₂; C₃₋₆ cycloalkyl optionally substituted with R^{4b}; pyrrolidinyl optionally substituted with R^{4b}, and morpholinyl optionally substituted with R^{4b}, and the piperidinyl is optionally substituted with R^{4b},
   R^{4a} is optionally substituted C₁₋₃ alkyl, and
   R^{4b} is optionally substituted C₁₋₃ alkyl, or halogen.
(12-11) The compound or a salt thereof according to (12-9),
   wherein A is N,
   X¹ is -O- or -NR^{X1}-,
   provided that when X¹ is -NR^{X1}-, R^{X1} and R⁴ present on the same nitrogen atom, together with the nitrogen atom adjacent thereto, form optionally substituted 4- to 11-membered saturated hetero ring group,
   R¹ is represented by the following formula (II), (III), or (IV): 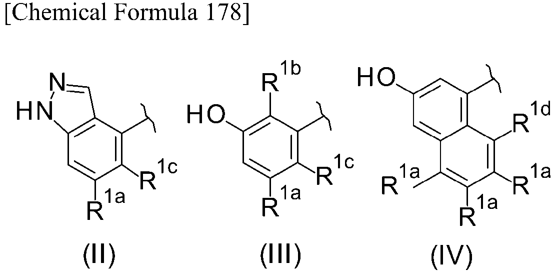
   R^{1a} is the same or different, and is H or F,
   R^{1b} is H,
   R^{1c} is methyl or cyclopropyl,
   R^{1d} is H,
   R² is cyclopropyl,
   R⁴ is C₁₋₆ alkyl or piperidinyl, and the C₁₋₆ alkyl is optionally substituted with a group selected from the group consisting of OH; OCH₃; N(R^{4a})₂; C₃₋₆ cycloalkyl optionally substituted with R^{4b}; pyrrolidinyl optionally substituted with R^{4b}; and morpholinyl optionally substituted with R^{4b}, and the piperidinyl is optionally substituted with R^{4b},
   R^{4a} is C₁₋₃ alkyl,
   R^{4b} is C₁₋₃ alkyl optionally substituted with OH, -O-(C₁₋₃ alkyl), or -N(C₁₋₃ alkyl)₂, or halogen,
   L and Z together are a group selected from the group consisting of the following formulas (XXXI-X), (XXXIII-A), (XXXIII-B), (XXXIV), and (XXXI-A): 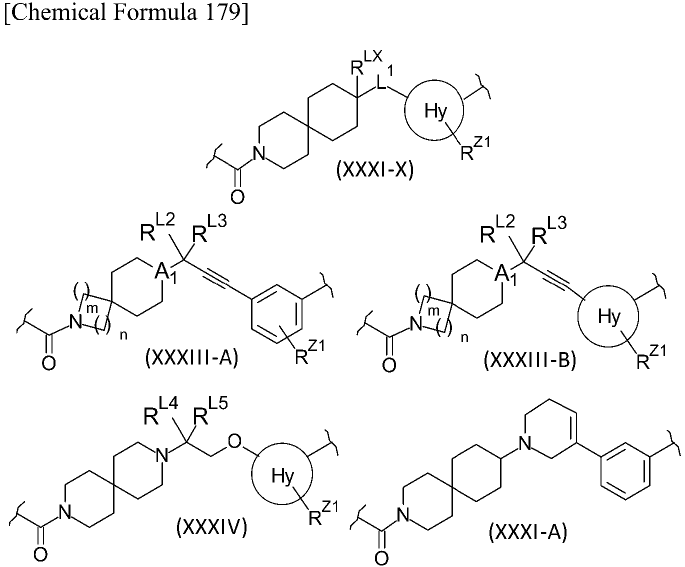
   L¹ is bond, C₁₋₃ alkylene, -C₁₋₃ alkylene-O-, or -CH₂-C=C-,
   R^{LX} is H or OH,
   ring Hy is 5- or 6-membered hetero ring,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   R^{Z1} is H, C₁₋₃ alkyl, or halogen,
   m and n are both 1 or both 2, and
   R^{L4} and R^{L5} are both H, or the R^{L4} and R^{L5} together with the carbon atom bonded thereto may form cyclopropane.
(12-12) The compound or a salt thereof according to (12-11) above,
   wherein X¹ is -O- or -NR^{X1}-,
   provided that when X¹ is -NR^{X1}-, R^{X1} and R⁴ present on the same nitrogen atom, together with the nitrogen atom adjacent thereto, form optionally substituted 4- to 11-membered saturated hetero ring group,
   L and Z together are a group selected from the group consisting of the following formulas (XXXI-1X), (XXXIII-A1), (XXXIII-B1), (XXXIV-1), and (XXXI-A): 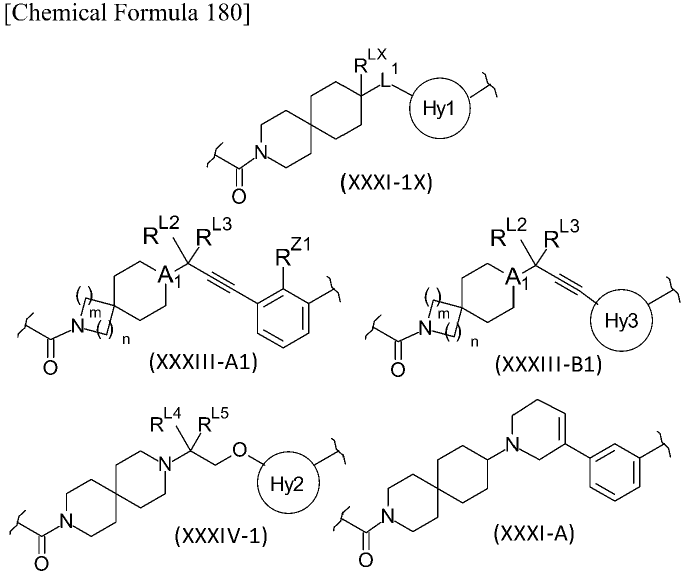
   L¹ is bond, C₁₋₃ alkylene, -C₁₋₃ alkylene-O-, or -CH₂-C=C-,
   R^{LX} is H or OH,
   ring Hy1 is a ring selected from the group consisting of the following formulas (XXXV), (XXXVI), (XXXVII), (XXXVII-2), (XXXVIII), and (XXXIX): 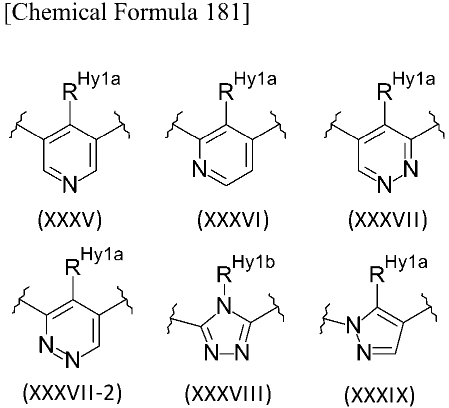
   R^{Hy1a} is H, C₁₋₃ alkyl, or halogen,
   R^{Hy1b} is H or C₁₋₃ alkyl,
   R^{Z1} is H, C₁₋₃ alkyl, or halogen,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 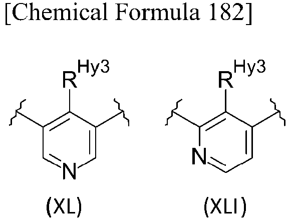
   R^{Hy3} is H, C₁₋₃ alkyl, or halogen,
   m and n are both 1 or both 2,
   ring Hy2 is of the following formula (XLIII): 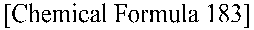 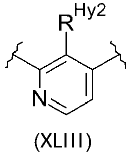
   R^{Hy2} is H, C₁₋₃ alkyl, or halogen, and
   R^{L4} and R^{L5} are both H, or the R^{L4} and R^{L5} together with the carbon atom bonded thereto may form cyclopropane.
(12-13) The compound or a salt thereof according to (12-12) above,
   L and Z together are a group selected from the group consisting of the following formulas (XXXI-1X), (XXXIII-A1), (XXXIII-B1), and (XXXIV-1): 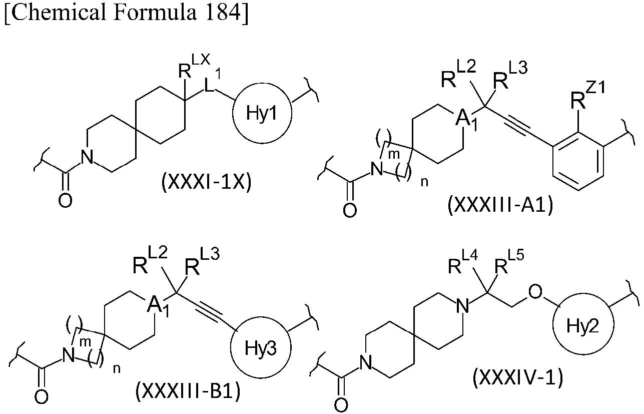
   L¹ is bond, C₁₋₃ alkylene, -C₁₋₃ alkylene-O-, or -CH₂-C=C-,
   R^{LX} is H or OH,
   ring Hy1 is a ring selected from the group consisting of the following formulas (XXXV), (XXXVI), (XXXVII), (XXXVII-2), (XXXVIII), and (XXXIX): 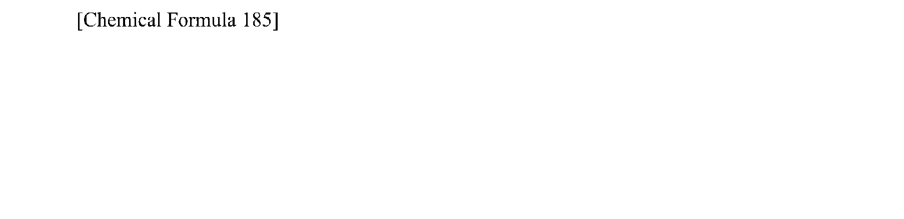 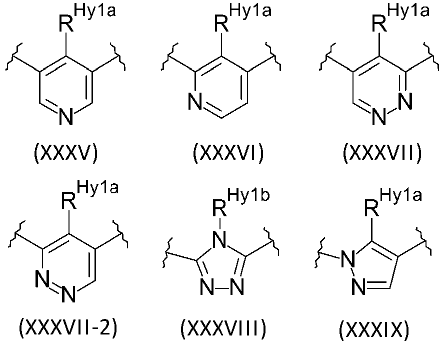
   R^{Hy1a} is H, C₁₋₃ alkyl, or halogen,
   R^{Hy1b} is H or C₁₋₃ alkyl,
   R^{Z1} is H or halogen,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   ring Hy3 is a ring selected from the group consisting of the following formulas (XL) and (XLI): 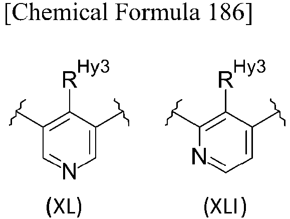
   R^{Hy3} is H or halogen,
   m and n are both 1 or both 2,
   ring Hy2 is of the following formula (XLIII): 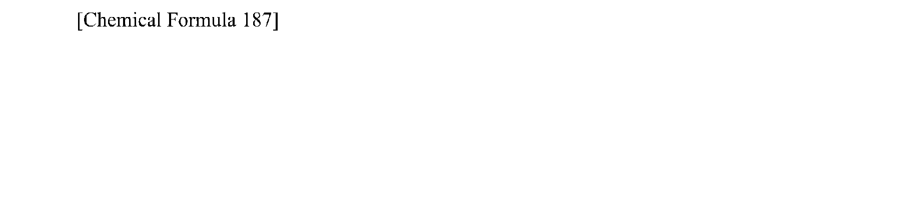 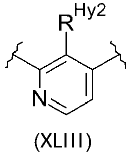
   R^{Hy2} is H or C₁₋₃ alkyl, and
   R^{L4} and R^{L5} are both H, or the R^{L4} and R^{L5} together with the carbon atom bonded thereto may form cyclopropane.
(12-14) The compound or a salt thereof according to (12-1), wherein the compound represented by formula (I) is any one of Examples 1 to 41 below.
(12-15) The compound or a salt thereof according to (12-1),
   wherein the compound represented by formula (I) is selected from the group consisting of:
   (2S)-1-[(7M)-6-cyclopropyl-2-[3-(dimethylamino)-2,2-dimethylpropoxy]-8-({4-[9-(2-{[4-(2,4-dioxo-1,3-diazinan-1-yl)pyridin-2-yl]oxy}ethyl)-3-azaspiro[5.5]undecane-3-carbonyl]phenyl}methoxy)-7-(6-fluoro-5-methyl-1H-indazol-4-yl)quinazolin-4-yl]azetidine-2-carboxamide,
   (2S)-1-[(7M)-6-cyclopropyl-2-[(3S)-3,4-dimethylpiperazin-1-yl]-8-({4-[9-(2-{[5-(2,4-dioxo-1,3-diazinan-1-yl)pyridin-3-yl]oxy}ethyl)-3-azaspiro[5.5]undecane-3-carbonyl]phenyl}methoxy)-7-(6-fluoro-5-methyl-1H-indazol-4-yl)quinazolin-4-yl]azetidine-2-carboxamide,
   (2S)-1-[(7M)-6-cyclopropyl-2-[(3S)-3,4-dimethylpiperazin-1-yl]-8-{[4-(9-{3-[4-(2,4-dioxo-1,3-diazinan-1-yl)pyridin-2-yl]prop-2-yn-1-yl}-3-azaspiro[5.5]undecane-3-carbonyl)phenyl]methoxy}-7-(6-fluoro-5-methyl-1H-indazol-4-yl)quinazolin-4-yl]azetidine-2-carboxamide,
   (2S)-1-[(7M)-6-cyclopropyl-2-[(3S)-3,4-dimethylpiperazin-1-yl]-8-({4-[9-(2-{[5-(2,4-dioxo-1,3-diazinan-1-yl)-4-methylpyridin-3-yl]oxy}ethyl)-3-azaspiro[5.5]undecane-3-carbonyl]phenyl}methoxy)-7-(6-fluoro-5-methyl-1H-indazol-4-yl)quinazolin-4-yl]azetidine-2-carboxamide, and
   (2S)-1-[(7M)-6-cyclopropyl-8-({4-[9-(2-{[5-(2,4-dioxo-1,3-diazinan-1-yl)pyridin-3-yl]oxy}ethyl)-3-azaspiro[5.5]undecane-3-carbonyl]phenyl}methoxy)-2-[(3S)-4-ethyl-3-methylpiperazin-1-yl]-7-(6-fluoro-5-methyl-1H-indazol-4-yl)quinazolin-4-yl]azetidine-2-carboxamide.
(12-16) The compound or a salt thereof according to (12-1), wherein the compound represented by formula (I) is selected from the group consisting of:
   (2S)-1-[(7M)-6-cyclopropyl-2-[3-(dimethylamino)-2,2-dimethylpropoxy]-8-({4-[9-(2-{[4-(2,4-dioxo-1,3-diazinan-1-yl)pyridin-2-yl]oxy}ethyl)-3-azaspiro[5.5]undecane-3-carbonyl]phenyl}methoxy)-7-(6-fluoro-5-methyl-1H-indazol-4-yl)quinazolin-4-yl]azetidine-2-carboxamide,
   (2S)-1-[(7M)-6-cyclopropyl-2-[(3S)-3,4-dimethylpiperazin-1-yl]-8-({4-[9-(2-{[5-(2,4-dioxo-1,3-diazinan-1-yl)pyridin-3-yl]oxy} ethyl)-3-azaspiro[5.5]undecane-3-carbonyl]phenyl}methoxy)-7-(6-fluoro-5-methyl-1H-indazol-4-yl)quinazolin-4-yl]azetidine-2-carboxamide, and
   (2S)-1-[(7M)-6-cyclopropyl-8-({4-[9-(2-{[5-(2,4-dioxo-1,3-diazinan-1-yl)pyridin-3-yl]oxy}ethyl)-3-azaspiro[5.5]undecane-3-carbonyl]phenyl}methoxy)-2-[(3S)-4-ethyl-3-methylpiperazin-1-yl]-7-(6-fluoro-5-methyl-1H-indazol-4-yl)quinazolin-4-yl]azetidine-2-carboxamide.

One aspect of the compound represented by formula (I-I) or a salt thereof according to the present invention will be shown below.
(13-1) A compound represented by formula (I-I) or a salt thereof, wherein TPB is a group having an ability to bind to target protein.
(13-1-1) The compound represented by formula (I-I) or a salt thereof, wherein TPB is a group having an ability to bind to RAS protein.
(13-2) The compound represented by formula (I-I) or a salt thereof, wherein TPB is the following formula (I-II), (I-III), or (I-IV): 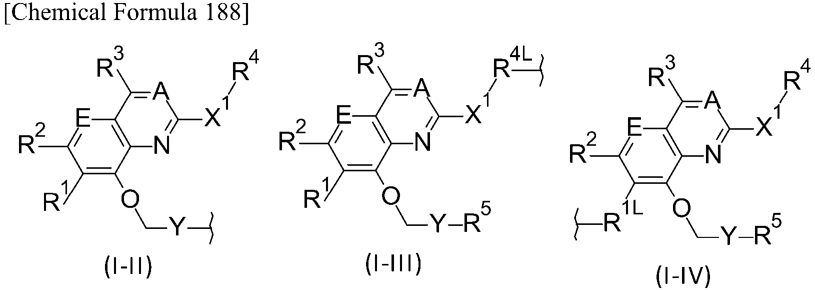
   wherein A, R^{A}, E, X¹, R^{X1}, R¹, R^{1a}, R^{1b}, R^{1c}, R^{1d}, R², R³, R^{3a}, R^{3b}, R^{3c}, R^{3d}, R^{3e}, R^{3f}, R^{3g}, R^{3h}, R³ⁱ, R^{N1}, R^{N2}, R^{3j}, X², X³, X⁴, n¹, p, q, R⁴, and Y are defined as in formula (I),
   R⁵ is H, CONR⁶R⁷, or a group selected from the group consisting of the following formulas (I-V), (I-VI), (I-VII), (I-VIII), (I-IX), (I-X), (I-XI), (I-XII), (I-XIII), (I-XIV), (I-XV), and (I-XVI): 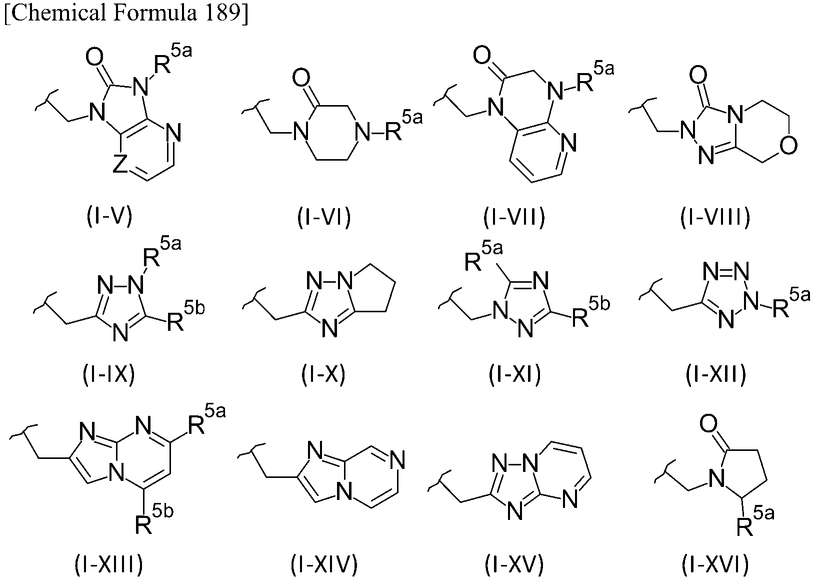
   R^{5a} and R^{5b} are the same or different, and are each H, optionally substituted C₁₋₃ alkyl, cyclopropyl, cyclopropylmethyl, oxetanyl, tetrahydropyranyl, optionally substituted oxazolyl, thiazolyl, or pyrazinyl,
   R⁶ and R⁷ are the same or different, and are each H or optionally substituted C₁₋₆ alkyl; or R⁶ and R⁷ together with a nitrogen atom bonded thereto form a 4- to 6-membered saturated hetero ring group, and the 4- to 6-membered saturated hetero ring group is optionally substituted with optionally substituted C₁₋₆ alkyl,
   R^{4L} is optionally substituted C₁₋₆ alkylene, or divalent group of optionally substituted saturated hetero ring,
   R^{1L} is the following formula (II-I), (III-I), (IV-I), (V-I), (VI-I), or (VII-I): 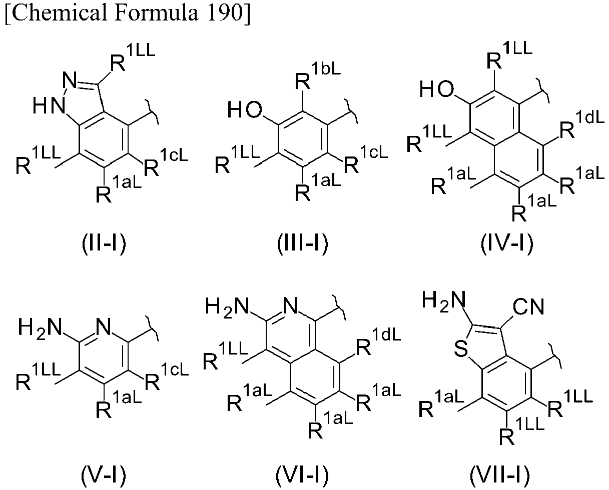
   R^{1aL} and R^{1bL} are the same or different, and are each H, methyl, F, Cl, or an attachment point to L,
   R^{1cL} is F, Cl, methyl, ethyl, trifluoromethyl, cyclopropyl, or an attachment point to L,
   R^{1dL} is H, methyl, ethyl, F, Cl, -C≡C-H, or an attachment point to L, and
   R^{1LL} is H or an attachment point to L,
   provided that only one attachment point to L is present in the above formula (II-I), (III-I), (IV-I), (V-I), (VI-I), or (VII-I).
(13-2-1) A compound represented by formula (I-I) or a salt thereof,
   wherein TPB is the following formula (I-II), (I-III-1), or (I-IV-1): 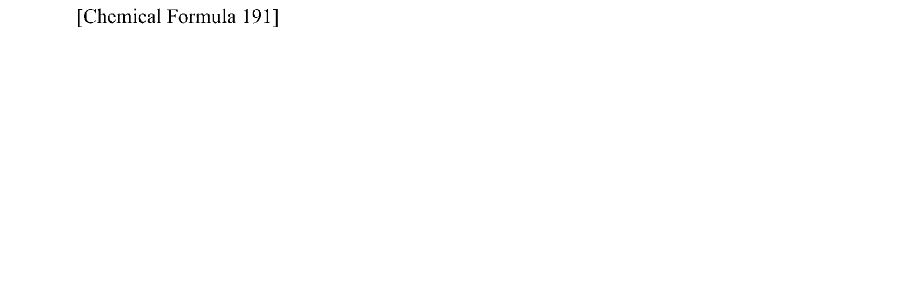 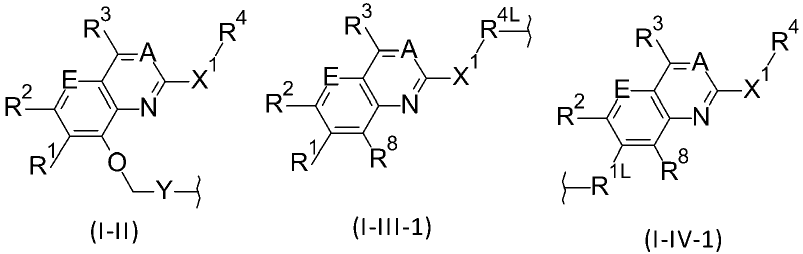
   wherein A, R^{A}, E, X¹, R^{X1}, R¹, R^{1a}, R^{1b}, R^{1c}, R^{1d}, R², R³, R^{3a}, R^{3b}, R^{3c}, R^{3d}, R^{3e}, R^{3f}, R^{3g}, R^{3h}, R³ⁱ, R^{N1}, R^{N2}, R^{3j}, X², X³, X⁴, n¹, p, q, and R⁴ are defined as in formula (I),
   R⁸ is H, halogen, -O-C₁₋₃ alkyl, or -O-CH₂-Y-R⁵,
   Y is defined as in formula (I),
   R⁵ is H, CONR⁶R⁷, or a group selected from the group consisting of the following formulas (I-V), (I-VI), (I-VII), (I-VIII), (I-IX), (I-X), (I-XI), (I-XII), (I-XIII), (I-XIV), (I-XV), and (I-XVI): 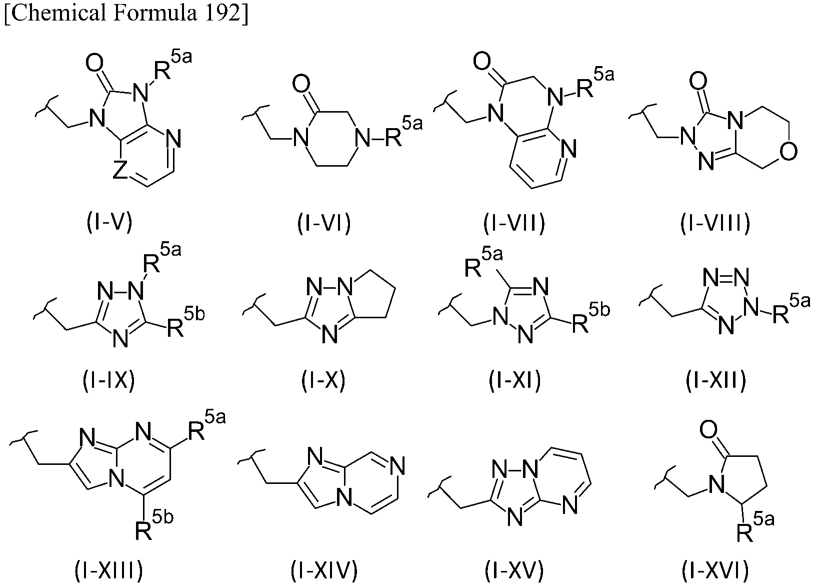
   R^{5a} and R^{5b} are the same or different, and are each H, optionally substituted C₁₋₃ alkyl, cyclopropyl, cyclopropylmethyl, oxetanyl, tetrahydropyranyl, optionally substituted oxazolyl, thiazolyl, or pyrazinyl,
   R⁶ and R⁷ are the same or different, and are each H or optionally substituted C₁₋₆ alkyl, or R⁶ and R⁷, together with a nitrogen atom bonded thereto, form 4- to 6-membered saturated hetero ring group, and the 4- to 6-membered saturated hetero ring group is optionally substituted with optionally substituted C₁₋₆ alkyl,
   R^{4L} is optionally substituted C₁₋₆ alkylene, or divalent group of optionally substituted saturated hetero ring,
   R^{1L} is the following formula (II-I), (III-I), (IV-I), (V-I), (VI-I), or (VII-I): 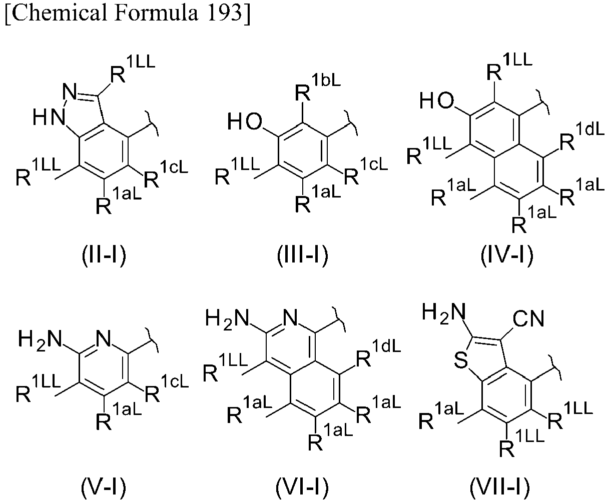
   R^{1aL} is the same or different, and is H, methyl, F, Cl, or an attachment point to L,
   R^{1bL} is H, methyl, F, Cl, or an attachment point to L,
   R^{1cL} is F, Cl, methyl, ethyl, trifluoromethyl, cyclopropyl, or an attachment point to L,
   R^{1dL} is H, methyl, ethyl, F, Cl, -C=C-H, or an attachment point to L, and
   R^{1LL} is the same or different, and is H or an attachment point to L,
   provided that only one attachment point to L is present in the above formula (II-I), (III-I), (IV-I), (V-I), (VI-I), or (VII-I).
(13-3) A compound represented by formula (I-I) or a salt thereof, wherein TPB is the following formula (I-II): 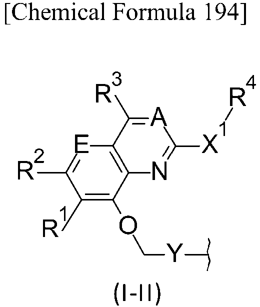 wherein A, R^{A}, E, X¹, R^{X1}, R¹, R^{1a}, R^{1b}, R^{1c}, R^{1d}, R², R³, R^{3a}, R^{3b}, R^{3c}, R^{3d}, R^{3e}, R^{3f}, R^{3g}, R^{3h}, R³ⁱ, R^{N1}, R^{N2}, R^{3j}, X², X³, X⁴, n¹, p, q, R⁴, and Y are defined as in formula (I).
(13-4) A compound represented by formula (I-I) or a salt thereof, wherein TPB is the following formula (I-III): 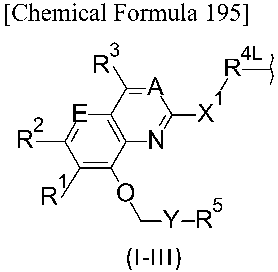
   wherein A, R^{A}, E, X¹, R^{X1}, R¹, R^{1a}, R^{1b}, R^{1c}, R^{1d}, R², R³, R^{3a}, R^{3b}, R^{3e}, R^{3d}, R^{3e}, R^{3f}, R^{3g}, R^{3h}, R³ⁱ, R^{N1}, R^{N2}, R^{3j}, X², X³, X⁴, n¹, p, q, and Y are defined as in formula (I),
   R⁵ is H, CONR⁶R⁷, or a group selected from the group consisting of the following formulas (I-V), (I-VI), (I-VII), (I-VIII), (I-IX), (I-X), (I-XI), (I-XII), (I-XIII), (I-XIV), (I-XV), and (I-XVI): 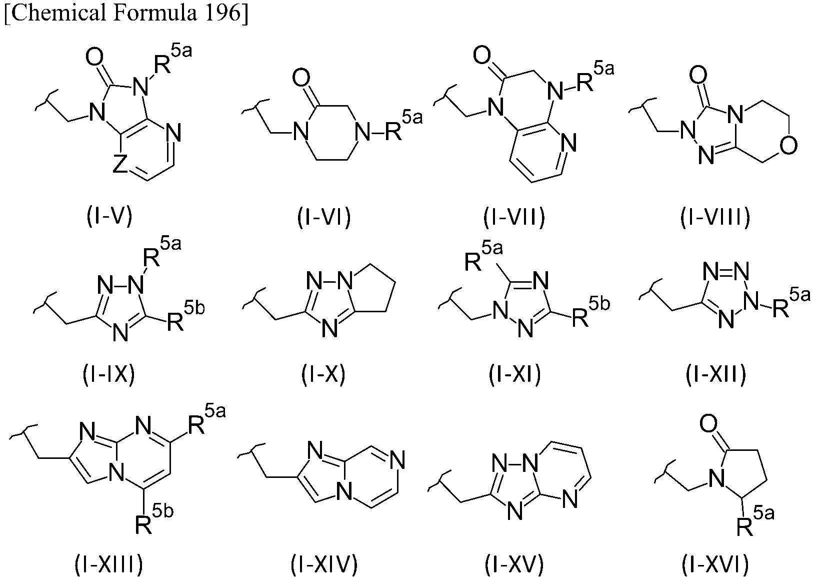
   R^{5a} and R^{5b} are the same or different, and are each H, optionally substituted C₁₋₃ alkyl, cyclopropyl, cyclopropylmethyl, oxetanyl, tetrahydropyranyl, optionally substituted oxazolyl, thiazolyl, or pyrazinyl,
   R⁶ and R⁷ are the same or different, and are each H or optionally substituted C₁₋₆ alkyl, or R⁶ and R⁷, together with a nitrogen atom bonded thereto, form 4- to 6-membered saturated hetero ring group, and the 4- to 6-membered saturated hetero ring group is optionally substituted with optionally substituted C₁₋₆ alkyl, and
   R^{4L} is optionally substituted C₁₋₆ alkylene, or divalent group of optionally substituted saturated hetero ring.
(13-4-1) A compound represented by formula (I-I) or a salt thereof, wherein TPB is the following formula (I-III-1): 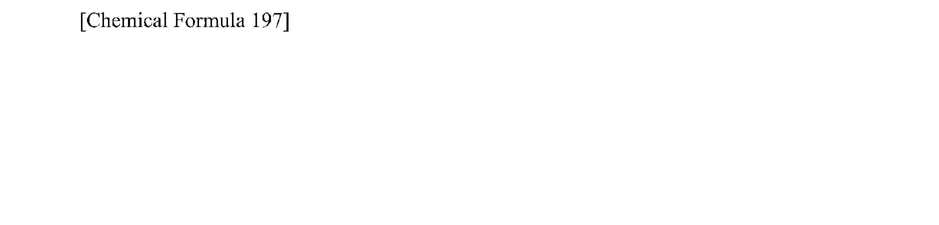 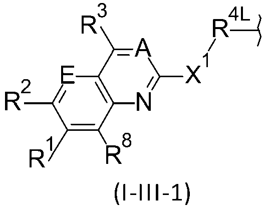
   wherein A, R^{A}, E, X¹, R^{X1}, R¹, R^{1a}, R^{1b}, R^{1c}, R^{1d}, R², R³, R^{3a}, R^{3b}, R^{3c}, R^{3d}, R^{3e}, R^{3f}, R^{3g}, R^{3h}, R³ⁱ, R^{N1}, R^{N2}, R^{3j}, X², X³, X⁴, n¹, p, and q are defined as in formula (I),
   R⁸ is H, halogen, -O-C₁₋₃ alkyl, or -O-CH₂-Y-R⁵,
   Y is defined as in formula (I),
   R⁵ is H, CONR⁶R⁷, or a group selected from the group consisting of the following formulas (I-V), (I-VI), (I-VII), (I-VIII), (I-IX), (I-X), (I-XI), (I-XII), (I-XIII), (I-XIV), (I-XV), and (I-XVI):

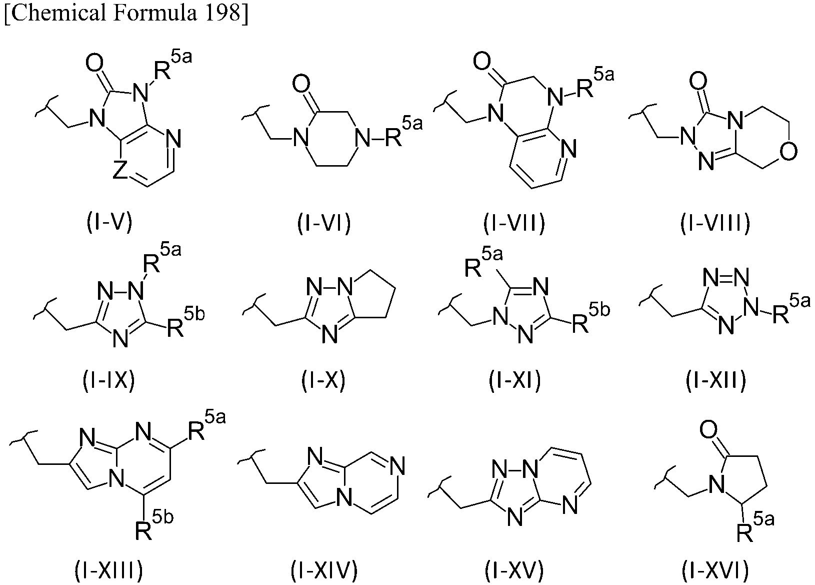

   R^{5a} and R^{5b} are the same or different, and are each H, optionally substituted C₁₋₃ alkyl, cyclopropyl, cyclopropylmethyl, oxetanyl, tetrahydropyranyl, optionally substituted oxazolyl, thiazolyl, or pyrazinyl,
   R⁶ and R⁷ are the same or different, and are each H or optionally substituted C₁₋₆ alkyl, or R⁶ and R⁷, together with a nitrogen atom bonded thereto, form 4- to 6-membered saturated hetero ring group, and the 4- to 6-membered saturated hetero ring group is optionally substituted with optionally substituted C₁₋₆ alkyl, and
   R^{4L} is optionally substituted C₁₋₆ alkylene, or divalent group of optionally substituted saturated hetero ring.
(13-5) A compound represented by formula (I-I) or a salt thereof, wherein TPB is the following formula (I-IV): 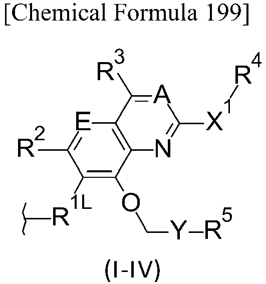
   wherein A, R^{A}, E, X¹, R^{X1}, R², R³, R^{3a}, R^{3b}, R^{3c}, R^{3d}, R^{3e}, R^{3f}, R^{3g}, R^{3h}, R³ⁱ, R^{N1}, R^{N2}, R^{3j}, X², X³, X⁴, n¹, p, q, R⁴, and Y are defined as in formula (I),
   R⁵ is H, CONR⁶R⁷, or a group selected from the group consisting of the following formulas (I-V), (I-VI), (I-VII), (I-VIII), (I-IX), (I-X), (I-XI), (I-XII), (I-XIII), (I-XIV), (I-XV), and (I-XVI): 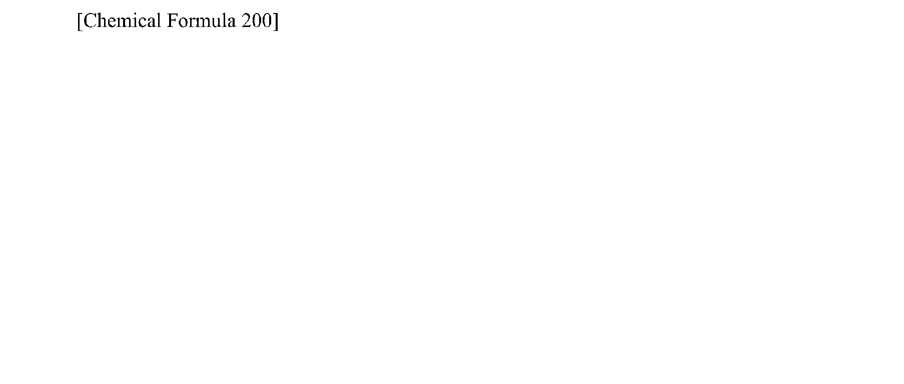 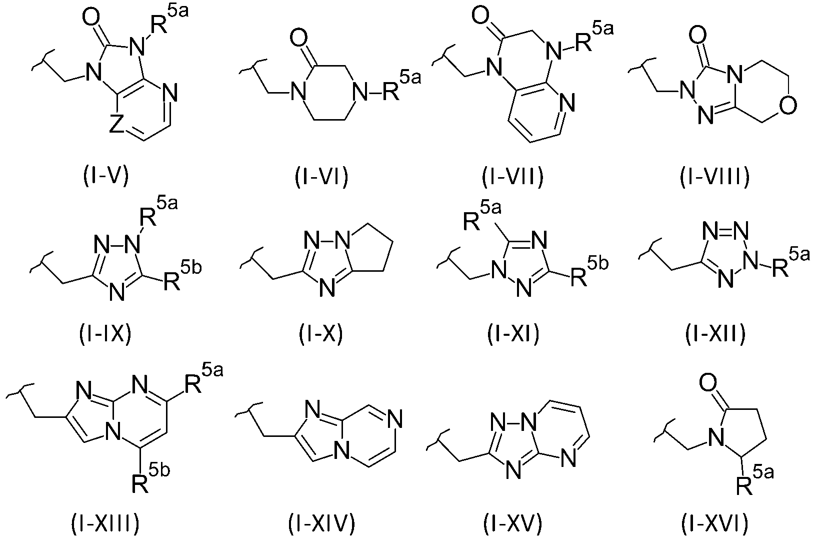
   R^{5a} and R^{5b} are the same or different, and are each H, optionally substituted C₁₋₃ alkyl, cyclopropyl, cyclopropylmethyl, oxetanyl, tetrahydropyranyl, optionally substituted oxazolyl, thiazolyl, or pyrazinyl,
   R⁶ and R⁷ are the same or different, and are each H or optionally substituted C₁₋₆ alkyl, or R⁶ and R⁷, together with a nitrogen atom bonded thereto, form 4- to 6-membered saturated hetero ring group, and the 4- to 6-membered saturated hetero ring group is optionally substituted with optionally substituted C₁₋₆ alkyl,
   R^{1L} is the following formula (II-I), (III-I), (IV-I), (V-I), (VI-I), or (VII-I): 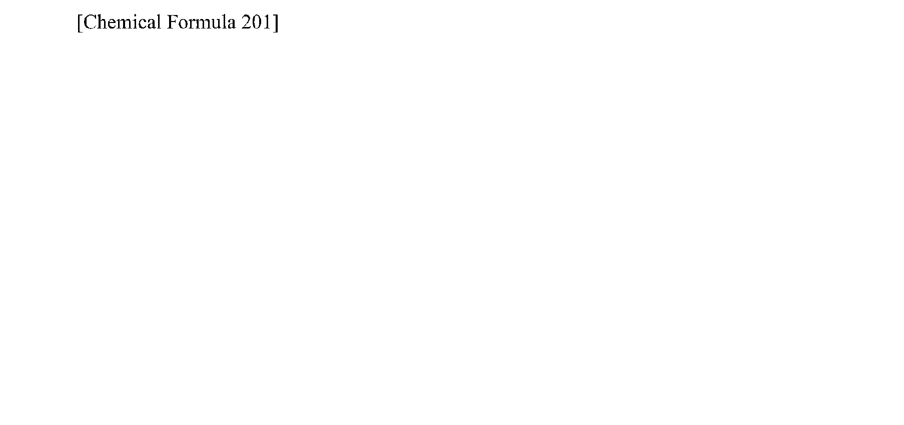 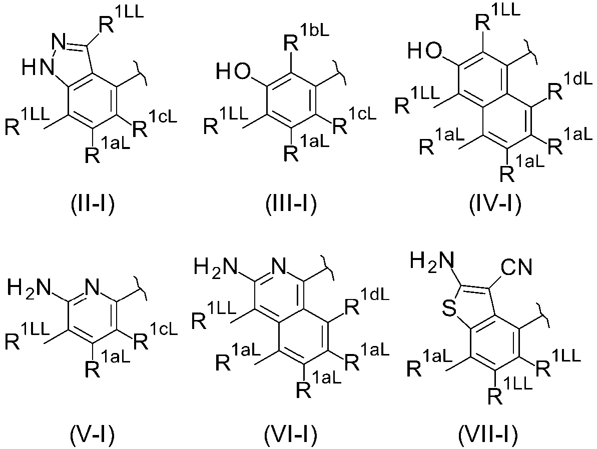
   R^{1aL} and R^{1bL} are the same or different, and are each H, methyl, F, Cl, or an attachment point to L,
   R^{1cL} is F, Cl, methyl, ethyl, trifluoromethyl, cyclopropyl, or an attachment point to L,
   R^{1dL} is H, methyl, ethyl, F, Cl, -C≡C-H, or an attachment point to L, and
   R^{1LL} is H or an attachment point to L,
   provided that only one attachment point to L is present in the above formula (II-I), (III-I), (IV-I), (V-I), (VI-I), or (VII-I).
(13-5-1) A compound represented by formula (I-I) or a salt thereof, wherein TPB is the following formula (I-IV-1): 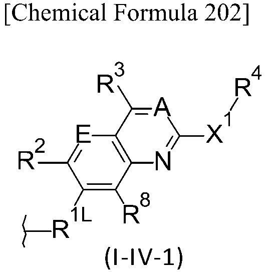
   wherein A, R^{A}, E, X¹, R^{X1}, R², R³, R^{3a}, R^{3b}, R^{3c}, R^{3d}, R^{3e}, R^{3f}, R^{3g}, R^{3h}, R³ⁱ, R^{N1}, R^{N2}, R^{3j}, X², X³, X⁴, n¹, p, q, and R⁴ are defined as in formula (I),
   R⁸ is H, halogen, -O-C₁₋₃ alkyl, or -O-CH₂-Y-R⁵,
   Y is defined as in formula (I),
   R⁵ is H, CONR⁶R⁷, or a group selected from the group consisting of the following formulas (I-V), (I-VI), (I-VII), (I-VIII), (I-IX), (I-X), (I-XI), (I-XII), (I-XIII), (I-XIV), (I-XV), and (I-XVI): 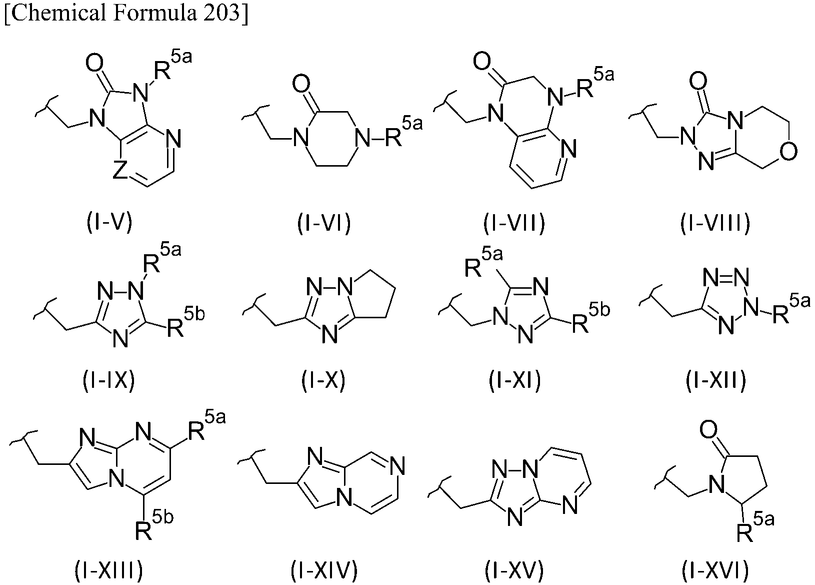
   R^{5a} and R^{5b} are the same or different, and are each H, optionallysubstituted C₁₋₃ alkyl, cyclopropyl, cyclopropylmethyl, oxetanyl, tetrahydropyranyl, optionally substituted oxazolyl, thiazolyl, or pyrazinyl,
   R⁶ and R⁷ are the same or different, and are each H or optionally substituted C₁₋₆ alkyl, or R⁶ and R⁷, together with a nitrogen atom bonded thereto, form 4- to 6-membered saturated hetero ring group, and the 4- to 6-membered saturated hetero ring group is optionally substituted with optionally substituted C₁₋₆ alkyl,
   R^{1L} is the following formula (II-I), (III-I), (IV-I), (V-I), (VI-I), or (VII-I): 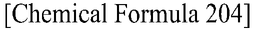 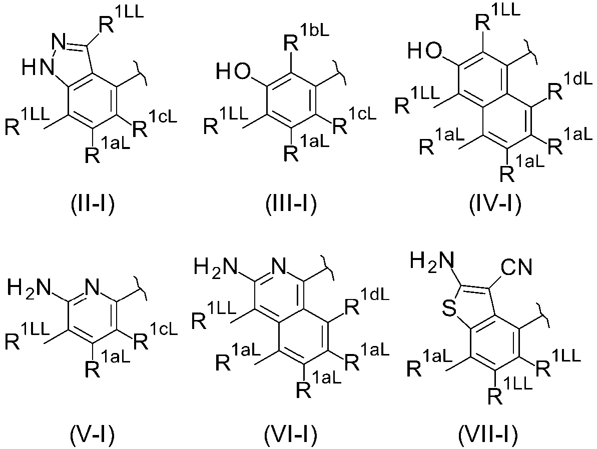
   R^{1aL} is the same or different, and is H, methyl, F, Cl, or an attachment point to L,
   R^{1bL} is H, methyl, F, Cl, or an attachment point to L,
   R^{1cL} is F, Cl, methyl, ethyl, trifluoromethyl, cyclopropyl, or an attachment point to L,
   R^{1dL} is H, methyl, ethyl, F, Cl, -C≡C-H, or an attachment point to L, and
   R^{1LL} is the same or different, and is H or an attachment point to L,
   provided that only one attachment point to L is present in the above formula (II-I), (III-I), (IV-I), (V-I), (VI-I), or (VII-I).
(14-1) A compound represented by formula (I-I) or a salt thereof, wherein L is -(L^{A}-L^{B}-L^{C}-L^{D}-L^{E}-L^{F})-,
   L^{A}, L^{B}, L^{C}, L^{D}, L^{E}, and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O, and
   R^{L1A} is H or C₁₋₃ alkyl.
(14-2) A compound represented by formula (I-I) or a salt thereof, wherein L is a group selected from the group consisting of the following formulas (XXX-L), (XXXI-L), (XXXII-L), (XXXIII-L), and (XXXIV-L): 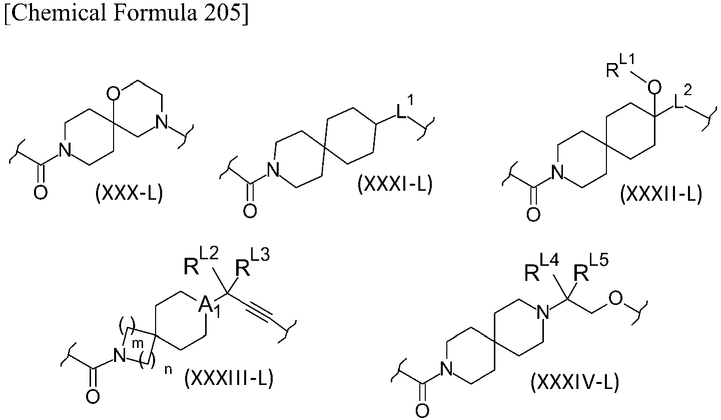
   L¹ is bond, C₁₋₃ alkylene, -C₁₋₃ alkylene-O-, -C₁₋₃ alkylene-C₂₋₃ alkynediyl, -C≡C-, or divalent group of optionally substituted saturated hetero ring,
   L² is -C₁₋₃ alkylene-C₂₋₃ alkynediyl or -C₁₋₃ alkylene-O-,
   R^{L1} is H or C₁₋₃ alkyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are the same or different, and are each 1 or 2, and
   R^{L4} and R^{L5} are the same or different, and are each H or C₁₋₃ alkyl, or the R^{L4} and R^{L5} together with the carbon atom bonded thereto may form C₃₋₆ cycloalkane, or the R^{L4} and R^{L5} together with the carbon atom bonded thereto may form carbonyl.
(14-2-1) A compound represented by formula (I-I) or a salt thereof, wherein L is a group selected from the group consisting of the following formulas (XXX-L), (XXXI-LX), (XXXII-L), (XXXIII-L), and (XXXIV-L): 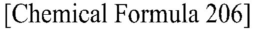 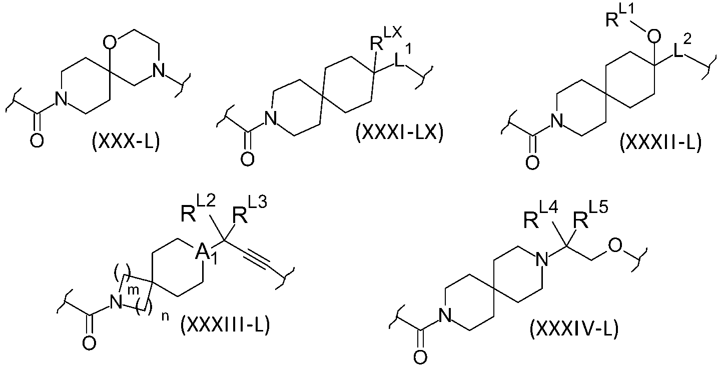
   L¹ is bond, C₁₋₃ alkylene, -C₁₋₃ alkylene-O-, -C₁₋₃ alkylene-C₂₋₃ alkynediyl, -C≡C-, or divalent group of optionally substituted saturated hetero ring,
   R^{LX} is H or OH,
   L² is -C₁₋₃ alkylene-C₂₋₃ alkynediyl or -C₁₋₃ alkylene-O-,
   R^{L1} is H or C₁₋₃ alkyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are the same or different, and are each 1 or 2, and
   R^{L4} and R^{L5} are the same or different, and are each H or C₁₋₃ alkyl, or the R^{L4} and R^{L5} together with the carbon atom bonded thereto may form C₃₋₆ cycloalkane, or the R^{L4} and R^{L5} together with the carbon atom bonded thereto may form carbonyl.
(14-3) A compound represented by formula (I-I) or a salt thereof, wherein L is a group selected from the group consisting of the following formulas (XXX-L), (XXXI-L), (XXXII-L), (XXXIII-L), and (XXXIV-L): 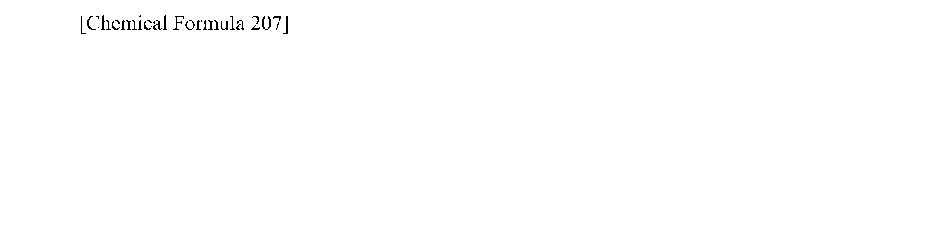 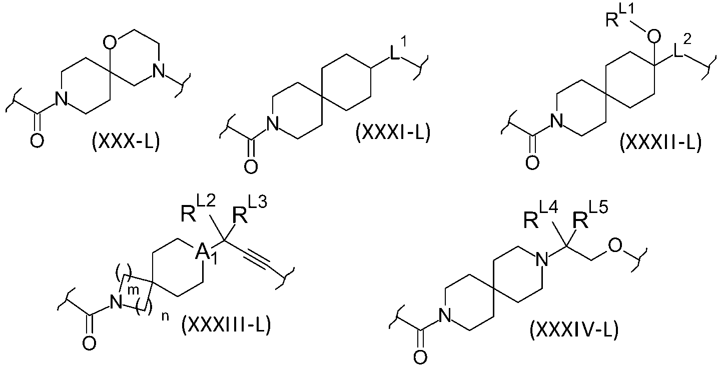
   L¹ is bond, C₁₋₃ alkylene, -CH₂-C≡C-, -C₁₋₃ alkylene-O-, or tetrahydropyridinediyl,
   L² is -CH₂-C≡C- or -CH₂CH₂-O-,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are both 1 or both 2, and
   R^{L4} and R^{L5} are both H, or the R^{L4} and R^{L5} together with the carbon atom bonded thereto may form cyclopropane.
(14-3-1) A compound represented by formula (I-I) or a salt thereof, wherein L is a group selected from the group consisting of the following formulas (XXX-L), (XXXI-LX), (XXXII-L), (XXXIII-L), and (XXXIV-L): 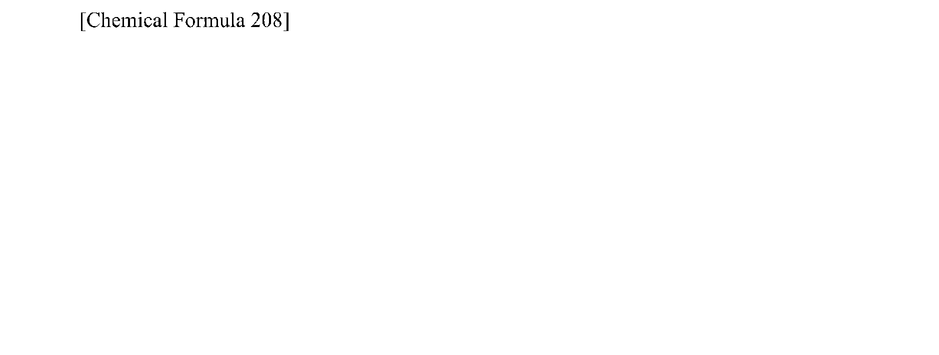 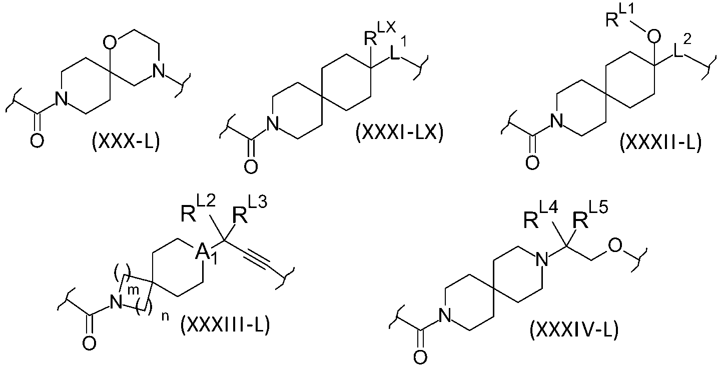
   L¹ is bond, C₁₋₃ alkylene, -CH₂-C≡C-, -C₁₋₃ alkylene-O-, or tetrahydropyridinediyl,
   R^{LX} is H or OH,
   L² is -CH₂-C≡C- or -CH₂CH₂-O-,
   R^{L1} is H or methyl,
   A₁ is CH or N,
   R^{L2} and R^{L3} are the same or different, and are each H, OH, or C₁₋₃ alkyl, provided that R^{L2} and R^{L3} are never OH at the same time, and R^{L2} and R^{L3} are never H at the same time,
   m and n are both 1 or both 2, and
   R^{L4} and R^{L5} are both H, or the R^{L4} and R^{L5} together with the carbon atom bonded thereto may form cyclopropane.
(14-4) A compound represented by formula (I-I) or a salt thereof, wherein L is a group represented by the following formula (XXXI-L): 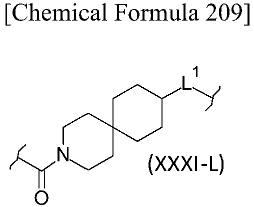 wherein L¹ is bond, C₁₋₃ alkylene, -CH₂-C≡C-, -C₁₋₃ alkylene-O-, or tetrahydropyridinediyl.
(14-4-1) A compound represented by formula (I-I) or a salt thereof, wherein L is a group represented by the following formula (XXXI-LX): 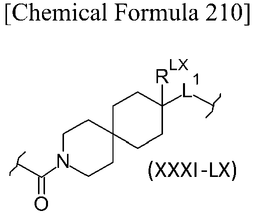
   wherein L¹ is bond, C₁₋₃ alkylene, -CH₂-C≡C-, -C₁₋₃ alkylene-O-, or tetrahydropyridinediyl, and
   R^{LX} is H or OH.
(14-5) A compound represented by formula (I-I) or a salt thereof, wherein L is a group represented by the following formula (XXXI-L): 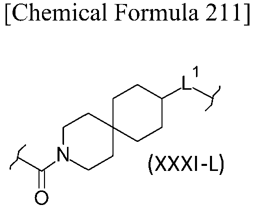 wherein L¹ is -CH₂CH₂-O-.
(14-6) A compound represented by formula (I-I) or a salt thereof, wherein L is a group represented by the following formula (XXXI-L): 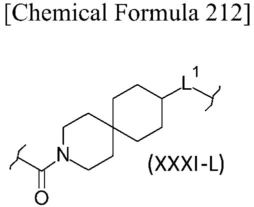 wherein L¹ is -CH₂-C≡C-.
(15-1) A compound represented by formula (I-I) or a salt thereof, wherein Z is a group selected from the group consisting of the following formulas (XVIII), (XIX), (XX), (XXI), (XXII), (XXIII), (XXIV), (XXV), (XXVI), (XXVII), (XXVIII), and (XXIX): 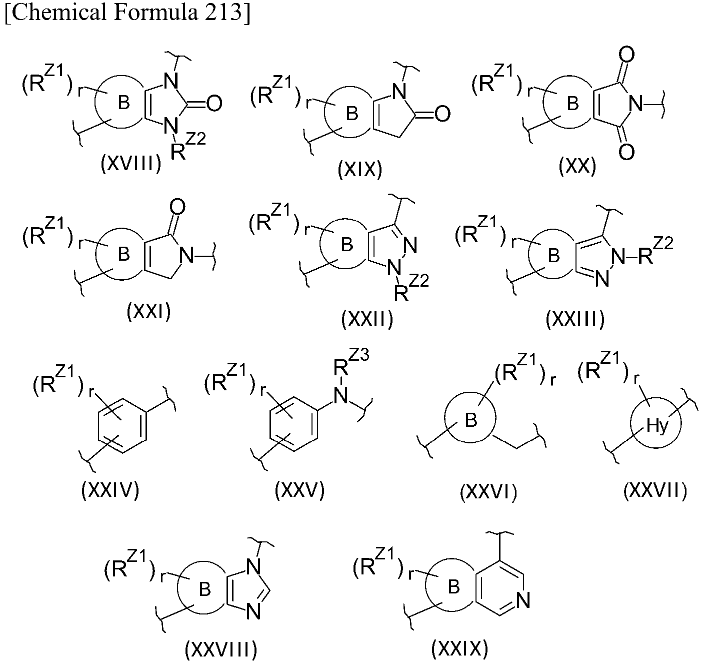
   wherein ring B is benzene ring or 6-membered hetero ring,
   ring Hy is 5- or 6-membered hetero ring,
   R^{Z1} is H, C₁₋₃ alkyl, halogen, -O-(C₁₋₃ alkyl), -NR^{Z4}₂, -CN, -CONR^{Z4}₂, or - NR^{Z4}COR^{Z5},
   R^{Z2} is H or C₁₋₃ alkyl,
   R^{Z3} is H or C₁₋₃ alkyl,
   R^{Z4} is the same or different, and is H or C₁₋₃ alkyl,
   R^{Z5} is C₁₋₃ alkyl,
   L is bonded to ring B in the above formulas (XVIII), (XIX), (XX), (XXI), (XXII), (XXIII), (XXVI), (XXVIII), and (XXIX), to the benzene ring in formulas (XXIV) and (XXV), and to ring Hy in formula (XXVII), and
   r is 1 or 2.
(15-2) A compound represented by formula (I-I) or a salt thereof, wherein Z is the following formula (XXVII-1): 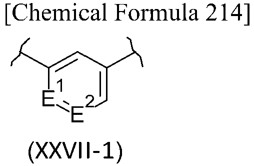 E¹ and E² are the same or different, and are each CH or N, provided that E¹ and E² are never CH at the same time.
(15-3) A compound represented by formula (I-I) or a salt thereof, wherein Z is the following formula (XXVII-1): 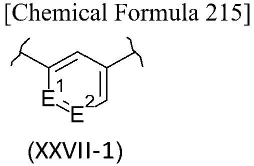 E¹ is CH, and E² is N.
(15-4) A compound represented by formula (I-I) or a salt thereof, wherein Z is the following formula (XXVII-1): 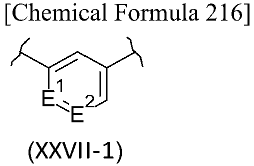 E¹ is N, and E² is CH.
(16-1) A compound represented by formula (I-I) or a salt thereof, wherein G is N or CH, and
   provided that when G is N, Z is formula (XVIII), (XXII), (XXIII), (XXIV), (XXVI), (XXVII), or (XXIX).
(16-2) A compound represented by formula (I-I) or a salt thereof, wherein G is N or CH, and
   provided that when G is N, Z is formula (XXVII).
(16-3) A compound represented by formula (I-I) or a salt thereof, wherein G is N, and Z is formula (XXVII).
(17) A compound represented by formula (I-I) or a salt thereof which is any noncontradictory combination of two or more of the aspects according to (13-1) to (13-5-1), (14-1) to (14-6), (15-1) to (5-4), and (16-1) to (16-3) above.

Examples of the combinations according to (17) specifically include the following aspects.
(17-0) A compound represented by formula (I-I) or a salt thereof: 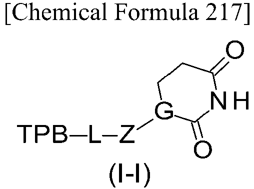 (wherein
   TPB is a group having an ability to bind to a target protein,
   L is -(L^{A} -L^{B}-L^{X}-L^{D}-L^{E}-L^{F})-,
   L^{A}, L^{B}, L^{C}, L^{D}, L^{E}, and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O,
   R^{L1A} is H or C₁₋₃ alkyl,
   Z is a group selected from the group consisting of the following formulas (XVIII), (XIX), (XX), (XXI), (XXII), (XXIII), (XXIV), (XXV), (XXVI), (XXVII), (XXVIII), and (XXIX): 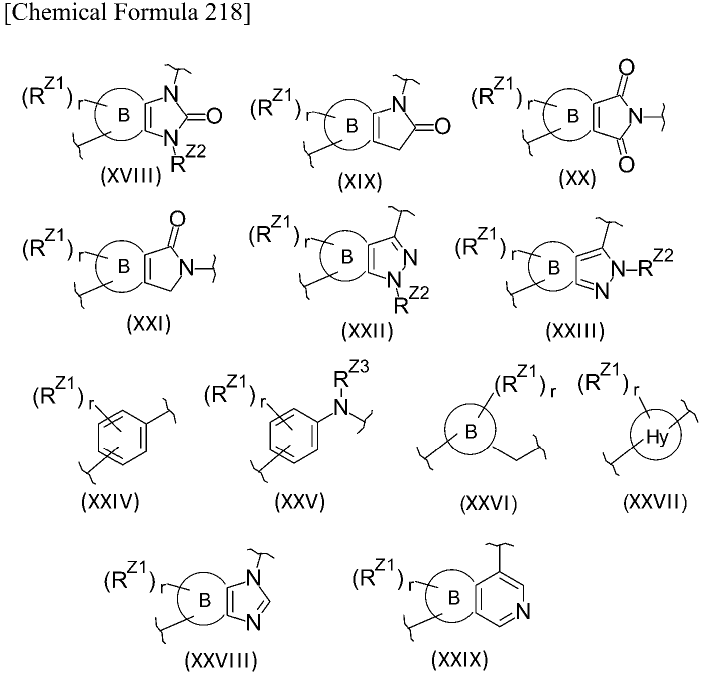
   ring B is benzene ring or 6-membered hetero ring,
   ring Hy is 5- or 6-membered hetero ring,
   R^{Z1} is H, C₁₋₃ alkyl, halogen, -O-(C₁₋₃ alkyl), -NR^{Z4}₂, -CN, -CONR^{Z4}₂, or - NR^{Z4}COR^{Z5},
   R^{Z2} is H or C₁₋₃ alkyl,
   R^{Z3} is H or C₁₋₃ alkyl,
   R^{Z4} is the same or different, and is H or C₁₋₃ alkyl,
   R^{Z5} is C₁₋₃ alkyl,
   L is bonded to ring B in the above formulas (XVIII), (XIX), (XX), (XXI), (XXII), (XXIII), (XXVI), (XXVIII), and (XXIX), to the benzene ring in formulas (XXIV) and (XXV), and to ring Hy in formula (XXVII),
   r is 1 or 2, and
   G is N or CH,
   provided that when G is N, Z is the above formula (XVIII), (XXII), (XXIII), (XXIV), (XXVI), (XXVII), or (XXIX))
(17-1) A compound represented by formula (I-I) or a salt thereof: 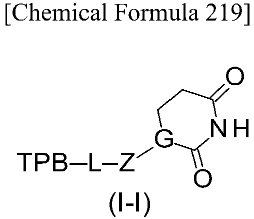 (wherein
   TPB is a group having an ability to bind to a RAS protein,
   L is -(L^{A}-L^{B}-L^{C}-L^{P}-L^{E}-L^{F})-,
   L^{A}, L^{B}, L^{C}, L^{D}, L^{E}, and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O,
   R^{L1A} is H or C₁₋₃ alkyl,
   Z is a group selected from the group consisting of the following formulas (XVIII), (XIX), (XX), (XXI), (XXII), (XXIII), (XXIV), (XXV), (XXVI), (XXVII), (XXVIII), and (XXIX): 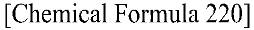 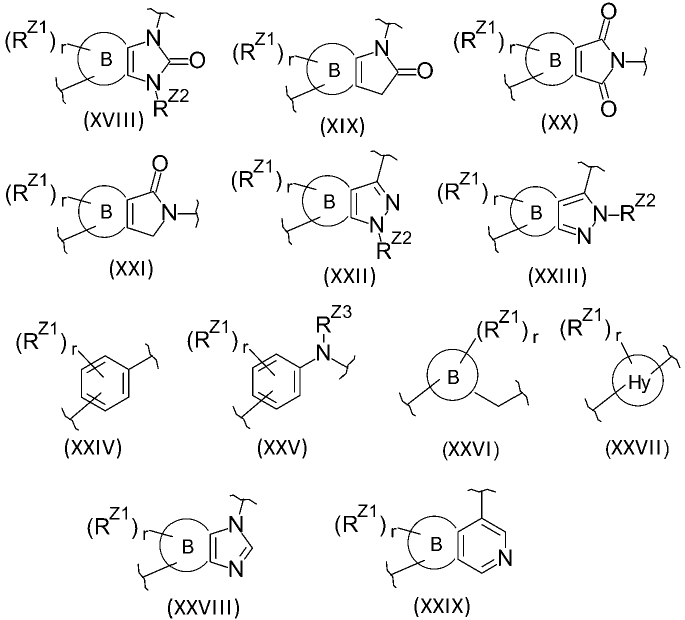
   ring B is benzene ring or 6-membered hetero ring,
   ring Hy is 5- or 6-membered hetero ring,
   R^{Z1} is H, C₁₋₃ alkyl, halogen, -O-(C₁₋₃ alkyl), -NR^{Z4}₂, -CN, -CONR^{Z4}₂, or - NR^{Z4}COR^{Z5},
   R^{Z2} is H or C₁₋₃ alkyl,
   R^{Z3} is H or C₁₋₃ alkyl,
   R^{Z4} is the same or different, and is H or C₁₋₃ alkyl,
   R^{Z5} is C₁₋₃ alkyl,
   L is bonded to ring B in the above formulas (XVIII), (XIX), (XX), (XXI), (XXII), (XXIII), (XXVI), (XXVIII), and (XXIX), to the benzene ring in formulas (XXIV) and (XXV), and to ring Hy in formula (XXVII),
   r is 1 or 2, and
   G is N or CH,
   provided that when G is N, Z is the above formula (XVIII), (XXII), (XXIII), (XXIV), (XXVI), (XXVII), or (XXIX))
(17-2) The compound or a salt thereof according to (17-1) above, wherein TPB is the following formula (I-II): 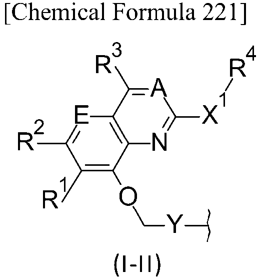
   wherein A, R^{A}, E, X¹, R^{X1}, R¹, R^{1a}, R^{1b}, R^{1x}, R^{1d}, R², R³, R^{3a}, R^{3b}, R^{3c}, R^{3d}, R^{3e}, R^{3f}, R^{3g}, R^{3h}, R³ⁱ, R^{N1}, R^{N2}, R^{3j}, X², X³, X⁴, n¹, p, q, R⁴, and Y are defined as in formula (I),
   Z is the following formula (XXVII-1) as (XXVII): 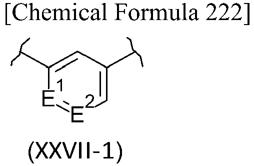
   E¹ and E² are the same or different, and are each CH or N, provided that E¹ and E² are never CH at the same time, and
   G is N.
(17-3) The compound or a salt thereof according to (17-1) above, wherein TPB is the following formula (I-III): 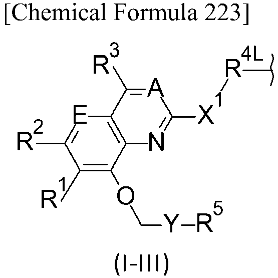
   wherein A, R^{A}, E, X¹, R^{X1}, R¹, R^{1a}, R^{1b}, R^{1c}, R^{1d}, R², R³, R^{3a}, R^{3b}, R^{3c}, R^{3d}, R^{3e}, R^{3f}, R^{3g}, R^{3h}, R³ⁱ, R^{N1}, R^{N2}, R^{3j}, X², X³, X⁴, n¹, p, q, and Y are defined as in formula (I),
   R⁵ is H, CONR⁶R⁷, or a group selected from the group consisting of the following formulas (I-V), (I-VI), (I-VII), (I-VIII), (I-IX), (I-X), (I-XI), (I-XII), (I-XIII), (I-XIV), (I-XV), and (I-XVI): 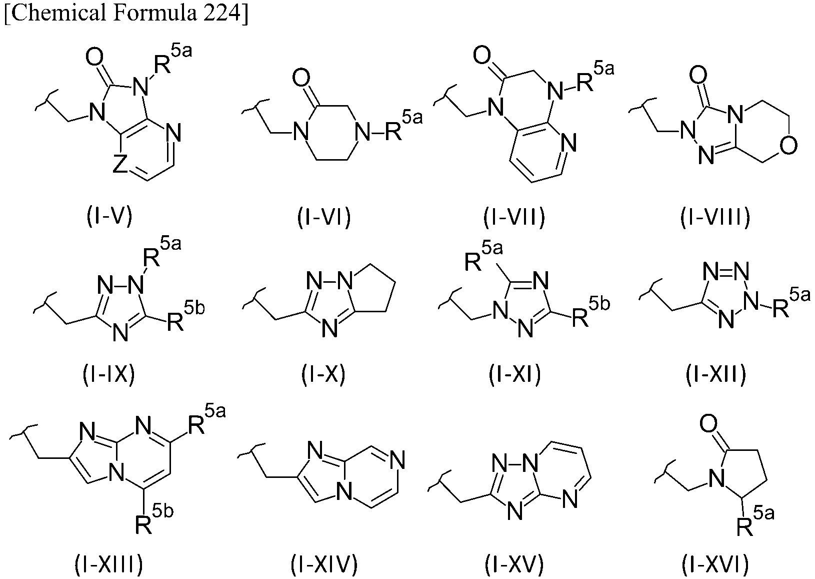
   R^{5a} and R^{5b} are the same or different, and are each H, optionally substituted C₁₋₃ alkyl, cyclopropyl, cyclopropylmethyl, oxetanyl, tetrahydropyranyl, optionally substituted oxazolyl, thiazolyl, or pyrazinyl,
   R⁶ and R⁷ are the same or different, and are each H or optionally substituted C₁₋₆ alkyl, or R⁶ and R⁷, together with a nitrogen atom bonded thereto, form 4- to 6-membered saturated hetero ring group, and the 4- to 6-membered saturated hetero ring group is optionally substituted with optionally substituted C₁₋₆ alkyl,
   R^{4L} is optionally substituted C₁₋₆ alkylene, or divalent group of optionally substituted saturated hetero ring,
   Z is the following formula (XXVII-1) as (XXVII): 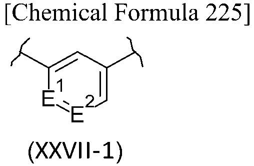
   E¹ and E² are the same or different, and are each CH or N, provided that E¹ and E² are never CH at the same time, and
   G is N.
(17-3-1) The compound or a salt thereof according to (17-1) above, wherein TPB is the following formula (I-III-1): 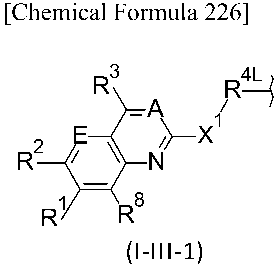
   wherein A, R^{A}, E, X¹, R^{X1}, R¹, R^{1a}, R^{1b}, R^{1c}, R^{1d}, R², R³, R^{3a}, R^{3b}, R^{3e}, R^{3d}, R^{3e}, R^{3f}, R^{3g}, R^{3h}, R³ⁱ, R^{N1}, R^{N2}, R^{3j}, X², X³, X⁴, n¹, p, and q are defined as in formula (I),
   R⁸ is H, halogen, -O-C₁₋₃ alkyl, or -O-CH₂-Y-R⁵,
   Y is defined as in formula (I),
   R⁵ is H, CONR⁶R⁷, or a group selected from the group consisting of the following formulas (I-V), (I-VI), (I-VII), (I-VIII), (I-IX), (I-X), (I-XI), (I-XII), (I-XIII), (I-XIV), (I-XV), and (I-XVI): 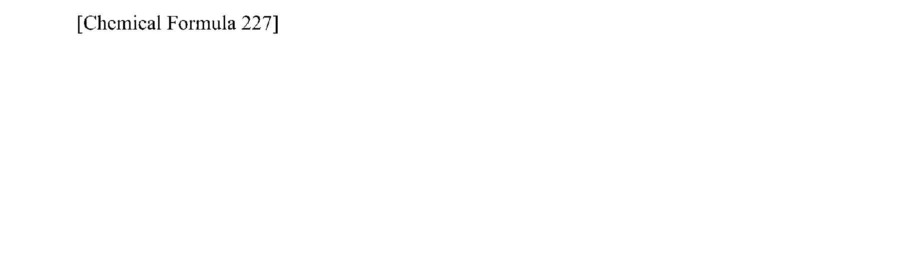 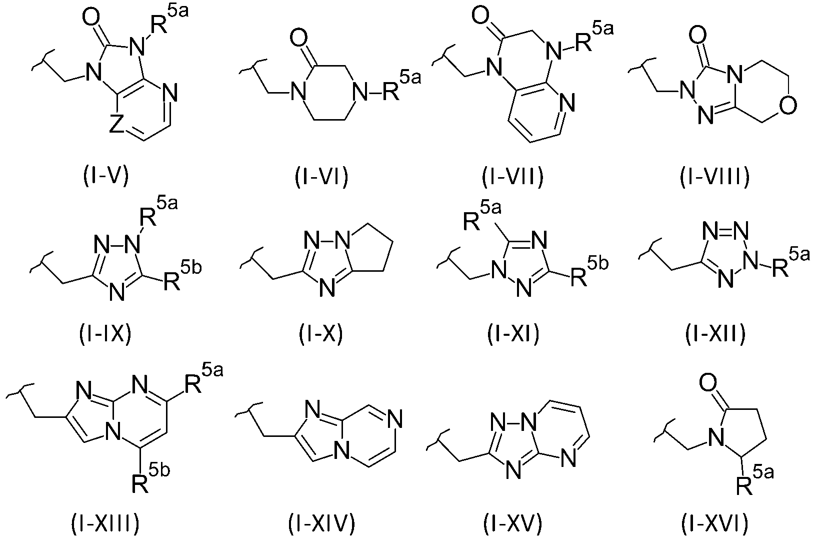
   R^{5a} and R^{5b} are the same or different, and are each H, optionally substituted C₁₋₃ alkyl, cyclopropyl, cyclopropylmethyl, oxetanyl, tetrahydropyranyl, optionally substituted oxazolyl, thiazolyl, or pyrazinyl,
   R⁶ and R⁷ are the same or different, and are each H or optionally substituted C₁₋₆ alkyl, or R⁶ and R⁷, together with a nitrogen atom bonded thereto, form 4- to 6-membered saturated hetero ring group, and the 4- to 6-membered saturated hetero ring group is optionally substituted with optionally substituted C₁₋₆ alkyl,
   R^{4L} is optionally substituted C₁₋₆ alkylene, or divalent group of optionally substituted saturated hetero ring,
   Z is the following formula (XXVII-1) as (XXVII): 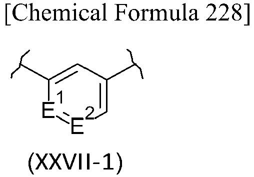
   E¹ and E² are the same or different, and are each CH or N, provided that E¹ and E² are never CH at the same time, and
   G is N.
(17-4) The compound or a salt thereof according to (17-1) above, wherein TPB is the following formula (I-IV): 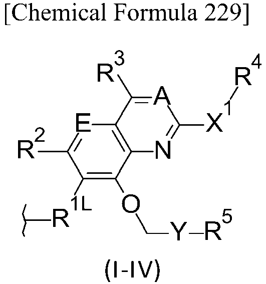
   wherein A, R^{A}, E, X¹, R^{X1}, R², R³, R^{3a}, R^{3b}, R^{3c}, R^{3d}, R^{3e}, R^{3f}, R^{3g}, R^{3h}, R³ⁱ, R^{N1}, R^{N2}, R^{3j}, X², X³, X⁴, n¹, p, q, R⁴, and Y are defined as in formula (I),
   R⁵ is H, CONR⁶R⁷, or a group selected from the group consisting of the following formulas (I-V), (I-VI), (I-VII), (I-VIII), (I-IX), (I-X), (I-XI), (I-XII), (I-XIII), (I-XIV), (I-XV), and (I-XVI): 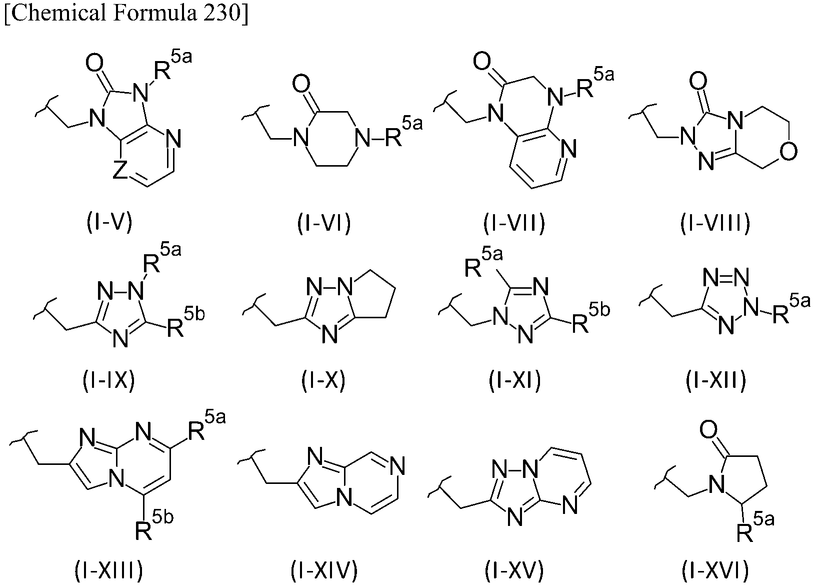
   R^{5a} and R^{5b} are the same or different, and are each H, optionally substituted C₁₋₃ alkyl, cyclopropyl, cyclopropylmethyl, oxetanyl, tetrahydropyranyl, optionally substituted oxazolyl, thiazolyl, or pyrazinyl,
   R⁶ and R⁷ are the same or different, and are each H or optionally substituted C₁₋₆ alkyl, or R⁶ and R⁷, together with a nitrogen atom bonded thereto, form 4- to 6-membered saturated hetero ring group, and the 4- to 6-membered saturated hetero ring group is optionally substituted with optionally substituted C₁₋₆ alkyl,
   R^{1L} is the following formula (II-I), (III-I), (IV-I), (V-I), (VI-I), or (VII-I): 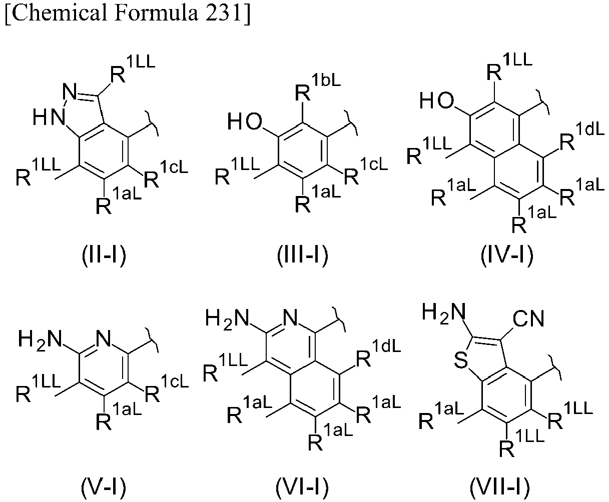
   R^{1aL} and R^{1bL} are the same or different, and are each H, methyl, F, Cl, or an attachment point to L,
   R^{1cL} is F, Cl, methyl, ethyl, trifluoromethyl, cyclopropyl, or an attachment point to L,
   R^{1dL} is H, methyl, ethyl, F, Cl, -C≡C-H, or an attachment point to L,
   R^{1LL} is H or an attachment point to L,
   provided that only one attachment point to L is present in the above formula (II-I), (III-I), (IV-I), (V-I), (VI-I), or (VII-I),
   Z is the following formula (XXVII-1) as (XXVII): 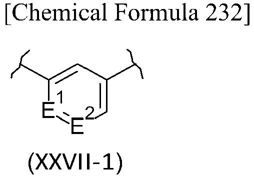
   E¹ and E² are the same or different, and are each CH or N, provided that E¹ and E² are never CH at the same time, and
   G is N.
(17-4-1) The compound or a salt thereof according to (17-1) above, wherein TPB is the following formula (I-IV-1): 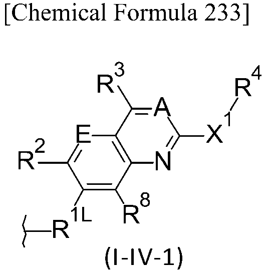
   wherein A, R^{A}, E, X¹, R^{X1}, R², R³, R^{3a}, R^{3b}, R^{3c}, R^{3d}, R^{3e}, R^{3f}, R^{3g}, R^{3h}, R³ⁱ, R^{N1}, R^{N2}, R^{3j}, X², X³, X⁴, n¹, p, q, and R⁴ are defined as in formula (I),
   R⁸ is H, halogen, -O-C₁₋₃ alkyl, or -O-CH₂-Y-R⁵,
   Y is defined as in formula (I),
   R⁵ is H, CONR⁶R⁷, or a group selected from the group consisting of the following formulas (I-V), (I-VI), (I-VII), (I-VIII), (I-IX), (I-X), (I-XI), (I-XII), (I-XIII), (I-XIV), (I-XV), and (I-XVI): 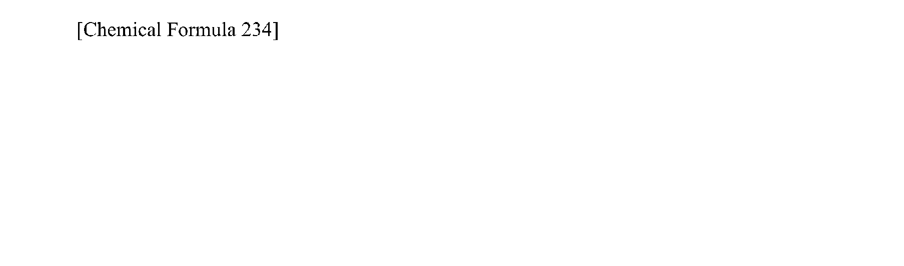 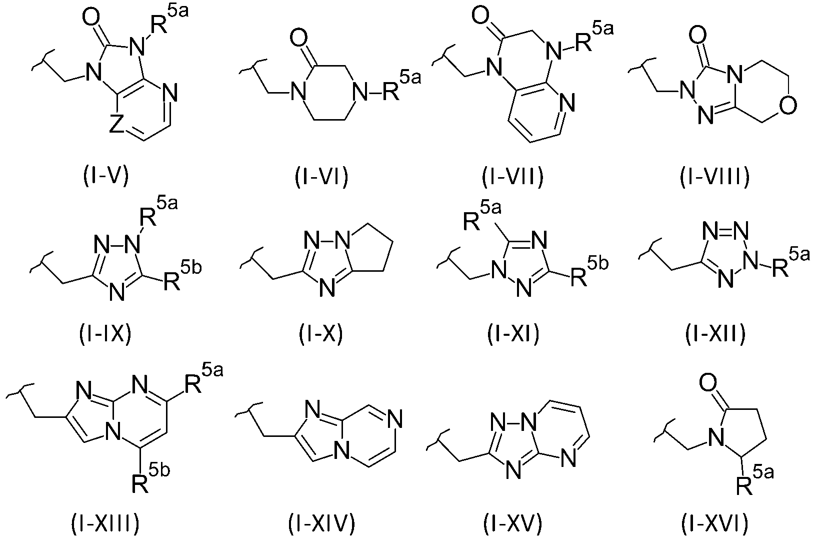
   R^{5a} and R^{5b} are the same or different, and are each H, optionally substituted C₁₋₃ alkyl, cyclopropyl, cyclopropylmethyl, oxetanyl, tetrahydropyranyl, optionally substituted oxazolyl, thiazolyl, or pyrazinyl,
   R⁶ and R⁷ are the same or different, and are each H or optionally substituted C₁₋₆ alkyl, or R⁶ and R⁷, together with a nitrogen atom bonded thereto, form 4- to 6-membered saturated hetero ring group, and the 4- to 6-membered saturated hetero ring group is optionally substituted with optionally substituted C₁₋₆ alkyl,
   R^{1L} is the following formula (II-I), (III-I), (IV-I), (V-I), (VI-I), or (VII-I): 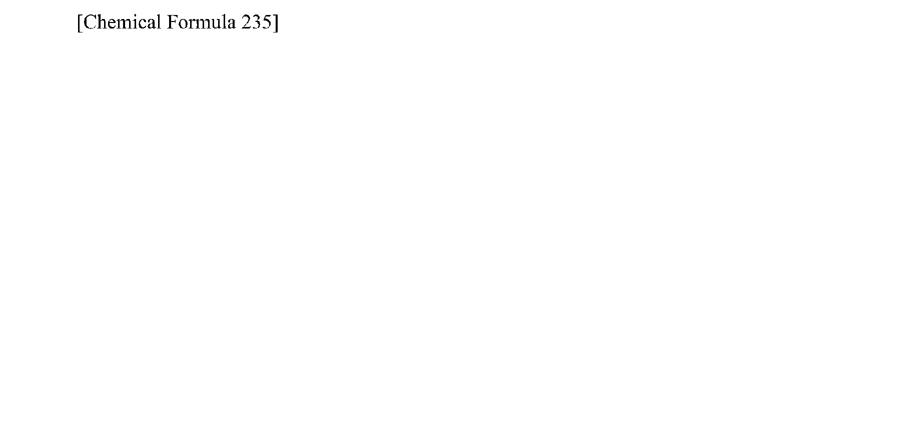 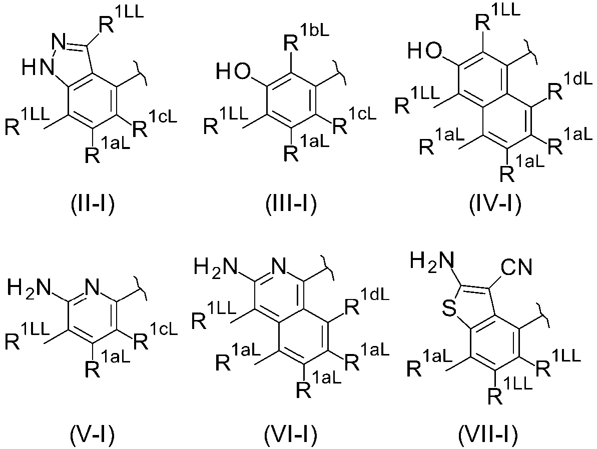
   R^{1aL} is the same or different, and is H, methyl, F, Cl, or an attachment point to L,
   R^{1bL} is H, methyl, F, Cl, or an attachment point to L,
   R^{1cL} is F, Cl, methyl, ethyl, trifluoromethyl, cyclopropyl, or an attachment point to L,
   R^{1dL} is H, methyl, ethyl, F, Cl, -C≡C-H, or an attachment point to L,
   R^{1LL} is the same or different, and is H or an attachment point to L,
   provided that only one attachment point to L is present in the above formula (II-I), (III-I), (IV-I), (V-I), (VI-I), or (VII-I),
   Z is the following formula (XXVII-1) as (XXVII): 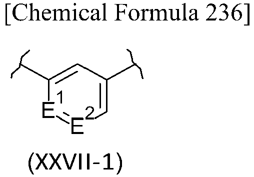
   E¹ and E² are the same or different, and are each CH or N, provided that E¹ and E² are never CH at the same time, and
   G is N.
(17-5-0) The compound or a salt thereof according to (17-0) above, wherein TPB is a group having an ability to bind to a target protein,
   L is -(L^{A}-L^{B}-L^{C}-L^{D}-L^{E}-L^{F})-,
   L^{A}, L^{B}, L^{C}, L^{D}, L^{F} and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O,
   R^{L1A} is H or C₁₋₃ alkyl,
   Z is the following formula (XXVII-1) as (XXVII): 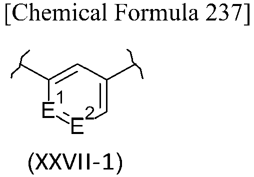
   E¹ and E² are the same or different, and are each CH or N, provided that E¹ and E² are never CH at the same time, and
   G is N.
(17-5) The compound or a salt thereof according to (17-1) above, wherein TPB is a group having an ability to bind to a RAS protein,
   L is -(L^{A}-L^{B}-L^{C}-L^{P}-L^{E}-L^{F})-,
   L^{A}, L^{B}, L^{C}, L^{D}, L^{E} and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O,
   R^{L1A} is H or C₁₋₃ alkyl,
   Z is the following formula (XXVII-1) as (XXVII): 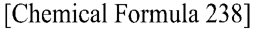 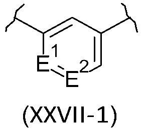
   E¹ and E² are the same or different, and are each CH or N, provided that E¹ and E² are never CH at the same time, and
   G is N.
(17-6-0) The compound or a salt thereof according to (17-0) above, wherein TPB is a group having an ability to bind to a target protein,
   L is -(L^{A}-L^{B}-L^{C}-L^{D}-L^{E}-L^{F})-,
   L^{A}, L^{B}, L^{C}, L^{D}, L^{E} and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O,
   R^{L1A} is H or C₁₋₃ alkyl,
   Z is the following formula (XXVII-1) as (XXVII): 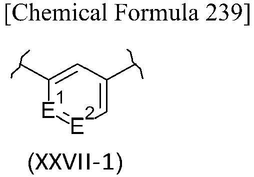
   E¹ is CH, and E² is N, and
   G is N.
(17-6) The compound or a salt thereof according to (17-1) above, wherein TPB is a group having an ability to bind to a RAS protein,
   L is -(L^{A}-L^{B}-L^{C}-L^{P}-L^{E}-L^{F})-,
   L^{A}, L^{B}, L^{C}, L^{D}, L^{E} and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O, R^{L1A} is H or C₁₋₃ alkyl,
   Z is the following formula (XXVII-1) as (XXVII): 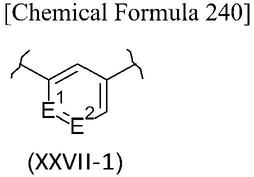
   E¹ is CH, and E² is N, and
   G is N.
(17-7-0) The compound or a salt thereof according to (17-0) above, wherein TPB is a group having an ability to bind to a target protein,
   L is -(L^{A}-L^{B}-L^{C}-L^{D}-L^{E}-L^{F})-,
   L^{A}, L^{B}, L^{C}, L^{D}, L^{E} and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O,
   R^{L1A} is H or C₁₋₃ alkyl,
   Z is the following formula (XXVII-1) as (XXVII): 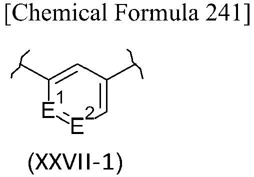
   E¹ is N, and E² is CH, and
   G is N.
(17-7) The compound or a salt thereof according to (17-1) above, wherein TPB is a group having an ability to bind to a RAS protein,
   L is -(L^{A}-L^{B}-L^{C}-L^{D}-L^{E}-L^{F})-,
   L^{A}, L^{B}, L^{C}, L^{D}, L^{F} and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O,
   R^{L1A} is H or C₁₋₃ alkyl,
   Z is the following formula (XXVII-1) as (XXVII): 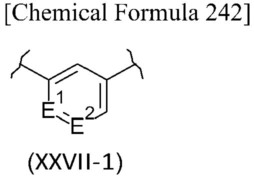
   E¹ is N, and E² is CH, and
   G is N.

In the compounds represented by formulas (I) and (I-I), tautomers and/or geometric isomers can be present depending on the type of the substituent. In this specification, although the compounds represented by formulas (I) and (I-I) are described as only one form of isomers in some cases, the present invention encompasses other isomers, and also encompasses separated isomers or a mixture thereof.

In some cases, the compounds represented by formulas (I) and (I-I) may have an asymmetric carbon atom or axial chirality, and a diastereomer based on this can be present. The present invention also encompasses the separated diastereomers of the compounds represented by formulas (I) and (I-I) or mixtures thereof.

Further, the present invention also encompasses pharmaceutically acceptable prodrugs of the compounds represented by formulas (I) and (I-I). The pharmaceutically acceptable prodrug is a compound having a group which can be converted into an amino group, a hydroxyl group, a carboxyl group, or the like by solvolysis or under a physiological condition. Examples of groups forming a prodrug include those described in Prog. Med., 5, 2157-2161 (1985) and "Iyakuhin no Kaihatsu" (Hirokawa Shoten, 1990) vol. 7, Bunshi Sekkei, 163-198.

Salts of the compounds represented by formulas (I) and (I-I) are pharmaceutically acceptable salts of the compounds represented by formulas (I) and (I-I), and depending on the type of substituents, these compounds may form an acid addition salt or a salt with a base in some cases. Examples of the salts include salts described in P. Heinrich Stahl, Handbook of Pharmaceutical Salts Properties, Selection, and Use, Wiley-VCH, 2008. Specifically, examples thereof include acid addition salts with an inorganic acid such as hydrochloric acid, hydrobromic acid, hydroiodic acid, sulfuric acid, nitric acid, or phosphoric acid or an organic acid such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluene sulfonic acid, aspartic acid, or glutamic acid; salts with a inorganic metal such as sodium, potassium, magnesium, calcium, or aluminum; salts with an organic base such as methylamine, ethylamine, or ethanolamine; salts with a variety of amino acids such as acetyl leucine, lysine, or ornithine or an amino acid derivative; and ammonium salts.

Further, the present invention also encompasses a variety of hydrates of the compounds represented by formulas (I) and (I-I) and salts thereof, solvates thereof, and crystal polymorphic substances thereof.

The present invention also encompasses all the pharmaceutically acceptable compounds represented by formulas (I) and (I-I) or salts thereof which are labelled with one or more pharmaceutically acceptable radioactive or non-radioactive isotopes. Examples of suitable isotopes used for isotopic labelling of the compound of the present invention include isotopes of hydrogen (such as ²H and ³H), carbon (such as ¹¹C, ¹³C, and ¹⁴C), nitrogen (such as ¹³N and ¹⁵N), oxygen (such as ¹⁵O, ¹⁷O, and ¹⁸O), fluorine (such as ¹⁸F), chlorine (such as ³⁶Cl), iodine (such as ¹²³I and ¹²⁵I), and sulfur (such as³⁵S).

The compounds according to the present invention labelled with an isotope can be used for research such as research on tissue distribution of drugs and/or substrates. For example, the radioactive isotopes such as tritium (³H) and carbon 14 (¹⁴C) can be used for this purpose because of easy labelling and convenience of detection.

In some cases, replacement by a heavier isotope, such as replacement by hydrogen deuterium (²H), improves metabolic stability, and thus may lead to therapeutic advantageous (e.g., an increase in half-life in vivo, a reduction in necessary amount, a reduction in drug interaction).

Replacement by a positron emitting isotope (such as ¹¹C, ¹⁸F, ¹⁵O, or ¹³N) can be used in a test using positron emission tomography (PET) in order to examine substrate receptor occupancy.

The isotope-labeled compound of the present invention can be generally produced by a conventional method known to persons skilled in the art, or by the same production method as in Examples or Production Examples or the like, using an appropriate isotopically-labelled reagent instead of a non-labelled reagent.

### (Production method)

Utilizing characteristics based on the basic structure of the compound or the type of substituent, the compounds represented by formulas (I) and (I-I) and salts thereof can be produced using a variety of known synthetic methods. In the production, depending on the type of functional group, it may be technically effective in the production to substitute the functional group by an appropriate protecting group (a group easily convertible to the functional group) in a stage in which the raw material is formed into an intermediate product. Examples of such a protecting group include protecting groups described in P. G. M. Wuts and T. W. Greene, "Greene's Protective Groups in Organic Synthesis", the 5th edition, John Wiley & Sons Inc., 2014, and these protecting groups can be appropriately selected according to the reaction conditions thereof for use. In such a method, a desired compound can be obtained by introducing the protecting group, performing the reaction, and then removing the protecting group as needed.

The pharmaceutically acceptable prodrug is a compound having a group which can be converted into an amino group, a hydroxyl group, a carboxyl group, or the like by solvolysis or under a physiological condition. Examples of groups forming a prodrug include those described in Prog. Med., 5, 2157-2161 (1985) and "Iyakuhin no Kaihatsu" (Hirokawa Shoten, 1990) vol. 7, Bunshi Sekkei, 163-198.

The prodrugs of the compounds represented by formulas (I) and (I-I) can be produced by introducing a specific group in a stage in which the raw material is formed into an intermediate product as in the production of the protecting group above or by further reacting the obtained compounds represented by formulas (I) and (I-I). The reaction can be performed using a method known by persons skilled in the art, such as standard esterification, amidation, or dehydration.

Hereinafter, representative methods for producing the compounds represented by formulas (I) and (I-I) will be described. The production methods can also be performed by referring to the reference attached to the description. The production method according to the present invention is not limited to the examples shown below.

In the present specification, the following abbreviations are sometimes used:
DMF: N,N-dimethylformamide, DMAc: N,N-dimethylacetamide, THF: tetrahydrofuran, MeCN: acetonitrile, MeOH: methanol, EtOH: ethanol, iPrOH or IPA: isopropyl alcohol, tBuOH: tert-butanol, DOX: 1,4-dioxane, DMSO: dimethyl sulfoxide, Et₃N or TEA: triethylamine, DIPEA: N,N-diisopropylethylamine, tBuOK: potassium tert-butoxide, tBuONa: sodium tert-butoxide, PdCl₂(dppf)·CH₂Cl₂: [1,1'-bis(diphenylphosphino)ferrocene]palladium(II) dichloride·dichloromethane adduct, Pd/C: palladium on carbon, PyBOP: (benzotriazol-1-yloxy)tripyrrolidinophosphonium hexafluorophosphate, PyAOP: (7-azabenzotriazol-1-yloxy)trispyrrolidinophosphonium hexafluorophosphate, SFC: supercritical fluid chromatography, NMM: N-methylmorpholine, CDI: 1,1'-carbonyldiimidazole, HATU: O-(7-azabenzotriazol-1-yl)-N,N,N',N'-tetramethyluronium hexafluorophosphate, DABCO: 1,4-diazabicyclo[2.2.2]octane, TFA: trifluoroacetic acid, DBU: 1,8-diazabicyclo[5.4.0]-7-undecene, TBAF: tetra-n-butylammonium fluoride, Pd(t-Bu₃P)₂: bis(tri-tert-butylphosphine)palladium(O), Pd₂(dba)₃: tris(dibenzylideneacetone)dipalladium(0), Xphos: 2-dicyclohexylphosphino-2',4',6'-triisopropylbiphenyl, NMP: N-ethyl-2-pyrrolidone, mCPBA: m-chloroperbenzoic acid, RuPhos: 2-dicyclohexylphosphino-2',6'-diisopropoxybiphenyl, RuPhos Pd G3: (2-dicyclohexylphosphino-2',6'-diisopropoxy-1,1'-biphenyl) [2-(2'-amino-1,1'-biphenyl)]palladium(II) methanesulfonate, PdCl₂(PPh₃)₂ dichlorobis(triphenylphosphine)palladium(II), DMP: Dess-Martin periodinane, EPhos: dicyclohexyl(3-isopropoxy-2',4',6'-triisopropyl-[1,1'-biphenyl]-2-yl)phosphane, Ephos Pd G4: [dicyclohexyl[3-(1-methylethoxy)-2',4',6'-tris(1-methylethyl)[1,1'-biphenyl]-2-yl]phosphine-κP](methanesulfonato-κO)[2'-(methylamino-κN)[1,1'-biphenyl]-2-yl-κC]palladium(II), XPhos: 2-dicyclohexylphosphino-2',4',6'-triisopropylbiphenyl, LHMDS: lithium bis(trimethylsilyl)amide, KHMDS: potassium bis(trimethylsilyl)amide, LAH: lithium aluminium hydride, HOBt: 1-hydroxybenzotriazole, EDC·HCl: 1-(3-dimethylaminopropyl)-3-ethylcarbodiimide hydrochloride, EtOAc: ethyl acetate, AcOH: acetic acid, TfOH: trifluoromethanesulfonic acid, TMEDA: N,N,N',N'-tetramethylethylenediamine, Sphos: dicyclohexyl(2',6'-dimethoxybiphenyl-2-yl)phosphine, ODS: octadecylsilyl, and MW: microwave.

### (Production Method 1)

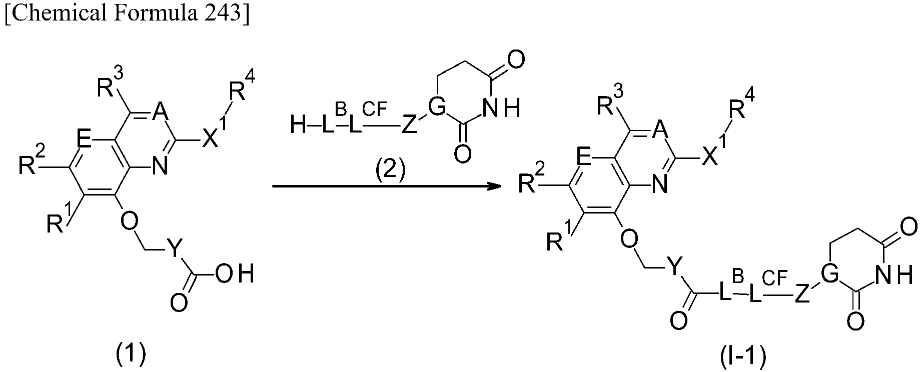

(wherein -L^{CF}- represents -L^{C}-L^{D}-L^{E}-L^{F}- contained in -L-. The same shall apply hereinafter)

This production method is a method for producing a compound of formula (I-1) included in the compound of formula (I), wherein A is N, -L^{A}- contained in -L- is CO, -L^{B}- is -N(R^{L1A})- or a saturated heterocyclic divalent group containing 1 or 2 nitrogen atoms optionally substituted, -L^{C}-L^{D}-L^{E}-L^{F}- is -L^{CF}-, and R^{L1A} is H or C₁₋₃ alkyl.

In this reaction, compound (1) and compound (2) are used in an equal amount or with one compound thereof in an excess amount, and the mixture of the compounds is stirred in the presence of a condensing agent, in a solvent inactive for the reaction, under a condition ranging from cooling to heating, preferably at -20°C to 60°C, generally for 0.1 hours to 5 days, to obtain an amide compound. Examples of the solvent include, but are not particularly limited to, an aromatic hydrocarbon, such as toluene, an ether, such as THF and DOX, a halogenated hydrocarbon, such as dichloromethane, an alcohol, DMF, DMSO, ethyl acetate, MeCN, and a mixture thereof. Examples of the condensing agent include PyBOP, HATU, 1-(3-dimethylaminopropyl)-3-ethylcarbodiimide or a hydrochloride (EDC·HCl) thereof, N,N'-dicyclohexylcarbodiimide (DCC), CDI, diphenylphosphoryl azide (DPPA), and PyAOP. Use of an additive (for example, HOBt or dimethylaminopyridine) is sometimes preferred for the reaction. Performing the reaction in the presence of an organic base, such as TEA, DIPEA and NMM, or an inorganic base, such as potassium carbonate, sodium carbonate and potassium hydroxide, is sometimes advantageous for smoothly promoting the reaction.

Alternatively, a method of converting compound (1) into a reactive derivative, followed by an acylation reaction, can be used. Examples of the reactive derivative of a carboxylic acid include an acid halide obtained by a reaction with a halogenating agent, such as phosphorus oxychloride and thionyl chloride, a mixed acid anhydride obtained by a reaction with isobutyl chloroformate or the like, and an active ester obtained by condensation with 1-hydroxybenzotriazole or the like. The reaction of the reactive derivative and compound (2) can be performed in a solvent inactive for the reaction, such as a halogenated hydrocarbon, an aromatic hydrocarbon and an ether, under a condition ranging from cooling to heating, preferably at -20°C to 120°C.

### [Reference]

S. R. Sandler and W. Karo, "Organic Functional Group Preparations", 2nd edition, Vol. 1, Academic Press Inc., 1991
The Chemical Society of Japan, "Jikken Kagaku Koza (lectures on experimental chemistry (5th edition)", Vol. 16 (2005) (Maruzen)

### (Production Method 2)

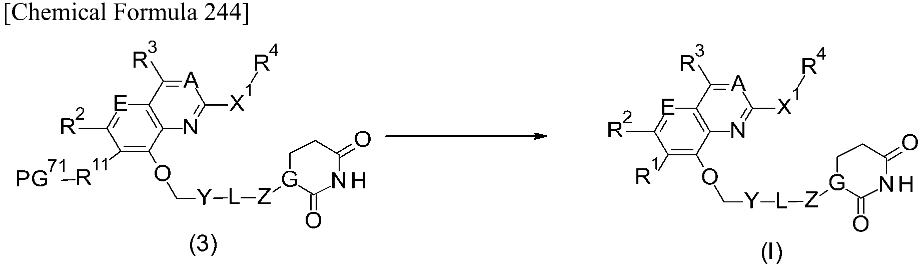

(PG⁷¹ represents a protecting group of NH contained in R¹, and R¹¹ represents divalent group formed by elimination of H from NH contained in R¹. The same shall apply hereinafter.)

This production method is another method for producing the compound of formula (I).

The compound of formula (I) can be obtained by subjecting compound (3) to a deprotection reaction. Examples of the protecting group shown here include a tert-butoxycarbonyl group, a triphenylmethyl group, a tetrahydro-2H-pyran-2-yl group, a methoxymethyl group, a dimethylmethanediyl group, and a tert-butylsulfinyl group and the like.

This deprotection reaction is performed by stirring the compound under a condition ranging from cooling to reflux with heat, generally for 0.1 hours to 5 days. Examples of the solvent used here include, but are not particularly limited to, an alcohol, such as MeOH and EtOH, a halogenated hydrocarbon, such as dichloromethane, 1,2-dichloroethane and chloroform, an ether, such as diethyl ether, THF, DOX and dimethoxyethane, DMF, DMSO, MeCN or water, and a mixture thereof. Examples of the deprotection reagent include, but are not particularly limited to, an acid, such as hydrogen chloride (DOX solution), trifluoroacetic acid, methanesulfonic acid, phosphoric acid, p-toluenesulfonic acid and trifluoromethanesulfonic acid and the like.

By selecting a protecting group, the deprotection can be performed by a catalytic hydrogenation reaction. Examples of the protecting group include a benzyl group, a p-methoxybenzyl group, and a benzyloxycarbonyl group and the like. Moreover, deprotection can also be performed with a fluoride ion source such as tetra-n-butylammonium fluoride. Examples of the protecting group include a tert-butyl(dimethyl)silyl group and a (trimethylsilyl)ethoxymethyl group. Furthermore, examples of the protecting group which can be deprotected under basic conditions include an acetyl group, a trifluoroacetyl group, a benzoyl group and the like.

For example, the following can be referred to as a reference.
P. G. M. Wuts and T. W. Greene, "Greene's Protective Groups in Organic Synthesis", 5th edition, John Wiley & Sons Inc., 2014
A. R. Katritzky and R. J. K. Taylor, "Comprehensive Organic Functional Group Transformations II", Vol. 2, Elsevier Pergamon, 2005

### (Production Method 3)

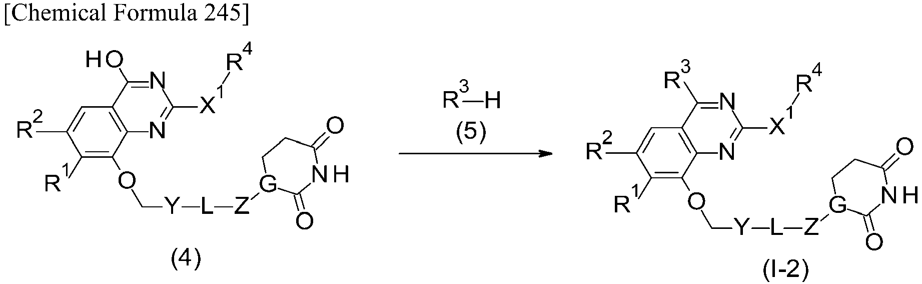

This production method is a method for producing a compound of formula (I-2) included in the compound of formula (I), wherein A and E are N and CH, respectively, by a reaction of compound (4) and compound (5).

In this reaction, compound (4) and compound (5) are used in an equal amount or with one compound thereof in an excess amount, and the mixture of the compounds is stirred in the presence of a condensing agent, in a solvent inactive for the reaction, under a condition ranging from cooling to heating, preferably at -20°C to 60°C, generally for 0.1 hours to 5 days. Examples of the solvent include, but are not particularly limited to, an aromatic hydrocarbon, such as toluene, an ether, such as THF and DOX, a halogenated hydrocarbon, such as dichloromethane, an alcohol, DMF, DMSO, ethyl acetate, MeCN, and a mixture thereof. Examples of the condensing agent include PyBOP, HATU, CDI, and PyAOP and the like. Performing the reaction in the presence of an organic base, such as TEA, DIPEA or NMM, or an inorganic base, such as potassium carbonate, sodium carbonate or cesium carbonate, is sometimes advantageous for smoothly promoting the reaction.

Alternatively, the compound of formula (I-2) can also be obtained by converting compound (4) into a reactive derivative in which a hydroxyl group at the 4-position of compound (4) is converted to chloro group using a chlorinating reagent, such as phosphorus oxychloride and thionyl chloride, followed by addition of compound (5) in the presence of an organic base, such as TEA, DIPEA and pyridine, or an inorganic base, such as potassium carbonate, cesium carbonate and potassium acetate. The reaction of the reactive derivative and compound (5) can be performed in a solvent inactive for the reaction, such as a halogenated hydrocarbon, an aromatic hydrocarbon and an ether, under a condition ranging from cooling to heating, preferably at -20°C to 120°C.

### (Production Method 4)

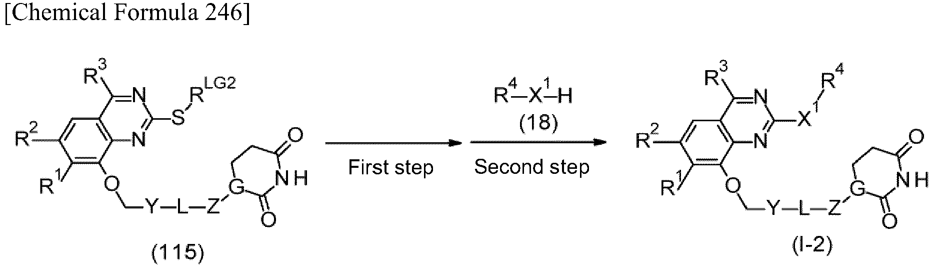

This production method is the second method for producing a compound of formula (I-2) included in the compound of formula (I), wherein A and E are N and CH, respectively, by subjecting compound (115) to an oxidation reaction in the first step, followed by a reaction with compound (18) in the second step.

In the first step of this reaction, the oxidation reaction can be carried out under the same conditions as in the seventh step of Raw Material Synthesis 1 described later.

In the second step, the compound of formula (I-2) can be produced by performing an ipso substitution reaction of compound (18) under the same conditions as in the third step of Raw Material Synthesis 1 described later.

### (Raw Material Synthesis 1)

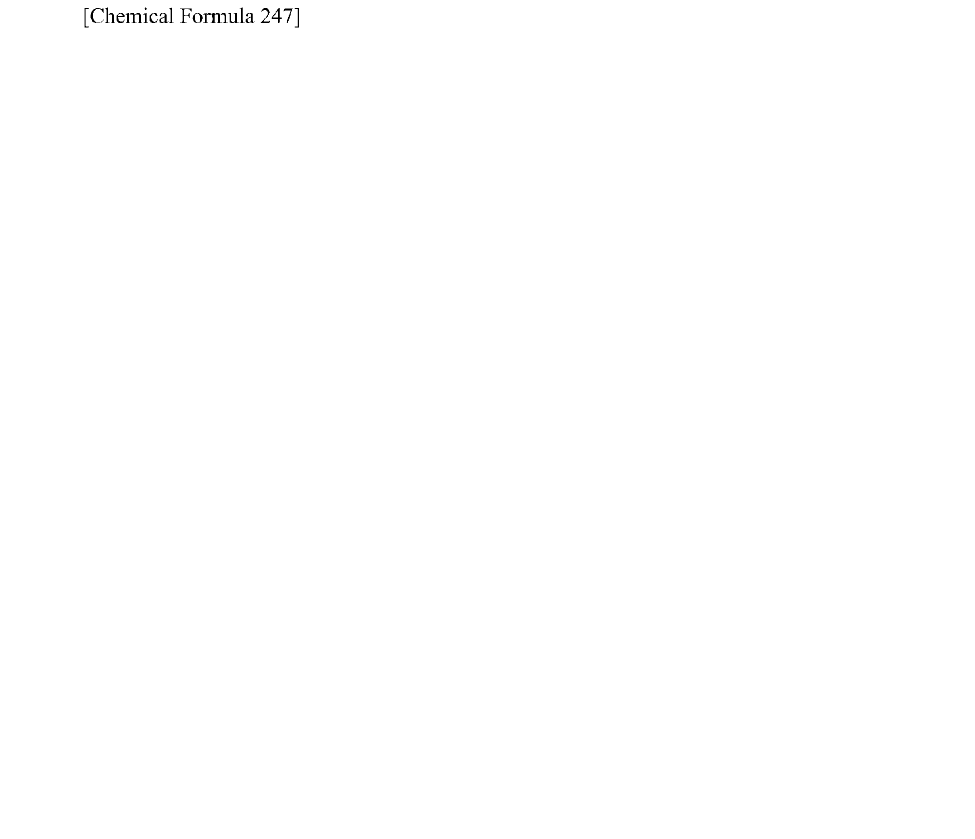

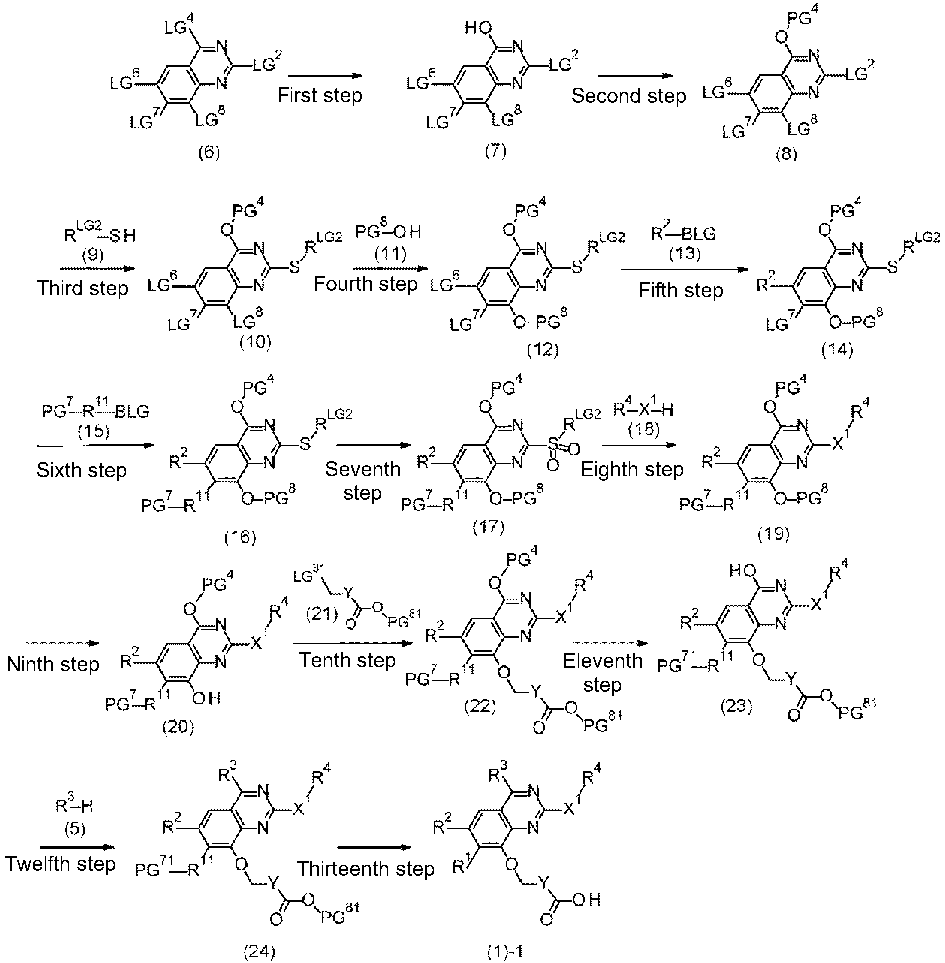

(wherein LG², LG⁴, LG⁶, LG⁷, LG⁸, and LG⁸¹, which are the same or different, each represent leaving group. PG⁴ represents protecting group of OH, PG⁷ and PG⁷¹ each represent protecting group of NH contained in R¹, R¹¹ represents divalent group formed by elimination of H from NH contained in R¹, PG⁸ represents protecting group which can be deprotected under catalytic hydrogenation reaction conditions, and PG⁸¹ represents protecting group of COOH. Moreover, the deprotection can also be performed in stages by selecting protecting groups deprotectable under different deprotection conditions as PG⁴, PG⁷, PG⁷¹, PG⁸, and PG⁸¹. R^{LG2} represents C₁₋₁₂ alkyl group, and BLG represents boronic acid group, boronic acid group protected with protecting group of boronic acid such as boronic acid pinacol ester group, or trifluoroborate group (hereinafter, sometimes described as boronic acid group or the like). Examples of the leaving group shown here include Cl, Br, I, methanesulfonyloxy group, and p-toluenesulfonyloxy group and the like. The same shall apply hereinafter)

This production method is the first method for producing compound (1)-1 included in compound (1), which is a raw material compound of Production Method 1, wherein A and E are N and CH, respectively.

### (First Step)

This step is a method for producing compound (7) from compound (6).

This reaction is performed by stirring compound (6) under a condition ranging from cooling to reflux with heat, generally for 0.1 hours to 5 days. Examples of the solvent used here include, but are not particularly limited to, an alcohol, acetone, DMF and THF. In addition, a mixed solvent of the above solvent and water is sometimes suitable for the reaction. Examples of the reagent used in this reaction include, but are not particularly limited to, aqueous sodium hydroxide and aqueous potassium hydroxide.

For example, the following can be referred to as a reference about this reaction.
The Chemical Society of Japan, "Jikken Kagaku Koza (Courses in Experimental Chemistry) (5th edition)", Vol. 16 (2005) (Maruzen)
Angew. Chem. Int. Ed. 2005, 44, p. 1378-1382.

### (Second Step)

This step is a method for producing compound (8) by protection of a hydroxyl group of compound (7) with the protecting group.

In the case of protection with a tert-butyl group, for example, this reaction is performed by stirring compound (7) in the presence of a tert-butyl protection reagent under a condition ranging from cooling to reflux with heat, generally for 0.1 hours to 5 days. Examples of the solvent used here include, but are not particularly limited to, an ether, such as THF and DOX, a halogenated hydrocarbon, such as dichloromethane, tBuOH, and DMF. Examples of the tert-butyl protection reagent include, but are not particularly limited to, isobutene, 2-tert-butyl-1,3-diisopropylisourea and the like.

Moreover, compound (8) can be produced by a dehydration condensation reaction of compound (7) and tBuOH.

For example, the following can be referred to as a reference about this reaction.
P. G. M. Wuts and T. W. Greene, "Greene's Protective Groups in Organic Synthesis", 5th edition, John Wiley & Sons Inc., 2014
Org. Lett., 2012, 14, 17, p. 4678-4681

### (Third Step)

This step is a method for producing compound (10) by an ipso substitution reaction of compound (8) and compound (9), R^{LG2}-SH.

Examples of the R^{LG2}-SH used here include C₁₋₁₂ alkylthiols, for example, ethanethiol and dodecanethiol.

In this reaction, compound (8) and compound (9) are used in an equal amount or with one compound thereof in an excess amount, and the mixture of the compounds is stirred in a solvent inactive for the reaction or with no solvent, under a condition ranging from cooling to reflux with heat, preferably at 0°C to 80°C, generally for 0.1 hours to 5 days. Examples of the solvent used here include, but are not particularly limited to, a halogenated hydrocarbon, such as dichloromethane, 1,2-dichloroethane and chloroform, an aromatic hydrocarbon, such as benzene, toluene and xylene, an ether, such as diethyl ether, THF, DOX and 1,2-dimethoxyethane, DMF, DMAc, DMSO, ethyl acetate, MeCN, NMP and a mixture thereof. Performing the reaction in the presence of an organic base, such as TEA, DIPEA, NMM, 1,4-diazabicyclo[2.2.2]octane (DABCO), tBuOK and KHMDS, or an inorganic base, such as sodium hydride, potassium carbonate, sodium carbonate and cesium carbonate, is sometimes advantageous for smoothly promoting the reaction.

### (Fourth Step)

This step is a method for producing compound (12) by an ipso substitution reaction of compound (10) and compound (11), PG⁸-OH. Examples of the PG⁸-OH used here include benzyl alcohol, p-methoxybenzyl alcohol and 1-phenylethanol.

The reaction conditions are the same as in the third step of Raw Material Synthesis 1.

### (Fifth Step)

This step is a method for producing compound (14) by a Suzuki-Miyaura coupling reaction of compound (12) and compound (13), a boronic acid derivative composed of an R²-boronic acid group or the like.

Examples of the boronic acid group or the like used here include, but are not particularly limited to, a boronic acid group, a boronic acid ester group, a boronic acid pinacol ester group, a triol borate salt group, and a trifluoroborate group.

In this reaction, compound (12) and the boronic acid derivative composed of the R²-boronic acid group or the like are used in an equal amount or with one compound thereof in an excess amount, and the mixture of the compounds is stirred in a solvent inactive for the reaction, in the presence of a base and a palladium catalyst, under a condition ranging from room temperature to reflux with heat, preferably at 20°C to 140°C, generally for 0.1 hours to 5 days. Examples of the solvent used here include, but are not particularly limited to, a halogenated hydrocarbon, such as dichloromethane, 1,2-dichloroethane or chloroform, an aromatic hydrocarbon, such as benzene, toluene or xylene, an ether, such as diethyl ether, THF, DOX or 1,2-dimethoxyethane, an alcohol, such as MeOH, EtOH, isopropyl alcohol, butanol or amyl alcohol, DMF, DMSO, MeCN, 1,3-dimethylimidazolidin-2-one, water and a mixture thereof. Examples of the base include an inorganic base, such as tripotassium phosphate, sodium carbonate, potassium carbonate, sodium hydroxide and barium hydroxide. Examples of the palladium catalyst include tetrakis(triphenylphosphine)palladium, PdCl₂(PPh₃)₂, PdCl₂(dppf)·CH₂Cl₂, Pd₂(dba)₃, RuPhos Pd G3, and palladium(II) acetate. Performing the reaction in the presence of a ligand, such as SPhos, RuPhos and 1,1'-bis(diphenylphosphino)ferrocene, is sometimes advantageous for smoothly promoting the reaction. In addition, heating the mixture by microwave irradiation is sometimes advantageous for smoothly promoting the reaction.

### [Reference]

J. Am. Chem. Soc., 2005, 127, p.4685-4696
Org. Lett. 2011, 13, p. 3948-3951
Org. Lett. 2012, 14, p. 1278-1281

When LG⁶ is halogen, compound (14) (where R² is hydrogen) can be produced by a dehalogenation reaction of compound (12) with Pd catalyst and a reducing agent.

### [Reference]

J. Org. Chem., 1977, 42, p.3491-3494
Tetrahedron Letters 2013, 54, 5207-5210

### (Sixth Step)

This step is a method for producing compound (16) by a Suzuki-Miyaura coupling reaction of compound (14) and compound (15).

The reaction conditions are the same as in the fifth step of Raw Material Synthesis 1. When compound (16) has an axial chirality, compound (16) is obtained as a mixture of stereoisomers, and each stereoisomer can be isolated by a common separation operation, for example, separation using ODS column chromatography or silica gel column chromatography.

### (Seventh Step)

This step is a method for producing compound (17) by an oxidation reaction of compound (16).

In this reaction, compound (16) is treated with an oxidant in an equal amount or an excess amount in a solvent inactive for the reaction, under a condition ranging from cooling to heating, preferably at -20°C to 80°C, generally for 0.1 hours to 3 days. In this reaction, oxidation with m-chloroperbenzoic acid, perbenzoic acid, peracetic acid, sodium hypochlorite or hydrogen peroxide is suitably used. Examples of the solvent include a halogenated hydrocarbon, such as dichloromethane, DMF, ethyl acetate, MeCN, and a mixture thereof. Other examples of the oxidant include cumene hydroperoxide, Oxone, active manganese dioxide, chromic acid, potassium permanganate, sodium periodate and the like.

### [Reference]

The Chemical Society of Japan, "Jikken Kagaku Koza (Courses in Experimental Chemistry)", 5th edition, Vol. 17, Maruzen, 2004
When compound (17) has an axial chirality, compound (17) may be obtained as a mixture of stereoisomers, and each stereoisomer can be isolated by a common separation operation, for example, separation using ODS column chromatography or silica gel column chromatography, and separation by SFC using a chiral column.

### (Eighth Step)

This step is a method for producing compound (19) by an ipso substitution reaction of compound (17) and compound (18).

The reaction conditions are the same as in the third step of Raw Material Synthesis 1.

### (Ninth Step)

This step is a method for producing compound (20) by deprotection by a catalytic hydrogenation reaction of compound (19).

This reaction can be performed by stirring compound (19) under hydrogen atmosphere, under a condition ranging from normal pressure to increased pressure, in a solvent inactive for the reaction, such as MeOH, EtOH, ethyl acetate and THF, in the presence of a metal catalyst, under a condition ranging from cooling to heating, preferably at room temperature, for 1 hour to 5 days. As the metal catalyst, a palladium catalyst, such as Pd/C or palladium black, a platinum catalyst, such as a platinum plate or platinum oxide, a nickel catalyst, such as reduced nickel or Raney nickel, or the like is used. The addition of an inorganic base, such as NaHCO₃, is sometimes suitable for the reaction to prevent deprotection of the protecting groups of PG⁴ and PG⁷.

### (Tenth Step)

This step is a method for producing compound (22) by a reaction of compound (20) and compound (21).

This reaction is performed using compound (20) and compound (21) in an equal amount or with one compound thereof in an excess amount by reacting a mixture of the compounds in the presence of a base, in a solvent inactive for the reaction, under a condition ranging from cooling to reflux with heat, preferably at 0°C to 80°C, generally for 0.1 hours to 5 days. Examples of the solvent used here include, but are not particularly limited to, an aromatic hydrocarbon, such as benzene, toluene and xylene, an alcohol, such as MeOH and EtOH, an ether, such as diethyl ether, THF, DOX and 1,2-dimethoxyethane, a halogenated hydrocarbon, such as dichloromethane, 1,2-dichloroethane and chloroform, DMF, DMSO, ethyl acetate, MeCN, and a mixture thereof. Examples of the base include, but are not particularly limited to, an organic base, such as TEA, DIPEA, 1,8-diazabicyclo[5.4.0]-7-undecene, n-butyllithium and tBuOK, and an inorganic base, such as sodium hydroxide, sodium carbonate, potassium carbonate, cesium carbonate and sodium hydride. Performing the reaction in the presence of a phase transfer catalyst, such as tetra-n-butylammonium chloride, is sometimes advantageous.

For example, the following can be referred to as a reference about this reaction.

The Chemical Society of Japan, "Jikken Kagaku Koza (Courses in Experimental Chemistry)", 5th edition, Vol. 14, Maruzen, 2005

Moreover, compound (21) in which LG⁸¹ is halogen can be produced by halogenation of a compound in which the moiety corresponding to LG⁸¹ is a hydroxy group. Examples of the halogenating agent used here include, but are not particularly limited to, thionyl chloride, phosphorus oxychloride, hydrobromic acid, phosphorus tribromide and the like.

For example, the following can be referred to as a reference about this reaction.

The Chemical Society of Japan, "Jikken Kagaku Koza (Courses in Experimental Chemistry)", 5th edition, Vol. 13, Maruzen, 2004

Furthermore, compound (21) in which LG⁸¹ is a sulfonyloxy group can be produced by sulfonylation of a compound in which the moiety corresponding to LG⁸¹ is a hydroxy group in the presence of a base. Examples of the sulfonylating reagent used here include, but are not particularly limited to, for example, methanesulfonyl chloride, p-toluenesulfonyl chloride, methanesulfonic anhydride and the like. Examples of the base include, but are not particularly limited to, for example, TEA, DIPEA, pyridine, tetramethylethylenediamine and the like.

For example, the following can be referred to as a reference about this reaction.

### Synthesis 1999, 9, p. 1633-1636

### (Eleventh Step)

This step is a method for producing compound (23) by performing deprotection of PG⁴ and PG⁷, which are the protecting groups of compound (22), and further protecting a deprotected NH group contained in R¹¹ with another protecting group PG⁷¹.

The reaction can be carried out under the same reaction conditions as in the step described in Production Method 2 by adding, in addition to compound (22) and the deprotection reagent, a protection reagent for protecting the NH group with a PG⁷¹ group.

### (Twelfth Step)

This step is a method for producing compound (24) by a reaction of compound (23) and compound (5).

The reaction conditions are the same as in the Production Method 3.

### (Thirteenth Step)

This step is a method for producing compound (1) by subjecting compound (24) to a deprotection reaction.

The reaction conditions are the same as in the step described in Production Method 2.

### (Raw Material Synthesis 2)

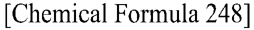

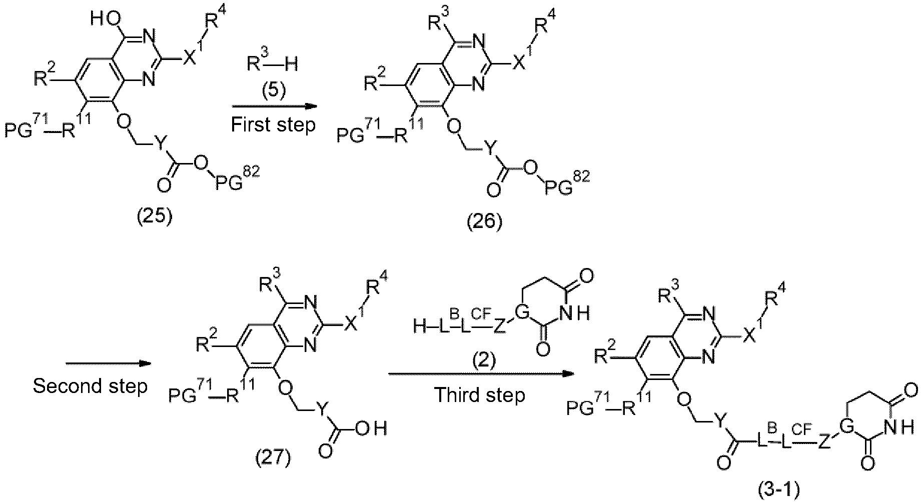

(wherein PG⁸² represents C₁₋₃ alkyl. The same shall apply hereinafter)

This production method is the first method for producing compound (3-1) included in compound (3), which is a raw material compound of Production Method 2, wherein A is N, -L^{A}- included in -L- is CO, -L^{B}- is -N(R^{L1A})- or a saturated heterocyclic divalent group containing 1 or 2 nitrogen atoms optionally substituted, -L^{C}-L^{D}-L^{E}-L^{F}- is -L^{CF}- and R^{L1A} is H or C₁₋₃ alkyl.

### (First Step)

This step is a method for producing compound (26) by a reaction of compound (25) and compound (5).

The reaction conditions are the same as in Production Method 3.

### (Second Step)

This step is a method for producing compound (27) by hydrolysis of compound (26) under basic conditions.

This reaction is carried out by stirring compound (26) under a condition ranging from cooling to reflux with heat, generally for 0.1 hours to 5 days. Examples of the solvent used here include, but are not particularly limited to, an alcohol, acetone, DMF and THF. In addition, a mixed solvent of the above solvent and water is sometimes suitable for the reaction. Examples of the hydrolysis reagent include, but are not particularly limited to, aqueous sodium hydroxide, aqueous potassium hydroxide, and trimethyltin hydroxide.

For example, the following can be referred to as a reference about this reaction.
The Chemical Society of Japan, "Jikken Kagaku Koza (Courses in Experimental Chemistry) (5th edition)", Vol. 16 (2005) (Maruzen)
Angew. Chem. Int. Ed. 2005, 44, p. 1378-1382.

### (Third Step)

This step is a method for producing compound (3-1) by subjecting compound (27) and compound (2) to an amidation reaction.

The reaction conditions are the same as in the step described in Production Method 1.

### (Raw Material Synthesis 3)

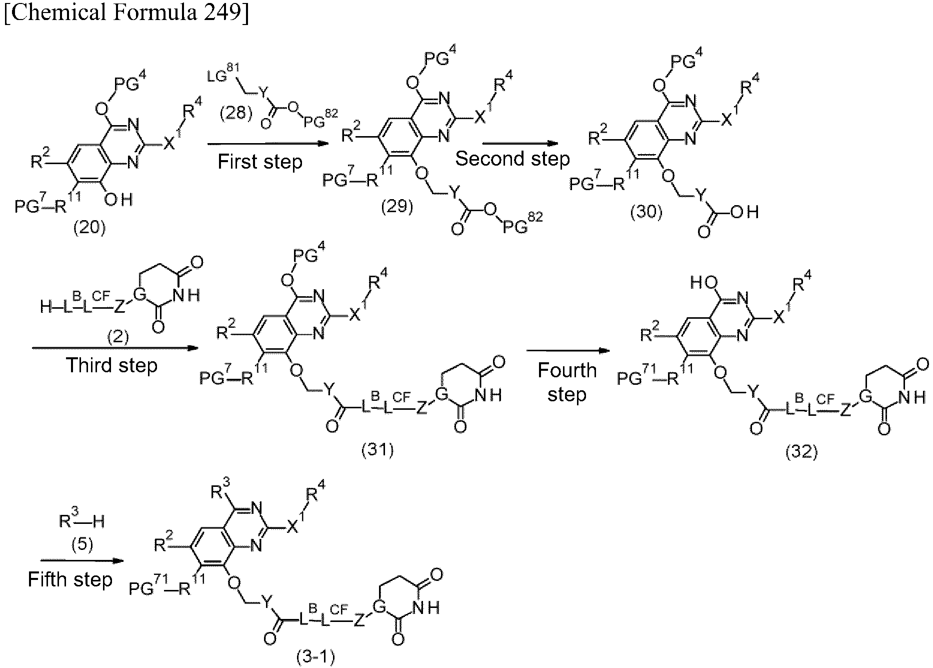

This production method is the second method for producing compound (3-1) included in compound (3), which is a raw material compound of Production Method 2, wherein A is N, -L^{A}- included in -L- is CO, -L^{B}- is -N(R^{1A})- or a saturated heterocyclic divalent group containing 1 or 2 nitrogen atoms optionally substituted, -L^{C}-L^{D}-L^{E}-L^{F}- is - L^{CF}-, and R^{L1A} is H or C₁₋₆ alkyl.

### (First Step)

This step is a method for producing compound (29) by a reaction of compound (20) and compound (28).

The reaction conditions are the same as in the tenth step of Raw Material Synthesis 1.

### (Second Step)

This step is a method for producing compound (30) by subjecting compound (29) to a deprotection reaction.

The reaction conditions are the same as in the second step of Raw Material Synthesis 2.

### (Third Step)

This step is a method for producing compound (31) by subjecting compound (30) and compound (2) to an amidation reaction.

The reaction conditions are the same as in the step described in Production Method 1.

### (Fourth Step)

This step is a method for producing compound (32) by performing deprotection of PG⁴ and PG⁷, which are the protecting groups of compound (31), and further protecting a deprotected NH group contained in R¹¹ with another protecting group PG⁷¹.

The reaction can be carried out under the same reaction conditions as in the step described in Production Method 2 by adding, in addition to compound (31) and the deprotection reagent, a protection reagent for protecting the NH group with the PG⁷¹ group.

### (Fifth Step)

This step is a method for producing compound (3-1) by a reaction of compound (32) and compound (5).

The reaction conditions are the same as in Production Method 3.

### (Raw Material Synthesis 4)

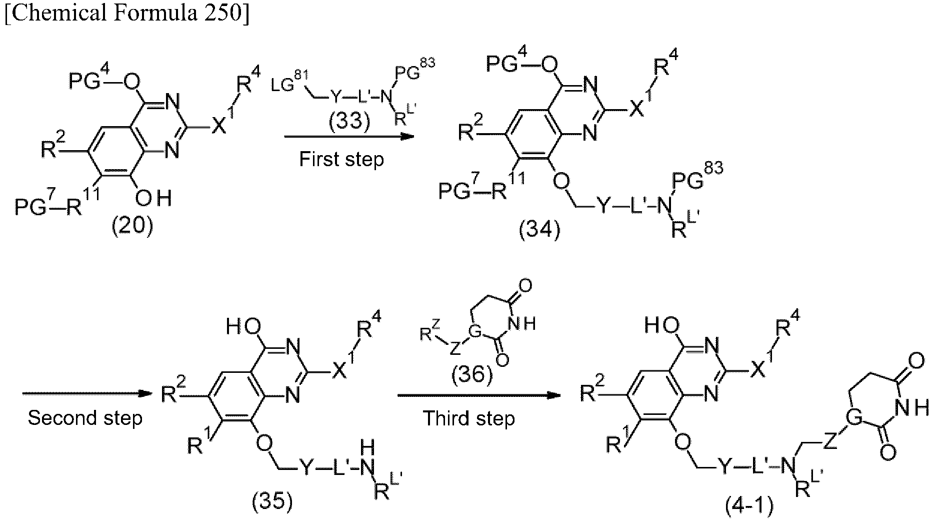

(wherein -L'- represents -L^{A}-L^{B}-L^{C}-L^{D}- contained in -L-, and PG⁸³ represents a protecting group of NH bonded to L', wherein R^{L}' is H or C₁₋₃ alkyl, or R^{L}' together with a nitrogen atom and L' adjacent thereto may form divalent group of saturated hetero ring containing 1 or 2 nitrogen atoms)

This production method is a method for producing compound (4-1) included in compound (4), which is a raw material compound of Production Method 3, wherein -L^{E}- and -L^{F}- contained in -L- are -N(R^{L}')- and -CH₂-, respectively.

### (First Step)

This step is a method for producing compound (34) by a reaction of compound (20) and compound (33).

The reaction conditions are the same as in the tenth step of Raw Material Synthesis 1.

### (Second Step)

This step is a method for producing compound (35) by subjecting compound (34) to a deprotection reaction.

The reaction conditions are the same as in the step described in Production Method 2.

### (Third Step)

This step is a step of producing compound (4-1) from compound (35) and compound (36) by a reductive amination reaction when -R^{Z} is -CHO or by an alkylation reaction when - R^{z} is -CH₂-LG⁸¹.

When -R^{Z} is -CHO, the reaction is carried out by stirring compound (35) and compound (36) in an equivalent amount or with one compound thereof in an excess equivalent amount in the presence of a reducing agent and acetic acid, in a solvent inactive for the reaction, under a condition ranging from ice-bath cooling to room temperature, generally for 1 hour to 5 days. Examples of the reducing agent used here include, but are not particularly limited to, NaBH(OAc)₃, 2-picoline borane, and NaBH₃CN. Examples of the solvent used here include, but are not particularly limited to, a halogenated hydrocarbon, such as dichloromethane, dichloroethane and chloroform, an ether-based solvent, such as THF, diethyl ether and DOX, an alcohol-based solvent, such as MeOH and EtOH, and MeCN.

When -R^{Z} is -CH₂-LG⁸¹, the reaction conditions are the same as in the tenth step of Raw Material Synthesis 1.

### (Raw Material Synthesis 5)

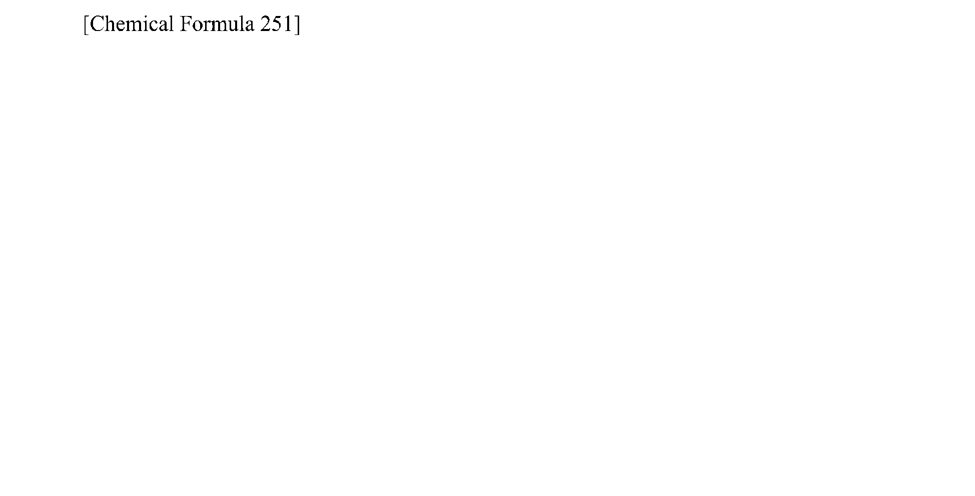

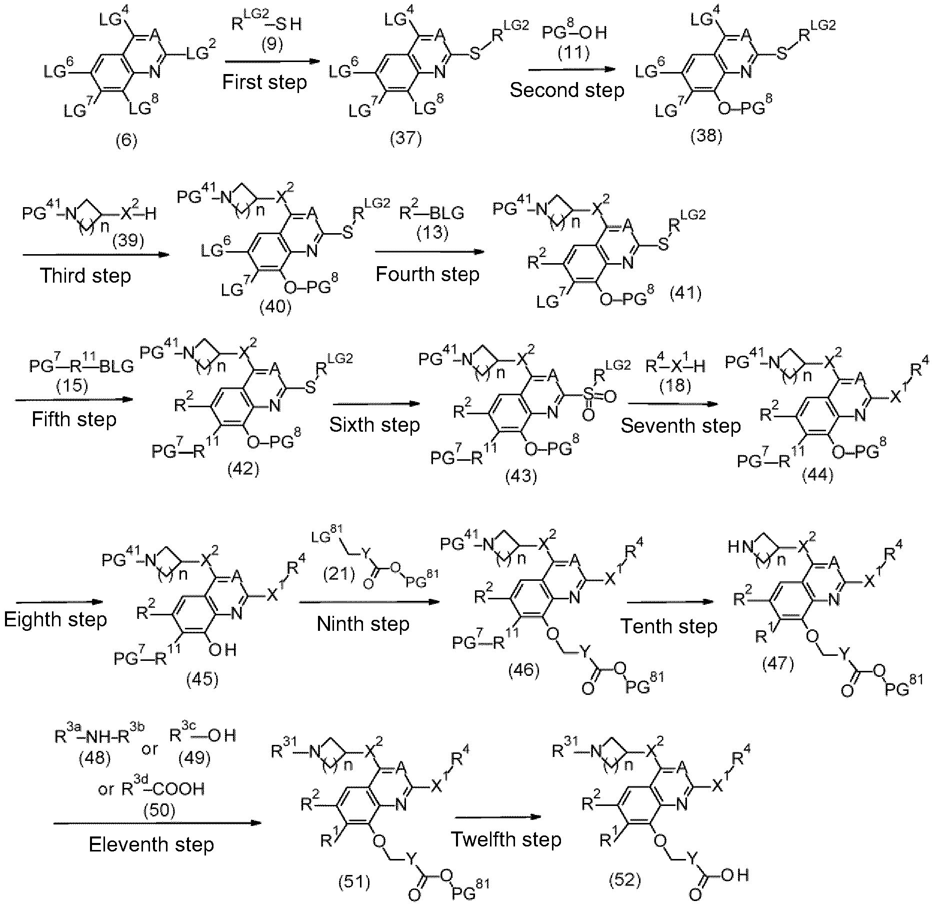

(wherein PG⁴¹ represents a protecting group of NH, and R³¹ represents R^{3a}-N(R^{3b})CO-, R^{3c}-OCO- or R^{3d}-CO-)

This production method is a method for producing compound (52) included in compound (1), which is a raw material compound of Production Method 1, wherein E is CH, R³ is formula (VIII), formula (IX) or formula (X), and X³ is O.

### (First Step)

This step is a method for producing compound (37) by an ipso substitution reaction of compound (6) and compound (9), R^{LG2}-SH.

The reaction conditions are the same as in the third step of Raw Material Synthesis 1.

### (Second Step)

This step is a method for producing compound (38) by an ipso substitution reaction of compound (37) and compound (11).

The reaction conditions are the same as in the fourth step of Raw Material Synthesis 1.

### (Third Step)

This step is a method for producing compound (40) by an ipso substitution reaction of compound (38) and compound (39), which has an amino group protected with the protecting group PG⁴¹.

In this reaction, compound (38) and compound (39) are used in an equal amount or with one compound thereof in an excess amount, and the mixture of the compounds is stirred in a solvent inactive for the reaction, under a condition ranging from cooling to heating, preferably at -20°C to 60°C, generally for 0.1 hours to 5 days. Examples of the solvent include, but are not particularly limited to, an aromatic hydrocarbon, such as toluene, a halogenated hydrocarbon, such as dichloromethane, DMF, DMSO, ethyl acetate, MeCN, and a mixture thereof. Performing the reaction in the presence of an organic base, such as TEA, DIPEA or NMM, or an inorganic base, such as potassium carbonate, sodium carbonate or cesium carbonate, is sometimes advantageous for smoothly promoting the reaction.

### (Fourth Step)

This step is a method for producing compound (41) by a Suzuki-Miyaura coupling reaction of compound (40) and compound (13), a boronic acid derivative composed of an R²-boronic acid group or the like.

The reaction conditions are the same as in the fifth step of Raw Material Synthesis 1.

### (Fifth Step)

This step is a method for producing compound (42) by a Suzuki-Miyaura coupling reaction of compound (41) and compound (15).

The reaction conditions are the same as in the fifth step of Raw Material Synthesis 1.

When compound (42) has an axial chirality, compound (16) is obtained as a mixture of stereoisomers, and each stereoisomer can be isolated by a common separation operation, for example, separation using ODS column chromatography or silica gel column chromatography.

### (Sixth Step)

This step is a method for producing compound (43) by an oxidation reaction of compound (42).

The reaction conditions are the same as in the seventh step of Raw Material Synthesis 1.

When compound (43) has an axial chirality, the compound may be obtained as a mixture of stereoisomers, and each stereoisomer can be isolated by a common separation operation, for example, separation using ODS column chromatography or silica gel column chromatography, and separation by SFC using a chiral column.

### (Seventh Step)

This step is a method for producing compound (44) by an ipso substitution reaction of compound (43) and compound (18).

The reaction conditions are the same as in the third step of Raw Material Synthesis 1.

### (Eighth Step)

This step is a method for producing compound (45) by deprotection by a catalytic hydrogenation reaction of compound (44).

The reaction conditions are the same as in the ninth step of Raw Material Synthesis 1.

### (Ninth Step)

This step is a method for producing compound (46) by a reaction of compound (45) and compound (21).

The reaction conditions are the same as in the tenth step of Raw Material Synthesis 1.

### (Tenth Step)

This step is a method for producing compound (47) by subjecting compound (46) to a deprotection reaction.

The reaction conditions are the same as in the step described in Production Method 2.

### (Eleventh Step)

This step is a step of producing compound (51) by subjecting compound (47) to a urea formation reaction using compound (48), a carbamate formation reaction using compound (49), and an amidation reaction using compound (50).

The urea formation reaction using compound (48) and the carbamate formation reaction using compound (49) were performed by using an equal amount of compound (48) or compound (49) with respect to compound (47) or one of them in excess over the other, and stirring the mixture of the compounds in the presence of a condensing agent, in a solvent inactive for the reaction, under a condition ranging from cooling to heating, preferably at - 20°C to 60°C, generally for 0.1 hours to 5 days. Examples of the solvent include, but are not particularly limited to, an aromatic hydrocarbon, such as toluene, an ether, such as THF and DOX, a halogenated hydrocarbon, such as dichloromethane, an alcohol, DMF, DMSO, ethyl acetate, MeCN, and a mixture thereof. Examples of the condensing agent include CDI, triphosgene, bis(4-nitrophenyl) carbonate, and 4-nitrophenyl chloroformate. Use of an additive (for example, HOBt or dimethylaminopyridine) is sometimes preferred for the reaction. Performing the reaction in the presence of an organic base, such as TEA, DIPEA and NMM, or an inorganic base, such as potassium carbonate, sodium carbonate and potassium hydroxide, is sometimes advantageous for smoothly promoting the reaction.

The reaction conditions for the amidation reaction using compound (50) are the same as in the step described in Production Method 1.

### (Twelfth Step)

This step is a method for producing compound (52) by subjecting compound (51) to a deprotection reaction.

The reaction conditions are the same as in the step described in Production Method 2.

### (Raw Material Synthesis 6)

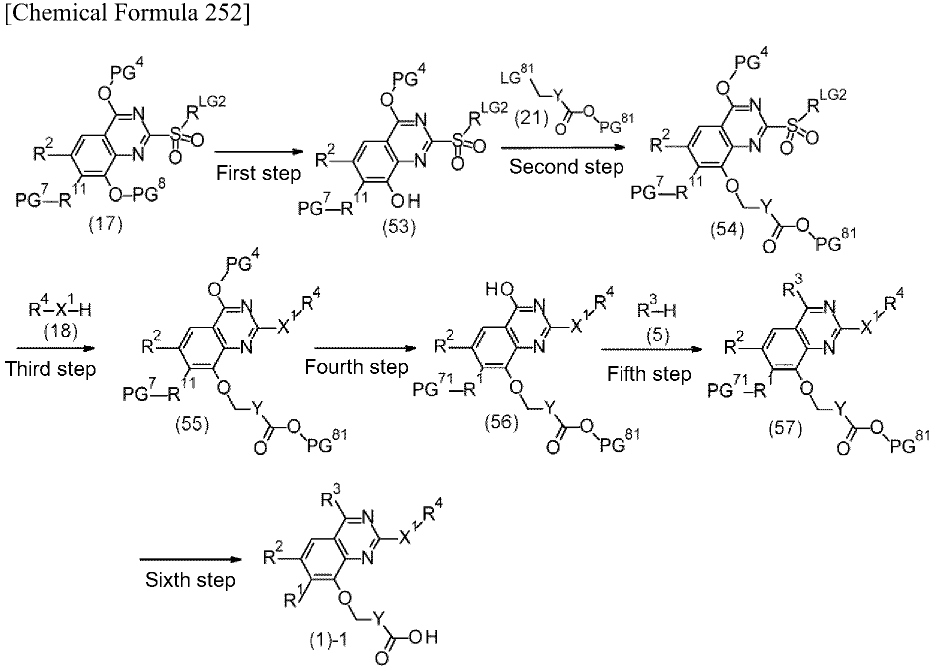

This production method is the second method for producing compound (1)-1 included in compound (1), which is a raw material compound of Production Method 1, wherein A and E are N and CH, respectively.

### (First Step)

This step is a method for producing compound (53) by deprotection by a catalytic hydrogenation reaction of compound (17).

The reaction conditions are the same as in the ninth step of Raw Material Synthesis 1.

### (Second Step)

This step is a method for producing compound (54) by a reaction of compound (53) and compound (21).

The reaction conditions are the same as in the tenth step of Raw Material Synthesis 1.

### (Third Step)

This step is a method for producing compound (55) by an ipso substitution reaction of compound (54) and compound (18).

The reaction conditions are the same as in the third step of Raw Material Synthesis 1.

The reaction in the fourth step can also be carried out without isolating compound (55) in this step.

### (Fourth Step)

This step is a method for producing compound (56) by subjecting compound (55) to a deprotection reaction.

The reaction can be carried out under the same reaction conditions as in the step described in Production Method 2 by adding, in addition to compound (55) and the deprotection reagent, a protection reagent such as dihydropyran for protecting the NH group with a PG⁷¹ group.

### (Fifth Step)

This step is a method for producing compound (57) by a reaction of compound (56) and compound (5).

The reaction conditions are the same as in Production Method 3.

### (Sixth Step)

This step is a method for producing compound (1)-1 by subjecting compound (57) to a deprotection reaction.

The reaction conditions are the same as in the step described in Production Method 2.

### (Raw Material Synthesis 7)

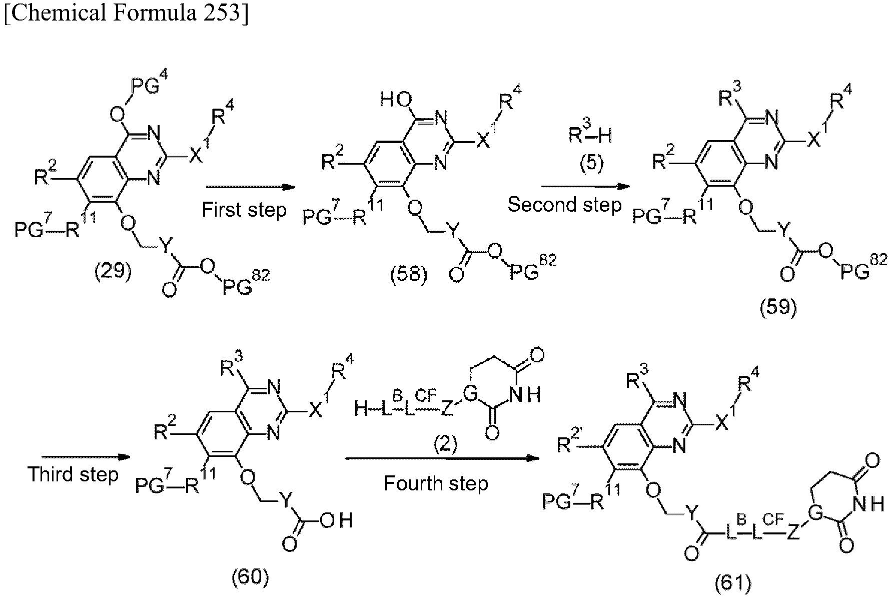

This production method is a method for producing compound (61) included in compound (3), which is a raw material compound of Production Method 2, wherein A is N, - L^{A}- contained in -L- is CO, -L^{B}- is -N(R^{L1A})- or divalent group of optionally substituted saturated hetero ring containing 1 or 2 nitrogen atoms, -L^{C}-L^{D}-L^{E}-L^{F}- is -L^{CF}-, and R^{L1A} is H or C₁₋₆ alkyl.

### (First Step)

This step is a method for producing compound (58) by subjecting compound (29) to a deprotection reaction.

The reaction conditions are the same as in the step described in Production Method 2.

### (Second Step)

This step is a method for producing compound (59) by a reaction of compound (58) and compound (5).

The reaction conditions are the same as in Production Method 3.

### (Third Step)

This step is a method for producing compound (60) by subjecting compound (59) to a deprotection reaction.

The reaction conditions are the same as in the second step of Raw Material Synthesis 2.

### (Fourth Step)

This step is a method for producing compound (61) by subjecting compound (60) and compound (2) to an amidation reaction.

The reaction conditions are the same as in the step described in Production Method 1.

### (Raw Material Synthesis 8)

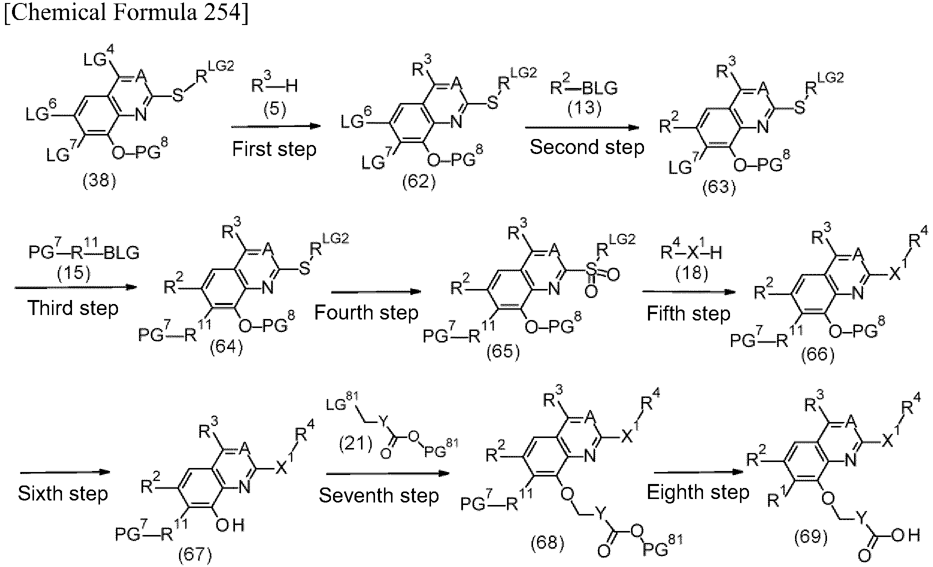

This production method is a method for producing compound (69), which is a raw material compound of Production Method 4.

### (First Step)

This step is a method for producing compound (62) by an ipso substitution reaction of compound (38) and compound (5).

The reaction conditions are the same as in the third step of Raw Material Synthesis 5.

### (Second Step)

This step is a method for producing compound (63) by a Suzuki-Miyaura coupling reaction of compound (62) and compound (13), a boronic acid derivative composed of an R²-boronic acid group or the like.

The reaction conditions are the same as in the fifth step of Raw Material Synthesis 1.

### (Third Step)

This step is a method for producing compound (64) by a Suzuki-Miyaura coupling reaction of compound (63) and compound (15).

The reaction conditions are the same as in the fifth step of Raw Material Synthesis 1.

When compound (64) has an axial chirality, compound (16) is obtained as a mixture of stereoisomers, and each stereoisomer can be isolated by a common separation operation, for example, separation using ODS column chromatography or silica gel column chromatography.

### (Fourth Step)

This step is a method for producing compound (65) by an oxidation reaction of compound (64).

The reaction conditions are the same as in the seventh step of Raw Material Synthesis 1.

When compound (65) has an axial chirality, the compound may be obtained as a mixture of stereoisomers, and each stereoisomer can be isolated by a common separation operation, for example, separation using ODS column chromatography or silica gel column chromatography, and separation by SFC using a chiral column.

### (Fifth Step)

This step is a method for producing compound (66) by an ipso substitution reaction of compound (65) and compound (18).

The reaction conditions are the same as in the third step of Raw Material Synthesis 1.

### (Sixth Step)

This step is a method for producing compound (67) by deprotection by a catalytic hydrogenation reaction of compound (66).

The reaction conditions are the same as in the ninth step of Raw Material Synthesis 1.

### (Seventh Step)

This step is a method for producing compound (68) by a reaction of compound (67) and compound (21).

The reaction conditions are the same as in the tenth step of Raw Material Synthesis 1.

### (Eighth Step)

This step is a method for producing compound (69) by subjecting compound (68) to a deprotection reaction.

The reaction conditions are the same as in the step described in Production Method 2.

### (Raw Material Synthesis 9)

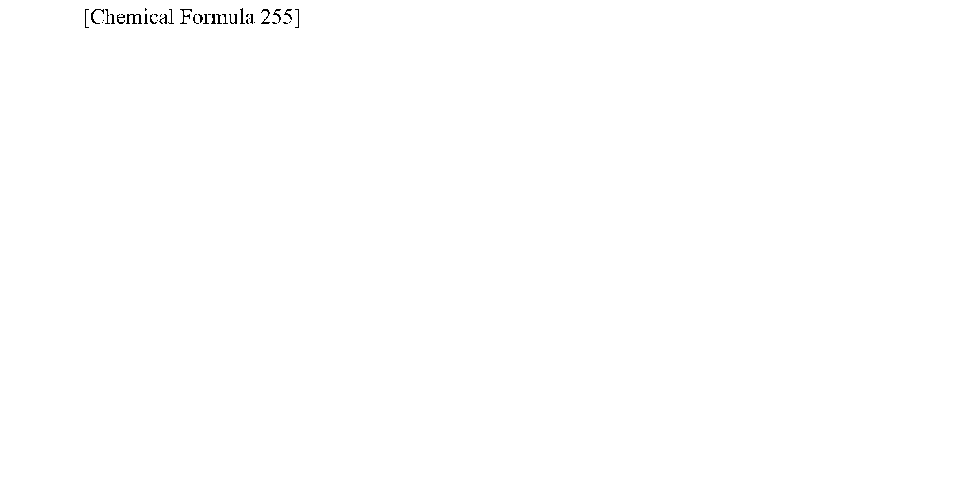

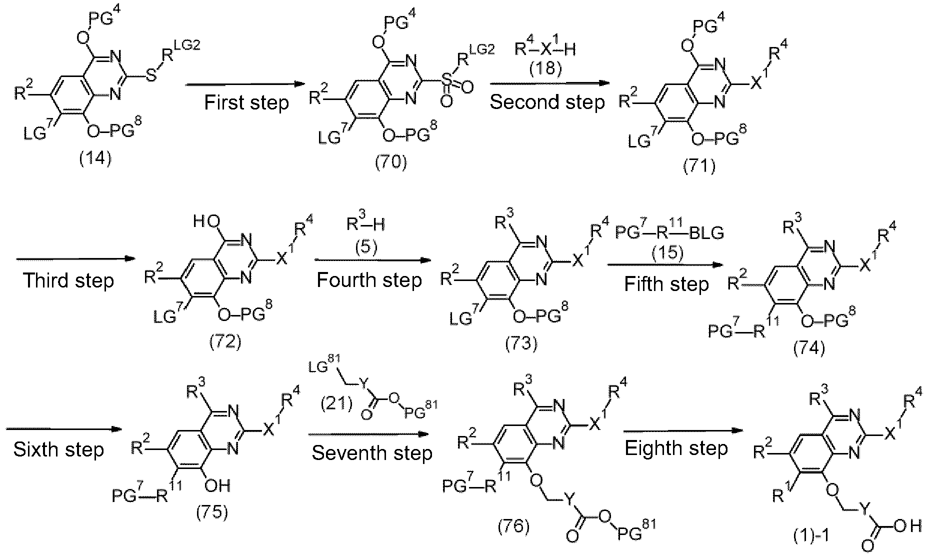

This production method is the third method for producing compound (1)-1 included in compound (1), which is a raw material compound of Production Method 1, wherein A and E are N and CH, respectively.

### (First Step)

This step is a method for producing compound (70) by an oxidation reaction of compound (14).

The reaction conditions are the same as in the seventh step of Raw Material Synthesis 1.

### (Second Step)

This step is a method for producing compound (71) by an ipso substitution reaction of compound (70) and compound (18).

The reaction conditions are the same as in the third step of Raw Material Synthesis 1.

### (Third Step)

This step is a method for producing compound (72) by a deprotection reaction of compound (71).

The reaction conditions are the same as in Production Method 2.

### (Fourth Step)

This step is a method for producing compound (73) by a reaction of compound (72) and compound (5).

The reaction conditions are the same as in the Production Method 3.

### (Fifth Step)

This step is a method for producing compound (74) by a Suzuki-Miyaura coupling reaction of compound (73) and compound (15).

The reaction conditions are the same as in the fifth step of Raw Material Synthesis 1.

When compound (74) has an axial chirality, compound (16) is obtained as a mixture of stereoisomers, and each stereoisomer can be isolated by a common separation operation, for example, separation using ODS column chromatography or silica gel column chromatography.

### (Sixth Step)

This step is a method for producing compound (75) by deprotection by a catalytic hydrogenation reaction of compound (74).

The reaction conditions are the same as in the ninth step of Raw Material Synthesis 1.

### (Seventh Step)

This step is a method for producing compound (76) by a reaction of compound (75) and compound (21).

The reaction conditions are the same as in the tenth step of Raw Material Synthesis 1.

### (Eighth Step)

This step is a method for producing compound (1)-1 by subjecting compound (76) to a deprotection reaction.

The reaction conditions are the same as in the step described in Production Method 2.

### (Raw Material Synthesis 10)

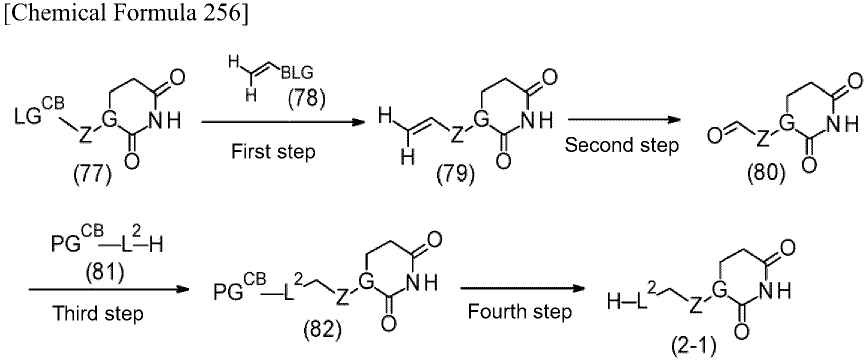

(wherein LG^{CB} represents a leaving group, and PG^{CB} represents a protecting group of NH contained in L²)

This production method is a method for producing compound (2-1) included in compound (2), which is a raw material compound of Production Method 1, wherein -L²- and -L³⁵- are divalent group of optionally substituted saturated hetero ring containing 2 nitrogen atoms and -CH₂-, respectively.

### (First Step)

This step is a method for producing compound (79) by a Suzuki-Miyaura coupling reaction of compound (77) and compound (78).

The reaction conditions are the same as in the fifth step of Raw Material Synthesis 1.

### (Second Step)

This step is a method for producing compound (80) which is an aldehyde from compound (79).

In this reaction, compound (79) and osmium tetroxide are reacted in the presence of an organic base, in a solvent inactive for the reaction, under a condition ranging from cooling to room temperature, to obtain a corresponding 1,2-diol compound, and then the 1,2-diol is oxidized by adding periodic acids to the reaction mixture to thus obtain compound (80), which is an aldehyde. Examples of the solvent used here include an alcohol, such as tBuOH, an ether, such as THF, DOX and 1,2-dimethoxyethane, an aromatic hydrocarbon, such as benzene, toluene and xylene, acetone, and a mixed solvent of these compounds and water. Examples of the organic base used here include pyridine and 2,6-lutidine, and examples of the periodic acid used here include sodium periodate and periodic acid.

In this reaction, compound (80) can also be produced by performing an oxidative cleavage reaction using ozone instead of osmium tetroxide and periodic acid. For example, the following can be referred to as a reference about this reaction:
Chem. Rev., 1958, 58, 5, p. 925-1010.

### (Third Step)

This step is a method for producing compound (82) by a reductive amination reaction using compound (80) and an amine compound, compound (81).

The reaction conditions are the same as when -R^{Z} is -CHO in third step of Raw Material Synthesis 4.

### (Fourth Step)

This step is a reaction step of producing compound (2-1) by subjecting compound (82) to a deprotection reaction.

The reaction conditions are the same as in the Production Method 2.

### (Raw Material Synthesis 11)

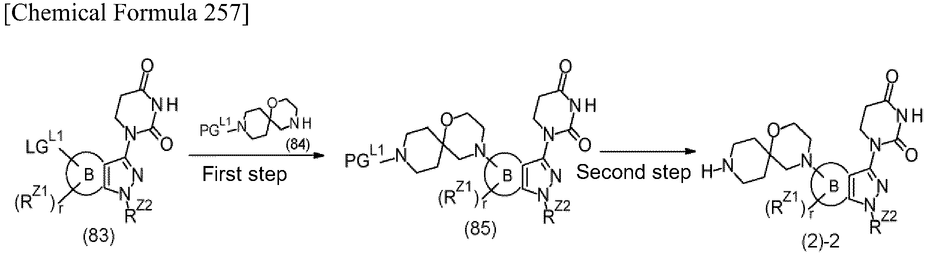

(PG^{L1} represents a protecting group of an amino group contained in compound (2)-2, for example, a tert-butoxycarbonyl group. LG^{L1} represents a leaving group, for example, halogen, and is Br in one embodiment.)

This production method is a method for producing compound (2)-2 included in compound (2), which is a raw material compound of Production Method 1, wherein L^{B} is a group represented by the formula (XXX-L^{B}), L^{CF} is bond, Z is a group represented by the following formula (XXII), and G is N.

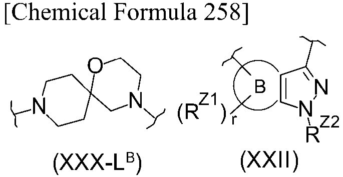

### (First Step)

This step is a method for producing compound (85) by reacting compound (83) and compound (84).

In this reaction, compound (83) and compound (84) are used in an equal amount or with one compound thereof in an excess amount, and the mixture of the compounds is stirred in a solvent inactive for the reaction, in the presence of a base and a palladium catalyst, under a condition ranging from room temperature to reflux with heat, preferably at 20°C to 140°C, generally for 0.1 hours to 5 days. Examples of the solvent used here include, but are not particularly limited to, a halogenated hydrocarbon, such as dichloromethane, 1,2-dichloroethane or chloroform, an aromatic hydrocarbon, such as benzene, toluene or xylene, an ether, such as diethyl ether, THF, DOX or 1,2-dimethoxyethane, an alcohol, such as MeOH, EtOH, isopropyl alcohol, butanol or amyl alcohol, DMF, DMSO, MeCN, 1,3-dimethylimidazolidin-2-one, water and a mixture thereof. Examples of the base include an inorganic base, such as tBuONa, tBuOK, cesium carbonate, tripotassium phosphate, sodium carbonate, potassium carbonate, sodium hydroxide and barium hydroxide and the like. Examples of the palladium catalyst include Pd(t-Bu₃P)₂, tetrakis(triphenylphosphine)palladium, bis(triphenylphosphine)palladium(II) dichloride, PdCl₂(dppf)·CH₂Cl₂, Pd₂(dba)₃, RuPhos Pd G3, and palladium(II) acetate. Performing the reaction in the presence of a ligand, such as SPhos, RuPhos, 1,1'-bis(diphenylphosphino)ferrocene and Xphos and the like, is sometimes advantageous for smoothly promoting the reaction. In addition, heating the mixture by microwave irradiation is sometimes advantageous for smoothly promoting the reaction.

### (Second Step)

This step is a method for producing compound (2)-2 by a deprotection reaction of compound (85).

The reaction conditions are the same as in the step described in Production Method 2.

### (Raw Material Synthesis 12)

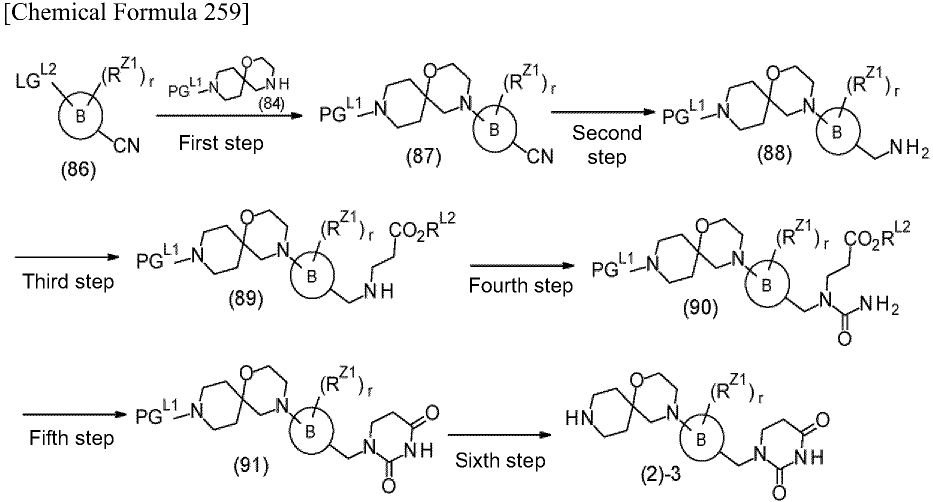

(LG^{L2} represents a leaving group, for example, halogen, and is F in one embodiment.)

This production method is a method for producing compound (2)-3 included in compound (2), which is a raw material compound of Production Method 1, wherein L^{B} is the following formula (XXX-L^{B}), L^{CF} is bond, Z is a group represented by formula (XXVI), and G is N.

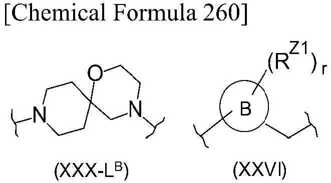

### (First Step)

This step is a method for producing compound (87) by an ipso substitution reaction of compound (86) and compound (84).

The reaction conditions are the same as in the third step of Raw Material Synthesis 5.

### (Second Step)

This step is a method for producing compound (88) by reducing compound (87).

The reaction is carried out by stirring compound (87) and a reducing agent in an equivalent amount or with one in an excess equivalent amount in a solvent inactive for the reaction, under a condition ranging from cooling to heating, preferably at -20°C to 100°C, generally for 1 hours to 5 days. Examples of the reducing agent used here include, but are not particularly limited to, BH₃·THF, LAH, and LiBH₄ and the like. Examples of the solvent used here include, but are not particularly limited to, a halogenated hydrocarbon, such as dichloromethane and dichloroethane, an ether-based solvent, such as THF, diethyl ether and DOX, and an aromatic hydrocarbon, such as toluene and benzene and the like.

Compound (88) can also be produced by reducing compound (87) through a hydrogenation reaction in the presence of a metal catalyst. In this case, Raney nickel and MeOH, for example, can be used as the metal catalyst and the solvent, respectively. In addition, addition of aqueous ammonia is sometimes suitable for the reaction.

### (Third Step)

This step is a method for producing compound (89) by reacting compound (88) with acrylic acid ester.

The reaction is carried out by stirring compound (88) and the acrylic acid ester in an equivalent amount or with one in an excess equivalent amount in a solvent inactive for the reaction, under a condition ranging from cooling to heating, preferably at -20°C to 150°C, generally for 1 hour to 5 days. Examples of the acrylic acid ester used here include methyl acrylate and ethyl acrylate. Examples of the solvent used here include, but are not particularly limited to, a halogenated hydrocarbon, such as dichloromethane, dichloroethane and chloroform, and an ether-based solvent, such as THF, diethyl ether and DOX and the like.

### (Fourth Step)

This step is a method for producing compound (90) by reacting compound (89) with a urea-forming reagent.

The reaction is carried out by stirring compound (89) and the urea-forming reagent in an equivalent amount or with one in an excess equivalent amount in a solvent inactive for the reaction, under a condition ranging from cooling to heating, preferably at -20°C to 150°C, generally for 1 hour to 5 days. Examples of the urea-forming reagent used here include trimethylsilyl isocyanate and chlorosulfonyl isocyanate and the like. Examples of the solvent used here include, but are not particularly limited to, a halogenated hydrocarbon, such as dichloromethane, dichloroethane and chloroform, and an ether-based solvent, such as THF, diethyl ether and DOX and the like.

Compound (90) can also be produced by reacting compound (89) with triphosgene or 1,1'-carbonyldiimidazole, followed by treatment with aqueous ammonia.

### (Fifth Step)

This step is a method for producing compound (91) from compound (90).

The reaction is carried out by stirring compound (90) and a weak base in an equivalent amount or with one in an excess equivalent amount in a solvent inactive for the reaction, under a condition ranging from cooling to heating, preferably at -20°C to 100°C, generally for 1 hour to 5 days. Examples of the weak base used here include benzyltrimethylammonium hydroxide, potassium carbonate, potassium trimethylsilanolate and the like. Examples of the solvent used here include, but are not particularly limited to, a halogenated hydrocarbon, such as dichloromethane, dichloroethane and chloroform, an ether-based solvent, such as THF, diethyl ether and DOX, and MeCN and the like.

### (Sixth Step)

This step is a method for producing compound (2)-3 by deprotection of compound (91).

The reaction conditions are the same as in the Production Method 2.

### (Raw Material Synthesis 13)

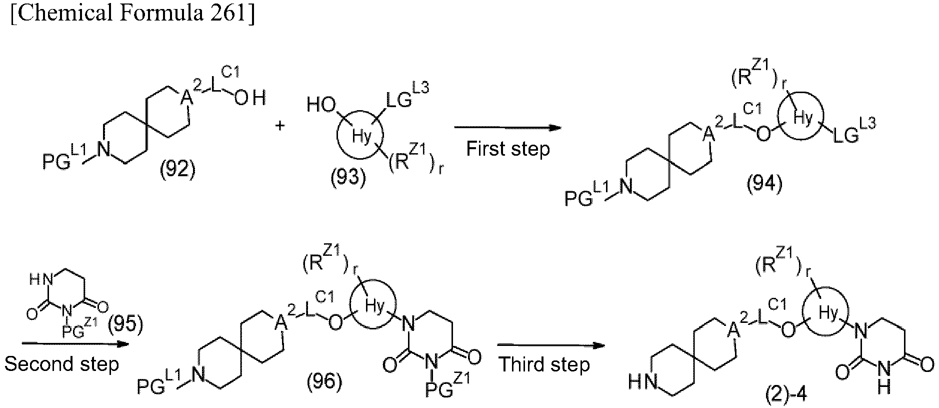

(LG^{L3} represents a leaving group, such as halogen, and is I in one embodiment. PG^{Z1} represents a protecting group of NH contained in compound (95), such as a p-methoxybenzyl group.)

This production method is the first method for producing compound (2)-4 included in compound (2), which is a raw material compound of Production Method 1, wherein L^{B} is a group represented by the following formula (XXXIII-L^{B2}), A² is CH or N, L^{CF} is -L^{C1}-O-, L^{C1} represents -L^{C}-L^{D}-L^{E}-, Z is a group represented by the following formula (XXVII), and G is N.

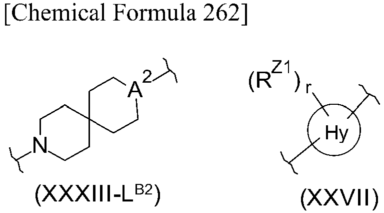

### (First Step)

This step is a method for producing compound (94) by a Mitsunobu reaction of compound (92) and compound (93).

The reaction is carried out by stirring compound (92) and compound (93) in an equivalent amount or with one compound thereof in an excess equivalent amount using a phosphine reagent and an azodicarboxylic acid ester in a solvent inactive for the reaction, under a condition ranging from cooling to heating, preferably at -20°C to 100°C, generally for 1 hour to 5 days. Examples of the phosphine reagent used here include triphenylphosphine, tricyclohexylphosphine, and tri-n-butylphosphine and the like. Examples of the azodicarboxylic acid ester used here include dimethyl azodicarboxylate, diethyl azodicarboxylate, and diisopropyl azodicarboxylate and the like. Examples of the solvent used here include, but are not particularly limited to, an ether-based solvent, such as THF, diethyl ether and DOX, benzene, and toluene and the like.

Note that an azodicarboxylic acid amide, such as N,N,N',N'-tetramethylazodicarboxamide and bis(piperidinocarbonyl)diimide, may also be used instead of the azodicarboxylic acid ester.

Moreover, compound (94) can be produced by an ipso substitution reaction of compound (92), in the presence of a base, with a compound in which OH of compound (93) is halogen. In this case, sodium hydride or tBuOK can be used as the base, and the other conditions are the same as in the third step of Raw Material Synthesis 1.

### (Second Step)

This step is a method for producing compound (96) by a reaction of compound (94) and compound (95).

This reaction is carried out by stirring compound (94) and compound (95) in an equivalent amount or with one compound thereof in an excess equivalent amount in the presence of a copper halide, a ligand and a base, in a solvent inactive for the reaction, under a condition ranging from ice-bath cooling to reflux with heat, generally for 1 hour to 5 days. Examples of the copper halide and the ligand used here include copper(I) iodide, 1,10-phenanthroline, and N,N,N',N'-tetramethylethylenediamine and the like. Examples of the base used here include, but are not particularly limited to, tripotassium phosphate and potassium carbonate and the like. Examples of the solvent used here include, but are not particularly limited to, an ether-based solvent, such as THF and DOX, benzene, toluene, and DOX and the like.

In addition, heating the mixture by microwave irradiation is sometimes advantageous for smoothly promoting the reaction.

In this step, the reaction can also be carried out in the same manner using a palladium catalyst. The reaction conditions are the same as in the third step of Raw Material Synthesis 16 described later.

### (Third Step)

This step is a method for producing compound (2)-4 by deprotection of compound (96).

The reaction conditions are the same as in the Production Method 2.

### (Raw Material Synthesis 14)

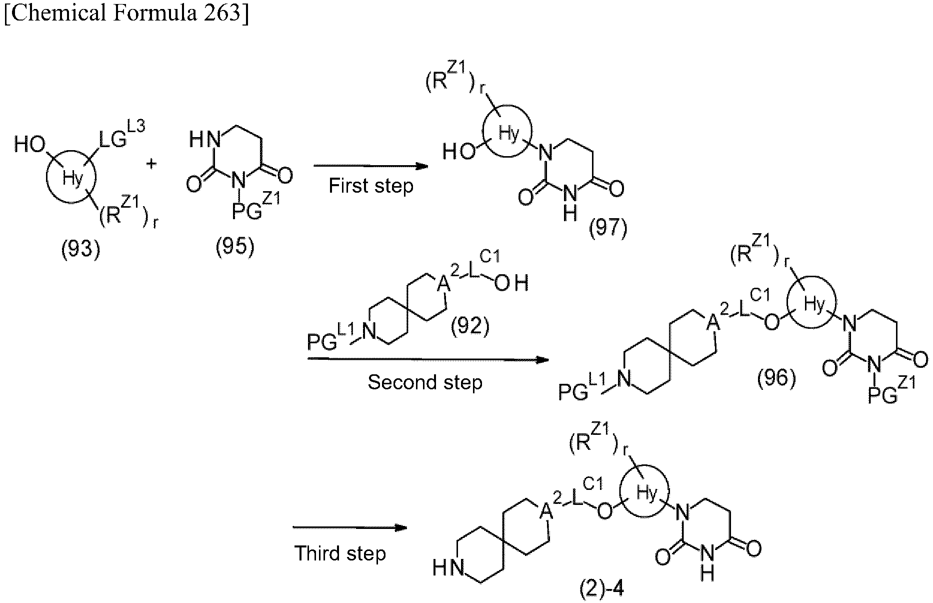

This production method is the second method for producing compound (2)-4 included in compound (2), which is a raw material compound of Production Method 1, wherein L^{B} is a group represented by formula (XXXIII-L^{B2}), A² is CH or N, L^{CF} is -L^{C1}-O-, L^{C1} represents -L^{C}-L^{D}-L^{E}-, Z is a group represented by formula (XXVII), and G is N.

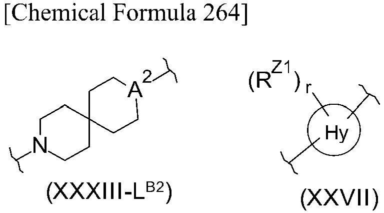

### (First Step)

This step is a method for producing compound (97) by a reaction of compound (93) and compound (95).

The reaction conditions are the same as in the second step of Raw Material Synthesis 13.

### (Second Step)

This step is a method for producing compound (96) by a Mitsunobu reaction of compound (97) and compound (92).

The reaction conditions are the same as in the first step of Raw Material Synthesis 13.

### (Third Step)

This step is a method for producing compound (2)-4 by deprotection of compound (96).

The reaction conditions are the same as in the Production Method 2.

### (Raw Material Synthesis 15)

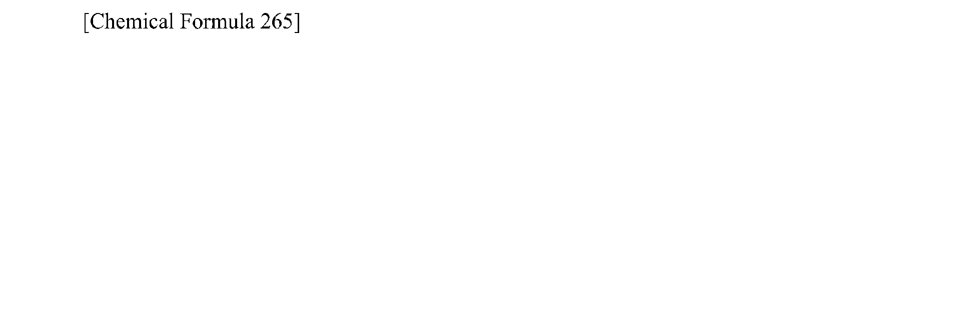

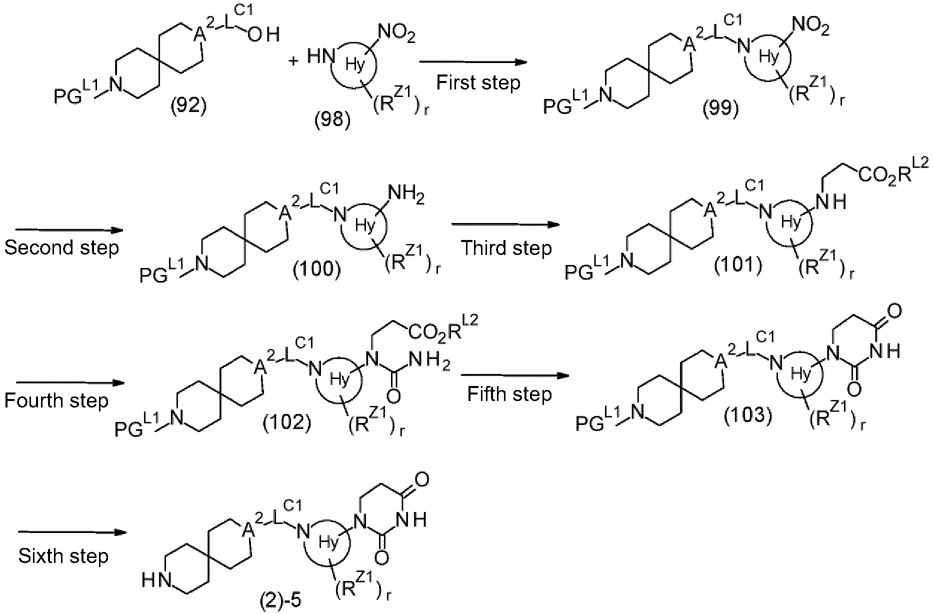

This production method is a method for producing compound (2)-5 included in compound (2), which is a raw material compound of Production Method 1, wherein L^{B} is a group represented by the following formula (XXXIII-L^{B2}), A² is CH or N, L^{CF} is -L^{C1}-, L^{C1} represents -L^{C}-L^{D}-L^{E}-L^{F}-, Z is a group represented by the following formula (XXVII)-1, and G is N.

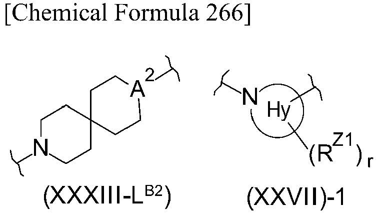

### (First Step)

This step is a method for producing compound (99) by a Mitsunobu reaction of compound (92) and compound (98).

The reaction conditions are the same as in the first step of Raw Material Synthesis 13.

### (Second Step)

This step is a method of reducing compound (99) by a catalytic hydrogenation reaction to produce compound (100).

The reaction conditions are the same as in the ninth step of Raw Material Synthesis 1.

### (Third Step)

This step is a method for producing compound (101) from compound (100).

The reaction conditions are the same as in the third step of Raw Material Synthesis 12.

### (Fourth Step)

This step is a method for producing compound (102) from compound (101).

The reaction conditions are the same as in the fourth step of Raw Material Synthesis 12.

### (Fifth Step)

This step is a method for producing compound (103) from compound (102).

The reaction conditions are the same as in the fifth step of Raw Material Synthesis 12.

### (Sixth Step)

This step is a method for producing compound (2)-5 by deprotection of compound (103).

The reaction conditions are the same as in the Production Method 2.

### (Raw Material Synthesis 16)

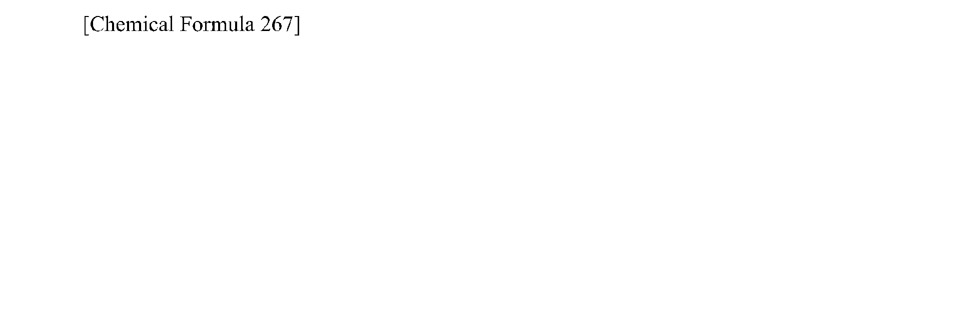

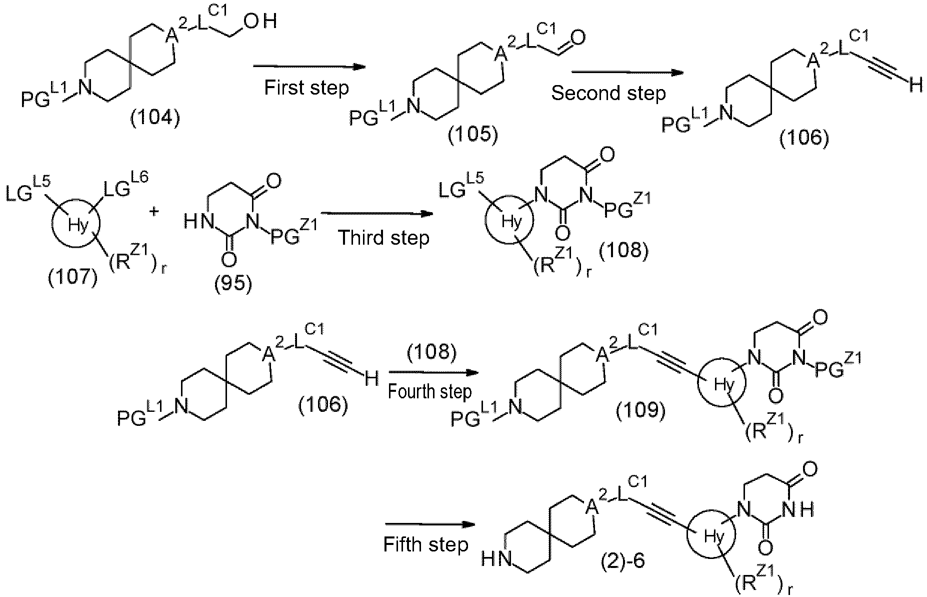

(LG^{L5} represents a leaving group, such as halogen, and is Br in one embodiment. Similarly, LG^{L6} represents a leaving group, such as halogen, and is I in one embodiment.)

This production method is a method for producing compound (2)-6 included in compound (2), which is a raw material compound of Production Method 1, wherein L^{B} is a group represented by formula (XXXIII-L^{B2}), A² is CH or N, L^{CF} is -L^{C1}-C ≡C-, L^{C1} represents -L^{C}-L^{D}-L^{E}-, Z is a group represented by formula (XXVII), and G is N.

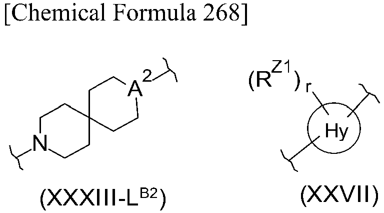

### (First Step)

This step is a method of oxidizing a hydroxyl group of compound (104) to produce compound (105).

This reaction is carried out by stirring compound (104) and an oxidant in an equivalent amount or with one in an excess equivalent amount in a solvent inactive for the reaction, under a condition ranging from ice-bath cooling to room temperature, generally for 1 hour to 5 days. Examples of the oxidant used here include Dess-Martin periodinane and the like. Examples of the solvent used here include, but are not particularly limited to, an aromatic hydrocarbon, such as benzene, and a halogenated hydrocarbon, such as dichloromethane.

### (Second Step)

This step is a method for producing compound (106) from compound (105) using an ethynyl group-introducing reagent.

This reaction is carried out by stirring compound (105) and the ethynyl group-introducing reagent in an equivalent amount or with one in an excess equivalent amount, in a solvent inactive for the reaction, under a condition ranging from ice-bath cooling to room temperature, generally for 1 hour to 5 days. Examples of the ethynyl group-introducing reagent used here include dimethyl (1-diazo-2-oxopropyl)phosphonate and the like. Examples of the solvent include, but are not particularly limited to, an alcohol, such as MeOH and EtOH, a halogenated hydrocarbon, such as dichloromethane, 1,2-dichloroethane and chloroform, an ether, such as THF, DOX and dimethoxyethane, DMF, DMSO, MeCN or water, and a mixture thereof.

### (Third Step)

This step is a method for producing compound (108) by a reaction of compound (107) and compound (95).

In this reaction, compound (107) and compound (95) are used in an equal amount or with one compound thereof in an excess amount, and the mixture of the compounds is stirred in a solvent inactive for the reaction, in the presence of a base and a palladium catalyst, under a condition ranging from at room temperature to reflux with heat, preferably at 20°C to 150°C, generally for 0.1 hours to 5 days. Examples of the solvent used here include, but are not particularly limited to, a halogenated hydrocarbon, such as dichloromethane, 1,2-dichloroethane or chloroform, an aromatic hydrocarbon, such as benzene, toluene or xylene, an ether, such as diethyl ether, THF, DOX or 1,2-dimethoxyethane, an alcohol, such as MeOH, EtOH, isopropyl alcohol, butanol or amyl alcohol, DMF, DMSO, MeCN, 1,3-dimethylimidazolidin-2-one, water and a mixture thereof. Examples of the base include an inorganic base, such as cesium carbonate, tripotassium phosphate, sodium carbonate, potassium carbonate, sodium hydroxide and barium hydroxide and the like. Examples of the palladium catalyst include tetrakis(triphenylphosphine)palladium, bis(triphenylphosphine)palladium(II) dichloride, PdCl₂(dppf)·CH₂Cl₂, Pd₂(dba)₃, RuPhos Pd G3, and palladium(II) acetate and the like. Performing the reaction in the presence of a ligand, such as SPhos, RuPhos, 1,1'-bis(diphenylphosphino)ferrocene, and Xphos and the like is sometimes advantageous for smoothly promoting the reaction. In addition, heating the mixture by microwave irradiation is sometimes advantageous for smoothly promoting the reaction.

### (Fourth Step)

This step is a method for producing compound (109) by a reaction of compound (106) and compound (108).

In this reaction, compound (106) and compound (108) are used in an equal amount or with one compound thereof in an excess amount, and the mixture of the compounds is stirred in a solvent inactive for the reaction, in the presence of a base, a palladium catalyst, and a copper catalyst, under a condition ranging from room temperature to reflux with heat, preferably at 20°C to 140°C, generally for 0.1 hours to 5 days. Examples of the solvent used here include, but are not particularly limited to, a halogenated hydrocarbon, such as dichloromethane, 1,2-dichloroethane or chloroform, an aromatic hydrocarbon, such as benzene, toluene or xylene, an ether, such as diethyl ether, THF, DOX or 1,2-dimethoxyethane, an alcohol, such as MeOH, EtOH, isopropyl alcohol, butanol or amyl alcohol, DMF, DMSO, MeCN, 1,3-dimethylimidazolidin-2-one, water and a mixture thereof. Examples of the base include an inorganic base, such as cesium carbonate, tripotassium phosphate, sodium carbonate, potassium carbonate, sodium hydroxide and barium hydroxide and the like. Examples of the palladium catalyst include tetrakis(triphenylphosphine)palladium, PdCl₂(PPh₃)₂, PdCl₂(dppf)·CH₂Cl₂, Pd₂(dba)₃, RuPhos Pd G3, and palladium(II) acetate and the like, and examples of the copper catalyst include copper(I) iodide and the like. Examples of the base include TEA and DIPEA and the like.

### (Fifth Step)

This step is a method for producing compound (2)-6 by deprotection of compound (109).

The reaction conditions are the same as in the Production Method 2.

### (Raw Material Synthesis 17)

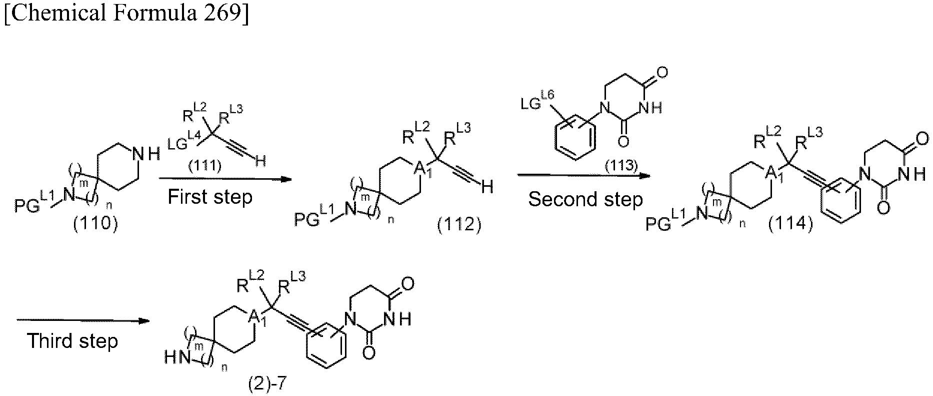

(LG^{L4} represents a leaving group, for example, halogen, and is Cl in one embodiment.)

This production method is a method for producing compound (2)-7 included in compound (2), which is a raw material compound of Production Method 1, wherein L^{B} is a group represented by formula (XXXIII-L^{B1}), L^{CF} is -C(R^{L2})(R^{L3})-C≡C-, Z is a group represented by formula (XXIV), and G is N.

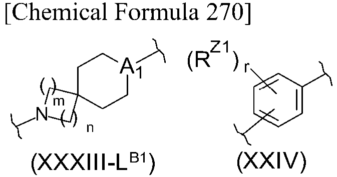

### (First Sep)

This step is a method for producing compound (112) from compound (110) and compound (111).

This reaction is carried out by stirring compound (110) and compound (111) in an equivalent amount or with one compound thereof in an excess equivalent amount in a solvent inactive for the reaction, in the presence of a base, under a condition ranging from ice-bath cooling to room temperature, generally for 1 hour to 5 days. Examples of the base used here include TEA and the like. Examples of the solvent include, but are not particularly limited to, an aromatic hydrocarbon, such as toluene, a halogenated hydrocarbon, such as dichloromethane, 1,2-dichloroethane and chloroform, an ether, such as THF, DOX and dimethoxyethane, DMF, DMSO, and MeCN and the like.

In addition, addition of a copper salt such as copper(I) chloride and the like is sometimes suitable for the reaction.

When R^{L2} and R^{L3} both are H, however, compound (112) can also be produced by a nucleophilic substitution reaction of compound (110) and compound (111) in the presence of a base. The reaction conditions in this case are the same as in the tenth step of Raw Material Synthesis 1.

### (Second Step)

This step is a method for producing compound (114) by a reaction of compound (112) and compound (113).

The reaction conditions are the same as in the fourth step of Raw Material Synthesis 16.

### (Third Step)

This step is a method for producing compound (2)-7 by deprotection of compound (114).

The reaction conditions are the same as in the Production Method 2.

### (Raw Material Synthesis 18)

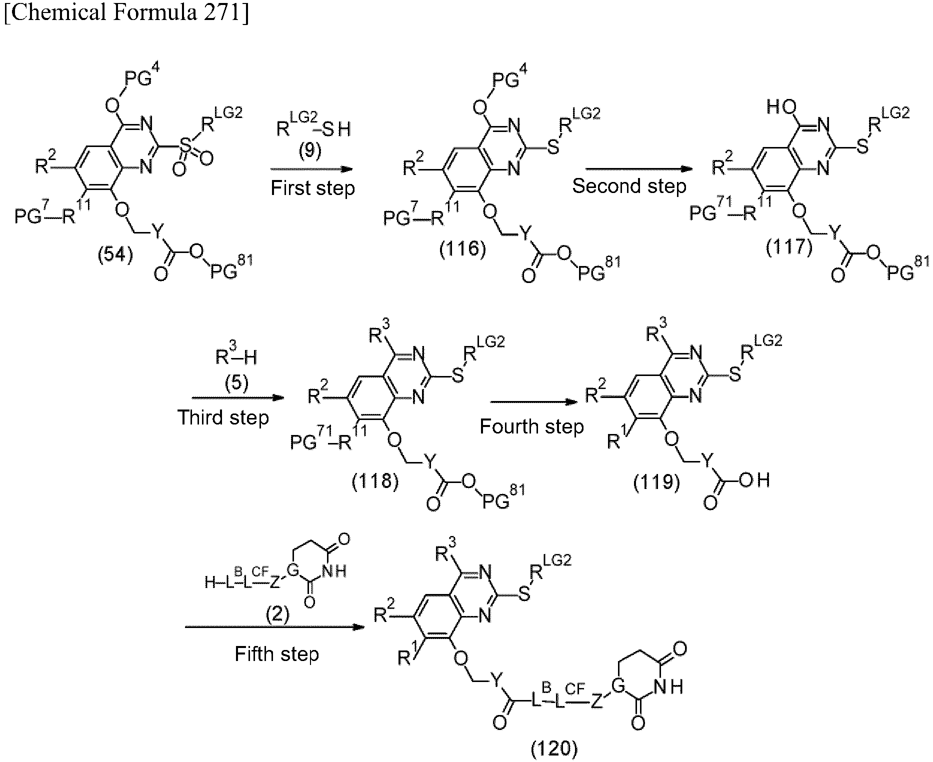

This production method is a method for producing compound (120) included in compound (115), which is a raw material compound of Production Method 4, wherein -L^{A}-included in -L- is CO, -L^{B}- is -N(R^{L1A})- or divalent group of optionally substituted saturated hetero ring containing 1 or 2 nitrogen atoms, and -L^{C}-L^{D}-L^{E}-L^{F}- is -L^{CF}-.

### (First Step)

This step is a step of producing compound (116) by reacting compound (54) and compound (9).

The reaction conditions are the same as in the third step of Raw Material Synthesis 1.

### (Second Step)

This step is a step of producing compound (117) by performing a deprotection reaction of compound (116).

The reaction conditions are the same as in the eleventh step of Raw Material Synthesis 1.

### (Third Step)

This step is a step of producing compound (118) by reacting compound (117) and compound (5).

The reaction conditions are the same as in the twelfth step of Raw Material Synthesis 1.

### (Fourth Step)

This step is a step of producing compound (119) by performing a deprotection reaction of compound (118).

The reaction conditions are the same as in the thirteenth step of Raw Material Synthesis 1.

### (Fifth Step)

This step is a step of producing compound (120) by reacting compound (119) and compound (2).

The reaction conditions are the same as in the third step of Raw Material Synthesis 2.

The pharmacological activities of the compounds of formula (I) and formula (I-I) were confirmed by the following tests.

### Test Example 1: Evaluation of KRAS degradation activity on human G12V mutant KRAS-positive pancreatic cancer line PA-TU-8902

The KRAS degradation activity of test compounds was evaluated by measuring the KRAS expression levels according to Cell ELISA.

PA-TU-8902 cells (DSMZ, ACC 179) were seeded at 36 µL each on 384-well plates (from Greiner bio-one) to give 1.5 × 10⁴ cells per well. As for the cell culture conditions, DMEM medium (from Sigma-Aldrich) containing 10% fetal bovine serum (from Cytiva) was used in the presence of 5% CO₂ at 37°C.

The next day, the test compounds (10 points having final concentrations in the range of 1 µM to 0.03 nM) and dimethyl sulfide (DMSO), which was the solvent for the test compounds, as a negative control were diluted 100-fold with a fresh medium and were added at 4 µL per well, followed by culturing for 24 hours.

The next day, the culture supernatant was removed, and 4% paraformaldehyde phosphate buffer (from FUJIFILM Wako Pure Chemical Corporation) was added at 20 µL per well. The plates were allowed to stand for 30 minutes at room temperature to thus immobilize the cells. Thereafter, the supernatant was removed, and 0.1% Polyoxyethylene(10) Octylphenyl Ether (from FUJIFILM Wako Pure Chemical Corporation)-containing phosphate buffered saline (PBS) was added at 20 µL per well. After the culture was allowed to stand for 10 minutes at room temperature, the supernatant was removed. PBS was added at 25 µL per well, and the supernatant was removed to thus wash each well. The washing was performed twice in total. Subsequent washing operations were performed in the same manner. Next, 0.5% sodium dodecyl sulfate (SDS; from ThermoFisher Scientific)-containing PBS was added at 20 µL per well. After the culture was allowed to stand for 10 minutes at room temperature, the supernatant was removed by centrifugation of the plates. After washing with PBS, the supernatant was removed by centrifugation, and Intercept Blocking Buffer (from LI-COR Biosciences) was added at 20 µL per well. After the culture was allowed to stand for 30 minutes at room temperature, the supernatant was removed by centrifugation, and a solution obtained by diluting, as primary antibodies, anti-KRAS antibody (EPR23474-76; Rabbit mAb; from Abcam; 1,000-fold diluted) and anti-β-actin antibody (Anti-beta Actin antibody; Mouse mAb; from Abcam; 1000-fold diluted) with Intercept Blocking Buffer was added as a mixed solution at 15 µL per well and allowed to stand overnight at 4°C.

The next day, the supernatant was removed by centrifugation and washed with PBS. The supernatant was removed by centrifugation, and a solution obtained by diluting, as secondary antibodies, anti-rabbit IgG antibody (IRDye 800CW Goat anti-Rabbit IgG; from LI-COR Biosciences) and anti-mouse IgG antibody (IRDye 680RD Donkey anti-Mouse IgG; from LI-COR Biosciences) 1,000-fold with Intercept Blocking Buffer was added as a mixed solution at 15 µL per well. After the culture was allowed to stand for 1 hour at room temperature, the supernatant was removed by centrifugation and washed with PBS. After removing the supernatant, the plates were dried with air at room temperature for 2 hours or more, and the fluorescent signals at 721 to 740 nm and 816 to 840 nm were measured with Odyssey M Imaging System (from Li-COR Biosciences).

The degradation rate of KRAS was calculated based on the KRAS signaling value of 0% at the time of addition of DMSO, which was corrected with the signaling value of β-actin, and the KRAS signaling value of 100% when stained only with the anti-β-actin antibody. The 50% degradation concentration (DC₅₀) of KRAS was calculated by Sigmoid-Emax model nonlinear regression analysis. The results for some test compounds of formula (I) and formula (I-I) are shown in tables below.

**[Table 1-1]**

| Ex | DC₅₀ (nM) | Ex | DC₅₀ (nM) | Ex | DC₅₀ (nM) |
|---|---|---|---|---|---|
| 1 | 1.5 | 13 | 2.4 | 25 | 2.1 |
| 2 | 1.4 | 14 | 0.92 | 26 | 0.87 |
| 3 | 1.8 | 15 | 4.0 | 27 | 2.9 |
| 4 | 1.3 | 16 | 5.0 | 28 | 0.64 |
| 5 | 2.5 | 17 | 2.3 | 29 | 0.35 |
| 6 | 5.2 | 18 | 0.93 | 30 | 0.89 |
| 7 | 1.1 | 19 | 0.91 | 31 | 6.0 |
| 8 | 5.6 | 20 | 0.96 | 32 | 1.2 |
| 9 | 1.6 | 21 | 1.8 | 33 | 4.9 |
| 10 | 8.3 | 22 | 2.7 | 34 | 1.7 |
| 11 | 0.90 | 23 | 1.5 | | |
| 12 | 1.3 | 24 | 1.2 | | |

**[Table 1-2]**

| Ex | DC₅₀ (nM) |
|---|---|
| 35 | 1.8 |
| 36 | 0.24 |
| 37 | 0.45 |
| 38 | 0.31 |
| 39 | 0.93 |
| 40 | 0.25 |
| 41 | 0.68 |

Test Example 2: Evaluation of non-anchorage-dependent cell growth inhibitory effect on human G12V mutant KRAS-positive pancreatic cancer line PA-TU-8902, human G12D mutant KRAS-positive pancreatic cancer line PK-59, human G12C mutant KRAS-positive pancreatic cancer line MIA PaCa-2, human G12C mutant KRAS-positive lung cancer line NCI-H358, human G12D mutant KRAS-positive colorectal cancer line GP2d, human G13D mutant KRAS-positive colorectal cancer line LoVo, or human wild-type KRAS gene amplification-positive gastric cancer line MKN1
The non-anchorage-dependent cell growth inhibitory effect of test compounds was evaluated by spheroid 3D cell culture.

PA-TU-8902 cells or GP2d cells (ECACC, 95090714), Pk-59 cells (RIKEN BRC, RCB1901), MIA PaCa-2 cells (RIKEN BRC, RCB2094), NCI-H358 cells (ATCC, CRL-5807), LoVo cells (ATCC, CCL-229), or MKN1 cells (JCRB, JCRB0252) were seeded on low-cell-adhesive round bottom 384-well plates (Prime Surface: from Sumitomo Bakelite) at 36 µL/well to give 5 × 10² cells per well, and the PA-TU-8902 cells were cultured under the same conditions as in Test Example 1. The GP2d cells were cultured using DMEM medium containing 10% fetal bovine serum in the presence of 5% CO₂ at 37°C. The PK-59 cells, MIA PaCa-2 cells, LoVo cells, NCI-H358 cells, or MKN1 cells were cultured using RPMI-1640 medium (from Sigma-Aldrich) containing 10% fetal bovine serum in the presence of 5% CO₂ at 37°C.

The next day, the test compounds (10 points having final concentrations in the range of 10 µM to 0.03 nM) and DMSO, which was the solvent for the test compounds, as a negative control were diluted 100-fold with a fresh medium and were added at 4 µL to each well containing the PA-TU-8902 cells, PK-59 cells, MIA PaCa-2 cells, LoVo cells, NCI-H358 cells, or MKN1 cells. After culturing in the presence of 5% CO₂ at 37°C for 6 days, CellTiter Glo 2.0 (from Promega) was added at 20 µL per well. After stirring with a plate mixer at room temperature for 1 hour, the luminescent signals were measured with ARVO X3 (from PerkinElmer).

With the signaling value in treatment with DMSO taken as a survival rate of 100% and with the signaling value in the medium alone without cells taken as a survival rate of 0%, the 50% inhibitory concentration (IC₅₀) was calculated by Sigmoid-Emax model nonlinear regression analysis. The results for some test compounds of formula (I) and formula (I-I) are shown in tables below.

**[Table 2-1]**

| Ex | G12V (PA-TU-8902) | G12D (PK-59) | G12C (MIA PaCa-2) | G13D (LoVo) |
|---|---|---|---|---|
| | IC₅₀ (nM) | IC₅₀ (nM) | IC₅₀ (nM) | IC₅₀ (nM) |
| 1 | 16 | 7.3 | 4.6 | 30 |
| 2 | 11 | 11 | 2.8 | 130 |
| 3 | 20 | 49 | 8.0 | 140 |
| 4 | 9.1 | 16 | 2.9 | 70 |
| 5 | 18 | 66 | 7.3 | 160 |
| 6 | 36 | 25 | 7.2 | 300 |
| 7 | 10 | 4.6 | 3.2 | 40 |
| 8 | 32 | 18 | 24 | 47 |
| 9 | 12 | 67 | 8.8 | 140 |
| 10 | 59 | 79 | 19 | 610 |
| 11 | 5.7 | 4.8 | 2.8 | 40 |
| 12 | 16 | 15 | 4.2 | 91 |
| 13 | 21 | 30 | 6.7 | 120 |
| 14 | 8.9 | 5.1 | 1.8 | 33 |
| 15 | 20 | 9.6 | 4.6 | 75 |
| 16 | 41 | 41 | 11 | 170 |
| 17 | 29 | 30 | 6.9 | 230 |
| 18 | 7.3 | 5.6 | 2.2 | 35 |
| 19 | 11 | 5.7 | 3.9 | 51 |
| 20 | 8.5 | 4.1 | 3.1 | 46 |
| 21 | 13 | 11 | 9.7 | 39 |
| 22 | 33 | 22 | 12 | 270 |
| 23 | 12 | 7.3 | 4.9 | 47 |
| 24 | 11 | 6.2 | 4.6 | 73 |
| 25 | 20 | 17 | 9.3 | 230 |
| 26 | 6.7 | 12 | 3.0 | 42 |
| 27 | 25 | 39 | 11 | 190 |
| 28 | 5.9 | 7.4 | 2.1 | 32 |
| 29 | 2.0 | 7.7 | 1.6 | 28 |
| 30 | 9.6 | 14 | 3.8 | 42 |
| 31 | 36 | 76 | 18 | 670 |
| 32 | 12 | 6.0 | 2.9 | 31 |
| 33 | 30 | 17 | 9.6 | 200 |
| 34 | 20 | 13 | 5.1 | 55 |

**[Table 2-2]**

| Ex | G12C (NCI-H358) | Wild-type amplification (MKN1) |
|---|---|---|
| | IC₅₀ (nM) | IC₅₀ (nM) |
| 1 | 2.9 | 3.1 |
| 7 | 1.9 | 2.9 |
| 14 | 1.5 | 2.1 |
| 20 | 2.8 | 2.8 |
| 24 | 1.7 | 1.9 |

### Test Example 3: Evaluation of anti-tumor activity in human G12D mutant KRAS-positive colorectal cancer line GP2d tumor-bearing mice

GP2d cells were cultured under the same conditions as in Test Example 2. The GP2d cells were collected and suspended in PBS, and a cell suspension prepared at 4.0 × 10⁷ cells/mL with two equal volumes of VitroGel Hydrogel Matrix (from TheWell Bioscience) was subcutaneously inoculated into 4- to 5-week-old male nude mice (BALB/c-nu (nu/nu), from The Jackson Laboratory Japan, Inc.) in a volume of 100 µL. About two weeks after the inoculation, the mice were divided into groups so that all the groups had approximately the same tumor volume and body weight, and administration of a test compound was started on the next day. The study was conducted for 5 mice each of a vehicle control group and test compound administration groups. The test compounds were dissolved in a solvent containing ethanol (from FUJIFILM Wako Pure Chemical Corporation), a 5% glucose solution (from Otsuka Pharmaceutical), 1 M hydrochloric acid, a 50% solution of (2-hydroxypropyl)-β-cyclodextrin (HP-βCD) (from ROQUETTE), HCO-40 (from Nikko Chemicals Co., Ltd.), and 1 M sodium hydroxide solution at a liquid amount ratio of 4:84.4:1.1:1:9:0.5. The test compound or vehicle control was administered into the tail vein. The administration was performed once a week and twice in total. The tumor sizes and the body weights were measured twice a week. The tumor volumes were calculated using the following equation. 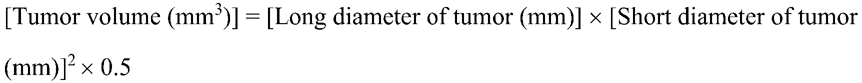 [Tumor volume (mm3)] = [Long diameter of tumor (mm)] × [Short diameter of tumor (mm)]2 × 0.5

The tumor growth inhibition rate (%) by the test compound was calculated with the tumor volume of the test compound administration group on the previous day of the start of the administration taken as 100% inhibition and the tumor volumes of the vehicle groups two weeks after the day of grouping taken as 0% inhibition. In addition, when the tumor volume of a test compound administration group was smaller than the tumor volume on the previous day of the start of the administration, the tumor regression rate (%) by the test compound was calculated with the tumor volume on the previous day of the start of the administration taken as 0% regression and with the tumor volume 0 taken as 100% regression. The results for some test compounds of formula (I) and formula (I-I) are shown in a table below.

**[Table 3]**

| Ex | Dose (mg/kg) | Anti-tumor activity |
|---|---|---|
| 1 | 3 | 85% inhibition |
| 7 | 3 | 24% regression |
| 14 | 3 | 97% inhibition |
| 20 | 3 | 18% regression |
| 24 | 3 | 29% regression |

### Test Example 4: Evaluation of anti-tumor activity in human G12V mutant KRAS-positive pancreatic cancer line PA-TU-8988S tumor-bearing mice

PA-TU-8988S cells are cultured using DMEM medium containing 10% fetal bovine serum in the presence of 5% CO₂ at 37°C. The PA-TU-8988S cells are collected and suspended in PBS, and a cell suspension prepared at 5.0 × 10⁷ cells/mL with Cultrex Basement Membrane Extract (from R&D systems) to have a final concentration of 5.0 mg/mL is subcutaneously inoculated into 4- to 5-week-old male nude mice (BALB/c-nu (nu/nu), from The Jackson Laboratory Japan, Inc.) in a volume of 100 µL. About two weeks after the inoculation, the mice are divided into groups so that all the groups have approximately the same tumor volume and body weight, and administration of a test compound is started on the next day. The study is conducted for 5 mice each of a vehicle control group and test compound administration groups. The test compound is dissolved in the same solvent as in Test Example 3. The test compound or vehicle control is administered into the tail vein. The administration is performed two or three times at an interval of once a week. The tumor sizes and the body weights were measured twice a week. The tumor volumes, the tumor growth inhibition rate (%) by the test compound, and the tumor regression rate (%) by the test compound are calculated in the same manner as in Test Example 3.

### Test Example 5: Evaluation of anti-tumor activity on human G12C mutant KRAS-positive lung cancer line NCI-H358 tumor-bearing mice

NCI-H358 cells are cultured in the same manner as in Test Example 2. The NCI-H358 cells are collected and suspended in PBS, and a cell suspension prepared at 5.0 × 10⁷ cells/mL with two equal volumes of VitroGel Hydrogel Matrix (from TheWell Bioscience) is subcutaneously inoculated into 4- to 5-week-old male nude mice (BALB/c-nu (nu/nu), from The Jackson Laboratory Japan, Inc.) in a volume of 100 µL. About three weeks after the inoculation, the mice are divided into groups so that all the groups have approximately the same tumor volume and body weight, and administration of test compounds is started on the next day. The study is conducted for 5 mice each of a vehicle control group and test compound administration groups. The test compound is dissolved in the same solvent as in Test Example 3. The test compound or vehicle control is administered into the tail vein. The administration is performed two or three times at an interval of once a week. The tumor sizes and the body weights are measured twice or three times a week. The tumor volumes, the tumor growth inhibition rate (%) by the test compound, and the tumor regression rate (%) by the test compound are calculated in the same manner as in Test Example 3.

As a result of the above tests, a degradation-inducing action of G12V mutant KRAS was observed for some compounds of formula (I) and formula (I-I). Moreover, cell growth inhibitory effects on human G12V mutant KRAS-positive cancer, human G12D mutant KRAS-positive cancer, human G12C mutant KRAS-positive cancer, and human G13D mutant KRAS-positive cancer were observed for some compound of formula (I) and formula (I-I). Further, anti-tumor activity in human G12D mutant KRAS-positive colorectal cancer line tumor-bearing mice was observed for some compounds of formula (I) and formula (I-I). Therefore, the compounds of formula (I) and formula (I-I) can be used for the treatment of cancer, in particular, G12C mutant, G12D mutant, G12V mutant and G13D mutant KRAS-positive cancer, and the like.

A pharmaceutical composition containing one or more compounds of formula (I) and formula (I-I) or a salt thereof as active ingredients can be prepared using an excipient usually used in the art, i.e., a pharmaceutical excipient, a pharmaceutical carrier or the like through a method usually used.

The administration may be either oral administration with a tablet, pill, capsule, granule, powder, liquid or other agent or parenteral administration with an intraarticular, intravenous, intramuscular or other injection, a transmucosal agent, an inhalant or the like.

As a solid composition for oral administration, a tablet, powder, granular or other agent is used. In such a solid composition, one or more active ingredients are mixed with at least one inert excipient. The composition may contain an inert additive, such as a lubricant, a disintegrant, a stabilizer and a dissolution aid according to a conventional method. A tablet or pill may be coated with a sugar coating or a film soluble in the stomach or intestine, as needed.

A liquid composition for oral administration includes a pharmaceutically acceptable emulsion, solution, suspension, syrup, or elixir agent and contains a generally used inactive diluent, for example, purified water or EtOH. The liquid composition may contain, in addition to the inactive diluent, an adjuvant, such as a solubilizer, a wetting agent and a suspending agent, a sweetening agent, a flavor, an aromatic, or a preservative.

The injection agent for parenteral administration contains a sterile aqueous or nonaqueous solution, a suspension, or an emulsion. Examples of the aqueous solvent include distilled water for injection or physiological saline. An example of the nonaqueous solvent is an alcohol, such as EtOH. Such a composition may further contain an isotonizing agent, a preservative, a wetting agent, an emulsifier, a dispersant, a stabilizer or a dissolution aid. These compositions are sterilized, for example, by filtration through a bacteria-retaining filter, incorporation of a microbicide or irradiation. In addition, such a composition can be produced as a sterile solid composition, which is dissolved or suspended in sterile water or a sterile solvent for injection before use.

The transmucosal agent, such as an inhalant and a transnasal agent, is used in a solid, liquid or semi-solid form and can be produced according to a conventionally known method. For example, a known excipient and in addition, a pH modifier, a preservative, a surfactant, a lubricant, a stabilizer, a thickener or the like may be appropriately added. The administration can be performed using a device for appropriate inhalation or insufflation. For example, the agent can be administered using a known device, such as a metering and administering inhalation device, or an atomizer, as a compound alone or a powder of a mixture formulated, or as a solution or a suspension in combination with a medically acceptable carrier. A dry powder inhaler or the like may be for a single administration or multiple administrations, and dry powder or powder-containing capsule can be used. Alternatively, the agent may be used in a form of a pressurized aerosol spray or the like using an appropriate ejection agent, for example, a suitable gas, such as a chlorofluoroalkane or carbon dioxide.

In the case of a common oral administration, the daily dose is appropriately about 0.001 to 100 mg/kg body weight, preferably 0.1 to 30 mg/kg body weight, further preferably 0.1 to 10 mg/kg body weight, and the dose is given once or is divided into two to four times in a day. In the case of intravenous administration, the daily dose is appropriately about 0.0001 to 10 mg/kg body weight and is given once or is divided into multiple times in a day. In addition, the daily dose of a transmucosal agent is about 0.001 to 100 mg/kg body weight and is given once or is divided into multiple times in a day. The dose is appropriately decided depending on the individual case taking the symptom, age, sex and the like into account.

Depending on the route of administration, dosage form, site of administration and types of excipient and additive, the pharmaceutical composition of the present invention contains 0.01 to 100% by weight, in one embodiment, 0.01 to 50% by weight, of one or more compounds of formula (I) and formula (I-I) or salts thereof which are active ingredients.

The compounds of formula (I) and formula (I-I) can be used in combination with various therapeutic or prophylactic agents for diseases in which the compounds of formula (I) and formula (I-I) are considered to exhibit efficacy. The combination use may be simultaneous administration or separate administration in succession, or administered at a desired interval. A simultaneous administration preparation may be a formulated agent or may be separately formulated.

### EXAMPLES

Hereinafter, the production methods of the compounds of formula (I) and compound (I-I) will be described in more detail based on Examples. Note that the present invention is not limited to compounds described in Examples below. The production methods of raw material compounds are also shown in the Production Examples. Although the production methods of the compounds of formula (I) and formula (I-I) are not limited only to the production methods of specific Examples described below, the compounds of formula (I) and formula (I-I) can also be produced by a combination of these production methods or methods obvious to a person skilled in the art.

Note that, in the present specification, a compound is sometimes named by using a naming soft, such as ACD/Name(R) (Advanced Chemistry Development, Inc.).

For the purpose of convenience, the concentration mol/L is expressed as M. For example, a 1 M aqueous sodium hydroxide solution means a 1 mol/L aqueous sodium hydroxide solution.

### Production Example 1

Under an argon atmosphere, to a mixed solution in MeOH (70 mL) and THF (70 mL) of (7M)-4-tert-butoxy-6-cyclopropyl-2-(ethanesulfonyl)-7-[6-fluoro-5-methyl-2-(triphenylmethyl)-2H-indazol-4-yl]-8-[(1S)-1-phenylethoxy]quinazoline (3 g) were added sodium hydrogen carbonate (1,551 mg) and 10% Pd/C (about 50% water content, 994 mg) at room temperature, and under a hydrogen atmosphere, the mixture was stirred overnight at ambient temperature and pressure. After argon replacement, the reaction mixture was filtered through celite(R) pad using CHCl₃/IPA (4/1, 100 mL) and EtOH/water (10/1, 100 mL), and the filtrate was concentrated under reduced pressure. After EtOAc and water were added, the separated organic layer was washed with saturated saline, was dried over anhydrous magnesium sulfate, and was filtered and concentrated under reduced pressure, thus obtaining (7M)-4-tert-butoxy-6-cyclopropyl-2-(ethanesulfonyl)-7-[6-fluoro-5-methyl-2-(triphenylmethyl)-2H-indazol-4-yl]quinazolin-8-ol (2,705 mg) as a foam-like solid.

### Production Example 2

To a DMF (150 mL) solution of (7M)-4-tert-butoxy-6-cyclopropyl-2-(ethanesulfonyl)-7-[6-fluoro-5-methyl-2-(triphenylmethyl)-2H-indazol-4-yl]quinazoline-8-ol (10.3 g) were added tert-butyl 4-(chloromethyl)benzoate (5.5 g) and cesium carbonate (18 g) under ice-bath cooling, and under an argon atmosphere, the mixture was stirred at 40°C overnight. The reaction was quenched with saturated aqueous ammonium chloride solution under ice-bath cooling. EtOAc and water were added to separate the organic layer from the aqueous layer, and the organic layer was washed once with saturated saline, was dried over anhydrous sodium sulfate, was filtered and was concentrated under reduced pressure. The residue was purified by silica gel column chromatography (hexane/EtOAc), thus obtaining tert-butyl 4-[({(7M)-4-tert-butoxy-6-cyclopropyl-2-(ethanesulfonyl)-7-[6-fluoro-5-methyl-2-(triphenylmethyl)-2H-indazol-4-yl] quinazolin-8-yl}oxy)methyl]benzoate (11.8 g) as a foam-like solid.

### Production Example 3

To a THF (10 mL) solution of tert-butyl 4-[({(7M)-4-tert-butoxy-6-cyclopropyl-2-(ethanesulfonyl)-7-[6-fluoro-5-methyl-2-(triphenylmethyl)-2H-indazol-4-yl]quinazolin-8-yl}oxy)methyl]benzoate (1 g) and 3-dimethylamino-2,2-dimethyl-1-propanol (0.36 mL) was added tBuOK (160 mg) under ice-bath cooling, and the mixture was stirred for 30 minutes under ice-bath cooling. The reaction was quenched with saturated aqueous ammonium chloride solution under ice-bath cooling. EtOAc and water were added to separate the organic layer from the aqueous layer, and the aqueous layer was extracted twice with EtOAc. The collected organic layer was dried over anhydrous sodium sulfate, was filtered and was concentrated under reduced pressure. The residue was purified by silica gel column chromatography (hexane/EtOAc), thus obtaining tert-butyl 4-[({(7M)-4-tert-butoxy-6-cyclopropyl-2-[3-(dimethylamino)-2,2-dimethylpropoxy]-7-[6-fluoro-5-methyl-2-(triphenylmethyl)-2H-indazol-4-yl]quinazolin-8-yl}oxy)methyl]benzoate (790 mg) as a foam-like solid.

### Production Example 4

To a THF (10 mL) solution of tert-butyl 4-[({(7M)-4-tert-butoxy-6-cyclopropyl-2-[3-(dimethylamino)-2,2-dimethylpropoxy]-7-[6-fluoro-5-methyl-2-(triphenylmethyl)-2H-indazol-4-yl]quinazolin-8-yl}oxy)methyl]benzoate (790 mg) were added 3,4-dihydro-2H-pyrane (0.5 mL) and p-toluenesulfonic acid monohydrate (270 mg) under ice-bath cooling, and under an argon atmosphere, the mixture was stirred at room temperature overnight. After Et₃N (1.3 mL) was added under ice-bath cooling, the reaction mixture was concentrated under reduced pressure. The residue was purified by silica gel column chromatography (CHCl₃/MeOH), thus obtaining tert-butyl 4-[({(7M)-6-cyclopropyl-2-[3-(dimethylamino)-2,2-dimethylpropoxy]-7-[6-fluoro-5-methyl-1-(oxan-2-yl)-1H-indazol-4-yl]-4-hydroxyquinazolin-8-yl}oxy)methyl]benzoate (520 mg) as a foam-like solid.

### Production Example 5

To a MeCN (10 mL) suspension of tert-butyl 4-[({(7M)-6-cyclopropyl-2-[3-(dimethylamino)-2,2-dimethylpropoxy]-7-[6-fluoro-5-methyl-1-(oxan-2-yl)-1H-indazol-4-yl]-4-hydroxyquinazolin-8-yl}oxy)methyl]benzoate (520 mg) and (2S)-azetidine-2-carboxamide (170 mg) were added DIPEA (0.9 mL) and PyAOP (720 mg) under ice-bath cooling, and under an argon atmosphere, the mixture was stirred at room temperature for 2 hours. The reaction mixture was concentrated under reduced pressure, and the residue was purified by silica gel column chromatography (CHCl₃/MeOH/28% aqueous ammonia), thus obtaining tert-butyl 4-[({(7M)-4-[(2S)-2-carbamoylazetidin-1-yl]-6-cyclopropyl-2-[3-(dimethylamino)-2,2-dimethylpropoxy]-7-[6-fluoro-5-methyl-1-(oxan-2-yl)-1H-indazol-4-yl]quinazolin-8-yl}oxy)methyl]benzoate (660 mg) as a foam-like solid.

### Production Example 6

To a CH₂Cl₂ (10 mL) solution of tert-butyl 4-[({(7M)-4-[(2S)-2-carbamoylazetidin-1-yl]-6-cyclopropyl-2-[3-(dimethylamino)-2,2-dimethylpropoxy]-7-[6-fluoro-5-methyl-1-(oxan-2-yl)-1H-indazol-4-yl]quinazolin-8-yl}oxy)methyl]benzoate (660 mg) was added TFA (2.4 mL) under ice-bath cooling, and the mixture was stirred at room temperature overnight. The reaction mixture was concentrated under reduced pressure, THF (3 mL) and saturated aqueous sodium hydrogen carbonate were added under ice-bath cooling, and the mixture was stirred under ice-bath cooling for 1 minute. CHCl₃/IPA (4/1) and water were added, the pH was adjusted to 8 to 9 by the addition of hydrochloric acid (1M), and the aqueous layer was extracted four times with CHCl₃/IPA (4/1). The collected organic layer was dried over anhydrous sodium sulfate, filtered, and concentrated under reduced pressure, thus obtaining 4-({[(7M)-4-[(2S)-2-carbamoylazetidin-1-yl]-6-cyclopropyl-2-[3-(dimethylamino)-2,2-dimethylpropoxy]-7-(6-fluoro-5-methyl-1H-indazol-4-yl)quinazolin-8-yl]oxy}methyl)benzoic acid (600 mg) as a solid.

### Production Example 7

At room temperature, 3-[(4-methoxyphenyl)methyl]-1,3-diazinane-2,4-dione (616 mg), 4-iodopyridin-2-ol (450 mg), CuI (402 mg) and tripotassium phosphate (900 mg) were suspended in DOX (6 mL) and TMEDA (316 µL) and stirred under microwave irradiation at 150°C for 1 hour. At room temperature, CuI (400 mg), tripotassium phosphate (450 mg) and TMEDA (0.31 mL) were added thereto, and the mixture was stirred under microwave irradiation at 150°C for 1.5 hours. Water and EtOAc were added to the reaction mixture at room temperature. The mixture was filtered, and the filtrate was extracted three times with EtOAc. Then, the organic layer was washed with saturated saline, dried over anhydrous sodium sulfate, filtered, and concentrated under reduced pressure. The resulting residue was purified by silica gel column chromatography (CHCl₃/MeOH), thus obtaining 1-(2-hydroxypyridin-4-yl)-3-[(4-methoxyphenyl)methyl]-1,3-diazinane-2,4-dione (204 mg) as a foam-like solid.

### Production Example 8

At room temperature, 1-(2-hydroxypyridin-4-yl)-3-[(4-methoxyphenyl)methyl]-1,3-diazinane-2,4-dione (794 mg) and tert-butyl 9-(2-hydroxyethyl)-3-azaspiro[5.5]undecane-3-carboxylate (938 mg) were suspended in toluene (12 mL). Under ice-bath cooling, N,N,N',N'-tetramethylazodicarboxamide (1,253 mg) and tri-n-butylphosphine (1.97 mL) were added, and the mixture was stirred under an argon atmosphere at room temperature for 2 hours. Saturated aqueous sodium hydrogen carbonate was added to the reaction mixture at room temperature, and the mixture was extracted three times with EtOAc. The collected organic layer was washed with saturated saline, dried over anhydrous sodium sulfate, then filtered, and concentrated under reduced pressure. The resulting residue was purified by silica gel column chromatography (hexane/EtOAc), thus obtaining tert-butyl 9-{2-[(4-{3-[(4-methoxyphenyl)methyl]-2,4-dioxo-1,3-diazinan-1-yl}pyridin-2-yl)oxy]ethyl}-3-azaspiro[5.5]undecane-3-carboxylate (666 mg) as an oil.

### Production Example 9

At room temperature, tert-butyl 9-{2-[(4-{3-[(4-methoxyphenyl)methyl]-2,4-dioxo-1,3-diazinan-1-yl}pyridin-2-yl)oxy]ethyl}-3-azaspiro[5.5]undecane-3-carboxylate (1,750 mg) was dissolved in TFA (28 mL). TfOH (1.51 mL) was added at room temperature, and the mixture was stirred under an argon atmosphere at 60°C for 4 hours. The reaction mixture was concentrated under reduced pressure, Et₃N (4 mL) was added to the resulting residue under ice-bath cooling, and the mixture was purified by basic silica gel column chromatography (CHCl₃/MeOH/28% aqueous ammonium), thus obtaining 1-{2-[2-(3-azaspiro[5.5]undecan-9-yl)ethoxy]pyridin-4-yl}-1,3-diazinane-2,4-dione (1,451 mg) as a solid.

### Production Example 10

To a DMSO (3 mL) solution of 1-(6-bromo-5-fluoro-1-methyl-1H-indazol-3-yl)-1,3-diazinane-2, 4-dione (300 mg), tert-butyl 1-oxa-4,9-diazaspiro[5.5]undecane-9-carboxylate (340 mg) and Pd(tBu₃P)₂ (46 mg) was added tBuONa (170 mg) at room temperature, and the mixture was stirred under microwave irradiation at 100°C for 1 hour under an argon atmosphere. Water was added at room temperature, the mixture was extracted three times with EtOAc. The organic layer was washed with saturated saline, dried over anhydrous magnesium sulfate, filtered, and concentrated under reduced pressure. The resulting residue was purified by basic silica gel column chromatography (hexane/CHCl₃/MeOH), thus obtaining tert-butyl 4-[3-(2,4-dioxo-1,3-diazinan-1-yl)-5-fluoro-1-methyl-1H-indazol-6-tl]-1-oxa-4,9-diazaspiro[5.5]undecane-9-carboxylate (205 mg) as a solid.

### Production Example 11

Tert-butyl 4-[3-(2,4-dioxo-1,3-diazinan-1-yl)-5-fluoro-1-methyl-1H-indazol-6-yl]-1-oxa-4,9-diazaspiro[5.5]undecane-9-carboxylate (200 mg) was dissolved in CH₂Cl₂ (3 mL), TFA (1 mL) was added at room temperature, and the mixture was stirred at room temperature for 1 hour. The reaction mixture was concentrated under reduced pressure, and the residue was purified by basic silica gel column chromatography (hexane/CHCl₃/MeOH), thus obtaining 1-[5-fluoro-1-methyl-6-(1-oxa-4,9-diazaspiro[5.5]undecan-4-yl)-1H-indazol-3-yl]-1,3-diazinane-2,4-dione (130 mg) as a solid.

### Production Example 12

2,6-Dichloro-5-fluoropyridine-3-carbonitrile (400 mg) and tert-butyl 1-oxa-4,9-diazaspiro[5.5]undecane-9-carboxylate (540 mg) were dissolved in NMP (4 mL), DIPEA (720 µL) was added at room temperature, and the mixture was stirred at room temperature for 1 hour. Subsequently, ethylhydrazine dihydrochloride (835 mg) and DIPEA (3.5 mL) were added to the reaction mixture. The mixture was stirred at room temperature for 1 hour, at 60°C for 2 hours and at 120°C overnight. The reaction mixture was poured into ice water and stirred at room temperature for 30 minutes. Then, the resulting powder was collected by filtration and was dried under reduced pressure, thus obtaining tert-butyl 4-(3-amino-1-ethyl-5-fluoro-1H-pyrazolo[3,4-b]pyridin-6-yl)-1-oxa-4,9-diazaspiro[5.5]undecane-9-carboxylate (604 mg) as a solid.

### Production Example 13

A mixture of tert-butyl 4-(3-amino-1-ethyl-5-fluoro-1H-pyrazolo[3,4-b]pyridin-6-yl)-1-oxa-4,9-diazaspiro[5.5]undecane-9-carboxylate (600 mg), ethyl acrylate (2 mL), DBU (200 µL) and DL-lactic acid (100 µL) was stirred under microwave irradiation at 120°C for 4 hours and at 130°C for 2 hours. The reaction mixture was poured into EtOAc/water and extracted three times with EtOAc. The collected organic layer was washed with saturated saline, dried over anhydrous magnesium sulfate, then filtered, and concentrated under reduced pressure. The residue was purified by silica gel column chromatography (hexane/EtOAc). The resulting residue was dissolved in AcOH (4.5 mL) and water (1.5 mL), sodium cyanate (150 mg) was added in multiple portions under ice-bath cooling, and the mixture was stirred for 10 minutes under ice-bath cooling and for 1 hour at room temperature. Sodium cyanate (90 mg) was added under ice-bath cooling, and the mixture was stirred at room temperature for 30 minutes. Water was added to the reaction mixture, and the mixture was extracted twice with EtOAc. The organic layer was washed once with water, twice with aqueous sodium hydroxide (1M), once with saturated aqueous sodium hydrogen carbonate and once with saturated saline, dried over anhydrous magnesium sulfate, then filtered, and concentrated under reduced pressure. The resulting residue was dissolved in MeCN (10 mL), benzyltrimethylammonium hydroxide (40% MeOH solution, 250 µL) was added at room temperature, and the mixture was stirred at room temperature for 30 minutes. The reaction mixture was concentrated under reduced pressure, and the residue was purified by silica gel column chromatography (CHCl₃/MeOH), thus obtaining tert-butyl 4-[3-(2,4-dioxo-1,3-diazinan-1-yl)-1-ethyl-5-fluoro-1H-pyrazolo[3.4-b]pyridin-6-yl]-1-oxa-4,9-diazaspiro[5.5]undecane-9-carboxylate (273 mg) as a foam-like solid.

### Production Example 14

Tert-butyl 4-[3-(2,4-dioxo-1,3-diazinan-1-yl)-1-ethyl-5-fluoro-1H-pyrazolo[3.4-b]pyridin-6-yl]-1-oxa-4,9-diazaspiro[5.5]undecane-9-carboxylate (272 mg) was dissolved in CH₂Cl₂ (3 mL), TFA (500 µL) was added at room temperature, and the mixture was stirred at room temperature for 30 minutes. The reaction mixture was concentrated under reduced pressure, and EtOAc (3 mL) was added to the residue, followed by HCl/EtOAc (4 M, 500 µL) at room temperature. The mixture was stirred for 20 minutes at room temperature and then concentrated under reduced pressure. The residue was suspended in EtOAc, collected by filtration, and dried under reduced pressure to give 1-[1-ethyl-5-fluoro-6-(1-oxa-4,9-diazaspiro[5.5]undecan-4-yl)-1H-pyrazolo[3,4-b]pyridin-3-yl]-1,3-diazinane-2,4-dionen hydrochloride (204 mg) as a solid.

### Production Example 15

7-Bromo-4-tert-butoxy-6-cyclopropyl-2-(ethylsulfanyl)-8-[(1S)-1-phenylethoxy]quinazoline (4.7 g) and CH₂Cl₂ (90 mL) were added, followed by mCPBA (about 30% water content, 4.8 g) with stirring in an ice bath. Then, the mixture was stirred at room temperature for 1 hour. The reaction mixture was diluted with CH₂Cl₂, washed with saturated aqueous sodium thiosulfate and saturated aqueous sodium hydrogen carbonate, and separated into layers. The organic layer was dried over anhydrous magnesium sulfate and filtered, and the solvent was removed under reduced pressure. To the residue were added 3-dimethylamino-2,2-dimethyl-1-propanol (1.6 mL) and THF (50 mL), and tBuOK (2 g) was added in a MeOH/ice bath with stirring. Then, the mixture was stirred in the same bath under an argon atmosphere for 0.5 hours. Ice and saturated aqueous ammonium chloride were poured into the reaction mixture which was returned to room temperature, followed by extraction with EtOAc twice. The organic layer was washed with saturated saline, then dried over anhydrous magnesium sulfate, filtered, and concentrated under reduced pressure. The residue was purified by basic silica gel column chromatography (hexane/EtOAc), thus obtaining 3-({7-bromo-4-tert-butoxy-6-cyclopropyl-8-[(1S)-1-phenylethoxy]quinazolin-2-yl}oxy)-N,N,2,2-tetramethylpropan-1-amine (2.35 g) as an oil.

### Production Example 16

3-({7-Bromo-4-tert-butoxy-6-cyclopropyl-8-[(1S)-1-phenylethoxy]quinazolin-2-yl}oxy)-N,N,2,2-tetramethylpropan-1-amine (3.4 g) was dissolved in THF (50 mL), and p-toluenesulfonic acid monohydrate (1.4 g) was added with stirring at room temperature. Then, the mixture was stirred under an argon atmosphere at room temperature for 3 hours. After Et₃N (3 mL) was added to the reaction mixture, the solvent was concentrated under reduced pressure. The residue was purified by basic silica gel column chromatography (CHCl₃/MeOH), thus obtaining 7-bromo-6-cyclopropyl-2-[3-(dimethylamino)-2,2-dimethylpropoxy]-8-[(1S)-1-phenylethoxy]quinazolin-4-ol (2.97 g) as a foam-like solid.

### Production Example 18

(2S)-1-{7-Bromo-6-cyclopropyl-2-[3-(dimethylamino)-2,2-dimethylpropoxy]-8-[(1S)-1-phenylethoxy]quinazolin-4-yl}azetidine-2-carboxamide (200 mg), 2-[7-fluoro-3-(methoxymethoxy)naphthalen-1-yl]-4,4,5,5-tetramethyl-1,3,2-dioxaborolane (160 mg), RuPhos (25 mg), RuPhos Pd G3 (45 mg), tripotassium phosphate (250 mg), DOX (5 mL) and water (1 mL) were added, and the mixture was subjected to an operation of degassing under reduced pressure/filling with argon several times and was then stirred under an argon atmosphere at 80°C for 2 hours. To the cooled reaction mixture was poured ice water and extracted twice with EtOAc. The collected organic layer was washed with saturated saline, dried over anhydrous magnesium sulfate, then filtered, and concentrated under reduced pressure. The residue was purified by basic silica gel column chromatography (hexane/EtOAc), thus obtaining (2S)-1-{6-cyclopropyl-2-[3-(dimethylamino)-2,2-dimethylpropoxy]-7-[7-fluoro-3-(methoxymethoxy)naphthalen-1-yl]-8-[(1S)-1-phenylethoxy]quinazolin-4-yl}azetidine-2-carboxamide (diastereomeric mixture derived from axial chirality, 189 mg) as a foam-like solid.

### Production Example 19

To (2S)-1-{6-cyclopropyl-2-[3-(dimethylamino)-2,2-dimethylpropoxy]-7-[7-fluoro-3-(methoxymethoxy)naphthalen-1-yl]-8-[(1S)-1-phenylethoxy]quinazolin-4-yl} azetidine-2-carboxamide (186 mg) were added MeOH (5 mL) and 20% Pd(OH)₂/C (about 50% water content, 60 mg), and the mixture was stirred under a hydrogen atmosphere at ambient temperature and pressure for 1 hour. To the reaction mixture was added celite(R) and was filtered through celite(R) pad while washing with EtOAc and CHCl₃. Toluene (about 5 mL) was added to the filtrate, and the solvent was removed under reduced pressure, thus obtaining (2S)-1-{6-cyclopropyl-2-[3-(dimethylamino)-2,2-dimethylpropoxy]-7-[7-fluoro-3-(methoxymethoxy)naphthalen-1-yl]-8-hydroxyquinazolin-4-yl}azetidine-2-carboxamide (164 mg) as a foam-like solid.

### Production Example 21

Triisopropylsilane (0.12 mL) and CH₂Cl₂ (3 mL) were poured into tert-butyl 4-[({4-[(2S)-2-carbamoylazetidin-1-yl]-6-cyclopropyl-2-[3-(dimethylamino)-2,2-dimethylpropoxy]-7-[7-fluoro-3-(methoxymethoxy)naphthalen-1-yl]quinazolin-8-yl}oxy)methylbenzoate (159 mg), and in a MeOH/ice bath, TFA (1 mL) was added dropwise thereto with stirring. Then, the mixture was stirred under an argon atmosphere at room temperature overnight. The solvent was concentrated under reduced pressure, a small amount of ice water was poured into the residue, and the pH was adjusted to 5 to 6 with saturated aqueous sodium hydrogen carbonate, followed by extraction with CHCl₃/MeOH (511) three times. The collected organic layer was dried over anhydrous magnesium sulfate and filtered, and the solvent was concentrated under reduced pressure, thus obtaining 4-[({4-[(2S)-2-carbamoylazetidin-1-yl]-6-cyclopropyl-2-[3-(dimethylamino)-2,2-dimethylpropoxy]-7-(7-fluoro-3-hydroxynaphthalen- 1-yl)quinazolin-8-yl} oxy)methyl]benzoic acid (67 mg) as a solid.

### Production Example 26

At room temperature, tert-butyl 1-oxa-4,9-diazaspiro[5.5]undecane-9-carboxylate (1,400 mg) and 4-chloro-5-fluoropyridine-2-carbonitrile (780 mg) were suspended in MeCN (10 mL) and DIPEA (1.7 mL) and stirred under microwave irradiation at 150°C for 1.5 hours under an argon atmosphere. Saturated aqueous sodium hydrogen carbonate was added to the reaction mixture at room temperature, and the mixture was extracted three times with EtOAc. The organic layer was washed with saturated saline, dried over anhydrous sodium sulfate, then filtered, and concentrated under reduced pressure. The resulting residue was purified by silica gel column chromatography (hexane/EtOAc), thus obtaining tert-butyl 4-(4-chloro-6-cyanopyridin-3-yl)-1-oxa-4,9-diazaspiro[5.5]undecane-9-carboxylate (1,501 mg) as a foam-like solid.

### Production Example 27

At room temperature, tert-butyl 4-(4-chloro-6-cyanopyridin-3-yl)-1-oxa-4,9-diazaspiro[5.5]undecane-9-carboxylate(1,450 mg) was dissolved in THF (16 mL), BH₃-THF complex (0.9 M THF solution, 16 mL) was added under ice-bath cooling, and the mixture was stirred under an argon atmosphere at 60°C for 1 hour. MeOH was added to the reaction mixture under ice-bath cooling, and the mixture was stirred overnight and was concentrated under reduced pressure. The resulting residue was purified by basic silica gel column chromatography (hexane/EtOAc), thus obtaining tert-butyl 4-[6-(aminomethyl)-4-chloropyridin-3-yl]-1-oxa-4,9-diazaspiro[5.5]undecane-9-carboxylate (293 mg) as a foam-like solid.

### Production Example 28

At room temperature, tert-butyl 4-[6-(aminomethyl)-4-chloropyridin-3-yl]-1-oxa-4,9-diazaspiro[5.5]undecane-9-carboxylate (293 mg) was dissolved in EtOH (7 mL), ethyl acrylate (0.15 mL) was added at room temperature, and the mixture was stirred under an argon atmosphere at room temperature overnight. The reaction mixture was concentrated under reduced pressure, the residue was dissolved in THF (7 mL) at room temperature. Trimethylsilyl isocyanate (1.5 mL) was added at room temperature, and under an argon atmosphere, the mixture was stirred at 60°C over the weekend. The reaction mixture was concentrated under reduced pressure, and the residue was purified by silica gel column chromatography (CHCl₃/MeOH), thus obtaining tert-butyl 4-(4-chloro-6- {[carbamoyl(3-ethoxy-3-oxopropyl)amin]methyl}pyridin-3-yl)-1-oxa-4,9-diazaspiro[5.5]undecane-9-carboxylate (296 mg) as a foam-like solid.

### Production Example 29

At room temperature, tert-butyl 4-(4-chloro-6-{[carbamoyl(3-ethoxy-3-oxopropyl)amin]methyl}pyridin-3-yl)-1-oxa-4,9-diazaspiro[5.5]undecane-9-carboxylate (296 mg) was dissolved in MeCN (5.5 mL), benzyltrimethylammonium hydroxide (40% MeOH solution, 0.27 mL) was added at room temperature, and the mixture was stirred under an argon atmosphere for 1 hour. The reaction mixture was concentrated under reduced pressure, and the residue was purified by silica gel column chromatography (CHCl₃/MeOH), thus obtaining tert-butyl 4-{4-chloro-6-[(2,4-dioxo-1,3-diazinan-1-yl)methyl]pyridin-3-yl}-1-oxa-4,9-diazaspiro[5.5]undecane-9-carboxylate (259 mg) as a foam-like solid.

### Production Example 30

In CH₂Cl₂ (5 mL), tert-butyl 4-{4-chloro-6-[(2,4-dioxo-1,3-diazinan-1-yl)methyl]pyridin-3-yl}-1-oxa-4,9-diazaspiro[5.5]undecane-9-carboxylate (259 mg) was dissolved at room temperature. HCl/DOX (4 M, 2.6 mL) was added at room temperature, and the mixture was stirred under an argon atmosphere at room temperature overnight. The reaction mixture was concentrated under reduced pressure, thus obtaining 1-{[4-chloro-5-(1-oxa-4,9-diazaspiro[5.5]undecan-4-yl)pyridin-2-yl]methyl}-1,3-diazinane-2,4-dione n hydrochloride (255 mg) as a foam-like solid.

### Production Example 31

Tert-butyl 4-[({(7M)-4-tert-butoxy-6-cyclopropyl-2-(ethanesulfonyl)-7-[6-fluoro-5-methyl-2-(triphenylmethyl)-2H-indazol-4-yl]quinazolin-8-yl}oxy)methyl]benzoate (160 mg), NMP (2 mL), (S)-1,2-dimethylpiperazine (120 mg) and DIPEA (300 µL) were added at room temperature, and the mixture was stirred under microwave irradiation at 150°C for 3 hours. EtOAc and water were added to separate the organic layer from the aqueous layer by a separation operation, and the organic layer was washed twice with saturated saline, dried over anhydrous sodium sulfate, filtered, and concentrated under reduced pressure. The residue was dissolved in THF (5 mL), 3,4-dihydro-2H-pyrane (280 µL) and p-toluenesulfonic acid monohydrate (200 mg) were added under ice-bath cooling, and under an argon atmosphere, the mixture was stirred at room temperature overnight. After Et₃N (1.5 mL) was added under ice-bath cooling, the reaction mixture was concentrated under reduced pressure. The residue was purified by silica gel column chromatography (CHCl₃/MeOH), thus obtaining tert-butyl 4-[({(7M)-6-cyclopropyl-2-[(3S)-3,4-dimethylpiperazin-1-yl]-7-[6-fluoro-5-methyl-1-(oxan-2-yl)-1H indazol-4-yl]-4-hydroxyquinazolin-8-yl}oxy)methyl]benzoate (150 mg) as an oil.

### Production Example 32

To a MeCN (5 mL) suspension of tert-butyl 4-[({(7M)-6-cyclopropyl-2-[(3S)-3,4-dimethylpiperazin-1-yl]-7-[6-fluoro-5-methyl-1-(oxan-2-yl)-1H indazol-4-yl]-4-hydroxyquinazolin-8-yl} oxy)methyl]benzoate (150 mg) and (2S)-azetidine-2-carboxamide (95 mg) were added DIPEA (400 µL) and PyAOP (430 mg) under ice-bath cooling, and under an argon atmosphere, the mixture was stirred at 50°C for 6 hours. The reaction mixture was concentrated under reduced pressure, and the residue was purified by silica gel column chromatography (CHCl₃/MeOH/28% aqueous ammonia), thus obtaining tert-butyl 4-[({(7M)-4-[(2S)-2-carbamoylazetidin-1-yl]-6-cyclopropyl-2-[(3S)-3,4-dimethylpiperazin-1-yl]-7-[6-fluoro-5-methyl-1-(oxan-2-yl)-1H-indazol-4-yl]quinazolin-8-yl}oxy)methyl]benzoate (100 mg) as a foam-like solid.

### Production Example 33

To a CH₂Cl₂ (3 mL) solution of tert-butyl 4-[({(7M)-4-[(2S)-2-carbamoylazetidin-1-yl]-6-cyclopropyl-2-[(3S)-3,4-dimethylpiperazin-1-yl]-7-[6-fluoro-5-methyl-1-(oxan-2-yl)-1H-indazol-4-yl]quinazolin-8-yl}oxy)methyl]benzoate (72 mg) was added TFA (0.25 mL) under ice-bath cooling, and the mixture was stirred at room temperature overnight. The reaction mixture was concentrated under reduced pressure, THF (3 mL) and saturated aqueous sodium hydrogen carbonate were added under ice-bath cooling, and the mixture was stirred under ice-bath cooling for 1 minute. CHCl₃/IPA (4/1) and water were added, the pH was adjusted to 8 to 9 by the addition of hydrochloric acid (1M), and the aqueous layer was extracted four times with CHCl₃/IPA (4/1). The collected organic layer was dried over anhydrous sodium sulfate, filtered, and concentrated under reduced pressure, thus obtaining 4-({[7M)-4-[(2S)-2-carbamoylazetidin-1-yl]-6-cyclopropyl-2-[(3S)-3,4-dimethylpiperazin-1-yl]-7-(6-fluoro-5-methyl-1H-indazol-4-yl)quinazolin-8-yl]oxy}methyl)benzoate (60 mg) as a solid.

### Production Example 34

To a suspension in toluene (10 mL) and THF (10 mL) of tert-butyl 9-(2-hydroxyethyl)-3-azaspiro[5.5] undecane-3-carboxylate (1.3 g) and 5-iodopyridin-3-ol (750 mg) were added tri-n-butylphosphine (2 mL) and N,N,N',N'-tetramethylazodicarboxamide (1.4 g) under ice-bath cooling, and under an argon atmosphere, the mixture was stirred at room temperature for 1 hour. The reaction mixture was concentrated under reduced pressure, and the residue was purified by silica gel column chromatography (hexane/EtOAc), thus obtaining tert-butyl 9-{2-[(5-iodopyridin-3-yl)oxy]ethyl}-3-azaspiro[5.5]undecane-3-carboxylate (1.88 g) as a foam-like solid.

### Production Example 35

At room temperature, 3-[(4-methoxyphenyl)methyl]-1,3-diazinane-2,4-dione (750 mg), tert-butyl 9-{2-[(5-iodopyridin-3-yl)oxy]ethyl}-3-azaspiro[5.5]undecane-3-carboxylate (1.25 g), CuI (950 mg), 1,10-phenanthroline (950 mg), DOX (10 mL) and potassium carbonate (1,125 mg) were added, and the mixture was stirred under microwave irradiation at 150°C for 2 hours. At room temperature, CuI (950 mg) and 1,10-phenanthroline (950 mg) were added thereto, and the mixture was stirred under microwave at 150°C for 2 hours. The reaction mixture was filtered through celite (R) pad using EtOAc. The filtrate was concentrated under reduced pressure, and the residue was purified by basic silica gel column chromatography (hexane/EtOAc), thus obtaining tert-butyl 9-{2-[(5-{3-[(4-methoxyphenylmethyl]-2,4-dioxo-1,3-diazinan-1-yl}pyridin-3-yl)oxy]ethyl}-3-azaspiro[5.5]undecane-3-carboxylate (760 mg) as a foam-like solid.

### Production Example 36

To tert-butyl 9-{2-[(5-{3-[(4-methoxyphenyl)methyl]-2,4-dioxo-1,3-diazinan-1-yl}pyridin-3-yl)oxy]ethyl}-3-azaspiro[5.5]undecane-3-carboxylate (680 mg) were added TFA (7 mL) and TfOH (0.45 mL) under ice-bath cooling, and the mixture was stirred at 65°C for 1 hour. The reaction mixture was concentrated under reduced pressure, and Et₃N (5 mL) was slowly added under ice-bath cooling. Then, the mixture was purified by basic silica gel column chromatography (CHCl₃/MeOH), thus obtaining 1-{5-[2-(3-azaspiro[5.5]undecan-9-yl)ethoxy]pyridin-3-yl}-1,3-diazinane-2,4-dione (330 mg) as a solid.

### Production Example 41

At room temperature, 1-(3-bromophenyl)-1,3-diazinane-2,4-dione (300 mg), tert-butyl 5-(4,4,5,5-tetramethyl-1,3,2-dioxaborolan-2-yl)-3,6-dihydropyridine-1(2H)-carboxylate (689 mg), PdCl₂(dppf)·CH₂Cl₂ (91 mg) and potassium carbonate (462 mg) were suspended in DOX (4 mL) and water (1 mL). The reaction mixture was degassed and was stirred at 90°C overnight under an argon atmosphere. Water was added to the reaction mixture at room temperature, the mixture was extracted three times with EtOAc, washed twice with saturated saline, dried over anhydrous sodium sulfate, then filtered, and concentrated under reduced pressure. The resulting residue was purified by basic silica gel column chromatography (hexane/EtOAc), thus obtaining tert-butyl 5-[3-(2,4-dioxo-1,3-diazinan-1-yl)phenyl]-3,6-dihydropyridine-1(2H)-carboxylate (397 mg) as a foam-like solid.

### Production Example 43

In CH₂Cl₂ (8 mL), 1-[3-(1,2,5,6-tetrahydropyridin-3-yl)phenyl]-1,3-diazinane-2,4-dione n hydrochloride (150 mg) was suspended, DIPEA (250 µL) was added at room temperature, and the mixture was stirred at room temperature for 10 minutes. Subsequently, tert-butyl 9-oxo-3-azaspiro[5.5]undecane-3-carboxylate (195 mg) and acetic acid (250 µL) were added, and the mixture as stirred at room temperature for 10 minutes. Then, sodium triacetoxyborohydride (250 mg) was added at room temperature, and the mixture was stirred at room temperature for 1 hour. THF (2 mL) was added at room temperature and the mixture was stirred at room temperature for 1 hour. DMF (2 mL) was further added, and the mixture was stirred at room temperature overnight. Tert-butyl 9-oxo-3-azaspiro[5.5]undecane-3-carboxylate (80 mg) and sodium triacetoxyborohydride (80 mg) were added thereto, and the mixture was stirred at room temperature overnight. Saturated aqueous sodium hydrogen carbonate was added to the reaction mixture, followed by extraction with EtOAc five times. The collected organic layer was washed with saturated saline, dried over anhydrous sodium sulfate, and filtered, and the filtrate was concentrated under reduced pressure. The residue was purified by silica gel column chromatography (CHCl₃/MeOH), thus obtaining tert-butyl 9-{5-[3-(2,4-dioxo-1,3-diazinan-1-yl)phenyl]-3,6-dihydropyridin-1(2H)-yl}-3-azaspiro[5.5]undecane-3-carboxylate (232 mg) as a foam-like solid.

### Production Example 55

Under an argon atmosphere, to a THF (10 mL) solution of trimethylsilylacetylene (1.35 mL) was added n-butyllithium (1.6 M n-hexane solution, 5.7 mL) under cooling in a dry ice/acetone bath, and the mixture was stirred at the same temperature for 15 minutes. Next, a THF (5 mL) solution of tert-butyl 9-formyl-3-azaspiro[5.5]undecane-3-carboxylate (920 mg) was added at the same temperature, and the mixture was heated to room temperature and stirred for 2 hours. Saturated aqueous ammonium chloride was added, and the mixture was extracted with EtOAc. The organic layer was dried over anhydrous magnesium sulfate, followed by filtration, and the solvent was removed under reduced pressure. The resulting residue was dissolved in MeOH (10 mL), potassium carbonate (1,130 mg) was added at room temperature, and the mixture was stirred for 3 hours. THF (5 mL), water (5 mL), and di-tert-butyl dicarbonate (714 mg) were added at room temperature, and the mixture was stirred for 30 minutes. The aqueous layer was extracted with EtOAc, and the collected organic layer was dried over anhydrous magnesium sulfate, followed by filtration, and the solvent was removed under reduced pressure. The residue was purified by silica gel column chromatography (hexane/EtOAc), thus obtaining tert-butyl rac-9-[(1R)-1-hydroxyprop-2-yn-1-yl]-3-azaspiro[5.5]undecane-3-carboxylate (160 mg) as an oil.

### Production Example 56

Under an argon atmosphere, to a THF (5 mL) solution of 1-(3-iodophenyl)-1,3-diazinane-2,4-dione (157 mg) were added CuI (19 mg), Et₃N (416 µL), and PdCl₂(PPh₃)₂ (35 mg) at room temperature. Under an argon atmosphere, to this solution was added a THF (2 mL) solution of tert-butyl rac-9-[(1R)-1-hydroxyprop-2-yn-1-yl]-3-azaspiro[5.5]undecane-3-carboxylate (153 mg) in 10 portions every 5 minutes with stirring at room temperature. Water was added, and the mixture was extracted with EtOAc. The organic layer was dried over anhydrous magnesium sulfate, followed by filtration, and the solvent was removed under reduced pressure. The residue was purified by silica gel column chromatography (hexane/EtOAc), thus obtaining tert-butyl rac-9-{(1R)-3-[3-(2,4-dioxo-1,3-diazinan-1-yl)phenyl]-1-hydroxyprop-2-yn-1-yl}-3-azaspiro[5.5]undecane-3-carboxylate (88 mg) as a solid.

### Production Example 58

At room temperature, tert-butyl 9-(2-hydroxyethyl)-3-azaspiro[5.5]undecane-3-carboxylate (3,000 mg) was dissolved in CH₂Cl₂ (38 mL). DMP (8.55 g) was added under ice-bath cooling, and the mixture was stirred under an argon atmosphere at room temperature for 1 hour. To the reaction mixture were added saturated aqueous sodium hydrogen carbonate and saturated aqueous sodium thiosulfate at room temperature, and the mixture was extracted three times with EtOAc, dried over anhydrous sodium sulfate, then filtered, and concentrated under reduced pressure. The resulting residue was purified by silica gel column chromatography (hexane/EtOAc), thus obtaining tert-butyl 9-(2-oxoethyl)-3-azaspiro[5.5]undecane-3-carboxylate (2,813 mg) as an oil.

### Production Example 59

To a MeOH (10 mL) solution of tert-butyl 9-(2-oxoethyl)-3-azaspiro[5.5]undecane-3-carboxylate (300 mg) were added dimethyl (1-diazo-2-oxopropyl)phosphonate (0.2 mL) and potassium carbonate (285 mg) under ice-bath cooling, and under an argon atmosphere, the mixture was stirred at room temperature for 2 hours. The reaction was quenched by adding saturated aqueous ammonium chloride to the reaction mixture under ice-bath cooling. Water and EtOAc were added to separate the organic layer from the aqueous layer by a separation operation, and the organic layer was washed once with saturated saline, dried over anhydrous sodium sulfate, filtered, and concentrated under reduced pressure. The residue was purified by silica gel column chromatography (hexane/EtOAc), thus obtaining tert-butyl 9-(prop-2-yn-1-yl)-3-azaspiro[5.5]undecane-3-carboxylate (170 mg) as an oil.

### Production Example 60

At room temperature, 2-bromo-4-iodopyridine (1,000 mg), 3-[(4-methoxyphenyl)methyl]-1,3-diazinane-2,4-dione (908 mg), Pd₂(dba)₃ (316 mg), XPhos (368 mg), and cesium carbonate (2,298 mg) were suspended in toluene (35 mL) and stirred overnight at 100°C under an argon atmosphere. Water and CHCl₃ were added to the reaction mixture at room temperature, and the mixture was extracted with a phase separator and was concentrated under reduced pressure. The resulting residue was purified by silica gel column chromatography (hexane/EtOAc), thus obtaining 1-(2-bromopyridin-4-yl)-3-[(4-methoxyphenyl)methyl]-1,3-diazinane-2,4-dione (250 mg) as a foam-like solid.

### Production Example 61

At room temperature, 1-(2-bromopyridin-4-yl)-3-[(4-methoxyphenyl)methyl]-1,3-diazinane-2,4-dione (365 mg) and tert-butyl 9-(prop-2-yn-1-yl)-3-azaspiro[5.5]undecane-3-carboxylate (409 mg) were dissolved in DOX (3 mL). At room temperature, PdCl₂(PPh₃)₂ (66 mg), CuI (36 mg), and Et₃N (0.77 mL) were added, and under an argon atmosphere, the mixture was stirred at 60°C for 6 hours. Water was added to the reaction mixture at room temperature, and the mixture was extracted three times with EtOAc. The collected organic layer was washed with saturated saline, dried over anhydrous sodium sulfate, then filtered, and concentrated under reduced pressure. The resulting residue was purified by basic silica gel column chromatography (hexane/EtOAc), thus obtaining tert-butyl 9-[3-(4- {3-[(4-methoxyphenyl)methyl]-2,4-dioxo-1,3-diazinan-1-yl} pyridin-2-yl)prop-2-yn-1-yl]-3-azaspiro[5.5]undecane-3-carboxylate (536 mg) as a foam-like solid.

### Production Example 62

Tert-butyl 9-[3-(4-{3-[(4-methoxyphenyl)methyl]-2,4-dioxo-1,3-diazinan-1-yl}pyridin-2-yl)prop-2-yn-1-yl]-3-azaspiro[5.5]undecane-3-carboxylate (536 mg) was dissolved in TFA (9 mL) at room temperature. TfOH (0.93 mL) was added at room temperature, and the mixture was stirred under an argon atmosphere at 60°C for 2 hours. The reaction mixture was concentrated under reduced pressure, Et₃N (1.9 mL) was added to the resulting residue under ice-bath cooling, and the mixture was purified by basic silica gel column chromatography (CHCl₃/MeOH), thus obtaining 1-{2-[3-(3-azaspiro[5.5]undecan-9-yl)prop-1-yn-1-yl]pyridin-4-yl}-1,3-diazinane-2,4-dione (275 mg) as a solid.

### Production Example 63

Mg (1.1 g) and ZnBr₂ (510 mg) were dried by heating with a heat gun under reduced pressure for 5 minutes and allowed to cool to room temperature. THF (100 mL) was added, followed by addition of 3-bromo-1-(trimethylsilyl)-1-propyne (3.4 mL) under ice-bath cooling, and the mixture was stirred at room temperature for 1 hour under an argon atmosphere. To the reaction mixture was added tert-butyl 9-oxo-3-azaspiro[5.5]undecane-3-carboxylate (4 g) under ice-bath cooling, and under an argon atmosphere, the mixture was stirred at room temperature overnight. The reaction mixture was filtered through celite(R) pad mixed with a small amount of basic silica gel using EtOAc, and the filtrate was concentrated under reduced pressure. The residue was purified by silica gel column chromatography (hexane/EtOAc), thus obtaining tert-butyl 9-hydroxy-9-[3-(trimethylsilyl)prop-2-yn-1-yl]-3-azaspiro[5.5]undecane-3-carboxylate (3.49 g) as a foam-like solid.

### Production Example 64

To a MeOH (50 mL) solution of tert-butyl 9-hydroxy-9-[3-(trimethylsilyl)prop-2-yn-1-yl]-3-azaspiro[5.5]undecane-3-carboxylate (3.49 g) was added potassium carbonate (3.8 g) under ice-bath cooling, and the mixture was stirred at room temperature overnight. Saturated aqueous ammonium chloride was added under ice-bath cooling, and the mixture was diluted with EtOAc and water and extracted three times with EtOAc. The collected organic layer was dried over anhydrous sodium sulfate, filtered, and concentrated under reduced pressure, thus obtaining tert-butyl 9-hydroxy-9-(prop-2-yn-1-yl)-3-azaspiro[5.5]undecane-3-carboxylate (3.28 g) as an oil.

### Production Example 65

2-Fluoro-3-iodoaniline (937 mg), ethyl acrylate (2.2 mL), DBU (591 µL), and DL-lactic acid (294 µL) were added, and the mixture was stirred under microwave irradiation at 140°C for 8 hours. Saturated aqueous sodium hydrogen carbonate was added to the reaction mixture at room temperature, and the mixture was extracted with EtOAc. The organic layer was washed with saturated saline, dried over anhydrous sodium sulfate, then filtered, and concentrated under reduced pressure. The resulting residue was purified by silica gel column chromatography (hexane/EtOAc), thus obtaining ethyl 3-(2-fluoro-3-iodoanilino)propanoate (750 mg) as an oil.

### Production Example 66

Ethyl 3-(2-fluoro-3-iodoanilino)propanoate (750 mg) was dissolved in AcOH (10 mL) and water (3 mL). Sodium cyanate (437 mg) was added at room temperature, and the mixture was stirred at room temperature for 3 hours. Sodium cyanate (434 mg) was added thereto at room temperature, and the mixture was stirred at room temperature overnight. Water was added to the reaction mixture, and the mixture was extracted with EtOAc. The organic layer was washed with water, twice with saturated aqueous sodium hydrogen carbonate and with saturated saline, dried over anhydrous magnesium sulfate, filtered, and concentrated under reduced pressure. The residue was suspended in MeCN (10 mL), benzyltrimethylammonium hydroxide (40% MeOH solution, 558 mg) was added at room temperature, and the mixture was stirred at room temperature overnight. The reaction mixture was concentrated, and the residue was purified by silica gel column chromatography (hexane/EtOAc to CHCl₃/MeOH), thus obtaining 1-(2-fluoro-3-iodophenyl)-1,3-diazinane-2,4-dione (465 mg) as a solid.

### Production Example 82

Under an argon atmosphere, to a THF (120 mL) solution of EtOAc (4 mL) was added LHMDS (1.1 M THF solution, 35 mL) in a dry ice/acetone bath, and the mixture was stirred at the same temperature for 5 minutes. Under an argon atmosphere, a THF (30 mL) solution of tert-butyl 9-oxo-3-azaspiro[5.5]undecane-3-carboxylate (7 g) was added in a dry ice/acetone bath, and under an argon atmosphere, the mixture was stirred at room temperature overnight. The reaction was quenched with saturated aqueous ammonium chloride under ice-bath cooling. Water and EtOAc were added, and the mixture was extracted twice with EtOAc. The collected organic layer was dried over anhydrous sodium sulfate, filtered, and concentrated under reduced pressure. The residue was purified by silica gel column chromatography (hexane/EtOAc), thus obtaining tert-butyl 9-(2-ethoxy-2-oxoethyl)-9-hydroxy-3-azaspiro[5.5]undecane-3-carboxylate (7.96 g) as an oil.

### Production Example 83

To a CH₂Cl₂ (150 mL) suspension of tert-butyl 9-(2-ethoxy-2-oxoethyl)-9-hydroxy-3-azaspiro[5.5]undecane-3-carboxylate (7.96 g) and 1,8-bis(dimethylamino)naphthalene (19.9 g) was added trimethyloxonium tetrafluoroborate (11.94 g) under ice-bath cooling, and under an argon atmosphere, the mixture was stirred at room temperature overnight. Water was added under ice-bath cooling, and the mixture was extracted three times with CHCl₃. The collected organic layer was dried over anhydrous sodium sulfate, filtered, and concentrated under reduced pressure. The residue was purified by silica gel column chromatography (hexane/EtOAc), thus obtaining tert-butyl 9-(2-ethoxy-2-oxoethyl)-9-methoxy-3-azaspiro[5.5]undecane-3-carboxylate (1.76 g) as an oil.

### Production Example 84

To a THF (40 mL) suspension of LAH (400 mg) was slowly added a THF (20 mL) solution of tert-butyl 9-(2-ethoxy-2-oxoethyl)-9-methoxy-3-azaspiro[5.5]undecane-3-carboxylate (1.76 g) under ice-bath cooling, and the mixture was stirred under ice-bath cooling for 30 minutes. Under ice-bath cooling, a small amount of saturated aqueous ammonium chloride was slowly added until foaming ceased. The reaction mixture was filtered through celite(R) pad using CHCl₃, and the filtrate was concentrated under reduced pressure. The residue was purified by silica gel column chromatography (hexane/EtOAc), thus obtaining tert-butyl 9-(2-hydroxyethyl)-9-methoxy-3-azaspiro[5.5]undecane-3-carboxylate (1.25 g) as a solid.

### Production Example 85

To a DMF (4.5 mL) solution of tert-butyl 9-(2-hydroxyethyl)-9-methoxy-3-azaspiro[5.5]undecane-3-carboxylate (300 mg) was added NaH (60%, dispersed in liquid paraffin, 60 mg) under ice-bath cooling, and under an argon atmosphere, the mixture was stirred under ice-bath cooling for 5 minutes and at room temperature for 5 minutes. Under ice-bath cooling, 1-fluoro-3-nitrobenzene (200 µL) was added, and under an argon atmosphere, the mixture was stirred under ice-bath cooling for 5 minutes, at room temperature for 1 hour, and at 40°C for 24 hours. Water was added under ice-bath cooling, the mixture was stirred at room temperature for 10 minutes, and the resulting powder was collected by filtration. The powder was dissolved in EtOAc, washed with saturated saline, dried over anhydrous magnesium sulfate, then filtered, and concentrated under reduced pressure, thus obtaining tert-butyl 9-methoxy-9-[2-(3-nitrophenoxy)ethyl]-3-azaspiro[5.5]undecane-3-carboxylate (421 mg) as a solid.

### Production Example 86

To a solution in EtOH (6 mL) and THF (6 mL) of tert-butyl 9-methoxy-9-[2-(3-nitrophenoxy)ethyl]-3-azaspiro[5.5]undecane-3-carboxylate (420 mg) was added 10% Pd/C (about 50% water content, 100 mg) at room temperature, and the mixture was stirred at room temperature for 1 hour under a hydrogen atmosphere at normal pressure. The reaction vessel was purged with argon gas, Celite(R) was added. The mixture was filtered through celite(R) pad and washed with CHCl₃, and the filtrate was concentrated under reduced pressure to give tert-butyl 9-[2-(3-aminophenoxy)ethyl]-9-methoxy-3-azaspiro[5.5]undecane-3-carboxylate (402 mg) as an oil.

### Production Example 89

At room temperature, 5-iodo-4-methylpyridin-3-ol (500 mg) and tert-butyl 9-(2-hydroxyethyl)-3-azaspiro[5.5]undecane-3-carboxylate (822 mg) were suspended in toluene (11 mL). Under ice-bath cooling, N,N,N',N'-tetramethylazodicarboxamide (1,100 mg) and tri-n-butylphosphine (1.7 mL) were added, and the mixture was stirred under an argon atmosphere at room temperature for 2 hours. Saturated aqueous sodium hydrogen carbonate was added to the reaction mixture at room temperature, and the mixture was extracted three times with EtOAc. The organic layer was washed with saturated saline, dried over anhydrous sodium sulfate, then filtered, and concentrated under reduced pressure. The resulting residue was purified by basic silica gel column chromatography (hexane/EtOAc), thus obtaining tert-butyl 9-{2-[(5-iodo-4-methylpyridin-3-yl)oxy]ethyl}-3-azaspiro[5.5]undecane-3-carboxylate (1,301 mg) as an oil.

### Production Example 90

Tert-butyl 9-{2-[(5-iodo-4-methylpyridin-3-yl)oxy]ethyl}-3-azaspiro[5.5]undecane-3-carboxylate (605 mg), 3-[(4-methoxyphenyl)methyl]-1,3-diazinane-2,4-dione (347 mg), CuI (229 mg), 1,10-phenanthroline (225 mg) and potassium carbonate (470 mg) were suspended in DMF (5 mL) at room temperature and stirred under microwave irradiation at 150°C for 2 hours. CHCl₃/IPA was added to the reaction mixture at room temperature, and the mixture was filtered and was concentrated under reduced pressure. The resulting residue was purified by basic silica gel column chromatography (hexane/EtOAc), thus obtaining tert-butyl 9-{2-[(5-{3-[(4-methoxyphenyl)methyl]-2,4-dioxo-1,3-diazinan-1-yl}-4-methylpyridin-3-yl)oxy]ethyl}-3-azaspiro[5.5]undecane-3-carboxylate (115 mg) as a foam-like solid.

### Production Example 91

At room temperature, tert-butyl 9-{2-[(5-{3-[(4-methoxyphenyl)methyl]-2,4-dioxo-1,3-diazinan-1-yl}-4-methylpyridin-3-yl)oxy]ethyl}-3-azaspiro[5.5]undecane-3-carboxylate (372 mg) was dissolved in TFA (6 mL). TfOH (0.26 mL) was added at room temperature, and the mixture was stirred under an argon atmosphere at 60°C for 1 hour. TfOH (0.31 mL) was added at room temperature, and the mixture was stirred under an argon atmosphere at 60°C for 2 hours and was concentrated under reduced pressure. Et₃N (1.2 mL) was added to the resulting residue under ice-bath cooling, and the mixture was purified by basic silica gel column chromatography (CHCl₃/MeOH), thus obtaining 1-{5-[2-(3-azaspiro[5.5]undecan-9-yl)ethoxy]-4-methylpyridin-3-yl}-1,3-diazinane-2,4-dione (212 mg) as a solid.

### Production Example 97

Tert-butyl 9-{2-[(3-fluoro-4-iodopyridin-2-yl)oxy]ethyl} -3-azaspiro[5 .5]undecane-3-carboxylate (310 mg) was dissolved in DMF (2 mL). At room temperature, 3-[(4-methoxyphenyl)methyl]-1,3-diazinane-2,4-dione (185 mg), CuI (60 mg) and potassium carbonate (250 mg) were added, and finally trans-N,N'-dimethylcyclohexane-1,2-diamine (50 µL) was added. The mixture was stirred under microwave irradiation at 120°C for 2 hours. The reaction mixture was poured into water and was stirred at room temperature for 10 minutes, and the resulting powder was collected by filtration. The powder was dissolved in CHCl₃, silica gel was added thereto, and the mixture was concentrated under reduced pressure. The residue was purified by silica gel column chromatography (hexane/EtOAc), thus obtaining tert-butyl 9-{2-[(3-fluoro-4-{3-[(4-methoxyphenyl)methyl]-2,4-dioxo-1,3-diazinan-1-yl}pyridin-2-yl)oxy]ethyl}-3-azaspiro[5.5]undecane-3-carboxylate (187 mg) as a solid.

### Production Example 109

Tert-butyl 4-[({(7M)-4-tert-butoxy-6-cyclopropyl-2-(ethanesulfonyl)-7-[6-fluoro-5-methyl-2-(triphenylmethyl)-2H-indazol-4-yl]quinazolin-8-yl}oxy)methyl]benzoate (160 mg), NMP (3 mL), (2S)-1-ethyl-2-methylpiperazine dihydrochloride (210 mg) and DIPEA (480 µL) were added at room temperature, and the mixture was stirred under microwave irradiation at 150°C for 3 hours. EtOAc and water were added to separate the organic layer from the aqueous layer by a separation operation, and the organic layer was washed twice with saturated saline, dried over anhydrous sodium sulfate, filtered, and concentrated under reduced pressure. The residue was dissolved in THF (5 mL), 3,4-dihydro-2H-pyrane (320 µL) and p-toluenesulfonic acid monohydrate (200 mg) were added under ice-bath cooling, and under an argon atmosphere, the mixture was stirred at room temperature overnight. After Et₃N (1.6 mL) was added under ice-bath cooling, the reaction mixture was concentrated under reduced pressure. The residue was purified by silica gel column chromatography (CHCl₃/MeOH), thus obtaining tert-butyl 4-[({(7M)-6-cyclopropyl-2-[(3S)-4-ethyl-3-methylpiperazin-1-yl]-7-[6-fluoro-5-methyl-1-(oxan-2-yl)-1H indazol-4-yl]-4-hydroxyquinazolin-8-yl} oxy)methyl]benzoate (103 mg) as a foam-like solid.

### Production Example 110

To a MeCN (5 mL) suspension of tert-butyl 4-[({(7M)-6-cyclopropyl-2-[(3S)-4-ethyl-3-methylpiperazin-1-yl]-7-[6-fluoro-5-methyl-1-(oxan-2-yl)-1H indazol-4-yl]-4-hydroxyquinazolin-8-yl}oxy)methyl]benzoate (103 mg) and (2S)-azetidine-2-carboxamide (65 mg) were added DIPEA (300 µL) and PyAOP (290 mg) under ice-bath cooling, and under an argon atmosphere, the mixture was stirred at 50°C for 6 hours. The reaction mixture was concentrated under reduced pressure, and the residue was purified by silica gel column chromatography (CHCl₃/MeOH/28% aqueous ammonia), thus obtaining tert-butyl 4-[({(7M)-4-[(2S)-2-carbamoylazetidin-1-yl]-6-cyclopropyl-2-[(3S)-4-ethyl-3-methylpiperazin-1-yl]-7-[6-fluoro-5-methyl-1-(oxan-2-yl)-1H-indazol-4-yl]quinazolin-8-yl}oxy)methyl]benzoate (110 mg) as a foam-like solid.

### Production Example 111

To a CH₂Cl₂ (5 mL) solution of tert-butyl 4-[({(7M)-4-[(2S)-2-carbamoylazetidin-1-yl]-6-cyclopropyl-2-[(3S)-4-ethyl-3-methylpiperazin-1-yl]-7-[6-fluoro-5-methyl-1-(oxan-2-yl)-1H-indazol-4-yl]quinazolin-8-yl}oxy)methyl]benzoate (110 mg) was added TFA (0.4 mL) under ice-bath cooling, and the mixture was stirred at room temperature overnight. The reaction mixture was concentrated under reduced pressure, THF (3 mL) and saturated aqueous sodium hydrogen carbonate were added under ice-bath cooling, and the mixture was stirred under ice-bath cooling for 1 minute. CHCl₃/IPA (4/1) and water were added, the pH was adjusted to 8 to 9 by the addition of hydrochloric acid (1M), and the aqueous layer was extracted four times with CHCl₃/IPA (4/1). The collected organic layer was dried over anhydrous sodium sulfate, filtered, and concentrated under reduced pressure, thus obtaining 4-({[(7M)-4-[(2S)-2-carbamoylazetidin-1-yl]-6-cyclopropyl-2-[(3S)-4-ethyl-3-methylpiperazin-1-yl]-7-(6-fluoro-5-methyl-1H-indazol-4-yl)quinazolin-8-yl]oxy}methyl)benzoate (117 mg) as a solid.

### Production Example 112

A DOX (7 mL) solution of 3-(methanesulfonyl)-4-methyl-4H-1,2,4-triazol (200 mg) and tert-butyl 9-(2-hydroxyethyl)-3-azaspiro[5.5]undecane-3-carboxylate (383 mg) was stirred under ice-bath cooling, to which tBuOK (153 mg) was added. After stirring at room temperature for 5 minutes, the mixture was stirred at 100°C for 1.5 hours. Saturated aqueous ammonium chloride was added under ice-bath cooling, and the mixture was stirred. EtOAc and water were added to separate the organic layer from the aqueous layer, and the aqueous layer was extracted with EtOAc. The collected organic layer was dried over anhydrous magnesium sulfate, then filtered, and concentrated under reduced pressure. The residue was dissolved in THF (15 mL) and stirred in a dry ice/MeCN bath under an argon atmosphere. Subsequently, 2,2,6,6-tetramethylpiperidinylmagnesium chloride lithium chloride complex (1M THF/toluene solution, 10 mL) was gently added dropwise, and the mixture was stirred for 2 hours while being cooled at the same temperature. The reaction mixture was transferred under ice-bath cooling, iodine (450 mg) was added, and the mixture was stirred for 15 minutes. Saturated aqueous ammonium chloride was added while cooling with ice, the mixture was stirred, and EtOAc and water were added to separate the organic layer from the aqueous layer. Then, the aqueous layer was extracted once with EtOAc. The collected organic layer was dried over anhydrous magnesium sulfate, then filtered, and concentrated under reduced pressure. The residue was purified by silica gel chromatography (hexane/EtOAc), thus obtaining tert-butyl 9-{2-[(5-iodo-4-methyl-4H-1,2,4-triazol-3-yl)oxy]ethyl}-3-azaspiro[5.5]undecane-3-carboxylate (158 mg) as a solid.

### Production Example 119

To a THF (30 mL) solution of tert-butyl 3,9-diazaspiro[5.5]undecane-3-carboxylate (1.5 g) and 3-chloro-3-methyl-1-butyne (1 mL) were added Et₃N (2.5 mL) and CuCl (60 mg) under ice-bath cooling, and under an argon atmosphere, the mixture was stirred at room temperature for 2 hours. The reaction mixture was filtered through celite(R) pad mixed with a small amount of basic silica gel using EtOAc. The filtrate was concentrated under reduced pressure, and the residue was purified by silica gel column chromatography (hexane/EtOAc), thus obtaining tert-butyl 9-(2-methylbut-3-yn-2-yl)-3,9-diazaspiro[5.5]undecane-3-carboxylate (1.56 g) as a solid.

### Production Example 132

At room temperature, tert-butyl 3,9-diazaspiro[5.5]undecane-3-carboxylate (10 g), 2-bromoethanol (4.3 mL) and potassium carbonate (16.3 g) were suspended in MeCN (60 mL) and stirred at 80°C for 9 hours under an argon atmosphere. The reaction mixture was filtered and was concentrated under reduced pressure, and the resulting residue was purified by silica gel column chromatography (hexane/EtOAc), thus obtaining tert-butyl 9-(2-hydroxyethyl)-3,9-diazaspiro[5.5]undecane-3-carboxylate (11.63 g) as an oil.

### Production Example 136

Under an argon atmosphere, tBuOK (513 mg) was added to a THF (10 mL) solution of tert-butyl 9-(2-hydroxyethyl)-3-azaspiro[5.5]undecane-3-carboxylate (675 mg) and 5-chloro-3-iodopyridazine (540 mg) under ice-bath cooling, and the mixture was stirred for 15 minutes under ice-bath cooling. Saturated aqueous ammonium chloride was added, and the mixture was extracted with EtOAc. The organic layer was dried over anhydrous magnesium sulfate, followed by filtration, and the solvent was removed under reduced pressure. The residue was purified by basic silica gel column chromatography (hexane/EtOAc), thus obtaining tert-butyl 9-{2-[(6-iodopyridazin-4-y1)oxy]ethyl}-3-azaspiro[5.5]undecane-3-carboxylate (666 mg) as a foam-like solid.

### Production Example 143

To a THF (50 mL) solution of tert-butyl 3,9-diazaspiro[5.5]undecane-3-carboxylate (3 g) were added a solution in water (12.5 mL) of potassium carbonate (6.5 g) and benzyloxyacetyl chloride (2.38 mL), and the mixture was stirred at the same temperature for 30 minutes. Water was added, and the mixture was extracted with EtOAc. The organic layer was dried over anhydrous magnesium sulfate, followed by filtration, and the solvent was removed under reduced pressure. The resulting residue was purified by silica gel column chromatography (hexane/EtOAc), thus obtaining tert-butyl 9-[(benzyloxy)acetyl]-3,9-diazaspiro[5.5]undecane-3-carboxylate (4.873 g) as an oil.

### Production Example 144

Under a nitrogen atmosphere, to a THF (100 mL) solution of tert-butyl 9-[(benzyloxy)acetyl]-3,9-diazaspiro[5.5]undecane-3-carboxylate (4.05 g) was added titanium(IV) isopropoxide (3 mL) in a MeOH/ice bath, and then ethylmagnesium bromide (1M THF solution, 30 mL) was added dropwise over 5 minutes. Thereafter, the MeOH/ice bath was taken off, and the mixture was stirred at room temperature for 3 hours. Saturated aqueous potassium sodium tartrate was added thereto, the mixture was stirred for 30 minutes, and the aqueous layer was extracted with EtOAc. The organic layer was dried over anhydrous magnesium sulfate, filtered, and concentrated under reduced pressure. The residue was purified by silica gel column chromatography (hexane/EtOAc), thus obtaining tert-butyl 9-{1-[(benzyloxy)methyl]cyclopropyl}-3,9-diazaspiro[5.5]undecane-3-carboxylate (2.41 g) as an oil which also contained impurities.

### Production Example 145

To tert-butyl 9-{1-[(benzyloxy)methyl]cyclopropyl}-3,9-diazaspiro[5.5]undecane-3-carboxylate (2.41 g) and pentamethylbenzene (1,206 mg) were added TFA (9 mL) and TfOH (1 mL) under ice-bath cooling, and the mixture was stirred at the same temperature for 15 minutes. The reaction mixture was diluted with THF (10 mL), and aqueous sodium hydroxide (8 M, 20 mL) was added under ice-bath cooling to adjust the pH to about 10. Next, di-tert-butyl dicarbonate (2.54 g) was added, and the mixture was stirred at the same temperature for 30 minutes. Hydrochloric acid (1 M) was added to adjust the pH to about 8, and the aqueous layer was extracted with EtOAc. The organic layer was dried over anhydrous magnesium sulfate, followed by filtration, and the solvent was removed under reduced pressure. The residue was purified by silica gel column chromatography (CHCl₃/MeOH), thus obtaining tert-butyl 9-[1-(hydroxymethyl)cyclopropyl]-3,9-diazaspiro[5.5]undecane-3-carboxylate (1.94 g) as a solid which also contained impurities.

### Production Example 154

To a solution in water (25 mL) of tert-butyl 4-({[(7M)-4-tert-butoxy-6-cyclopropyl-7-[6-fluoro-5-methyl-2-(triphenylmethyl)-2H-indazol-4-yl]-2- {3-[(3R)-3-fluoropyrrolidin-1-yl]-2,2-dimethylpropoxy}quinazolin-8-yl]oxy}methyl)benzoate (2.5 g) was added AcOH (52.45 g) at 25°C, and under a nitrogen atmosphere, the mixture was stirred at 60°C for 2 hours. The reaction mixture was diluted with water at 0°C, and the pH was adjusted to 7 by the addition of saturated aqueous sodium hydrogen carbonate, followed by extraction with EtOAc twice. The collected organic layer was washed with saturated saline, dried over anhydrous sodium sulfate, filtered, and concentrated under reduced pressure. Then, the residue was purified by ODS column chromatography (0.05% formic acid in water/MeCN). The eluate containing the target compound was extracted three times with EtOAc. The collected organic layer was washed with saturated saline, dried over anhydrous sodium sulfate, filtered, and concentrated under reduced pressure, thus obtaining tert-butyl 4-({[(7M)-6-cyclopropyl-7-(6-fluoro-5-methyl-1H-indazol-4-yl)-2-{3-[(3R)-3-fluoropyrrolidin-1-yl]-2,2-dimethylpropoxy } -4-hydroxyquinazolin-8-yl]oxy} methyl)benzoic acid n formate (1.2 g) as a solid.

### Production Example 156

To a CH₂Cl₂ (20 mL) solution of tert-butyl 4-({[(7M)-4-[(2S)-2-carbamoylazetidin-1-yl]-6-cyclopropyl-7-(6-fluoro-5-methyl-1H-indazol-4-yl)-2- {3-[(3 R)-3-fluoropyrrolidin-1-yl]-2,2-dimethylpropoxy}quinazolin-8-yl]oxy}methyl)benzoate (1.18 g) was added TFA (6.56 mL), and the mixture was stirred at 25°C for 2 hours. The reaction mixture was concentrated under reduced pressure, and the residue was purified by ODS column chromatography (0.05% TFA in water/MeCN), thus obtaining 4-({[(7M)-4-[(2S)-2-carbamoylazetidin-1-yl]-6-cyclopropyl-7-(6-fluoro-5-methyl-1H-indazol-4-yl)-2-{3-[(3R)-3-fluoropyrrolidin-1-yl]-2,2-dimethylpropoxylquinazolin-8-yl]oxylmethyl)benzoic acid n (trifluoroacetate) (1.169 g) as a solid.

### Production Example 159

At room temperature, tert-butyl 9-{2-[(4-bromopyridin-2-yl)oxy]ethyl}-9-hydroxy-3-azaspiro[5.5]undecane-3-carboxylate (904 mg), 5,6-dihydrouracil (650 mg), cesium carbonate (1.35 g), EPhos Pd G4 (266 mg), Ephos (155 mg), and DOX (20 mL) were added. The mixture was subjected five times to a degassing operation of decompression and argon gas replacement with stirring and was then stirred under an argon atmosphere at 100°C for 6 hours. The reaction mixture was returned to room temperature and was filtered through celite(R) pad using CHCl₃. The filtrate was concentrated under reduced pressure, and the residue was purified by silica gel column chromatography (hexane/EtOAc), thus obtaining tert-butyl 9-(2-{[4-(2,4-dioxo-1,3-diazinan-1-yl)pyridin-2-yl]oxy}ethyl)-9-hydroxy-3-azaspiro[5.5]undecane-3-carboxylate (665 mg) as a foam-like solid.

### Production Example 161

To a CH₂Cl₂ (40 mL) solution of tert-butyl 4-[({(7M)-4-[(2S)-2-carbamoylazetidin-1-yl]-6-cyclopropyl-2-[2,2-dimethyl-3-(morpholin-4-yl)propoxy]-7-[6-fluoro-5-methyl-1-(oxan-2-yl)-1H-indazol-4-yl]quinazolin-8-yl}oxy)methyl]benzoate (2.04 g) was added TFA (8 mL) under ice-bath cooling, and the mixture was stirred at room temperature for 4 hours. The reaction mixture was concentrated under reduced pressure, and the residue was dissolved in EtOAc (20 mL). HCl/DOX (4 M, 3 mL) was added under ice-bath cooling, and the mixture was concentrated under reduced pressure. The residue was purified by ODS column chromatography (0.1% formic acid in water/0.1% formic acid in MeCN) and then lyophilized, thus obtaining 4-({[(7M)-4-[(2S)-2-carbamoylazetidin-1-yl]-6-cyclopropyl-2-[2,2-dimethyl-3-(morpholin-4-yl)propoxy]-7-(6-fluoro-5-methyl-1H-indazol-4-yl)quinazolin-8-yl]oxy}methyl)benzoic acid n formate (941 mg) as a solid.

### Production Example 165

To a solution in water (5.3 mL) of tert-butyl 4-({[(7M)-4-tert-butoxy-6-cyclopropyl-7-[6-fluoro-5-methyl-2-(triphenylmethyl)-2H-indazol-4-yl]-2-{3-[(3S)-3-fluoropyrrolidin-1-yl]-2,2-dimethylpropoxy}quinazolin-8-yl]oxy}methyl)benzoate (4.60 g) was added AcOH (10.5 mL) at 25°C, and under a nitrogen atmosphere, the mixture was stirred at 60°C for 1 hour. Saturated aqueous sodium hydrogen carbonate was added to the reaction mixture at 0 to 4°C to adjust the pH to 6 to 7, followed by extraction with EtOAc three times. The collected organic layer was washed with saturated saline, dried over anhydrous sodium sulfate, filtered, and concentrated under reduced pressure. Then, the residue was purified by silica gel column chromatography (AcOEt), thus obtaining tert-butyl 4-({[(7M)-6-cyclopropyl-7-(6-fluoro-5-methyl-1H-indazol-4-yl)-2-{3-[(3S)-3-fluoropyrrolidin-1-yl]-2,2-dimethylpropoxy}-4-hydroxyquinazolin-8-yl]oxy}methyl)benzoate (2.6 g) as a solid.

### Production Example 168

Under an argon atmosphere, tert-butyl 4-[({(7M)-4-tert-butoxy-6-cyclopropyl-2-(ethanesulfonyl)-7-[6-fluoro-5-methyl-2-(triphenylmethyl)-2H-indazol-4-yl]quinazolin-8-yl}oxy)methyl]benzoate (870 mg), MeCN (10 mL), and ethanethiol (90 µL) were added, followed by cesium carbonate (430 mg) and DABCO (15 mg) with stirring at room temperature. The mixture was stirred for 50 minutes. Ice and saturated aqueous ammonium chloride were poured into a reaction vessel, followed by extraction with EtOAc twice. The collected organic layer was washed with saturated saline and then dried over anhydrous magnesium sulfate. The solvent was removed under reduced pressure. THF (10 mL), 3,4-dihydro-2H-pyrane (1 mL), and p-toluenesulfonic acid monohydrate (100 mg) were added to the resulting residue. Then, the mixture was stirred under an argon atmosphere at room temperature overnight. After Et₃N (1 mL) was added to the reaction mixture, the solvent was concentrated under reduced pressure. The residue was purified by silica gel column chromatography (hexane/EtOAc), thus obtaining tert-butyl 4-[({(7M)-6-cyclopropyl-2-(ethylsulfanyl)-7-[6-fluoro-5-methyl-1-(oxan-2-yl)-1H-indazol-4-yl]-4-hydroxyquinazolin-8-yl}oxy)methyl]benzoate (552 mg) as a solid.

### Production Example 177

To a CH₂Cl₂ (5 mL) solution of tert-butyl 4-[({(7M)-4-[(2S)-2-carbamoylazetidin-1-yl]-6-cyclopropyl-7-[6-fluoro-5-methyl-1-(oxan-2-yl)-1H-indazol-4-yl]-2-[(2S)-2-methylmorpholin-4-yl]quinazolin-8-yl}oxy)methyl]benzoate (180 mg) was added TFA (0.25 mL) under ice-bath cooling, and the mixture was stirred at room temperature for 3 hours. The reaction mixture was concentrated under reduced pressure, and the residue was dissolved in EtOAc(5 mL). HCl/DOX (4 M, 0.2 mL) was added under ice-bath cooling. The insoluble material was collected by filtration using toluene under a stream of nitrogen and dried under reduced pressure at 40°C overnight, thus obtaining 4-[({(7M)-4-[(2S)-2-carbamoylazetidin-1-yl]-6-cyclopropyl-7-(6-fluoro-5-methyl-1H-indazol-4-yl)-2-[(2S)-2-methylmorpholin-4-yl]quinazolin-8-yl}oxy)methyl]benzoic acid n hydrochloride (72 mg) as a solid.

### Production Example 181

Tert-butyl (3S,5S)-3,5-dimethylpiperazine-1-carboxylate (200 mg), iodoethane (190 mg), MeCN (4 mL), and potassium carbonate (195 mg) were added, and under an argon atmosphere, the mixture was stirred at room temperature for 3 days. Thereafter, the reaction mixture was stirred at 50°C for 4 hours. The cooled reaction suspension was filtered through celite(R) pad, and the resulting filtrate was concentrated under reduced pressure. The residue was purified by basic silica gel column chromatography (CHCl₃/MeOH), thus obtaining tert-butyl (3S,5S)-4-ethyl-3,5-dimethylpiperazine-1-carboxylate (204 mg) as an oil.

### Production Example 182

To a CH₂Cl₂(2 mL) solution of tert-butyl (3S,5S)-4-ethyl-3,5-dimethylpiperazine-1-carbonate (200 mg) was added HCl/DOX (4M, 2 mL) dropwise with stirring at room temperature. Then, the mixture was stirred under an argon atmosphere at room temperature overnight. The reaction mixture was concentrated under reduced pressure, and the residue was subjected to azeotropic distillation with toluene twice, thus obtaining (2S,6S)-1-ethyl-2,6-dimethylpiperazine n hydrochloride (184 mg) as a solid.

### Example 1

At room temperature, 4-({[(7M)-4-[(2S)-2-carbamoylazetidin-1-yl]-6-cyclopropyl-2-[3-(dimethylamino)-2,2-dimethylpropoxy]-7-(6-fluoro-5-methyl-1H-indazol-4-yl)quinazolin-8-yl]oxy}methyl)benzoic acid (50 mg) and 1-{2-[2-(3-azaspiro[5.5]undecan-9-yl)ethoxy]pyridin-4-yl}-1,3-diazinane-2,4-dione (42 mg) were dissolved in DMF (1 mL) and DIPEA (123 µL), PyAOP (50 mg) was added at room temperature, and the mixture was stirred at room temperature for 30 minutes. The reaction mixture was dissolved in MeCN/water and purified by ODS column chromatography (0.1% formic acid in water/MeCN). The target fraction was neutralized with saturated aqueous sodium hydrogen carbonate, and the mixture was extracted three times with CHCl₃/IPA (4/1). The collected organic layer was dried over anhydrous sodium sulfate, then filtered, and concentrated under reduced pressure. The resulting residue was dissolved in a small amount of EtOAc/IPA, followed by addition of hexane. The precipitated solid was collected by filtration, washed with water, and was dried under reduced pressure at 30°C overnight, thus obtaining (2S)-1-[(7M)-6-cyclopropyl-2-[3-(dimethylamino)-2,2-dimethylpropoxy]-8-({4-[9-(2-{[4-(2,4-dioxo-1,3-diazinan-1-yl)pyridin-2-yl]oxy}ethyl)-3-azaspiro[5.5]undecane-3-carbonyl]phenyl}methoxy)-7-(6-fluoro-5-methyl-1H-indazol-4-yl)quinazolin-4-yl]azetidine-2-carboxamide (27.5 mg) as a solid.

### Example 4

To 4-[({4-[(2S)-2-carbamoylazetidin-1-yl]-6-cyclopropyl-2-[3-(dimethylamino)-2,2-dimethylpropoxy]-7-(7-fluoro-3-hydroxynaphthalen-1-yl)quinazolin-8-yl}oxy)methyl]benzoic acid (63 mg) were added 1-[5-fluoro-1-methyl-6-(1-oxa-4,9-diazaspiro[5.5]undecan-4-yl)-1H-indazol-3-yl]-1,3-diazinane-2,4-dione (45 mg), DMF (2 mL) and DIPEA (0.1 mL) under a nitrogen atmosphere, and HOBt (20 mg) and EDC·HCl (29 mg) were added thereto with stirred at room temperature. Then, the mixture was stirred for 4 days. The reaction mixture was diluted with MeCN/water and purified by ODS column chromatography (0.1% formic acid in water/MeCN). Saturated aqueous sodium hydrogen carbonate was added to a fraction containing the low polar peak to adjust the pH to about 6, and the mixture was then extracted three times with CHCl₃/MeOH (4/1). The collected organic layer was dried over anhydrous magnesium sulfate and filtered, and the solvent was removed under reduced pressure, thus obtaining (2S)-1-{6-cyclopropyl-2-[3-(dimethylamino)-2,2-dimethylpropoxy]-8-[(4-{4-[3-(2,4-dioxo-1,3-diazinan-1-yl)-5-fluoro-1-methyl-1H-indazol-6-yl]-1-oxa-4,9-diazaspiro[5.5]undecane-9-carbonyl}phenyl)methoxy]-7-(7-fluoro-3-hydroxynaphthalen-1-yl)quinazolin-4-yl}azetidine-2-carboxamide (single diastereomer with undetermined axial chirality configuration, 19 mg) as a solid.

### Example 7

To a DMF (1 mL) solution of 4-({[(7M)-4-[(2S)-2-carbamoylazetidin-1-yl]-6-cyclopropyl-2-[(3S)-3,4-dimethylpiperazin-1-yl]-7-(6-fluoro-5-methyl-1H-indazol-4-yl)quinazolin-8-yl]oxy}methyl)benzoic acid (60 mg) and 1-{5-[2-(3-azaspiro[5.5]undecan-9-yl)ethoxy]pyridin-3-yl}-1,3-diazinane-2,4-dione (35 mg) were added DIPEA (110 µL) and PyAOP (50 mg) at room temperature, and the mixture was stirred at room temperature for 20 minutes. The reaction mixture was diluted with MeCN/water and purified by ODS column chromatography (0.1% formic acid in water/MeCN). Saturated aqueous sodium hydrogen carbonate was added to a fraction of the target compound, and the mixture was extracted three times with CHCl₃/IPA (4/1). The collected organic layer was dried over anhydrous sodium sulfate, filtered, and concentrated under reduced pressure. The residue was taken up in a small amount of EtOAc/IPA, followed by addition of hexane. The precipitated solid was collected by filtration, washed with water, and was dried under reduced pressure at 30°C overnight, thus obtaining (2S)-1-[(7M)-6-cyclopropyl-2-[(3S)-3,4-dimethylpiperazin-1-yl]-8-({4-[9-(2-{[5-(2,4-dioxo-1,3-diazinan-1-yl)pyridin-3-yl]oxy}ethyl)-3-azaspiro[5.5]undecane-3-carbonyl]phenyl}methoxy)-7-(6-fluoro-5-methyl-1H-indazol-4-yl)quinazolin-4-yl]azetidine-2-carboxamide (39.3 mg) as a solid.

### Example 14

At room temperature, 4-({[(7M)-4-[(2S)-2-carbamoylazetidin-1-yl]-6-cyclopropyl-2-[(3S)-3,4-dimethylpiperazin-1-yl]-7-(6-fluoro-5-methyl-1H-indazol-4-yl)quinazolin-8-yl]oxy}methyl)benzoic acid (16 mg) and 1-{2-[3-(3-azaspiro[5.5]undecan-9-yl))prop-1-yn-1-yl]pyridin-4-yl}-1,3-diazinane-2,4-dione (11 mg) were suspended in DMF (0.6 mL) and DIPEA (0.05 mL). PyAOP (17 mg) was added at room temperature, and the mixture was stirred at room temperature for 15 minutes. The reaction mixture was dissolved in MeCN/water and purified by ODS column chromatography (0.1% formic acid in water/MeCN). The target fraction was neutralized with saturated aqueous sodium hydrogen carbonate, and the mixture was extracted three times with CHCl₃/IPA (4/1). The collected organic layer was dried over anhydrous sodium sulfate, then filtered, and concentrated under reduced pressure. The residue was suspended in water, collected by filtration, and dried under reduced pressure at 40°C overnight, thus obtaining (2S)-1-[(7M)-6-cyclopropyl-2-[(3S)-3,4-dimethylpiperazin-1-yl]-8-{[4-(9-{3-[4-(2,4-dioxo-1,3-diazinan-1-yl)pyridin-2-yl]prop-2-yn-1-yl}-3-azaspiro[5.5]undecane-3-carbonyl)phenyl]methoxy}-7-(6-fluoro-5-methyl-1H-indazol-4-yl)quinazolin-4-yl]azetidine-2-carboxamide (5.5 mg) as a solid.

### Example 20

To a DMF (1 mL) solution of 4-({[(7M)-4-[(2S)-2-carbamoylazetidin-1-yl]-6-cyclopropyl-2-[(3S)-3,4-dimethylpiperazin-1-yl]-7-(6-fluoro-5-methyl-1H-indazol-4-yl)quinazolin-8-yl]oxy}methyl)benzoic acid (33 mg) and 1-{5-[2-(3-azaspiro[5.5]undecan-9-yl)ethoxy]-4-methylpyridin-3-yl}-1,3-diazinane-2,4-dione (21 mg) were added DIPEA (60 µL) and PyAOP (30 mg) at room temperature, and the mixture was stirred at room temperature for 20 minutes. The reaction mixture was diluted with MeCN/water and purified by ODS column chromatography (0.1% formic acid in water/MeCN). Saturated aqueous sodium hydrogen carbonate was added to a fraction of the target compound, and the mixture was extracted three times with CHCl₃/IPA (4/1). The collected organic layer was dried over anhydrous sodium sulfate, filtered, and concentrated under reduced pressure. The residue was suspended in a small amount of water, and the insoluble material was collected by filtration and dried under reduced pressure at 30°C overnight, thus obtaining (2S)-1-[(7M)-6-cyclopropyl-2-[(3S)-3,4-dimethylpiperazin-1-yl]-8-({4-[9-(2-{[5-(2,4-dioxo-1,3-diazinan-1-yl)-4-methylpyridin-3-yl]oxy}ethyl)-3-azaspiro[5.5]undecane-3-carbonyl]phenyl}methoxy)-7-(6-fluoro-5-methyl-1H-indazol-4-yl)quinazolin-4-yl]azetidine-2-carboxamide (20 mg) as a solid.

### Example 24

To a DMF (1 mL) solution of 4-({[(7M)-4-[(2S)-2-carbamoylazetidin-1-yl]-6-cyclopropyl-2-[(3S)-4-ethyl-3-methylpiperazin-1-yl]-7-(6-fluoro-5-methyl-1H-indazol-4-yl)quinazolin-8-yl]oxy}methyl)benzoic acid (35 mg) and 1-{5-[2-(3-azaspiro[5.5]undecan-9-yl)ethoxy]pyridin-3-yl}-1,3-diazinane-2,4-dione (20 mg) were added DIPEA (60 µL) and PyAOP (33 mg) at room temperature, and the mixture was stirred at room temperature for 20 minutes. The reaction mixture was diluted with MeCN/water and purified by ODS column chromatography (0.1% formic acid in water/MeCN). Saturated aqueous sodium hydrogen carbonate was added to a fraction of the target compound, and the mixture was extracted three times with CHCl₃/IPA (4/1). The collected organic layer was dried over anhydrous sodium sulfate, filtered, and concentrated under reduced pressure. The residue was suspended in water, and the insoluble material was collected by filtration and dried under reduced pressure at 30°C overnight, thus obtaining (2S)-1-[(7M)-6-cyclopropyl-8-({4-[9-(2-{[5-(2,4-dioxo-1,3-diazinan-1-yl)pyridin-3-yl]oxy}ethyl)-3-azaspiro[5.5]undecane-3-carbonyl]phenyl}methoxy)-2-[(3 S)-4-ethyl-3-methylpiperazin-1-yl]-7-(6-fluoro-5-methyl-1H-indazol-4-yl)quinazolin-4-yl]azetidine-2-carboxamide (19 mg) as a solid.

### Example 39

(2S)-1-[(7M)-6-Cyclopropyl-8-({4-[9-(2-{[5-(2,4-dioxo-1,3-diazinan-1-yl)pyridin-3-yl]oxy}ethyl)-3-azaspiro[5.5]undecane-3-carbonyl]phenyl}methoxy)-2-(ethylsulfanyl)-7-(6-fluoro-5-methyl-1H-indazol-4-yl)quinazolin-4-yl]azetidine-2-carboxamide (35 mg) and CH₂Cl₂ (1 mL) were added, followed by mCPBA (about 30% water content, 9 mg) with stirring in an ice bath. Then, the mixture was stirred for 30 minutes at room temperature. The reaction mixture was diluted with CH₂Cl₂, washed with saturated aqueous sodium thiosulfate and saturated aqueous sodium hydrogen carbonate, and separated into layers. The organic layer was dried over anhydrous magnesium sulfate, and the solvent was removed under reduced pressure, thus obtaining a sulfoxide intermediate. In another reaction vessel, (cyclopropane-1,1-diyl)dimethanol (15 mg) and THF (1 mL) were added under an argon atmosphere, and tBuOK (14 mg) was added in a MeOH/ice bath with stirring. Then, the mixture was stirred at room temperature for 5 minutes. A solution in THF (1 mL) and MeCN (0.5 mL) containing the sulfoxide intermediate was added portionwise while stirring the reaction mixture in the MeOH/ice bath. Then, the mixture was stirred under an argon atmosphere at room temperature for 2 hours. Ice and saturated aqueous ammonium chloride were poured into the reaction vessel, followed by extraction with CHCl₃/MeOH (5/1) twice. The collected organic layer was dried over anhydrous magnesium sulfate, and the solvent was concentrated under reduced pressure. The residue was purified by basic silica gel column chromatography (CHCl₃/MeOH), thus obtaining (2S)-1-[(7M)-6-cyclopropyl-8-({4-[9-(2-{[5-(2,4-dioxo-1,3-diazinan-1-yl)pyridin-3-yl]oxy}ethyl)-3-azaspiro[5.5]undecane-3-carbonyl]phenyl}methoxy)-7-(6-fluoro-5-methyl-1H-indazol-4-yl)-2-{[1-(hydroxymethyl)cyclopropyl]methoxy}quinazolin-4-yl]azetidine-2-carboxamide (20 mg) as a solid.

In the same manner as in the production methods of the Examples described above, the Example compounds shown in the tables presented later were produced. In addition, the production method and the physiochemical data of the compound of each Example are shown in tables presented later.

In the tables presented below, the following abbreviations are sometimes used:
PEx: Production Example No., Ex: Example No., PSyn: Production Example No. produced in the same method, Syn: Example No. produced in the same method (for example, Syn 1 represents that it was produced by the same method as for Example 1), Str: chemical structural formula (a compound with "#" in the chemical structural formula represents a diastereomeric mixture derived from axial chirality, and a compound with "##" in the chemical structural formula represents that the compound is single structure with regard to the axial chirality but has an undetermined configuration), n HCl: n hydrochloride (the compound with Production Example No. shows that the compound is monohydrochloride to trihydrochloride), DAT: physiochemical data, ESI+: m/z value in mass spectrometry (ionization method ESI, [M+H]⁺ unless otherwise specified), ESI-: m/z value in mass spectrometry (ionization method ESI, [M-H]⁻ unless otherwise specified), NMR: δ value (ppm) of peak in ¹H-NMR (500 MHz) in DMSO-d₆ at 27°C, s: singlet (spectrum), d: doublet (spectrum), dd: double doublet (spectrum), ddd: double double doublet (spectrum), t: triplet (spectrum), dt: double triplet (spectrum), q: quartet (spectrum), m: multiplet (spectrum), and br: broad (spectrum).

**[Table 3-1]**

| PEx | Str |
|---|---|
| 1 | 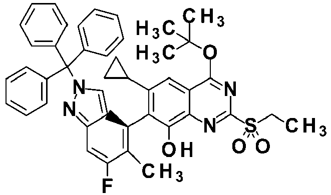 |
| 2 | 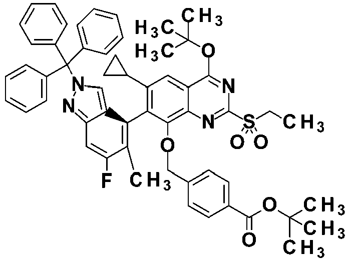 |
| 3 | 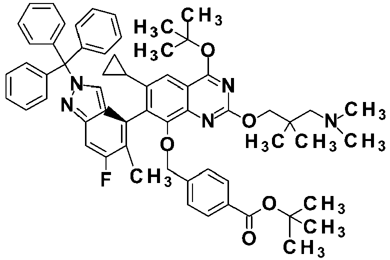 |
| 4 | 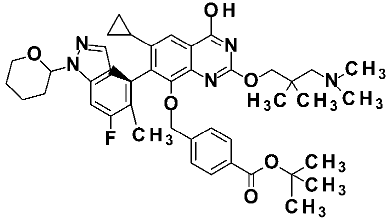 |
| 5 | 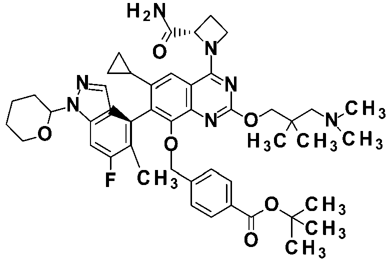 |

**[Table 3-2]**

| PEx | Str |
|---|---|
| 6 | 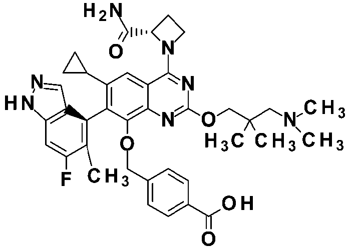 |
| 7 | 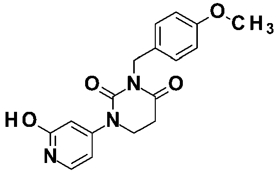 |
| 8 | 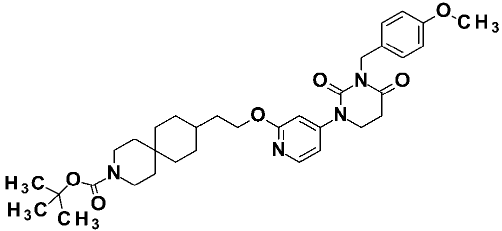 |
| 9 | 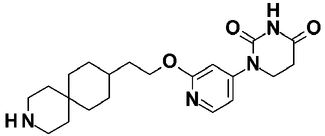 |
| 10 | 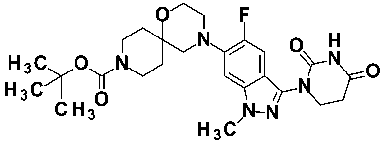 |
| 11 | 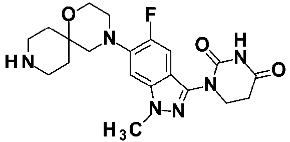 |
| 12 | 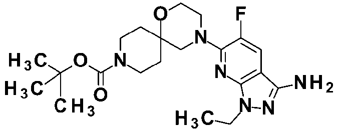 |

**[Table 3-3]**

| PEx | Str |
|---|---|
| 13 | 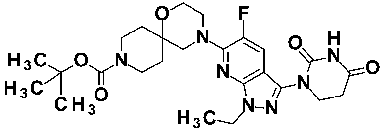 |
| 14 | 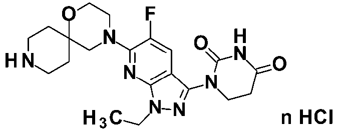 |
| 15 | 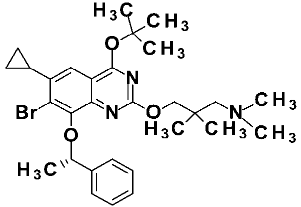 |
| 16 | 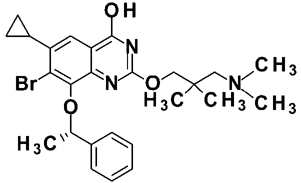 |
| 17 | 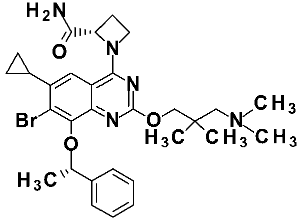 |
| 18 | 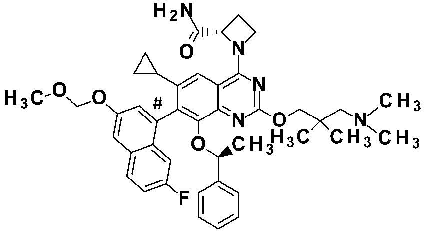 |

**[Table 3-4]**

| PEx | Str |
|---|---|
| 19 | 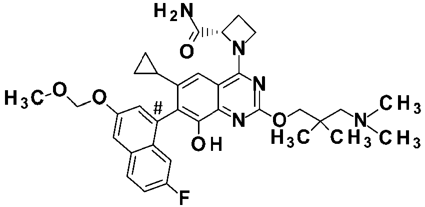 |
| 20 | 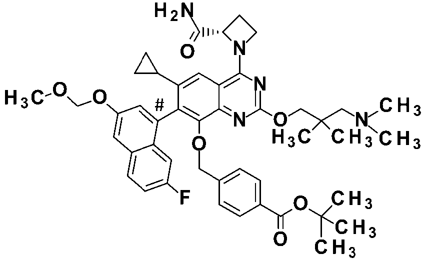 |
| 21 | 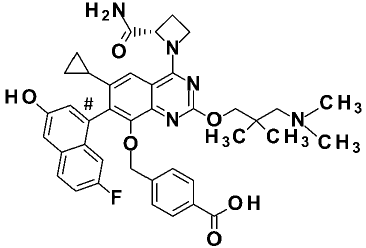 |
| 22 | 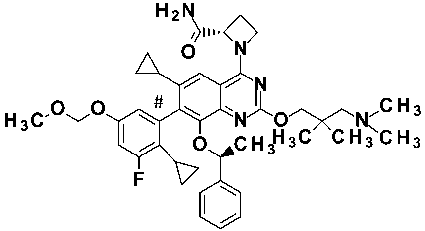 |
| 23 | 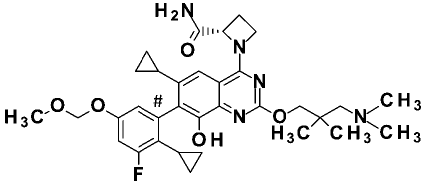 |

**[Table 3-5]**

| PEx | Str |
|---|---|
| 24 | 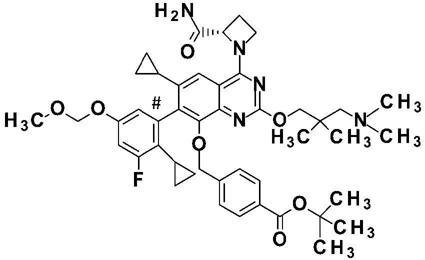 |
| 25 | 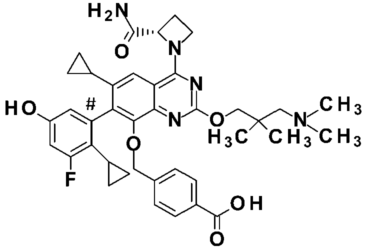 |
| 26 | 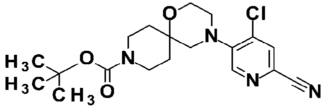 |
| 27 | 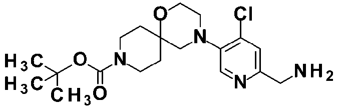 |
| 28 | 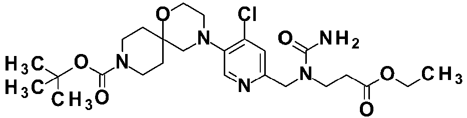 |
| 29 | 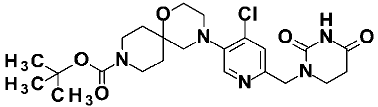 |
| 30 | 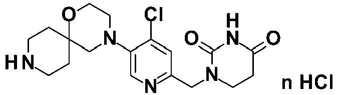 |

**[Table 3-6]**

| PEx | Str |
|---|---|
| 31 | 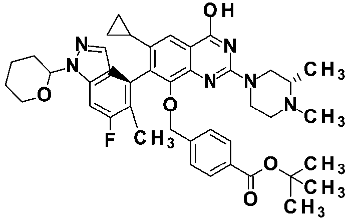 |
| 32 | 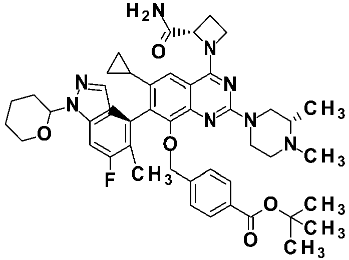 |
| 33 | 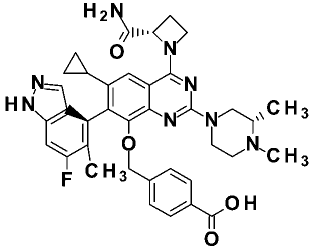 |
| 34 | 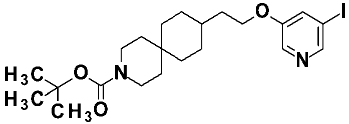 |
| 35 | 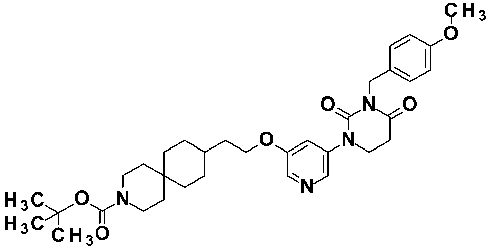 |
| 36 | 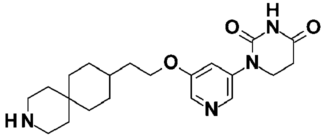 |

**[Table 3-7]**

| PEx | Str |
|---|---|
| 37 | 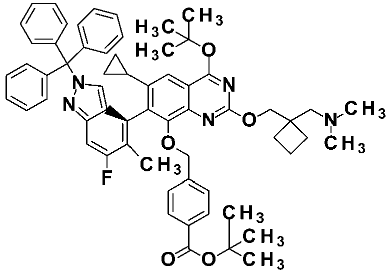 |
| 38 | 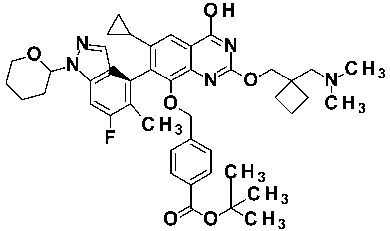 |
| 39 | 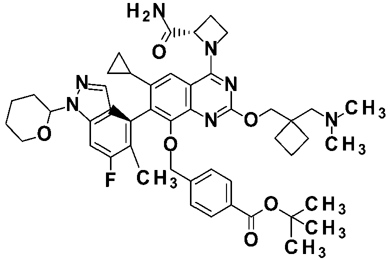 |
| 40 | 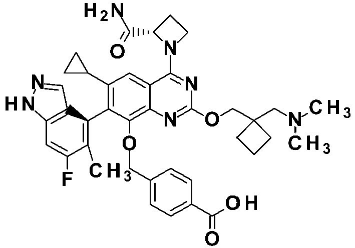 |
| 41 | 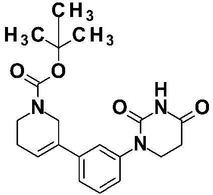 |

**[Table 3-8]**

| PEx | Str |
|---|---|
| 42 | 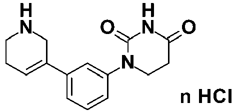 |
| 43 | 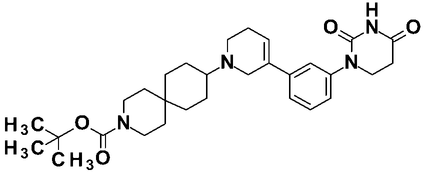 |
| 44 | 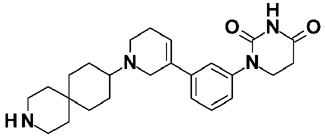 |
| 45 | 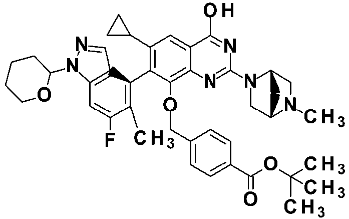 |
| 46 | 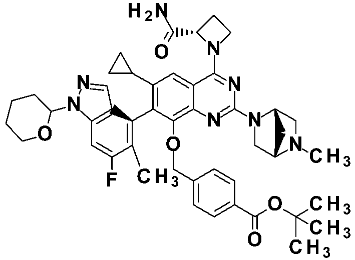 |
| 47 | 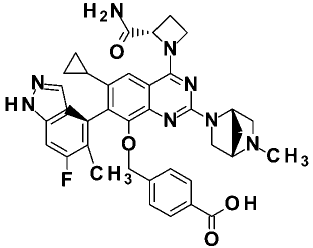 |

**[Table 3-9]**

| PEx | Str |
|---|---|
| 48 | 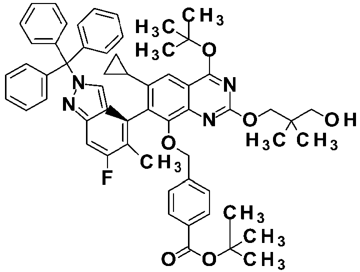 |
| 49 | 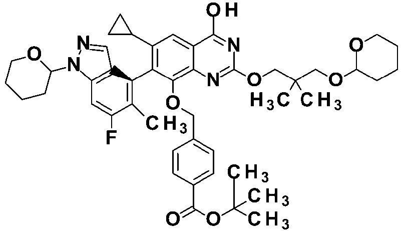 |
| 50 | 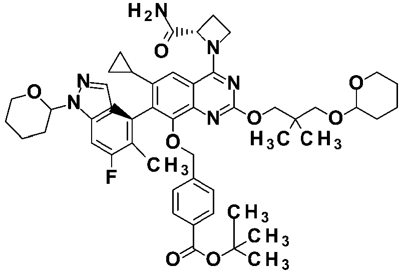 |
| 51 | 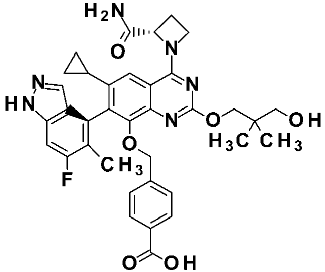 |
| 52 | 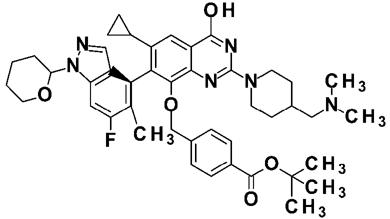 |

**[Table 3-10]**

| PEx | Str |
|---|---|
| 53 | 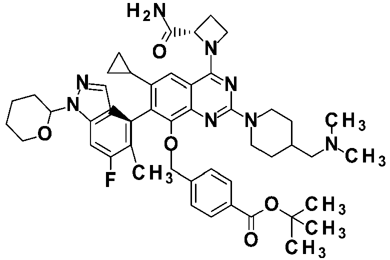 |
| 54 | 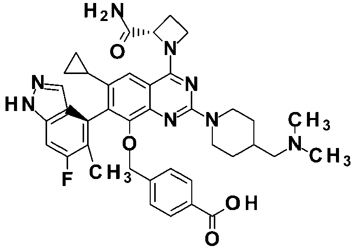 |
| 55 | 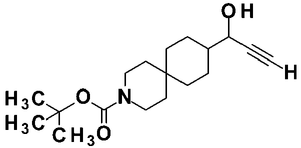 |
| 56 | 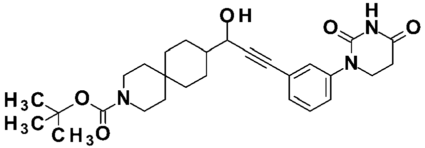 |
| 57 | 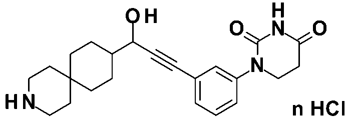 |
| 58 | 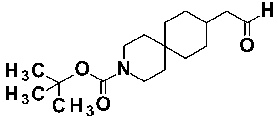 |
| 59 | 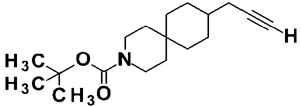 |

**[Table 3-11]**

| PEx | Str |
|---|---|
| 60 | 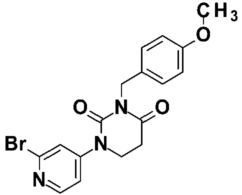 |
| 61 | 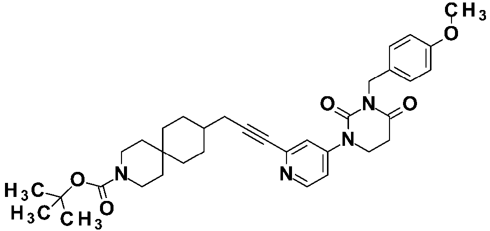 |
| 62 | 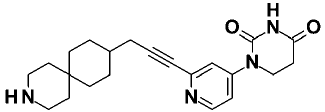 |
| 63 | 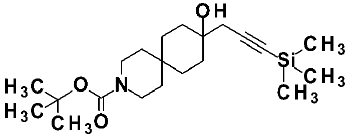 |
| 64 | 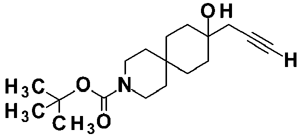 |
| 65 | 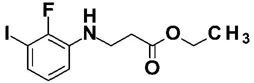 |
| 66 | 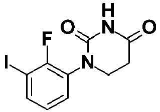 |
| 67 | 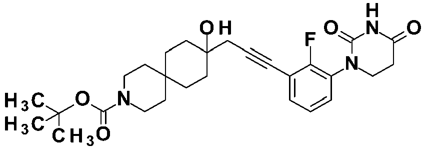 |

**[Table 3-12]**

| PEx | Str |
|---|---|
| 68 | 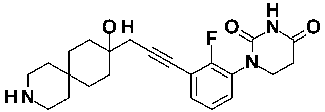 |
| 69 | 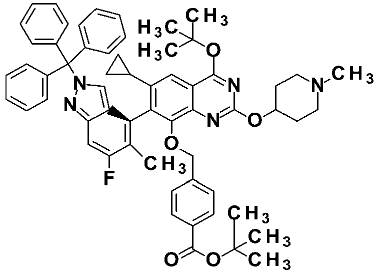 |
| 70 | 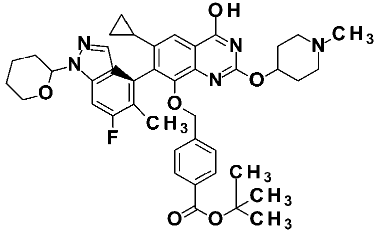 |
| 71 | 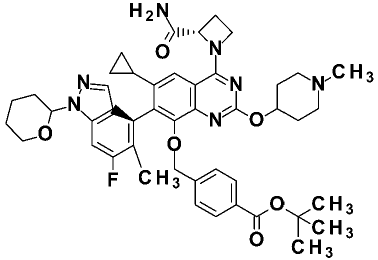 |
| 72 | 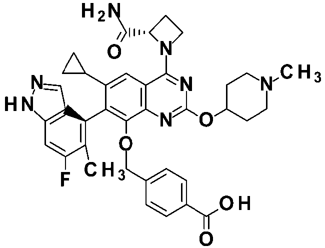 |

**[Table 3-13]**

| PEx | Str |
|---|---|
| 73 | 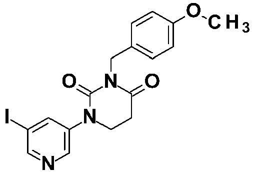 |
| 74 | 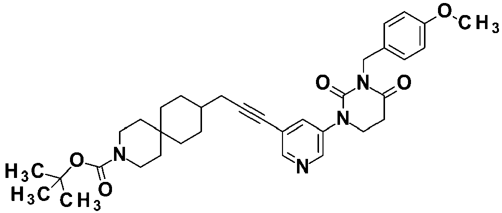 |
| 75 | 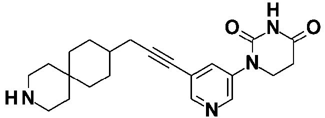 |
| 76 | 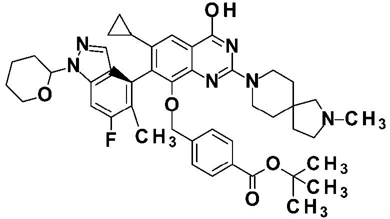 |
| 77 | 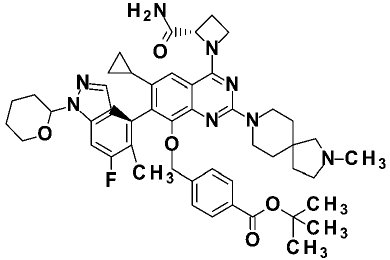 |
| 78 | 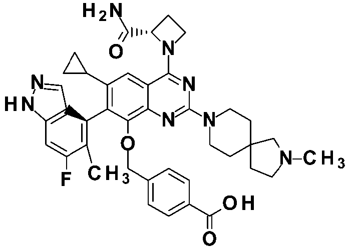 |

**[Table 3-14]**

| PEx | Str |
|---|---|
| 79 | 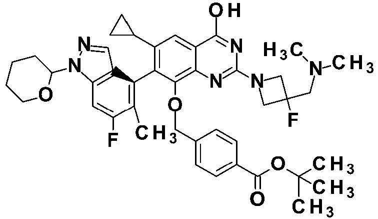 |
| 80 | 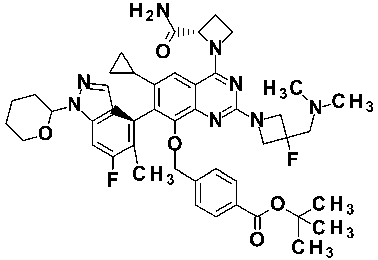 |
| 81 | 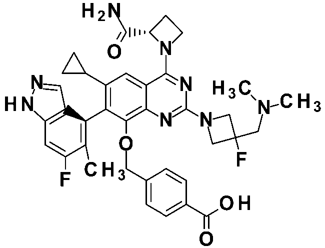 |
| 82 | 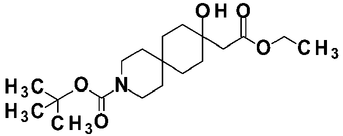 |
| 83 | 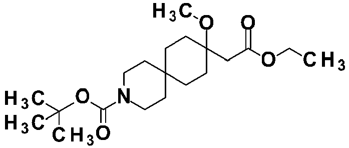 |
| 84 | 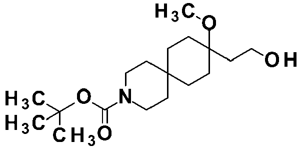 |

**[Table 3-15]**

| PEx | Str |
|---|---|
| 85 | 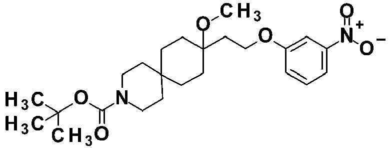 |
| 86 | 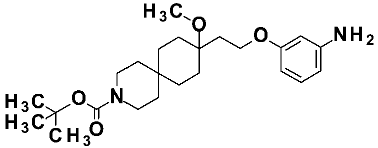 |
| 87 | 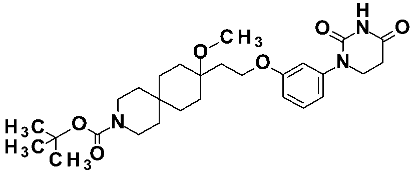 |
| 88 | 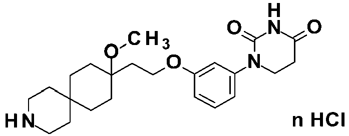 |
| 89 | 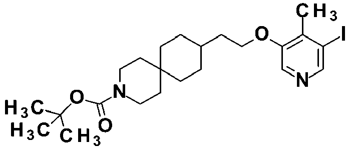 |
| 90 | 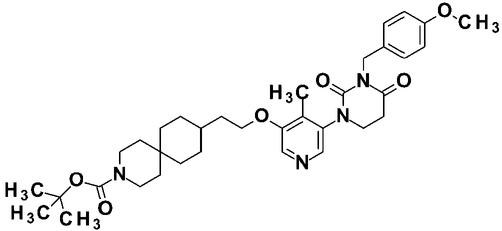 |
| 91 | 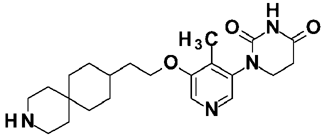 |

**[Table 3-16]**

| PEx | Str |
|---|---|
| 92 | 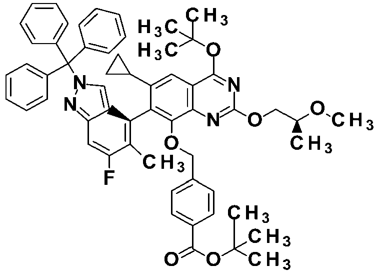 |
| 93 | 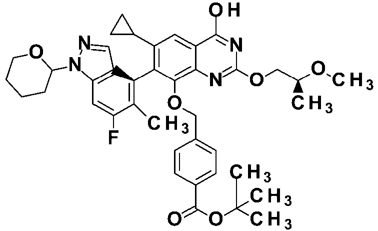 |
| 94 | 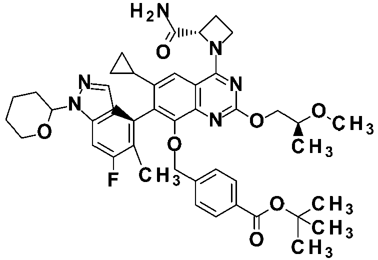 |
| 95 | 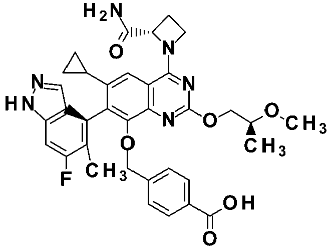 |
| 96 | 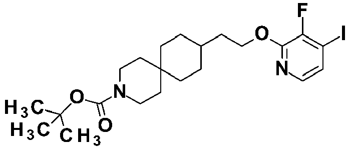 |

**[Table 3-17]**

| PEx | Str |
|---|---|
| 97 | 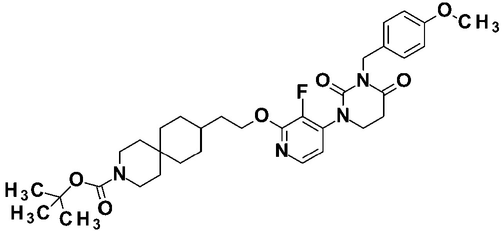 |
| 98 | 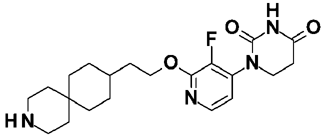 |
| 99 | 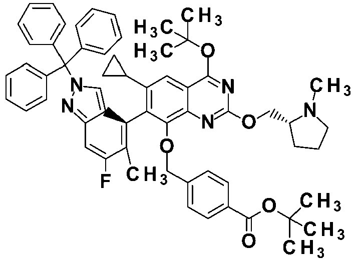 |
| 100 | 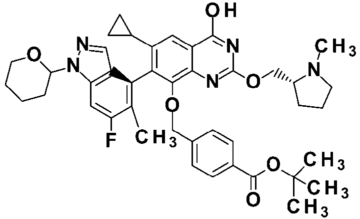 |
| 101 | 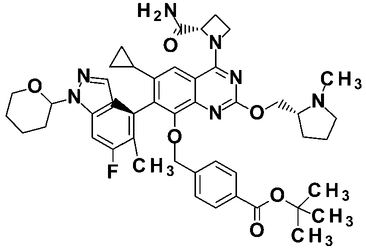 |

**[Table 3-18]**

| PEx | Str |
|---|---|
| 102 | 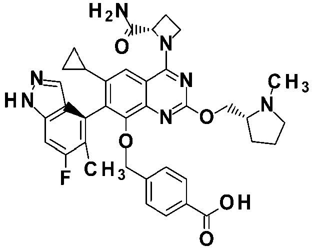 |
| 103 | 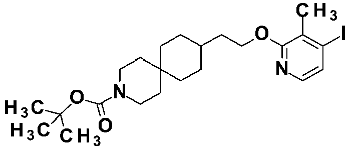 |
| 104 | 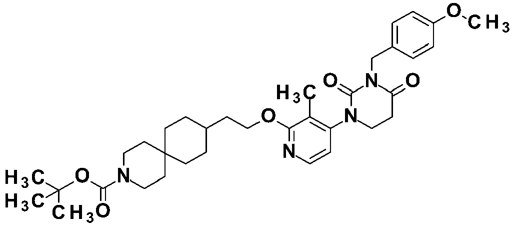 |
| 105 | 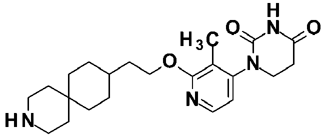 |
| 106 | 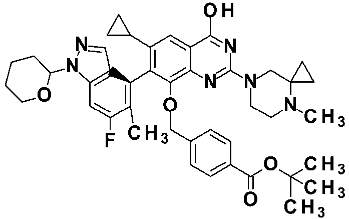 |
| 107 | 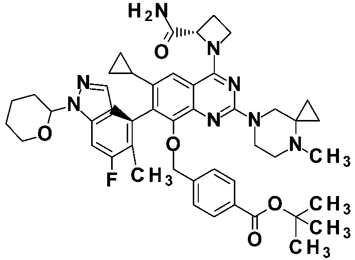 |

**[Table 3-19]**

| PEx | Str |
|---|---|
| 108 | 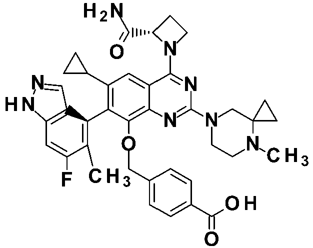 |
| 109 | 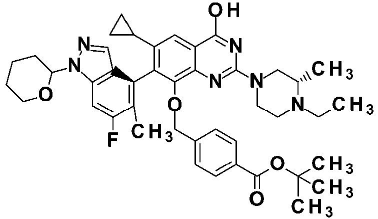 |
| 110 | 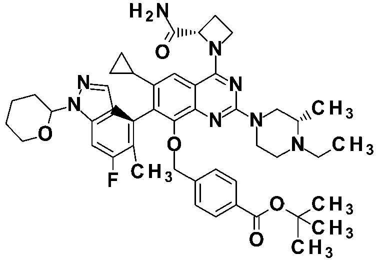 |
| 111 | 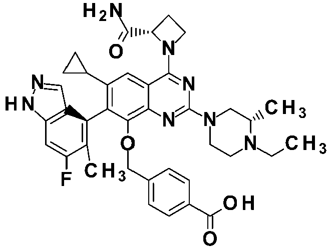 |
| 112 | 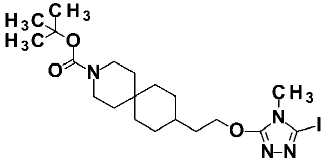 |

**[Table 3-20]**

| PEx | Str |
|---|---|
| 113 | 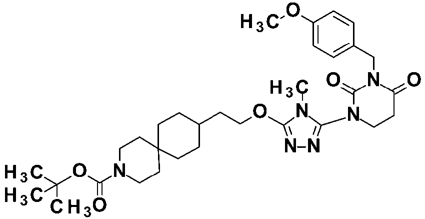 |
| 114 | 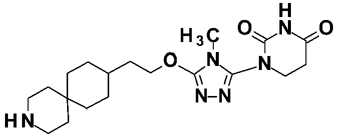 |
| 115 | 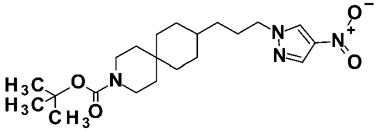 |
| 116 | 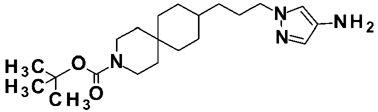 |
| 117 | 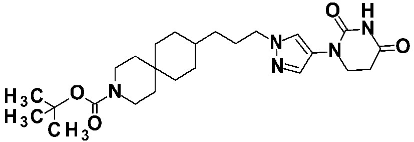 |
| 118 | 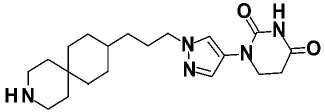 |
| 119 | 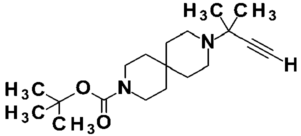 |
| 120 | 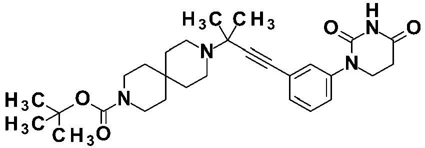 |

**[Table 3-21]**

| PEx | Str |
|---|---|
| 121 | 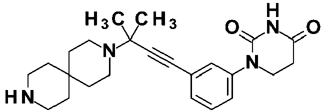 |
| 122 | 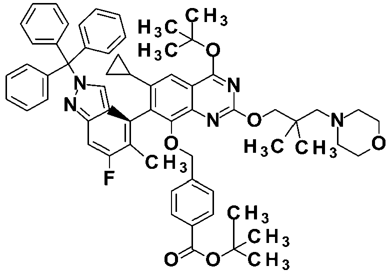 |
| 123 | 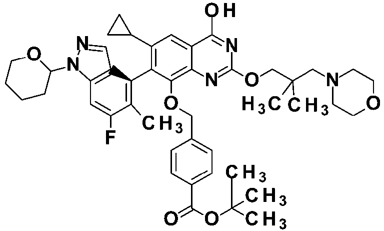 |
| 124 | 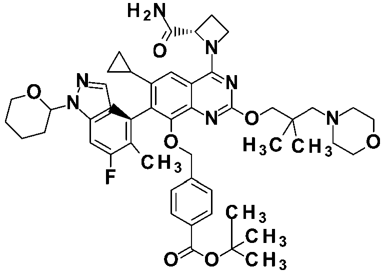 |
| 125 | 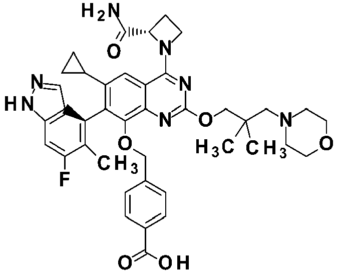 |

**[Table 3-22]**

| PEx | Str |
|---|---|
| 126 | 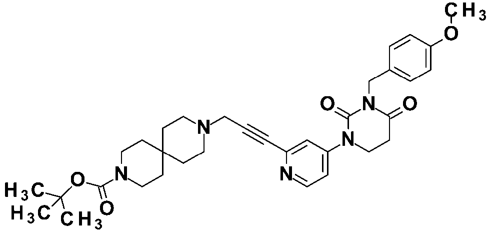 |
| 127 | 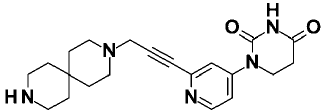 |
| 128 | 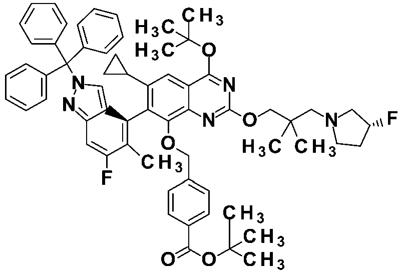 |
| 129 | 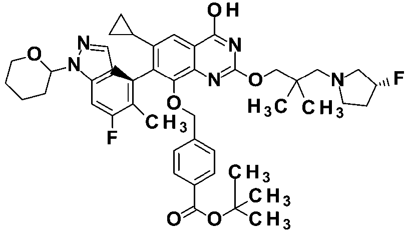 |
| 130 | 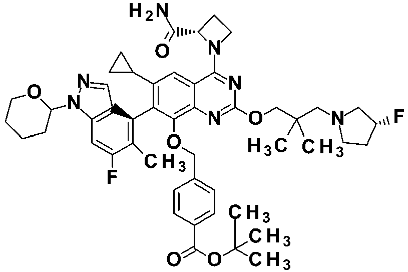 |

**[Table 3-23]**

| PEx | Str |
|---|---|
| 131 | 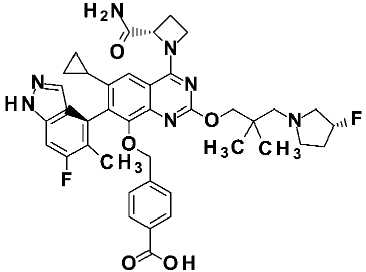 |
| 132 | 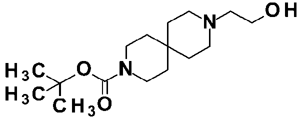 |
| 133 | 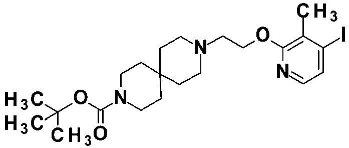 |
| 134 | 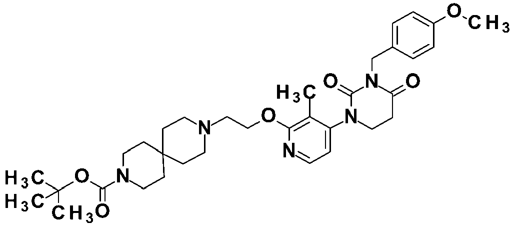 |
| 135 | 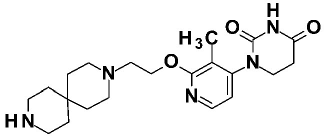 |
| 136 | 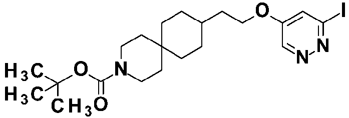 |
| 137 | 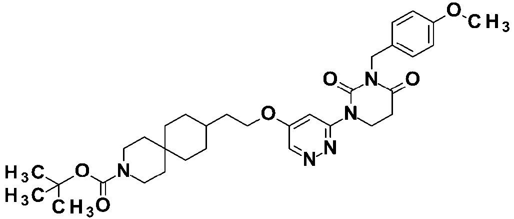 |

**[Table 3-24]**

| PEx | Str |
|---|---|
| 138 | 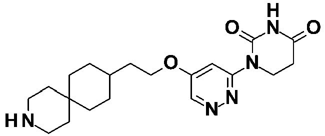 |
| 139 | 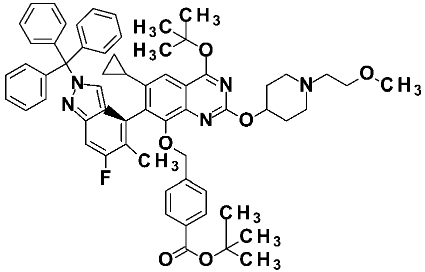 |
| 140 | 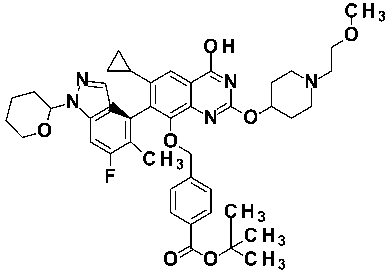 |
| 141 | 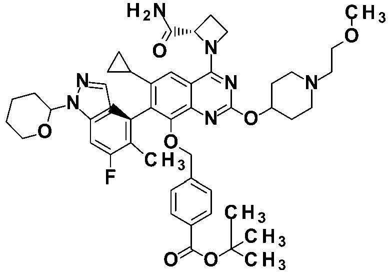 |
| 142 | 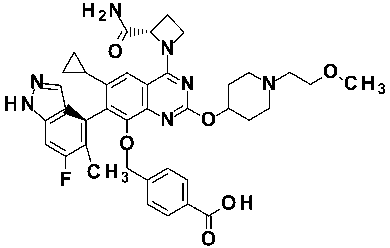 |

**[Table 3-25]**

| PEx | Str |
|---|---|
| 143 | 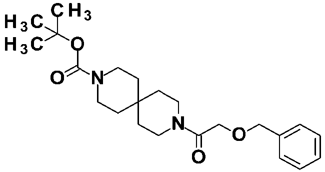 |
| 144 | 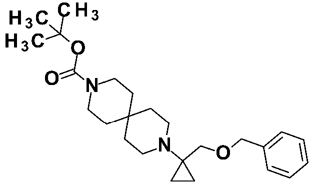 |
| 145 | 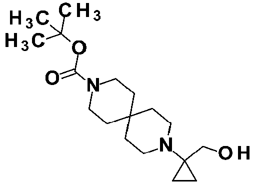 |
| 146 | 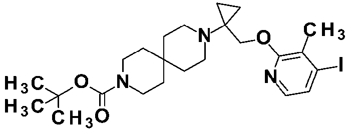 |
| 147 | 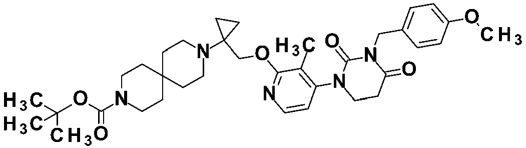 |
| 148 | 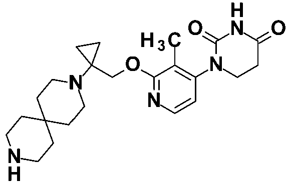 |
| 149 | 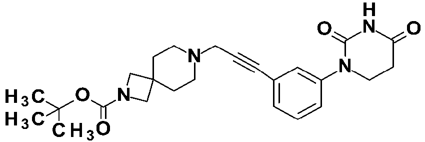 |

**[Table 3-26]**

| PEx | Str |
|---|---|
| 150 | 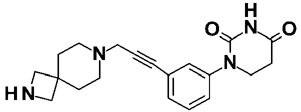 |
| 151 | 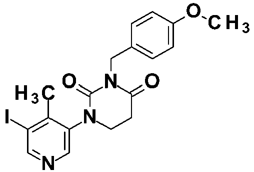 |
| 152 | 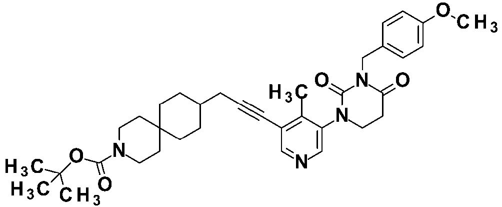 |
| 153 | 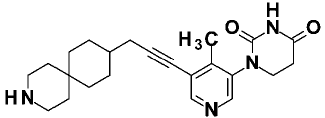 |

**[Table 3-27]**

| PEx | Str |
|---|---|
| 154 | 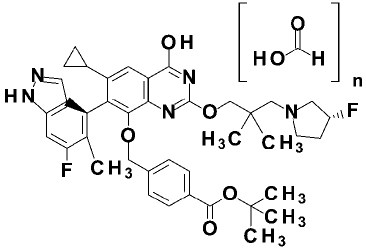 |
| 155 | 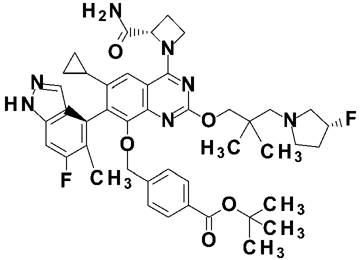 |
| 156 | 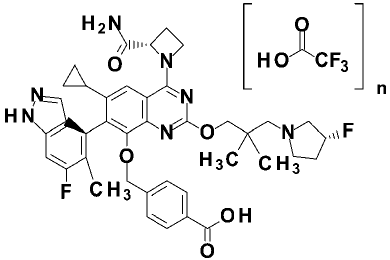 |
| 157 | 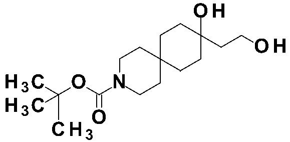 |
| 158 | 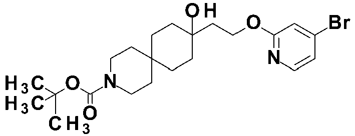 |

**[Table 3-28]**

| PEx | Str |
|---|---|
| 159 | 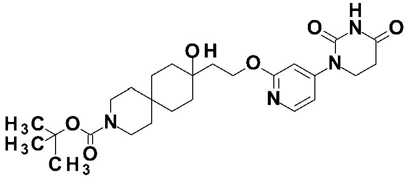 |
| 160 | 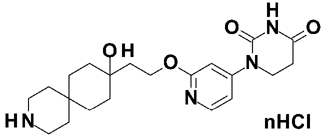 |
| 161 | 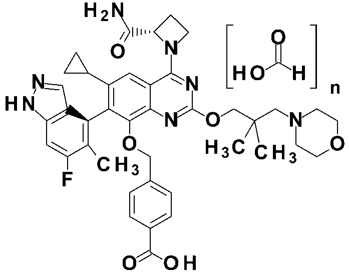 |
| 162 | 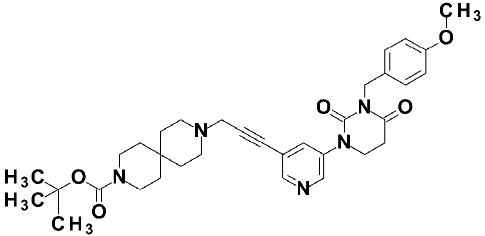 |
| 163 | 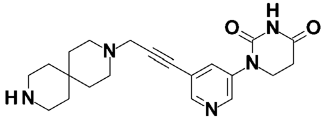 |

**[Table 3-29]**

| PEx | Str |
|---|---|
| 164 | 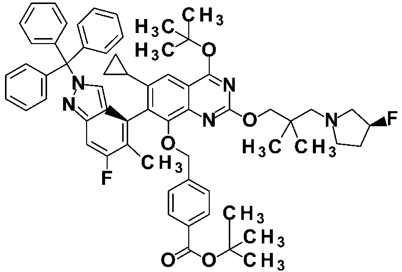 |
| 165 | 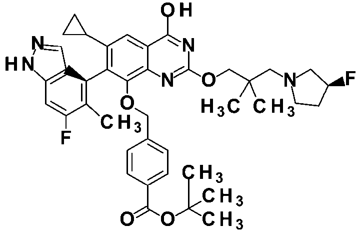 |
| 166 | 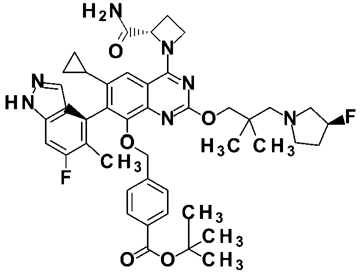 |
| 167 | 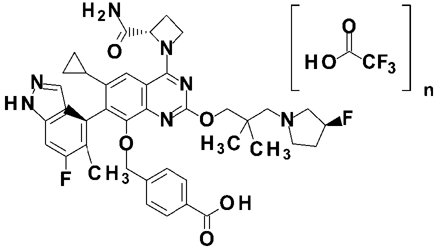 |

**[Table 3-30]**

| PEx | Str |
|---|---|
| 168 | 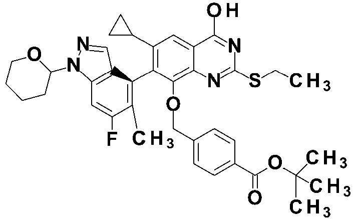 |
| 169 | 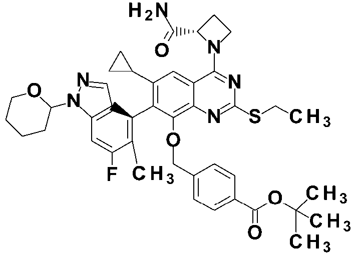 |
| 170 | 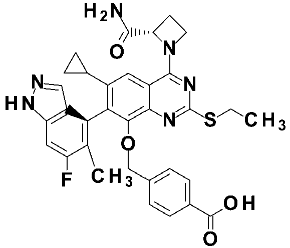 |
| 171 | 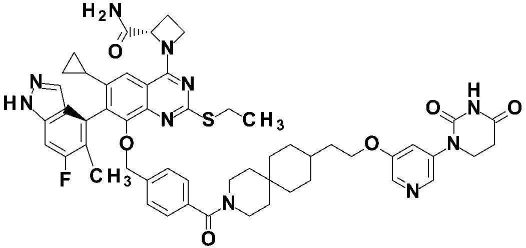 |
| 172 | 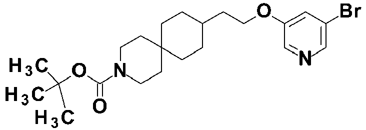 |

**[Table 3-31]**

| PEx | Str |
|---|---|
| 173 | 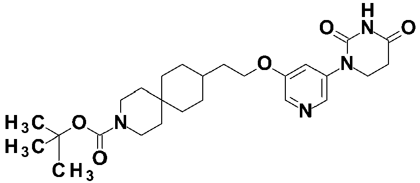 |
| 174 | 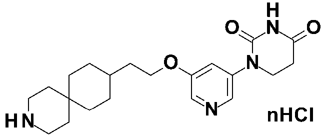 |
| 175 | 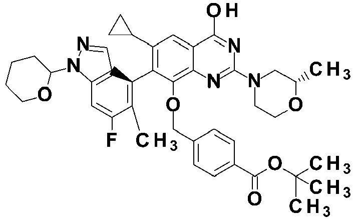 |
| 176 | 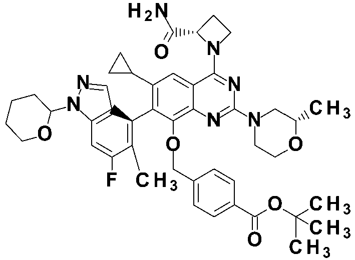 |
| 177 | 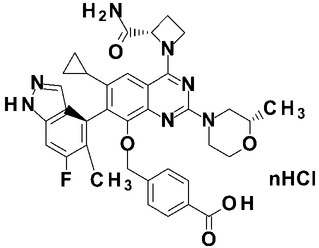 |

**[Table 3-32]**

| PEx | Str |
|---|---|
| 178 | 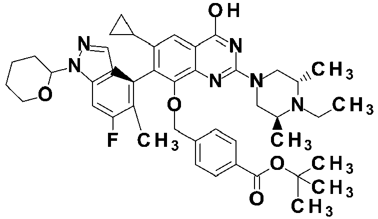 |
| 179 | 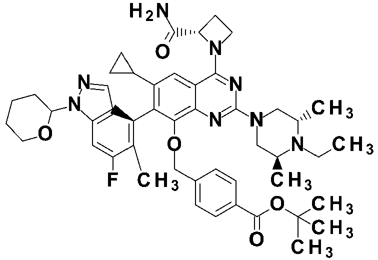 |
| 180 | 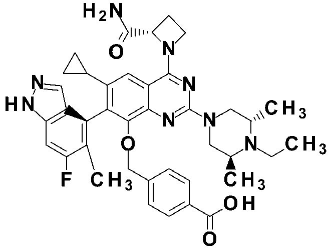 |
| 181 | 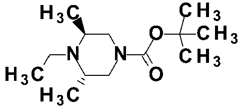 |
| 182 | 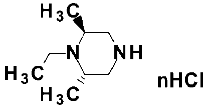 |
| 183 | 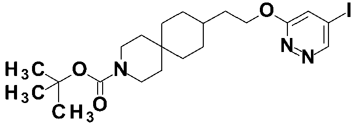 |

**[Table 3-33]**

| PEx | Str |
|---|---|
| 184 | 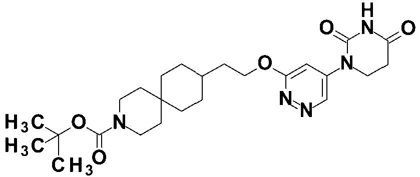 |
| 185 | 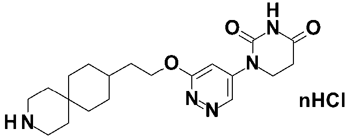 |

**[Table 4-1]**

| PEx | PSyn | DAT |
|---|---|---|
| 1 | 1 | ESI+: 763.5 [M+Na]+ |
| 2 | 2 | ESI+: 953.6 [M+Na]+ |
| 3 | 3 | ESI+: 968.7 |
| 4 | 4 | ESI+: 754.6 |
| 5 | 5 | ESI+: 836.7 |
| 6 | 6 | ESI+: 696.6 |
| 7 | 7 | ESI+: 328.2 |
| 8 | 8 | ESI+: 607.6 |
| 9 | 9 | ESI+: 387.4 |
| 10 | 10 | ESI+: 517.4 |
| 11 | 11 | ESI+: 417.3 |
| 12 | 12 | ESI+: 435.4 |
| 13 | 13 | ESI+: 532.5 |
| 14 | 14 | ESI+: 432.4 |
| 15 | 15 | ESI+: 572.4 |
| 16 | 16 | ESI+: 516.4 |
| 17 | 5 | ESI+: 598.4 |
| 18 | 18 | ESI+: 722.6 |
| 19 | 19 | ESI+: 618.6 |
| 20 | 2 | ESI+: 808.6 |
| 21 | 21 | ESI+: 708.6 |
| 22 | 18 | ESI+: 712.7 |
| 23 | 19 | ESI+: 608.5 |
| 24 | 2 | ESI+: 798.7 |
| 25 | 21 | ESI+: 698.6 |
| 26 | 26 | ESI+: 415.4 [M+Na]+ |
| 27 | 27 | ESI+: 397.4 |
| 28 | 28 | ESI+: 540.5 |
| 29 | 29 | ESI+: 494.4 |
| 30 | 30 | ESI+: 394.3 |
| 31 | 31 | ESI+: 737.6 |
| 32 | 32 | ESI+: 819.7 |
| 33 | 33 | ESI+: 679.6 |
| 34 | 34 | ESI+: 501.3 |
| 35 | 35 | ESI+: 607.5 |
| 36 | 36 | ESI+: 387.4 |
| 37 | 3 | ESI+: 980.8 |
| 38 | 4 | ESI+: 766.7 |
| 39 | 5 | ESI+: 848.7 |
| 40 | 6 | ESI+: 708.5 |
| 41 | 41 | ESI+: 394.2 [M+Na]+ |

**[Table 4-2]**

| PEx | PSyn | DAT |
|---|---|---|
| 42 | 30 | ESI+: 272.2 |
| 43 | 43 | ESI+: 523.5 |
| 44 | 11 | ESI+: 423.4 |
| 45 | 31 | ESI+: 735.6 |
| 46 | 32 | ESI+: 817.7 |
| 47 | 33 | ESI+: 677.5 |
| 48 | 3 | ESI+: 941.5 |
| 49 | 4 | ESI+: 833.6 [M+Na]+ |
| 50 | 5 | ESI+: 893.7 |
| 51 | 6 | ESI+: 669.5 |
| 52 | 31 | ESI+: 765.7 |
| 53 | 32 | ESI+: 847.8 |
| 54 | 33 | ESI+: 707.6 |
| 55 | 55 | ESI+: 330.3 [M+Na]+ |
| 56 | 56 | ESI+: 496.5 |
| 57 | 30 | ESI+: 396.4 |
| 58 | 58 | ESI+: 318.3 [M+Na]+ |
| 59 | 59 | ESI+: 314.3 [M+Na]+ |
| 60 | 60 | ESI+: 392.1 |
| 61 | 61 | ESI+: 601.5 |
| 62 | 62 | ESI+: 381.4 |
| 63 | 63 | ESI+: 402.4 [M+Na]+ |
| 64 | 64 | ESI+: 330.3 [M+Na]+ |
| 65 | 65 | ESI+: 338.1 |
| 66 | 66 | ESI+: 335.1 |
| 67 | 56 | ESI+: 514.4 |
| 68 | 11 | ESI+: 414.4 |
| 69 | 3 | ESI+: 952.7 |
| 70 | 4 | ESI+: 738.6 |
| 71 | 5 | ESI+: 820.7 |
| 72 | 6 | ESI+: 680.6 |
| 73 | 35 | ESI+: 438.2 |
| 74 | 56 | ESI+: 601.5 |
| 75 | 62 | ESI+: 381.3 |
| 76 | 31 | ESI+: 777.8 |
| 77 | 32 | ESI+: 859.7 |
| 78 | 33 | ESI+: 719.6 |
| 79 | 31 | ESI+: 755.7 |
| 80 | 32 | ESI+: 837.7 |
| 81 | 33 | ESI+: 697.5 |
| 82 | 82 | ESI+: 378.4 [M+Na]+ |
| 83 | 83 | ESI+: 392.4 [M+Na]+ |

**[Table 4-3]**

| PEx | PSyn | DAT |
|---|---|---|
| 84 | 84 | ESI+: 350.4 [M+Na]+ |
| 85 | 85 | ESI+: 471.4 [M+Na]+ |
| 86 | 86 | ESI+: 419.5 |
| 87 | 13 | ESI+: 538.5 [M+Na]+ |
| 88 | 14 | ESI+: 416.4 |
| 89 | 89 | ESI+: 515.4 |
| 90 | 90 | ESI+: 621.5 |
| 91 | 91 | ESI+: 401.4 |
| 92 | 3 | ESI+: 927.6 |
| 93 | 4 | ESI+: 713.5 |
| 94 | 5 | ESI+: 795.6 |
| 95 | 6 | ESI+: 655.4 |
| 96 | 8 | ESI+: 541.3 [M+Na]+ |
| 97 | 97 | ESI+: 647.5 [M+Na]+ |
| 98 | 9 | ESI+: 405.4 |
| 99 | 3 | ESI+: 952.7 |
| 100 | 4 | ESI+: 738.6 |
| 101 | 5 | ESI+: 820.7 |
| 102 | 21 | ESI+: 680.6 |
| 103 | 85 | ESI+: 537.3 [M+Na]+ |
| 104 | 97 | ESI+: 621.5 |
| 105 | 9 | ESI+: 401.4 |
| 106 | 31 | ESI+: 749.6 |
| 107 | 32 | ESI+: 831.6 |
| 108 | 33 | ESI+: 691.5 |
| 109 | 109 | ESI+: 751.6 |
| 110 | 110 | ESI+: 833.7 |
| 111 | 111 | ESI+: 693.6 |
| 112 | 112 | ESI+: 505.3 |
| 113 | 35 | ESI+: 611.5 |
| 114 | 9 | ESI+: 391.4 |
| 115 | 8 | ESI+: 429.4 [M+Na]+ |
| 116 | 86 | ESI+: 377.4 |
| 117 | 13 | ESI+: 496.5 [M+Na]+ |
| 118 | 11 | ESI+: 374.4 |
| 119 | 119 | ESI+: 321.4 |
| 120 | 56 | ESI+: 509.5 |
| 121 | 11 | ESI+: 409.5 |
| 122 | 3 | ESI+: 1032.8 [M+Na]+ |
| 123 | 4 | ESI+: 796.6 |
| 124 | 5 | ESI+: 878.7 |
| 125 | 6 | ESI+: 738.3 |

**[Table 4-4]**

| PEx | PSyn | DAT |
|---|---|---|
| 126 | 61 | ESI+: 602.5 |
| 127 | 9 | ESI+: 382.3 |
| 128 | 3 | ESI+: 1012.7 |
| 129 | 4 | ESI+: 798.6 |
| 130 | 5 | ESI+: 880.7 |
| 131 | 6 | ESI+: 740.5 |
| 132 | 132 | ESI+: 299.4 |
| 133 | 85 | ESI+: 516.3 |
| 134 | 97 | ESI+: 622.5 |
| 135 | 9 | ESI+: 402.4 |
| 136 | 136 | ESI+: 502.3 |
| 137 | 97 | ESI+: 608.5 |
| 138 | 9 | ESI+: 388.4 |
| 139 | 3 | ESI+: 996.6 |
| 140 | 4 | ESI+: 782.6 |
| 141 | 5 | ESI+: 864.7 |
| 142 | 6 | ESI+: 724.5 |
| 143 | 143 | ESI+: 403.4 |
| 144 | 144 | ESI+: 415.4 |
| 145 | 145 | ESI+: 325.3 |
| 146 | 136 | ESI+: 542.4 |
| 147 | 97 | ESI+: 648.6 |
| 148 | 9 | ESI+: 428.5 |
| 149 | 56 | ESI+: 453.4 |
| 150 | 11 | ESI+: 353.3 |
| 151 | 90 | ESI+: 452.2 |
| 152 | 56 | ESI+: 615.5 |
| 153 | 9 | ESI+: 395.4 |

**[Table 4-5]**

| PEx | PSyn | DAT |
|---|---|---|
| 154 | 154 | ESI+: 714.6 |
| 155 | 5 | ESI+: 796.7 |
| 156 | 156 | ESI+: 740.2 |
| 157 | 84 | ESI+: 336.3 [M+Na]+ |
| 158 | 85 | ESI+: 471.3 |
| 159 | 159 | ESI+: 503.5 |
| 160 | 14 | ESI+: 403.4 |
| 161 | 161 | ESI+: 738.6 |
| 162 | 56 | ESI+: 602.6 |
| 163 | 9 | ESI+: 382.4 |
| 164 | 3 | ESI+: 1012.8 |
| 165 | 165 | ESI+: 714.5 |
| 166 | 5 | ESI+: 796.3 |
| 167 | 156 | ESI+: 740.1 |
| 168 | 168 | ESI+: 685.4 |
| 169 | 5 | ESI+: 767.5 |
| 170 | 21 | ESI+: 627.4 |
| 171 | Ex1 | ESI+: 995.5 |
| 172 | 85 | ESI+: 455.2 |
| 173 | 159 | ESI+: 487.4 |
| 174 | 30 | ESI+: 387.3 |
| 175 | 31 | ESI+: 724.7 |
| 176 | 5 | ESI+: 806.7 |
| 177 | 177 | ESI+: 666.6 |
| 178 | 31 | ESI+: 765.5 |
| 179 | 5 | ESI+: 847.5 |
| 180 | 21 | ESI+: 707.5 |
| 181 | 181 | ESI+: 243.3 |
| 182 | 182 | ESI+: 143.1 |
| 183 | 136 | ESI+: 502.2 |
| 184 | 159 | ESI+: 488.4 |
| 185 | 30 | ESI+: 388.3 |

**[Table 5-1]**

| Ex | Str |
|---|---|
| 1 | 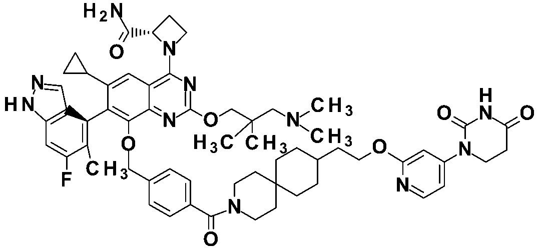 |
| 2 | 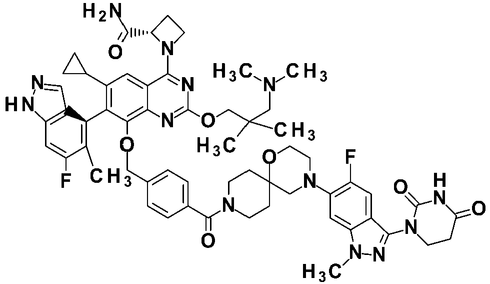 |
| 3 | 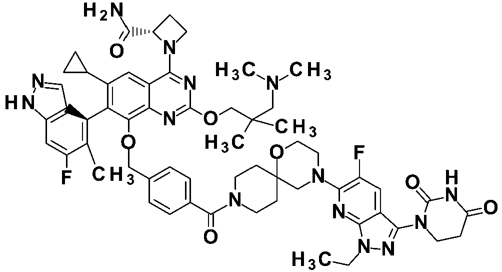 |
| 4 | 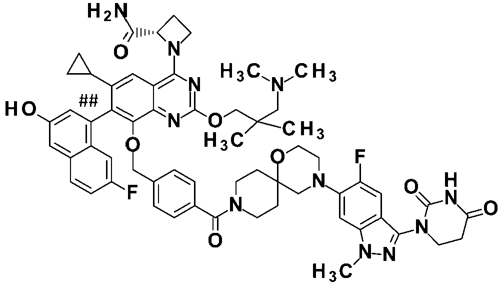 |

**[Table 5-2]**

| Ex | Str |
|---|---|
| 5 | 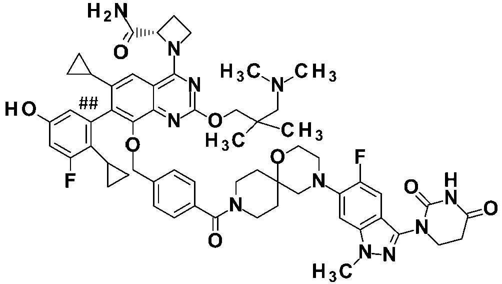 |
| 6 | 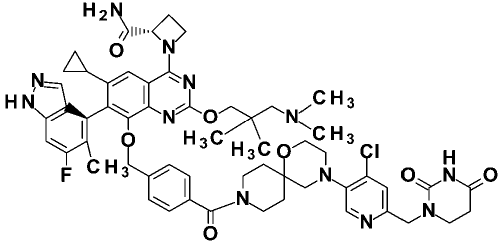 |
| 7 | 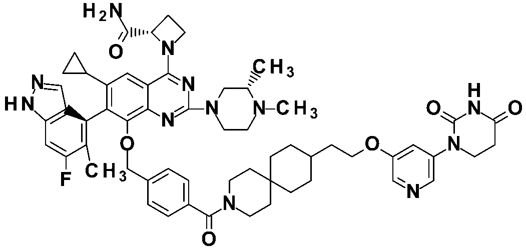 |
| 8 | 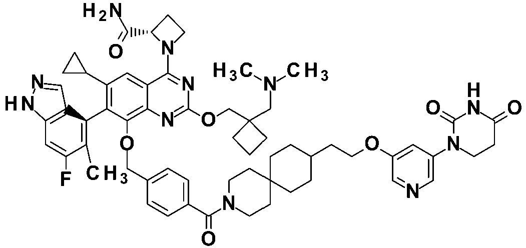 |
| 9 | 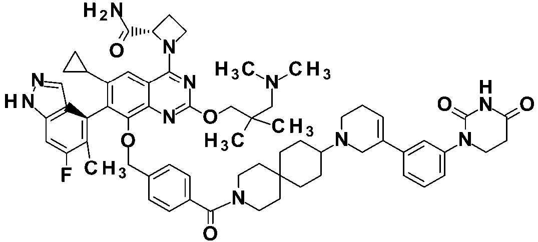 |

**[Table 5-3]**

| Ex | Str |
|---|---|
| 10 | 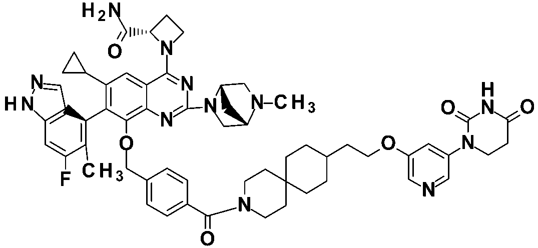 |
| 11 | 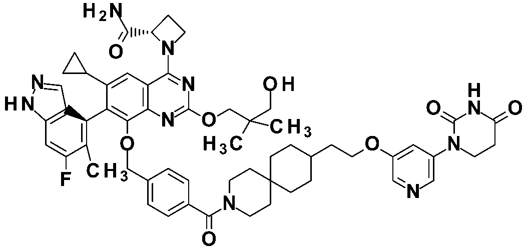 |
| 12 | 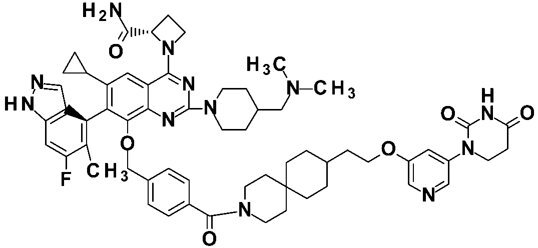 |
| 13 | 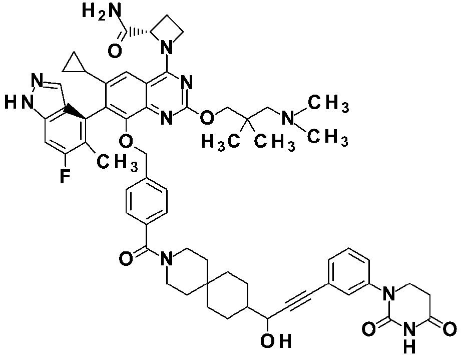 |

**[Table 5-4]**

| Ex | Str |
|---|---|
| 14 | 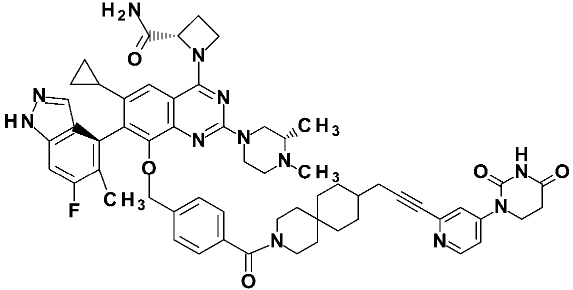 |
| 15 | 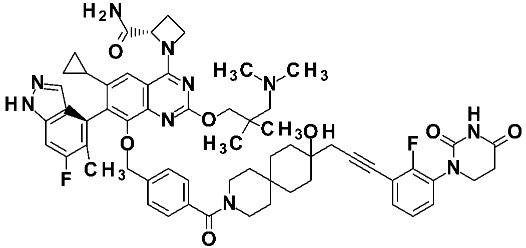 |
| 16 | 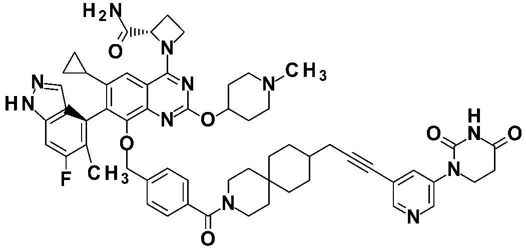 |
| 17 | 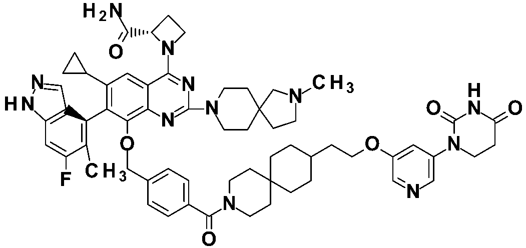 |

**[Table 5-5]**

| Ex | Str |
|---|---|
| 18 | 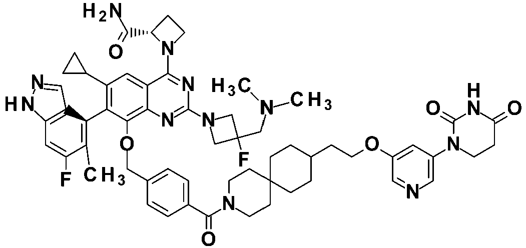 |
| 19 | 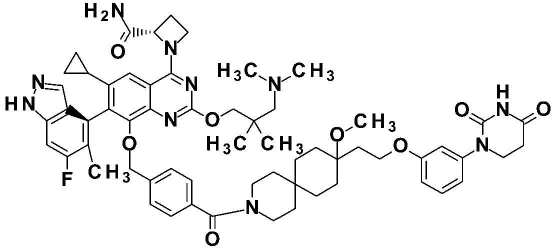 |
| 20 | 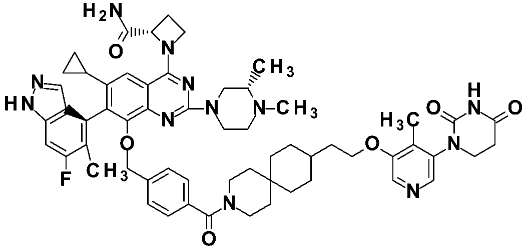 |
| 21 | 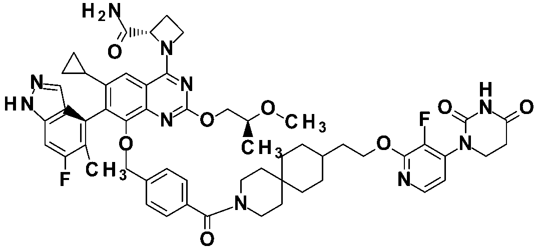 |
| 22 | 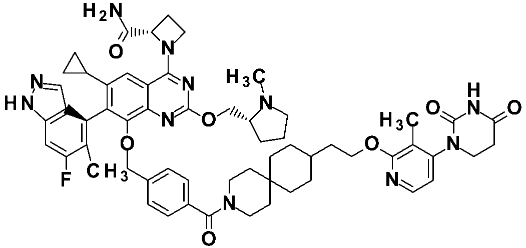 |

**[Table 5-6]**

| Ex | Str |
|---|---|
| 23 | 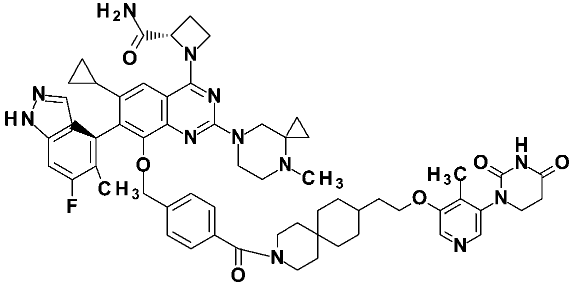 |
| 24 | 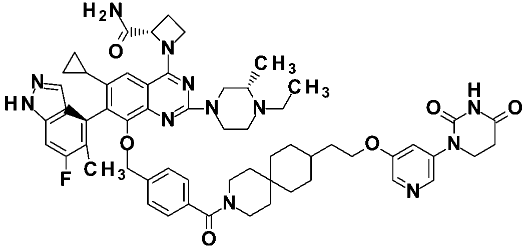 |
| 25 | 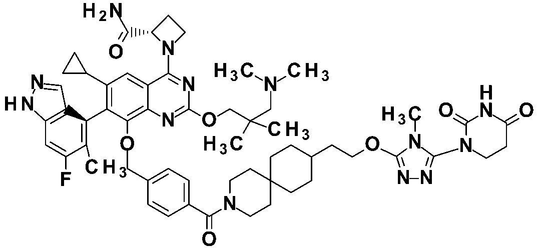 |
| 26 | 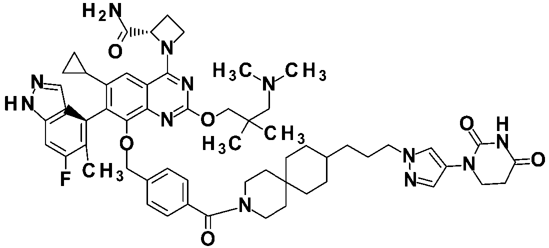 |

**[Table 5-7]**

| Ex | Str |
|---|---|
| 27 | 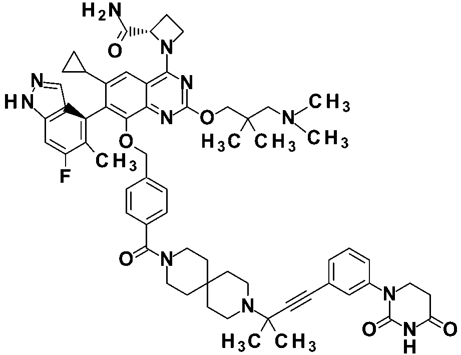 |
| 28 | 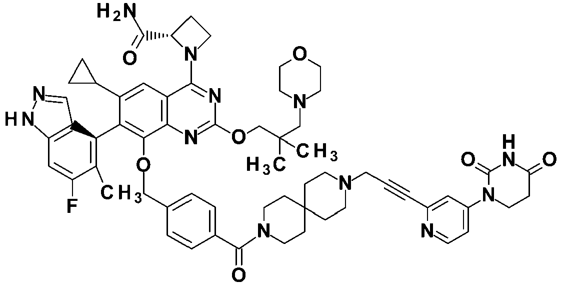 |
| 29 | 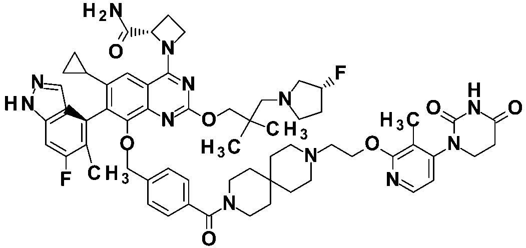 |
| 30 | 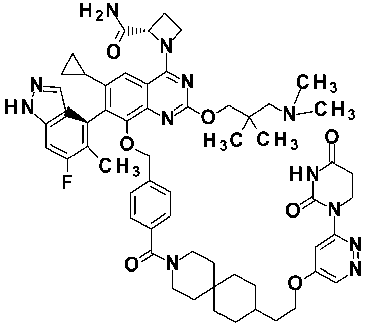 |

**[Table 5-8]**

| Ex | Str |
|---|---|
| 31 | 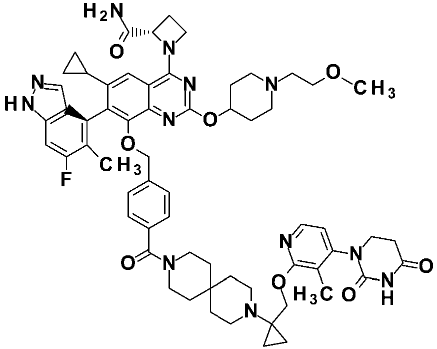 |
| 32 | 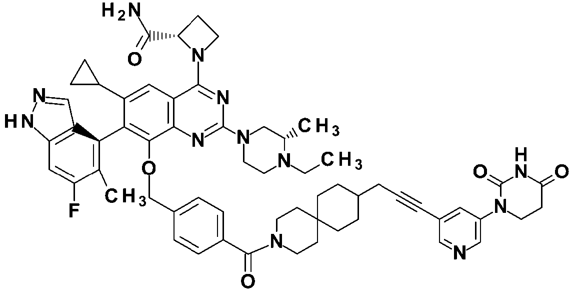 |
| 33 | 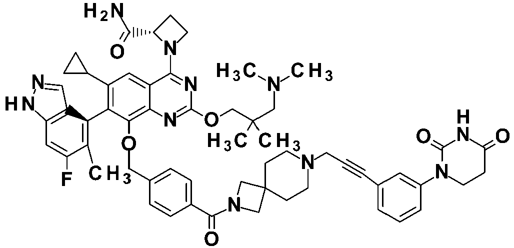 |
| 34 | 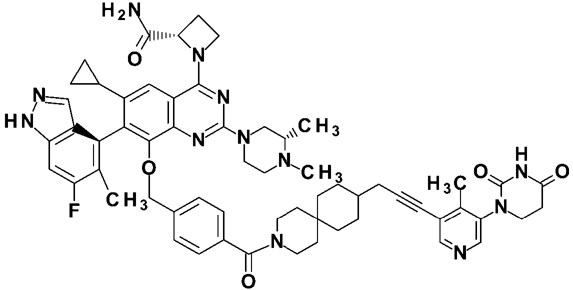 |

**[Table 5-9]**

| Ex | Str |
|---|---|
| 35 | 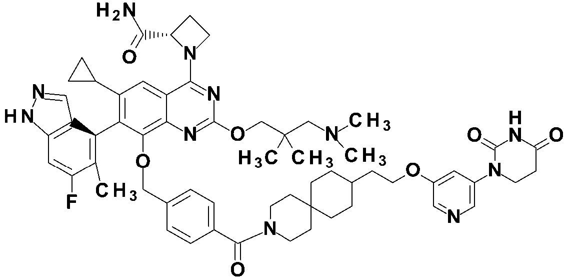 |
| 36 | 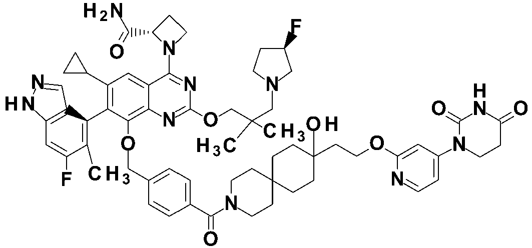 |
| 37 | 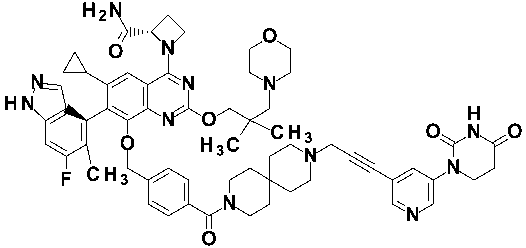 |
| 38 | 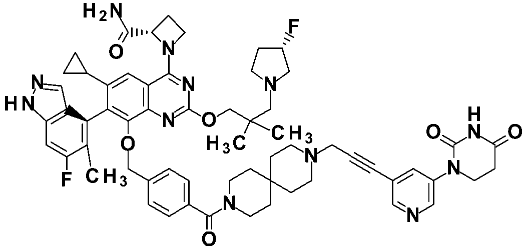 |

**[Table 5-10]**

| Ex | Str |
|---|---|
| 39 | 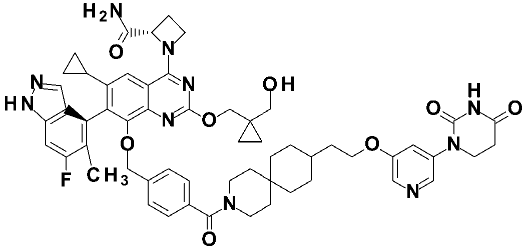 |
| 40 | 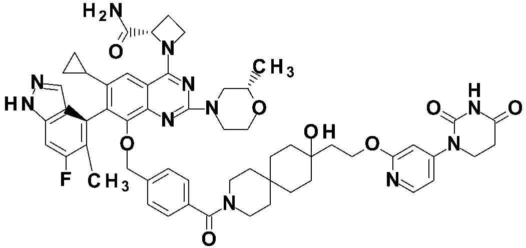 |
| 41 | 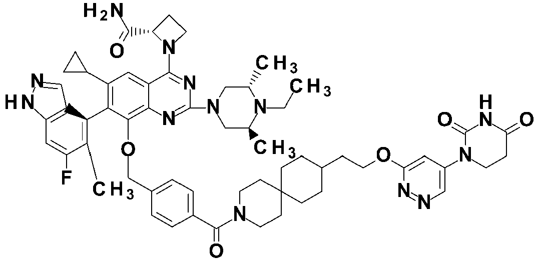 |

**[Table 6-1]**

| Ex | Syn | DAT |
|---|---|---|
| 1 | 1 | ESI+: 1086.8 [M+Na]+ |
| | | NMR: 0.47-0.57 (m, 1H), 0.57-0.72 (m, 3H), 0.92 (s, 6H), 1.00-1.79 (m, 16H), 1.96 (d, 3H), 2.07-2.35 (m, 9H), 2.65-2.80 (m, 3H), 3.20 (br, 2H), 3.54 (br, 2H), 3.80-3.89 (m, 2H), 4.01-4.23 (m, 2H), 4.23-4.35 (m, 2H), 4.49 (br, 1H), 4.64 (br, 1H), 4.86 (d, 1H), 5.01-5.28 (m, 2H), 6.74 (d, 1H), 6.86 (d, 2H), 7.02 (dd, 1H), 7.06-7.23 (m, 3H), 7.31 (br, 1H), 7.35 (d, 1H), 7.44 (s, 1H), 7.74 (br, 1H), 8.07 (d, 1H), 10.54 (s, 1H), 13.07 (s, 1H) |
| 2 | 1 | ESI+: 1094.6 |
| 3 | 1 | ESI-: 1107.8 |
| 4 | 4 | ESI+: 1107.7 |
| 5 | 4 | ESI+: 1096.9 |
| 6 | 1 | ESI-: 1069.4 |
| 7 | 7 | ESI-: 1045.8 |
| | | NMR: 0.41-0.52 (m, 1H), 0.52-0.66 (m, 3H), 0.90-1.61 (m, 15H), 1.61-1.76 (m, 4H), 1.85-2.00 (m, 4H), 2.00-2.25 (m, 4H), 2.25-2.34 (m, 1H), 2.56-2.82 (m, 5H), 2.99 (br, 1H), 3.20 (br, 2H), 3.54 (br, 2H), 3.78-3.88 (m, 2H), 4.01-4.14 (m, 2H), 4.31-4.59 (m, 3H), 4.66 (br, 1H), 4.82 (d, 1H), 4.96 (br, 1H), 5.19 (d, 1H), 6.82 (d, 2H), 7.04-7.16 (m, 3H), 7.21 (br, 1H), 7.33 (d, 1H), 7.37-7.40 (m, 1H), 7.45 (s, 1H), 7.64 (br, 1H), 8.14 (d, 1H), 8.18 (d, 1H), 10.48 (s, 1H), 13.03 (s, 1H) |
| 8 | 1 | ESI+: 1076.7 |
| 9 | 1 | ESI+: 1101.0 |
| 10 | 1 | ESI-: 1043.9 |
| 11 | 1 | ESI+: 1037.6 |
| 12 | 1 | ESI+: 1075.8 |
| 13 | 1 | ESI+: 1073.6 |
| 14 | 14 | ESI+: 1041.7 |
| | | NMR: 0.41-0.52 (m, 1H), 0.52-0.65 (m, 3H), 0.96 (d, 3H), 1.06-1.59 (m, 12H), 1.59-1.69 (m, 2H), 1.88-2.00 (m, 4H), 2.01-2.11 (m, 1H), 2.17 (s, 3H), 2.23-2.34 (m, 1H), 2.39 (br, 2H), 2.55-2.76 (m, 5H), 2.91-3.05 (m, 1H), 3.21 (br, 2H), 3.55 (br, 2H), 3.80-3.93 (m, 2H), 4.38-4.49 (m, 2H), 4.54 (br, 1H), 4.66 (br, 1H), 4.82 (d, 1H), 4.95 (br, 1H), 5.20 (d, 1H), 6.82 (d, 2H), 7.03-7.16 (m, 3H), 7.20 (br, 1H), 7.29-7.37 (m, 2H), 7.41-7.49 (m, 2H), 7.63 (br, 1H), 8.39-8.48 (m, 1H), 10.60 (s, 1H), 13.03 (s, 1H) |
| 15 | 1 | ESI+: 1091.9 |
| 16 | 1 | ESI+: 1042.8 |
| 17 | 1 | ESI+: 1087.8 |

**[Table 6-2]**

| Ex | Syn | DAT |
|---|---|---|
| 18 | 1 | ESI+: 1065.7 |
| 19 | 1 | ESI+: 1093.9 |
| 20 | 20 | ESI+: 1061.7 |
| | | NMR: 0.41-0.52 (m, 1H), 0.52-0.66 (m, 3H), 0.97 (d, 3H), 1.02-1.64 (m, 12H), 1.64-1.77 (m, 4H), 1.88-1.99 (m, 4H), 1.99-2.11 (m, 4H), 2.18 (s, 3H), 2.24-2.34 (m, 1H), 2.56-2.83 (m, 5H), 2.92-3.08 (m, 1H), 3.20 (br, 2H), 3.46-3.62 (m, 3H), 3.74-3.85 (m, 1H), 4.10-4.22 (m, 2H), 4.40-4.50 (m, 2H), 4.53 (br, 1H), 4.66 (br, 1H), 4.82 (d, 1H), 4.95 (br, 1H), 5.20 (d, 1H), 6.82 (d, 2H), 7.05-7.15 (m, 3H), 7.20 (br, 1H), 7.33 (d, 1H), 7.43-7.47 (m, 1H), 7.63 (br, 1H), 8.14 (s, 1H), 8.24 (s, 1H), 10.43 (s, 1H), 13.03 (s, 1H) |
| 21 | 1 | ESI+: 1041.8 |
| 22 | 1 | ESI-: 1060.7 |
| 23 | 1 | ESI+: 1073.8 |
| 24 | 24 | ESI+: 1061.8 |
| | | NMR: 0.40-0.51 (m, 1H), 0.51-0.65 (m, 3H), 0.88-1.02 (m, 6H), 1.01-1.78 (m, 16H), 1.97 (d, 3H), 2.11-2.22 (m, 1H), 2.23-2.35 (m, 3H), 2.59-2.68 (m, 1H), 2.68-2.79 (m, 4H), 2.79-2.90 (m, 1H), 3.08-3.25 (m, 3H), 3.54 (br, 2H), 3.77-3.89 (m, 2H), 4.02-4.15 (m, 2H), 4.20-4.35 (m, 2H), 4.53 (br, 1H), 4.66 (br, 1H), 4.82 (d, 1H), 4.95 (br, 1H), 5.20 (d, 1H), 6.82 (d, 2H), 7.04-7.15 (m, 3H), 7.20 (br, 1H), 7.33 (d, 1H), 7.37-7.41 (m, 1H), 7.43-7.47 (s, 1H), 7.63 (br, 1H), 8.14 (d, 1H), 8.18 (d, 1H), 10.48 (s, 1H), 13.03 (br, 1H) |
| 25 | 1 | ESI+: 1068.7 |
| 26 | 1 | ESI+: 1051.8 |
| 27 | 1 | ESI+: 1086.8 |
| 28 | 1 | ESI+: 1102.0 |
| 29 | 1 | ESI+: 1123.7 |
| 30 | 1 | ESI+: 1065.9 |
| 31 | 1 | ESI+: 1155.8 [M+Na]+ |
| 32 | 1 | ESI+: 1055.8 |
| 33 | 1 | ESI+: 1030.9 |
| 34 | 1 | ESI+: 1055.6 |

**[Table 6-3]**

| Ex | Syn | DAT |
|---|---|---|
| 35 | 1 | ESI+: 1064.8 |
| 36 | 1 | ESI+: 1124.6 |
| 37 | 1 | ESI-: 1099.8 |
| 38 | 1 | ESI-: 1102.0 |
| 39 | 39 | ESI+: 1035.6 |
| 40 | 1 | ESI+: 1050.8 |
| 41 | 1 | ESI+: 1076.6 |

### INDUSTRIAL APPLICABILITY

The compound or a salt thereof of the present invention has an excellent degradation-inducing action of a mutant KRAS protein, in particular, an excellent degradation-inducing action of a G12C mutant, G12D mutant, G12V mutant, and G13D mutant KRAS proteins as well as a wild-type KRAS protein, is useful as a mutant KRAS inhibitor, in particular, a G12C mutant, G12D mutant, G12V mutant, and G13D mutant KRAS inhibitor as well as an inhibitor of KRAS in the presence of amplification of wild-type KRAS gene, and can be used as an active ingredient of a pharmaceutical composition, for example, a pharmaceutical composition for treatment of cancer.

## Claims

1. A compound represented by formula (I) or a salt thereof: 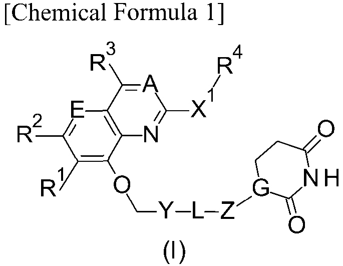 wherein
A is CR^{A} or N,
R^{A} is H, C₁₋₃ alkyl, -CN, or -O-(C₁₋₃ alkyl),
E is CH or N,
X¹ is -CH₂-, -O-, or -NR^{X1}-,
R^{X1} is H or optionally substituted C₁₋₃ alkyl,
provided that when X¹ is -NR^{X1}-, R^{X1} and R⁴ present on the same nitrogen atom, together with the nitrogen atom adjacent thereto, may form optionally substituted 4- to 11-membered saturated hetero ring group,
R¹ is represented by the following formula (II), (III), (IV), (V), (VI), or (VII): 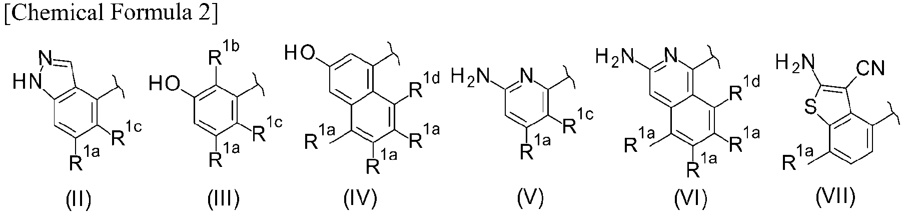
R^{1a} and R^{1b} are the same or different, and are each H, methyl, F, or Cl,
R^{1c} is F, Cl, methyl, ethyl, trifluoromethyl, or cyclopropyl,
R^{1d} is H, methyl, ethyl, F, Cl, or -C=C-H,
R² is H, halogen, optionally substituted C₁₋₃ alkyl, cyclopropyl, or vinyl,
R³ is a group selected from the group consisting of the following formulas (VIII), (XI), (X), (XI), (XII), (XIII), (XIV), (XV), (XVI), and (XVII): 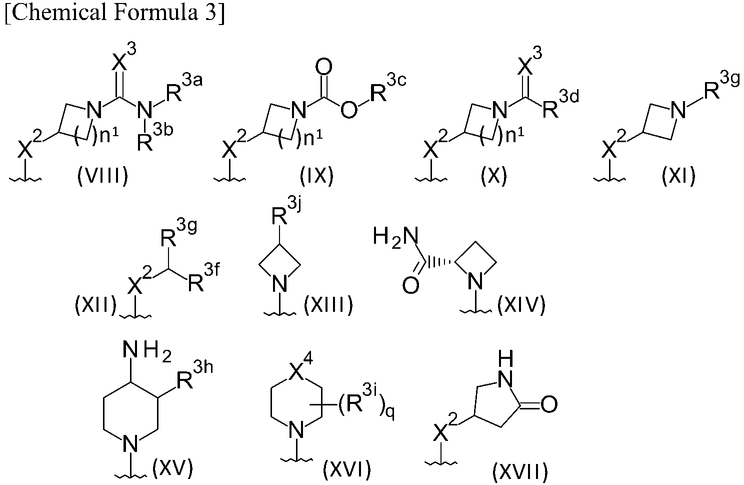
R^{3a} is -(CH₂)ₚCHR^{3e}-NR^{N1}R^{N2}; -(CH₂)ₚCHR^{3e}-OR^{3f}; **4-** to 6-membered saturated hetero ring group optionally substituted with a group selected from the group consisting of C₁₋₃ alkyl, -C₁₋₃ alkylene-OR^{3f}, -C₁₋₃ alkylene-NR^{N1}R^{N2}, and -NR^{N1}R^{N2}; or C₃₋₆ cycloalkyl optionally substituted with a group selected from the group consisting of C₁₋₃ alkyl, -C₁₋₃ alkylene-OR^{3f}, -C₁₋₃ alkylene-NR^{N1}R^{N2}, -OR^{3f}, and -NR^{N1}R^{N2},
R^{3b} is H or C₁₋₃ alkyl,
R^{3c} and R^{3d} are -(CH₂)ₚCHR^{3e}-NR^{N1}R^{N2}; -(CH₂)ₚCHR^{3e}-OR^{3f}; 4- to 6-membered saturated hetero ring group optionally substituted with a group selected from the group consisting of C₁₋₃ alkyl, -C₁₋₃ alkylene-OR^{3f}, -C₁₋₃ alkylene-NR^{N1}R^{N2}, and -NR^{N1}R^{N2}; or C₃₋₆ cycloalkyl optionally substituted with a group selected from the group consisting of C₁₋₃ alkyl, -C₁₋₃ alkylene-OR^{3f}, -C₁₋₃ alkylene-NR^{N1}R^{N2}, -OR^{3f}, and -NR^{N1}R^{N2},
R^{3e} is H, F, or C₁₋₃ alkyl,
R^{3f} is the same or different, and is H or C₁₋₃ alkyl,
R^{3g} is optionally substituted C₃₋₆ cycloalkyl, optionally substituted 5-membered heteroaryl, optionally substituted 6-membered heteroaryl, or optionally substituted 4- to 6-membered saturated hetero ring group,
R^{3h} is H, F, or C₁₋₃ alkyl,
R³ⁱ is the same or different, and is a group selected from the group consisting of H, OH, optionally substituted C₁₋₃ alkyl, -O-optionally substituted C₁₋₃ alkyl, -NH-optionally substituted C₁₋₃ alkyl, -N-(optionally substituted C₁₋₃ alkyl)₂, halogen, -CN, and oxo, or
two R³ⁱs present on the same carbon atom together with the carbon atom adjacent thereto may form spiro ring having a ring selected from the group consisting of C₃₋₆ cycloalkane and 4- to 6-membered saturated hetero ring, wherein the spiro ring is optionally substituted with 1 to 2 groups selected from the group of C₁₋₃ alkyl, -O-(C₁₋₃ alkyl), OH, halogen, and oxo, or
R³ⁱ present on two adjacent carbon atoms together with the two carbon atoms may form fused ring having ring selected from the group consisting of C₃₋₆ cycloalkane and 4- to 6-membered saturated hetero ring, wherein the fused ring is optionally substituted with 1 to 2 groups selected from the group consisting of C₁₋₃ alkyl, -O-(C₁₋₃ alkyl), OH, halogen, and oxo, or
R³ⁱ present on two non-adjacent carbon atoms together with the two carbon atoms may form bridged structure composed of 1 to 2 carbon atoms, and ring having the bridged structure is optionally substituted with 1 to 2 groups selected from the group consisting of C₁₋₃ alkyl, -O-(C₁₋₃ alkyl), OH, halogen, and oxo,
R^{N1} and R^{N2} are the same or different, and are each H or C₁₋₃ alkyl, or
R^{N1} and R^{N2} together with the nitrogen atom bonded thereto may form optionally substituted 4- to 6-membered saturated hetero ring group, or
R^{3e} and R^{N1} together with the carbon atom and the nitrogen atom bonded thereto may form optionally substituted 4- to 6-membered saturated hetero ring group,
R^{3j} is a group selected from the group consisting of H, OH, halogen, optionally substituted C₁₋₃ alkyl, -O-optionally substituted C₁₋₃ alkyl, optionally substituted C₃₋₆ cycloalkyl, optionally substituted 5-membered heteroaryl, optionally substituted 6-membered heteroaryl, and -CN,
X² is -O-, -NH-, or -N(C₁₋₃ alkyl)-,
X³ is O or S,
X⁴ is -CH₂-, -CH₂-CH₂-, -O-CH₂-, -O-, or -O-NH-,
n¹ is 1 or 2,
p is 1 or 2,
q is 1 to 8,
R⁴ is optionally substituted C₁₋₆ alkyl, optionally substituted piperidinyl, or optionally substituted tetrahydropyranyl,
Y is phenylene or pyridinediyl, wherein the phenylene is optionally substituted with F,
L is -(L^{A}-L^{B}-L^{C}-L^{P}-L^{E}-L^{F})-,
L^{A}, L^{B}, L^{C}, L^{D}, L^{E}, and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O,
R^{L1A} is H or C₁₋₃ alkyl,
Z is a group selected from the group consisting of the following formulas (XVIII), (XIX), (XX), (XXI), (XXII), (XXIII), (XXIV), (XXV), (XXVI), (XXVII), (XXVIII), and (XXIX): 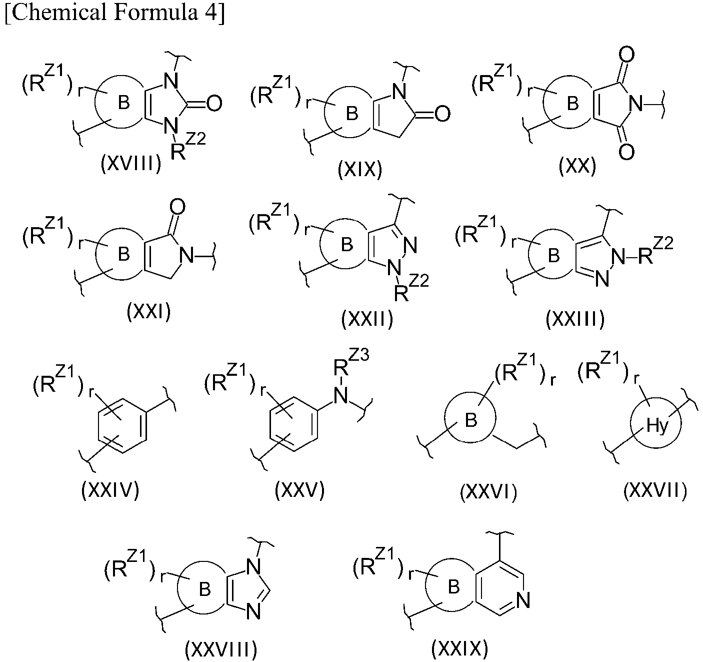
ring B is benzene ring or 6-membered hetero ring,
ring Hy is 5- or 6-membered hetero ring,
R^{Z1} is H, C₁₋₃ alkyl, halogen, -O-(C₁₋₃ alkyl), -NR^{Z4}₂, -CN, -CONR^{Z4}₂, or - NR^{Z4}COR^{Z5},
R^{Z2} is H or C₁₋₃ alkyl,
R^{Z3} is H or C₁₋₃ alkyl,
R^{Z4} is the same or different, and is H or C₁₋₃ alkyl,
R^{Z5} is C₁₋₃ alkyl,
L is bonded to ring B in the above formulas (XVIII), (XIX), (XX), (XXI), (XXII), (XXIII), (XXVI), (XXVIII), and (XXIX), to the benzene ring in formulas (XXIV) and (XXV), and to ring Hy in formula (XXVII),
r is 1 or 2, and
G is N or CH,
provided that when G is N, Z is the above formula (XVIII), (XXII), (XXIII), (XXIV), (XXVI), (XXVII), or (XXIX).

2. The compound or a salt thereof according to claim 1,
wherein E is CH,
R³ is a group represented by the following formula (XII), (XIII), (XIV), or (XVII): 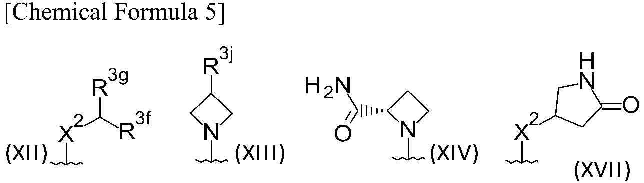
R^{3f} is the same or different, and is H or C₁₋₃ alkyl,
R^{3g} is optionally substituted C₃₋₆ cycloalkyl, optionally substituted 5-membered heteroaryl, optionally substituted 6-membered heteroaryl, or optionally substituted 4- to 6-membered saturated hetero ring group,
R^{3j} is a group selected from the group consisting of H, OH, halogen, optionally substituted C₁₋₃ alkyl, -O-optionally substituted C₁₋₃ alkyl, optionally substituted C₃₋₆ cycloalkyl, optionally substituted 5-membered heteroaryl, optionally substituted 6-membered heteroaryl, and -CN,
X² is -O-, -NH-, or -N(C₁₋₃ alkyl)-,
L is -(L^{A}-L^{B}-L^{C}-L^{D}-L^{E}-L^{F})-,
L^{A}, L^{B}, L^{C}, L^{D}, L^{E} and L^{F} are the same or different, and are each a group selected from the group consisting of bond, -O-, -NR^{L1A}-, divalent group of optionally substituted saturated hetero ring, optionally substituted C₁₋₃ alkylene, optionally substituted C₃₋₆ cycloalkylene, C₂₋₃ alkynediyl optionally substituted with C₁₋₃ alkyl, and C=O,
R^{L1A} is H or C₁₋₃ alkyl,
Z is a group selected from the group consisting of formulas (XXII), (XXIV), (XXVI), and (XXVII): 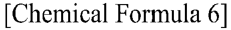 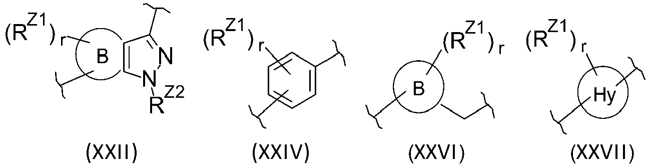
ring B is benzene ring or 6-membered hetero ring,
ring Hy is 5- or 6-membered hetero ring,
R^{Z1} is H, C₁₋₃ alkyl, halogen, -O-(C₁₋₃ alkyl), -NR^{Z4}₂, -CN, -CONR^{Z4}₂, or - NR^{Z4}COR^{Z5},
R^{Z2} is H or C₁₋₃ alkyl,
R^{Z4} is the same or different, and is H or C₁₋₃ alkyl,
R^{Z5} is C₁₋₃ alkyl,
r is 1 or 2,
L is bonded to ring B in the above formulas (XXII) and (XXVI), to the benzene ring in formulas (XXIV), and to ring Hy in formula (XXVII), and
G is N.

3. The compound or a salt thereof according to claim 2,
wherein X¹ is -O- or -NR^{X1}-,
provided that when X¹ is -NR^{X1}-,R^{X1} and R⁴ present on the same nitrogen atom, together with the nitrogen atom adjacent thereto, form optionally substituted 4- to 11-membered saturated hetero ring group,
R¹ is represented by the following formula (II), (III), or (IV): 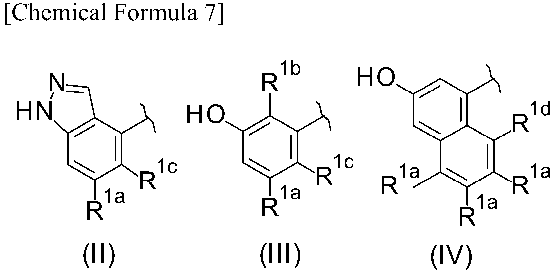
R^{1a} is the same or different, and is H or F,
R^{1b} is H,
R^{1c} is methyl or cyclopropyl,
R^{1d} is H,
R² is cyclopropyl,
R³ is a group represented by formula (XIV): 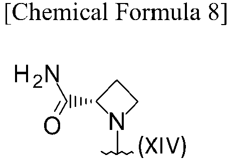
R⁴ is C₁₋₆ alkyl or piperidinyl, and the C₁₋₆ alkyl is optionally substituted with a group selected from the group consisting of OH; OCH₃; N(R^{4a})₂; C₃₋₆ cycloalkyl optionally substituted with R^{4b}; pyrrolidinyl optionally substituted with R^{4b}; and morpholinyl optionally substituted with R^{4b}, and the piperidinyl is optionally substituted with R^{4b},
R^{4a} is C₁₋₃ alkyl,
R^{4b} is C₁₋₃ alkyl optionally substituted with -O-(C₁₋₃ alkyl) or -N(C₁₋₃ alkyl)₂, or halogen,
Y is phenylene,
L is a group selected from the group consisting of the following formulas (XXX-L), (XXXI-L), (XXXII-L), (XXXIII-L), and (XXXIV-L): 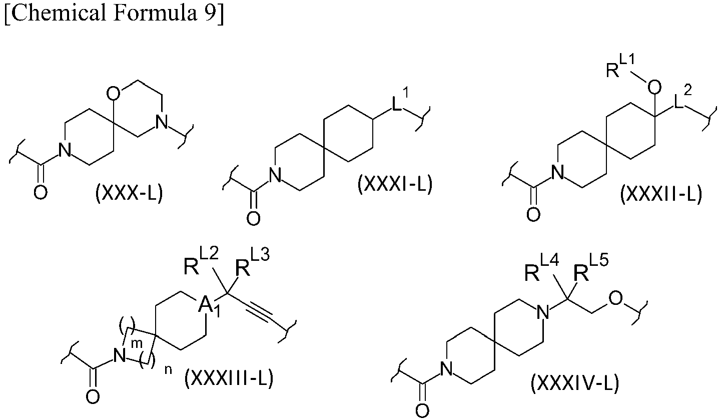
L¹ is bond, C₁₋₃ alkylene, -CH₂-C≡C-, -C₁₋₃ alkylene-O-, or tetrahydropyridinediyl,
L² is -CH₂-C≡C- or -CH₂CH₂-O-,
R^{L1} is H or methyl,
A₁ is CH or N,
R^{L2} and R^{L3} are the same or different, and are each H, OH, or C₁₋₃ alkyl, provided that R^{L2} and R^{L3} are never OH at the same time, and R^{L2} and R^{L3} are never H at the same time,
m and n are both 1 or both 2,
R^{L4} and R^{L5} are both H, or the R^{L4} and R^{L5} together with the carbon atom bonded thereto may form cyclopropane,
Z is a group selected from the group consisting of formulas (XXX-Z), (XXXI-Z), and (XXXII-Z): 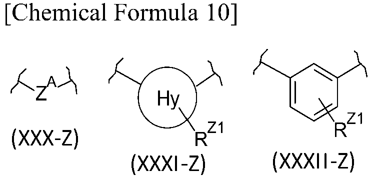
Z^{A} is a group represented by the following formula (XXII-1) or (XXVI-2): 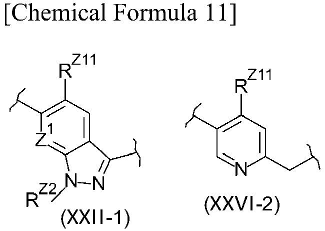
R^{Z11} is halogen,
Z¹ is CH or N,
R^{Z2} is C₁₋₃ alkyl,
ring Hy is 5- or 6-membered hetero ring, and
R^{Z1} is H, C₁₋₃ alkyl, or halogen.

4. The compound or a salt thereof according to claim 1, wherein E is CH,
X¹ is -O- or -NR^{X1}-,
R^{X1} is H or C₁₋₃ alkyl,
provided that when X¹ is -NR^{X1}-,R^{X1} and R⁴ present on the same nitrogen atom, together with the nitrogen atom adjacent thereto, may form optionally substituted 4- to 11-membered saturated hetero ring group,
R¹ is represented by the following formula (II), (III), or (IV): 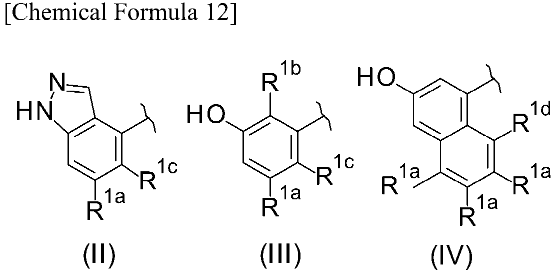
R^{1a} is the same or different, and is H or F,
R^{1b} is H,
R^{1c} is methyl or cyclopropyl,
R^{1d} is H,
R³ is a group represented by the following formula (XIV): 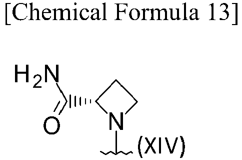
R⁴ is C₁₋₆ alkyl or piperidinyl, and the C₁₋₆ alkyl is optionally substituted with a group selected from the group consisting of F; OH; OCH₃; N(R^{4a})₂; C₃₋₆ cycloalkyl optionally substituted with R^{4b}; pyrrolidinyl optionally substituted with R^{4b}, and morpholinyl optionally substituted with R^{4b}, and the piperidinyl is optionally substituted with R^{4b},
R^{4a} is optionally substituted C₁₋₃ alkyl,
R^{4b} is optionally substituted C₁₋₃ alkyl, or halogen,
Y is phenylene,
L together with Z forms a group selected from the group consisting of the following formulas (XXX), (XXXI-X), (XXXII), (XXXIII-A), (XXXIII-B), (XXXIV), and (XXXI-A): 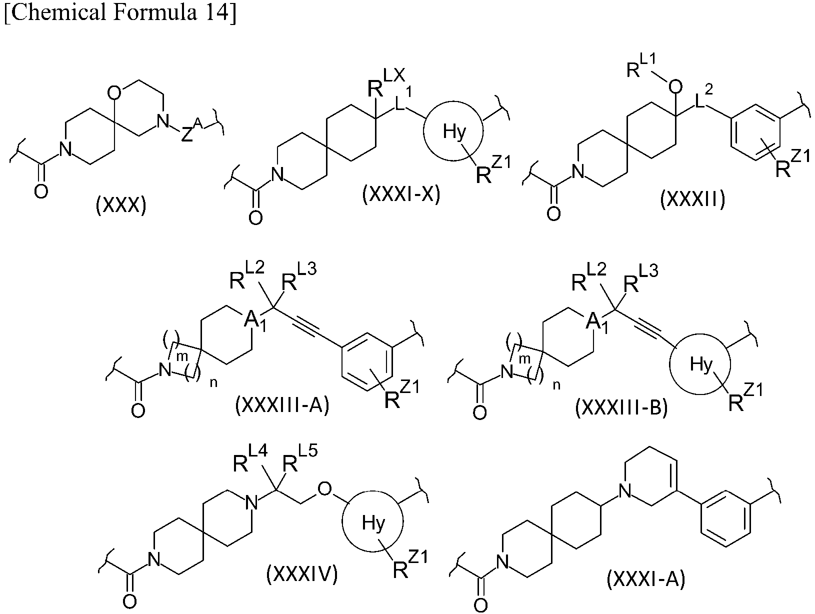
Z^{A} is a group represented by the following formula (XXII-1) or (XXVI-1): 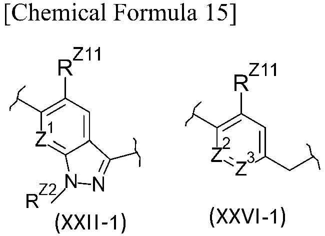
R^{Z11} is H or halogen,
Z¹, Z², and Z³ are the same or different, and are each CH or N,
R^{Z2} is C₁₋₃ alkyl,
L¹ is bond, C₁₋₃ alkylene, -C₁₋₃ alkylene-O-, -C₁₋₃ alkylene-C₂₋₃ alkynediyl, or -C=C-, R^{LX} is H or OH,
ring Hy is a 5- or 6-membered hetero ring,
L² is -C₁₋₃ alkylene-C₂₋₃ alkynediyl or -C₁₋₃ alkylene-O-,
R^{Z1} is H, halogen, C₁₋₃ alkyl, -O-(C₁₋₃ alkyl), or -CN,
R^{L1} is H or C₁₋₃ alkyl,
A₁ is CH or N,
R^{L2} and R^{L3} are the same or different, and are each H, OH, or C₁₋₃ alkyl, provided that R^{L2} and R^{L3} are never OH at the same time, and R^{L2} and R^{L3} are never H at the same time,
m and n are the same or different, and are each 1 or 2,
R^{L4} and R^{L5} are the same or different, and are each H or C₁₋₃ alkyl, or the R^{L4} and R^{L5} together with the carbon atom bonded thereto may form C₃₋₆ cycloalkane, or the R^{L4} and R^{L5} together with the carbon atom bonded thereto may form carbonyl, and
G is N.

5. The compound or a salt thereof according to claim 4,
wherein A is N,
X¹ is -O- or -NR^{X1}-,
provided that when X¹ is -NR^{X1}-,R^{X1} and R⁴ present on the same nitrogen atom, together with the nitrogen atom adjacent thereto, form optionally substituted 4- to 11-membered saturated hetero ring group,
R¹ is represented by the following formula (II), (III), or (IV): 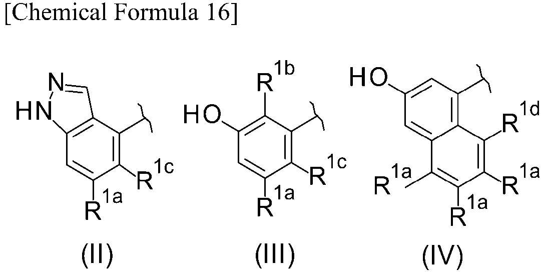
R^{1a} is the same or different, and is H or F,
R^{1b} is H,
R^{1c} is methyl or cyclopropyl,
R^{1d} is H,
R² is cyclopropyl,
R⁴ is C₁₋₆ alkyl or piperidinyl, and the C₁₋₆ alkyl is optionally substituted with a group selected from the group consisting of OH; OCH₃; N(R^{4a})₂; C₃₋₆ cycloalkyl optionally substituted with R^{4b}; pyrrolidinyl optionally substituted with R^{4b}; and morpholinyl optionally substituted with R^{4b}, and the piperidinyl is optionally substituted with R^{4b},
R^{4a} is C₁₋₃ alkyl,
R^{4b} is C₁₋₃ alkyl optionally substituted with a group selected from the group consisting of OH, -O-(C₁₋₃ alkyl) and -N(C₁₋₃ alkyl)₂, or halogen,
L and Z together are a group selected from the group consisting of the following formulas (XXX), (XXXI-X), (XXXII), (XXXIII-A), (XXXIII-B), (XXXIV), and (XXXI-A): 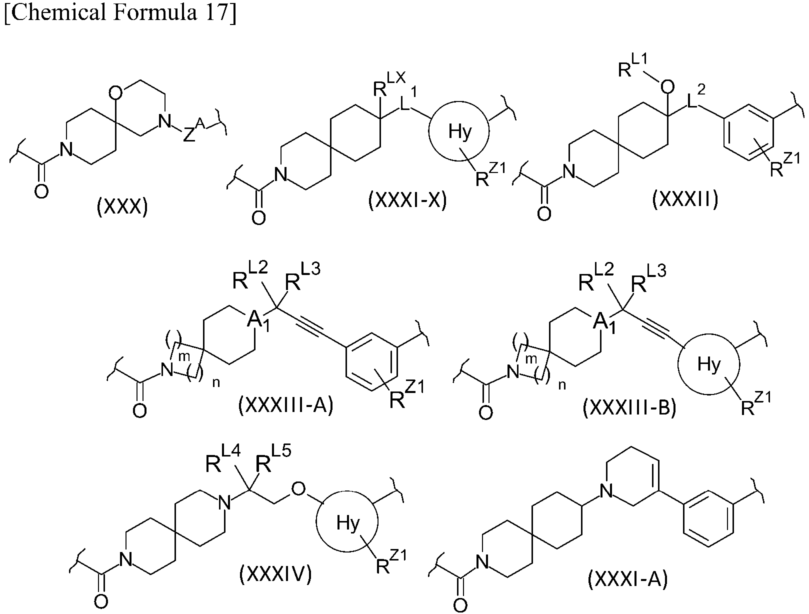
Z^{A} is a group represented by the following formula (XXII-1) or (XXVI-2): 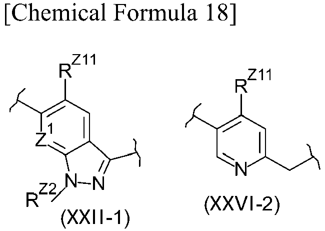
R^{Z11} is halogen,
Z¹ is CH or N,
R^{Z2} is C₁₋₃ alkyl,
L¹ is bond, C₁₋₃ alkylene, -C₁₋₃ alkylene-O-, or -CH₂-C≡C-,
R^{LX} is H or OH,
ring Hy is a 5- or 6-membered hetero ring,
L² is -CH₂-C≡C- or -CH₂CH₂-O-,
R^{Z1} is H, C₁₋₃ alkyl, or halogen,
R^{L1} is H or methyl,
A₁ is CH or N,
R^{L2} and R^{L3} are the same or different, and are each H, OH, or C₁₋₃ alkyl, provided that R^{L2} and R^{L3} are never OH at the same time, and R^{L2} and R^{L3} are never H at the same time,
m and n are both 1 or both 2, and
R^{L4} and R^{L5} are both H, or the R^{L4} and R^{L5} together with the carbon atom bonded thereto may form cyclopropane.

6. The compound or a salt thereof according to claim 5,
wherein X¹ is -O- or -NR^{X1}-,
provided that when X¹ is -NR^{X1}-,R^{X1} and R⁴ present on the same nitrogen atom, together with the nitrogen atom adjacent thereto, form optionally substituted 4- to 11-membered saturated hetero ring group,
L and Z together are a group selected from the group consisting of the following formulas (XXX), (XXXI-1X), (XXXII-1), (XXXIII-A1), (XXXIII-B1), (XXXIV-1), and (XXXI-A): 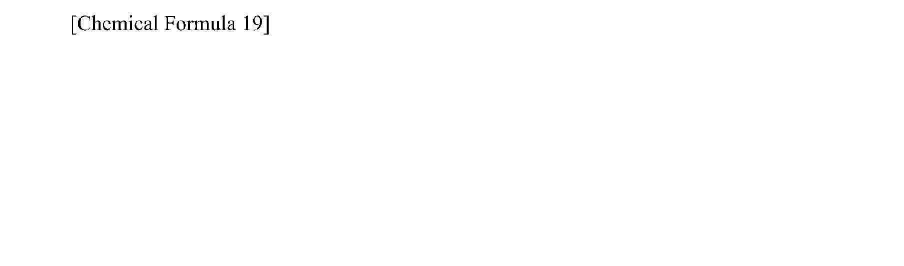 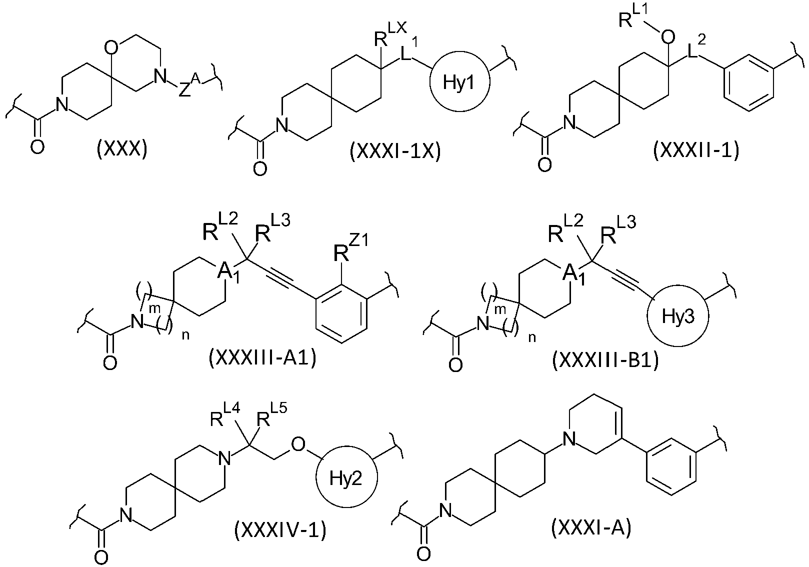
Z^{A} is a group represented by the following formula (XXII-1) or (XXVI-2): 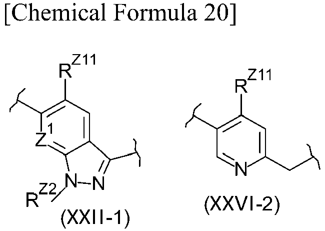
R^{Z11} is halogen,
Z¹ is CH or N,
R^{Z2} is C₁₋₃ alkyl,
L¹ is bond, C₁₋₃ alkylene, -C₁₋₃ alkylene-O-, or -CH₂-C≡C-,
R^{LX} is H or OH,
ring Hy1 is a ring selected from the group consisting of the following formulas (XXXV), (XXXVI), (XXXVII), (XXXVII-2), (XXXVIII), and (XXXIX): 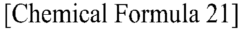 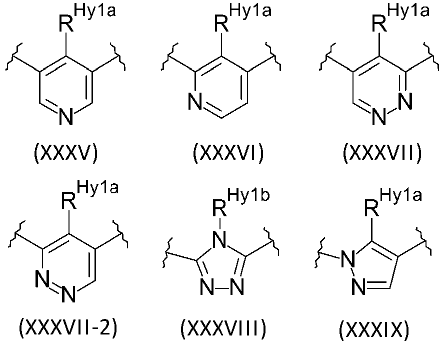
R^{Hy1a} is H, C₁₋₃ alkyl, or halogen,
R^{Hy1b} is H or C₁₋₃ alkyl,
L² is -CH₂-C≡C- or -CH₂CH₂-O-,
R^{Z1} is H, C₁₋₃ alkyl, or halogen,
R^{L1} is H or methyl,
A₁ is CH or N,
R^{L2} and R^{L3} are the same or different, and are each H, OH, or C₁₋₃ alkyl, provided that R^{L2} and R^{L3} are never OH at the same time, and R^{L2} and R^{L3} are never H at the same time,
ring Hy3 is a ring selected from the group consisting of the following formulas (XL) and (XLI): 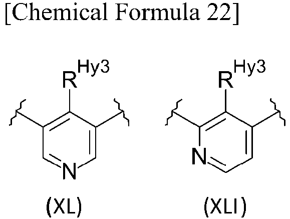
R^{Hy3} is H, C₁₋₃ alkyl, or halogen,
m and n are both 1 or both 2, and
ring Hy2 is of the following formula (XLIII): 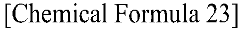 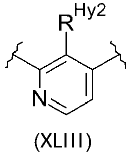
R^{Hy2} is H, C₁₋₃ alkyl, or halogen, and
R^{L4} and R^{L5} are both H, or the R^{L4} and R^{L5} together with the carbon atom bonded thereto may form cyclopropane.

7. The compound or a salt thereof according to claim 6, wherein L and Z together are a group represented by the formula (XXXI-1X): 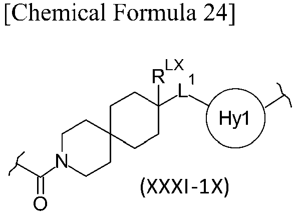
L¹ is -CH₂CH₂-O- or -CH₂-C≡C-,
R^{LX} is H,
ring Hy1 is ring selected from the group consisting of the following formulas (XXXV) and (XXXVI): 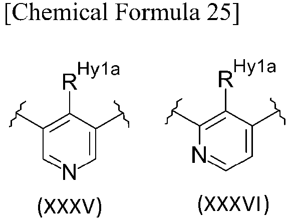
R^{Hy1a} is H, C₁₋₃ alkyl, or halogen.

8. The compound or a salt thereof according to claim 1,
wherein A is N,
X¹ is -O- or -NR^{X1}-,
provided that when X¹ is -NR^{X1}-, R^{X1} and R⁴ present on the same nitrogen atom, together with the nitrogen atom adjacent thereto, form optionally substituted 4- to 11-membered saturated hetero ring group,
R¹ is represented by the following formula (II), (III), or (IV): 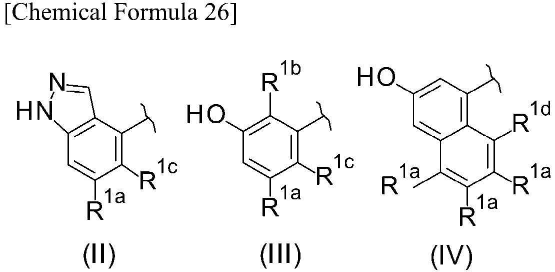
R^{1a} is the same or different, H or F,
R^{1b} is H,
R^{1c} is methyl or cyclopropyl,
R^{1d} is H,
R² is cyclopropyl,
R⁴ is C₁₋₆ alkyl or piperidinyl, and the C₁₋₆ alkyl is optionally substituted with a group selected from the group consisting of OH; OCH₃; N(R^{4a})₂; C₃₋₆ cycloalkyl optionally substituted with R^{4b}; pyrrolidinyl optionally substituted with R^{4b}; and morpholinyl optionally substituted with R^{4b}, and the piperidinyl is optionally substituted with R^{4b},
R^{4a} is C₁₋₃ alkyl,
R^{4b} is C₁₋₃ alkyl optionally substituted with -O-(C₁₋₃ alkyl) or -N(C₁₋₃ alkyl)₂, or halogen,
L and Z together are a group selected from the group consisting of the following formulas (XXX), (XXXI), (XXXII), (XXXIII-A), (XXXIII-B), (XXXIV), and (XXXI-A): 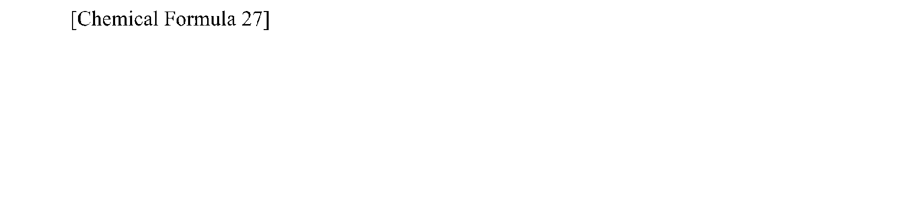 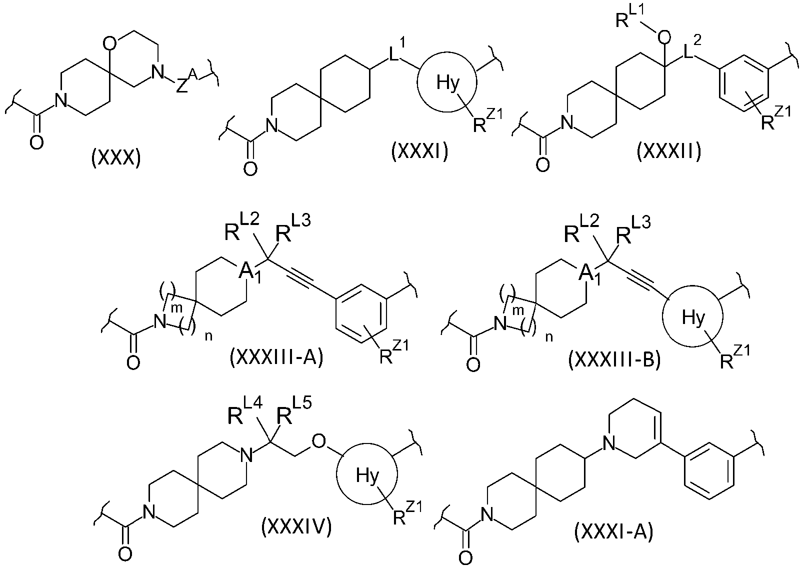
Z^{A} is a group represented by the following formula (XXII-1) or (XXVI-2): 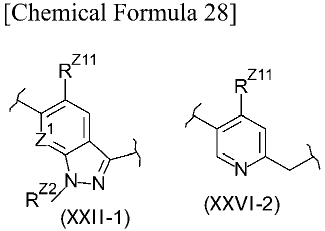
R^{Z11} is halogen,
Z¹ is CH or N,
R^{Z2} is C₁₋₃ alkyl,
L¹ is bond, C₁₋₃ alkylene, -C₁₋₃ alkylene-O-, or -CH₂-C≡C-,
ring Hy is 5- or 6-membered hetero ring,
L² is -CH₂-C≡C- or -CH₂CH₂-O-,
R^{Z1} is H, C₁₋₃ alkyl, or halogen,
R^{L1} is H or methyl,
A₁ is CH or N,
R^{L2} and R^{L3} are the same or different, and are each H, OH, or C₁₋₃ alkyl, provided that R^{L2} and R^{L3} are never OH at the same time, and R^{L2} and R^{L3} are never H at the same time,
m and n are both 1 or both 2, and
R^{L4} and R^{L5} are both H, or the R^{L4} and R^{L5} together with the carbon atom bonded thereto may form cyclopropane.

9. The compound or a salt thereof according to claim 1,
wherein the compound represented by formula (I) is selected from the group consisting of:
(2S)-1-[(7M)-6-cyclopropyl-2-[3-(dimethylamino)-2,2-dimethylpropoxy]-8-({4-[9-(2-{[4-(2,4-dioxo-1,3-diazinan-1-yl)pyridin-2-yl]oxy}ethyl)-3-azaspiro[5.5]undecane-3-carbonyl]phenyl}methoxy)-7-(6-fluoro-5-methyl-1H-indazol-4-yl)quinazolin-4-yl]azetidine-2-carboxamide,
(2S)-1-[(7M)-6-cyclopropyl-2-[(3S)-3,4-dimethylpiperazin-1-yl]-8-({4-[9-(2-{[5-(2,4-dioxo-1,3-diazinan-1-yl)pyridin-3-yl]oxy} ethyl)-3-azaspiro[5.5]undecane-3-carbonyl]phenyl}methoxy)-7-(6-fluoro-5-methyl-1H-indazol-4-yl)quinazolin-4-yl]azetidine-2-carboxamide,
(2S)-1-[(7M)-6-cyclopropyl-2-[(3S)-3,4-dimethylpiperazin-1-yl]-8-{[4-(9-{3-[4-(2,4-dioxo-1,3-diazinan-1-yl)pyridin-2-yl]prop-2-yn-1-yl}-3-azaspiro[5.5]undecane-3-carbonyl)phenyl]methoxy}-7-(6-fluoro-5-methyl-1H-indazol-4-yl)quinazolin-4-yl]azetidine-2-carboxamide,
(2S)-1-[(7M)-6-cyclopropyl-2-[(3S)-3,4-dimethylpiperazin-1-yl]-8-({4-[9-(2-{[5-(2,4-dioxo-1,3-diazinan-1-yl)-4-methylpyridin-3-yl]oxy}ethyl)-3-azaspiro[5.5]undecane-3-carbonyl]phenyl}methoxy)-7-(6-fluoro-5-methyl-1H-indazol-4-yl)quinazolin-4-yl]azetidine-2-carboxamide, and
(2S)-1-[(7M)-6-cyclopropyl-8-({4-[9-(2-{[5-(2,4-dioxo-1,3-diazinan-1-yl)pyridin-3-yl]oxy}ethyl)-3-azaspiro[5.5]undecane-3-carbonyl]phenyl}methoxy)-2-[(3S)-4-ethyl-3-methylpiperazin-1-yl]-7-(6-fluoro-5-methyl-1H-indazol-4-yl)quinazolin-4-yl]azetidine-2-carboxamide.

10. A pharmaceutical composition comprising the compound or a salt thereof according to claim 1, and one or more pharmaceutically acceptable excipients.

11. The pharmaceutical composition according to claim 10, which is a pharmaceutical composition for treating cancer.

12. Use of the compound or a salt thereof according to claim 1 for the manufacture of a pharmaceutical composition for treating cancer.

13. The compound or a salt thereof according to claim 1 for use in treatment of cancer.

14. Use of the compound or a salt thereof according to claim 1 for treating cancer.

15. A method for treating cancer, comprising administrating an effective amount of the compound or a salt thereof according to claim 1 to a subject.
